# EUROPEAN PATENT APPLICATION

(11) **EP 3 498 726 A1**
(43) Date of publication of application: **19.06.2019**
(21) Application number: 17839555.4
(22) Date of filing: 09.08.2017
(51) Int. Cl.: C07K 14/00, C12P 21/02, C12N 15/09

(54) **DNA-BINDING PROTEIN USING PPR MOTIF AND USE OF SAID DNA-BINDING PROTEIN**

(30) Priority: 10.08.2016 JP 2016157698
(71) Applicant: Fujifilm Wako Pure Chemical Corporation, Osaka-shi, Osaka 540-8605 (JP); Kyushu University, National University Corporation, Nishi-ku Fukuoka-shi Fukuoka 819-0395 (JP)
(72) Inventor: YAMANE Masayuki, Amagasaki-shi Hyogo 661-0963 (JP); NAKAMURA Takahiro, Fukuoka-shi Fukuoka 819-0395 (JP); YAGI Yusuke, Fukuoka-shi Fukuoka 819-0395 (JP)
(74) Representative: Hoffmann Eitle
(86) International application number: PCT/JP2017/028995
(87) International publication number: WO 2018/030488

(57) **Abstract**

The object of the present invention is to generalize and improve DNA-binding proteins using PPR. There is provided a protein that contains one or more PPR motifs having a structure of the following formula 1, wherein one PPR motif (Mₙ) contained in the protein is a PPR motif having a specific combination of amino acids corresponding to a target DNA base or target DNA base sequence as the three amino acids of No. 1 A.A., No. 4 A.A., and No. "ii" (-2) A.A, and satisfies at least one selected from the group consisting of the following conditions (a) to (h): (a) No. 7 A.A. of the PPR motif (Mₙ) is isoleucine (I); (b) No. 9A.A. of the PPR motif (Mₙ) is alanine (A); (c) No. 10 A.A. of the PPR motif (Mₙ) is tyrosine (Y); (d) No. 18 A.A. of the PPR motif (Mₙ) is lysine (K), arginine (R), or histidine (H); (e) No. 20 A.A. of the PPR motif (Mₙ) is glutamic acid (E), or aspartic acid (D); (f) No. 29 A.A. of the PPR motif (Mₙ) is glutamic acid (E), or aspartic acid (D); (g) No. 31 A.A. of the PPR motif (Mₙ) is isoleucine (I); and (h) No. 32 A.A. of the PPR motif (Mₙ) is lysine (K), arginine (R), or histidine (H).

## Description

### Technical Field

The present invention relates to a protein that can selectively or specifically bind to an intended DNA base or DNA sequence. According to the present invention, a pentatricopeptide repeat (PPR) motif is utilized. The present invention can be used for identification and design of a DNA-binding protein, identification of a target DNA of a protein having a PPR motif, and functional control of DNA. The present invention is useful in the fields of medicine, agricultural science, and so forth. The present invention also relates to a novel DNA-cleaving enzyme that utilizes a complex of a protein containing a PPR motif and a protein that defines a functional region.

### Background Art

In recent years, techniques of binding nucleic acid-binding protein factors elucidated through various analyses to an intended sequence have been established, and they are coming to be used. Use of this sequence-specific binding is enabling analysis of intracellular localization of a target nucleic acid (DNA or RNA), elimination of a target DNA sequence, or expression control (activation or inactivation) of a protein-encoding gene existing downstream of a target DNA sequence.

There are being conducted researches and developments using the zinc finger protein (Non-patent documents 1 and 2), TAL effecter (TALE, Non-patent document 3, Patent document 1), and CRISPR (Non-patent documents 4 and 5) as protein factors that act on DNA as materials for protein engineering. However, types of such protein factors are still extremely limited.

For example, the artificial enzyme, zinc finger nuclease (ZFN), known as an artificial DNA-cleaving enzyme, is a chimera protein obtained by binding a part that is constituted by linking 3 to 6 zinc fingers that specifically recognize a DNA consisting of 3 or 4 nucleotides and bind to it, and recognizes a nucleotide sequence in a sequence unit of 3 or 4 nucleotides with one DNA cleavage domain of a bacterial DNA-cleaving enzyme (for example, FokI) (Non-patent document 2). In such a chimera protein, the zinc finger domain is a protein domain that is known to bind to DNA, and it is based on the knowledge that many transcription factors have the aforementioned domain, and bind to a specific DNA sequence to control expression of a gene. By using two of ZFNs each having three zinc fingers, cleavage of one site per 70 billion nucleotides can be induced in theory.

However, because of the high cost required for the production of ZFNs, etc., the methods using ZFNs have not come to be widely used yet. Moreover, functional sorting efficiency of ZFNs is bad, and it is suggested that the methods have a problem also in this respect. Furthermore, since a zinc finger domain consisting of n of zinc fingers tends to recognize a sequence of (GNN)n, the methods also have a problem that degree of freedom for the target gene sequence is low.

An artificial enzyme, TALEN, has also been developed by binding a protein consisting of a combinatory sequence of module parts that can recognize every one nucleotide, TAL effecter (TALE), with a DNA cleavage domain of a bacterial DNA-cleaving enzyme (for example, FokI), and it is being investigated as an artificial enzyme that can replace ZFNs (Non-patent document 3). This TALEN is an enzyme generated by fusing a DNA binding domain of a transcription factor of a plant pathogenic *Xanthomonas* bacterium, and the DNA cleavage domain of the DNA restriction enzyme FokI, and it is known to bind to a neighboring DNA sequence to form a dimer and cleave a double strand DNA. Since, as for this molecule, the DNA binding domain of TALE found from a bacterium that infects with plants recognize one base with a combination of amino acids at two sites in the TALE motif consisting of 34 amino acid residues, it has a characteristic that binding property for a target DNA can be chosen by choosing the repetitive structure of the TALE module. TALEN using the DNA binding domain that has such a characteristic as mentioned above has a characteristic that it enables introduction of mutation into a target gene, like ZFNs, but the significant superiority thereof to ZFNs is that degree of freedom for the target gene (nucleotide sequence) is markedly improved, and the nucleotide to which it binds can be defined with a code.

However, since the total conformation of TALEN has not been elucidated, the DNA cleavage site of TALEN has not been identified at present. Therefore, it has a problem that cleavage site of TALEN is inaccurate, and is not fixed, compared with ZFNs, and it also cleaves even a similar sequence. Therefore, it has a problem that a nucleotide sequence cannot be accurately cleaved at an intended target site with a DNA-cleaving enzyme. For these reasons, it is desired to develop and provide a novel artificial DNA-cleaving enzyme free from the aforementioned problems.

On the basis of genome sequence information, PPR proteins (proteins having a pentatricopeptide repeat (PPR) motif) constituting a big family of no less than 500 members only for plants have been identified (Non-patent document 6). The PPR proteins are nucleus-encoded proteins, but are known to act on or involved in control, cleavage, translation, splicing, RNA editing, and RNA stability chiefly at an RNA level in organelles (chloroplasts and mitochondria) in a gene-specific manner. The PPR proteins typically have a structure consisting of about 10 contiguous 35-amino acid motifs of low conservativeness, i.e., PPR motifs, and it is considered that the combination of the PPR motifs is responsible for the sequence-selective binding with RNA. Almost all the PPR proteins consist only of repetition of about 10 PPR motifs, and any domain required for exhibiting a catalytic action is not found in many cases. Therefore, it is considered that the PPR proteins are essentially RNA adapters (Non-patent document 7).

In general, binding of a protein and DNA, and binding of a protein and RNA are attained by different molecular mechanisms. Therefore, a DNA-binding protein generally does not bind to RNA, whereas an RNA-binding protein generally does not bind to DNA. For example, in the case of the pumilio protein, which is known as an RNA-binding factor, and can encode RNA to be recognized, binding thereof to DNA has not been reported (Non-patent documents 8 and 9).

However, in the process of investigating properties of various kinds of PPR proteins, it became clear that it could be suggested that some types of the PPR proteins worked as DNA-binding factors.

On the other hand, the wheat p63 is a PPR protein having 9 PPR motifs, and it has been suggested that it binds with DNA in a sequence-specific manner, which has been proven by gel shift assay (Non-patent document 10). The GUN1 protein of *Arabidopsis thaliana* has 11 PPR motifs, and it has been suggested that it binds with DNA, which has been proven by pull-down assay (Non-patent document 11). It has been demonstrated by run-on assay that the *Arabidopsis thaliana* pTac2 (protein having 15 PPR motifs, Non-patent document 12) and *Arabidopsis thaliana* DG1 (protein having 10 PPR motifs, Non-patent document 13) directly participate in transcription for generating RNA by using DNA as a template, and they are considered to bind with DNA. An *Arabidopsis thaliana* strain deficient in the gene of GRP23 (protein having 11 PPR motifs, Non-patent document 14) shows a phenotype of embryonal death. It has been demonstrated that this protein physically interacts with the major subunit of the eukaryotic RNA transcription polymerase 2, which is a DNA-dependent RNA transcription enzyme, and therefore it is considered that GRP23 also acts in binding with DNA. The inventors of the present invention analyzed the structures and functions of p63 of wheat, GUN1 of *Arabidopsis thaliana,* pTac2 of *Arabidopsis thaliana,* DG1 of *Arabidopsis thaliana,* and so forth with a prediction that the RNA recognition rules of the PPR motifs can also be applied to the recognition of DNA, and proposed a method for designing a custom-made DNA-binding protein that binds to a desired sequence (Patent document 4).

### Prior art references

### Patent documents

Patent document 1: WO2011/072246
Patent document 2: WO2011/111829
Patent document 3: WO2013/058404
Patent document 4: WO2014/175284

### Non-patent documents

Non-patent document 1: Maeder, M.L., et al. (2008) Rapid "open-source" engineering of customized zinc-finger nucleases for highly efficient gene modification, Mol. Cell 31, 294-301
Non-patent document 2: Urnov, F.D., et al. (2010) Genome editing with engineered zinc finger nucleases, Nature Review Genetics, 11, 636-646
Non-patent document 3: Miller, J.C., et al. (2011) A TALE nuclease architecture for efficient genome editing, Nature Biotech., 29, 143-148
Non-patent document 4: Mali P., et al. (2013) RNA-guided human genome engineering via Cas9, Science, 339, 823-826
Non-patent document 5: Cong L., et al. (2013) Multiplex genome engineering using CRISPR/Cas systems, Science, 339, 819-823
Non-patent document 6: Small, I.D. and Peeters, N. (2000) The PPR motif - a TPR-related motif prevalent in plant organellar proteins, Trends Biochem. Sci., 25, 46-47
Non-patent document 7: Woodson, J.D., and Chory, J. (2008) Coordination of gene expression between organellar and nuclear genomes, Nature Rev. Genet., 9, 383-395
Non-patent document 8: Wang, X., et al. (2002) Modular recognition of RNA by a human pumilio-homology domain, Cell, 110, 501-512
Non-patent document 9: Cheong, C.G., and Hall and T.M. (2006) Engineering RNA sequence specificity of Pumilio repeats, Proc. Natl. Acad. Sci. USA 103, 13635-13639
Non-patent document 10: Ikeda T.M. and Gray M.W. (1999) Characterization of a DNA-binding protein implicated in transcription in wheat mitochondria, Mol. Cell Bio., 119 (12):8113-8122
Non-patent document 11: Koussevitzky S., et al. (2007) Signals from chloroplasts converge to regulate nuclear gene expression, Science, 316:715-719
Non-patent Document 12: Pfalz J, et al. (2006) PTAC2, -6, and -12 are components of the transcriptionally active plastid chromosome that are required for plastid gene expression, Plant Cell 18:176-197
Non-patent document 13: Chi W, et al. (2008) The pentatricopeptide repeat protein DELAYED GREENING 1 is involved in the regulation of early chloroplast development and chloroplast gene expression in Arabidopsis, Plant Physiol., 147:573-584 Non-patent document 14: Ding YH, et al. (2006) Arabidopsis GLUTAMINE-RICH PROTEIN 23 is essential for early embryogenesis and encodes a novel nuclear PPR motif protein that interacts with RNA polymerase II subunit III, Plant Cell, 18:815-830

### Summary of the Invention

### Object to be Achieved by the Invention

As actual dPPR proteins (DNA-binding proteins using PPR), there are only P63, GUN1, pTAC2, GRP23, and DG1 described in Patent document 4, and it is hard to say that they are sufficient for acquiring information for generalizing and improving the artificial nucleic acid-binding modules based on the PPR techniques.

### Means for Achieving the Object

Therefore, the inventors of the present invention decided to perform screening for searching PPR proteins having a DNA-binding ability to increase dPPR proteins. While the genes of the dPPR proteins accidentally found so far contain an intron, almost all the genes of rPPR proteins (RNA-binding proteins using PPR) do not have any intron. When the total genome sequences of the model plant, *Arabidopsis thaliana,* were analyzed by using the aforementioned fact as an index, there were found 42 types of PPR genes containing two or more introns. The inventors of the present invention analyzed the DNA-binding abilities of these 42 kinds of potential dPPR molecules to attempt to identify novel dPPR molecules. On the basis of the amino acid sequence information of the modules of the identified dPPR proteins, they also analyzed dPPR motif-specific amino acid sequences. They further investigated the DNA-binding abilities of modified type rPPRs containing a dPPR-specific amino acid sequence in order to verify whether the DNA-binding ability of PPR protein is increased by a dPPR-specific amino acid sequence. As a result, they accomplished the present invention.

The present invention provides the followings.
[1] A protein that can bind in a DNA base-selective manner or a DNA base sequence-specific manner, which contains one or more PPR motifs having a structure of the following formula 1:
   [Chemical Formula 1]

   (Helix A)-X-(Helix B)-L (Formula 1)

   (wherein, in the formula 1:
   Helix A is a part that can form an α-helix structure;
   X does not exist, or is a part consisting of 1 to 9 amino acids;
   Helix B is a part that can form an α-helix structure; and
   L is a part consisting of 2 to 7 amino acids),
   wherein,
   under the following definitions:
   the first amino acid of Helix A is referred to as No. 1 amino acid (No. 1 A.A.), the fourth amino acid as No. 4 amino acid (No. 4 A.A.), and
      - when a next PPR motif (Mₙ₊₁) contiguously exists on the C-terminus side of the PPR motif (Mₙ) (when there is no amino acid insertion between the PPR motifs), the -2nd amino acid counted from the end (C-terminus side) of the amino acids constituting the PPR motif (Mₙ);
      - when a non-PPR motif consisting of 1 to 20 amino acids exists between the PPR motif (Mₙ) and the next PPR motif (Mₙ₊₁) on the C-terminus side, the amino acid locating upstream of the first amino acid of the next PPR motif (Mₙ₊₁) by 2 positions, i.e., the -2nd amino acid; or
      - when any next PPR motif (Mₙ₊₁) does not exist on the C-terminus side of the PPR motif (Mₙ), or 21 or more amino acids constituting a non-PPR motif exist between the PPR motif (Mₙ) and the next PPR motif (Mₙ₊₁) on the C-terminus side, the 2nd amino acid counted from the end (C-terminus side) of the amino acids constituting the PPR motif (Mₙ)
         is referred to as No. "ii" (-2) amino acid (No. "ii" (-2) A.A.),
   one PPR motif (Mₙ) contained in the protein is a PPR motif having a specific combination of amino acids corresponding to a target DNA base or target DNA base sequence as the three amino acids of No. 1 A.A., No. 4 A.A., and No. "ii" (-2) A.A, and the protein satisfies at least one selected from the group consisting of the following conditions (a) to (h), preferably (b) to (h):
      (a) No. 7 A.A. of the PPR motif (Mₙ) is isoleucine (I);
      (b) No. 9 A.A. of the PPR motif (Mₙ) is alanine (A);
      (c) No. 10 A.A. of the PPR motif (Mₙ) is tyrosine (Y), phenylalanine (F), or tryptophan (W);
      (d) No. 18 A.A. of the PPR motif (Mₙ) is lysine (K), arginine (R), or histidine (H);
      (e) No. 20 A.A. of the PPR motif (Mₙ) is glutamic acid (E), or aspartic acid (D);
      (f) No. 29 A.A. of the PPR motif (Mₙ) is glutamic acid (E), or aspartic acid (D);
      (g) No. 31 A.A. of the PPR motif (Mₙ) is isoleucine (I), leucine (L), or valine (V); and
      (h) No. 32 A.A. of the PPR motif (Mₙ) is lysine (K), arginine (R), or histidine (H) (provided that a protein consisting of any one of the amino acid sequences of SEQ ID NOS: 1 to 5 and SEQ ID NOS: 291 to 308 is excluded).
[2] The protein according to [1], wherein the combination of the three amino acids of No. 1 A.A., No. 4 A.A., and No. "ii" (-2) A.A. is a combination corresponding to a target DNA base or target DNA base sequence, and the combination of amino acids is determined according to any one of the following definitions:
   (1-1) when No. 4 A.A. is glycine (G), No. 1 A.A. may be an arbitrary amino acid, and No. "ii" (-2) A.A. is aspartic acid (D), asparagine (N), or serine (S);
   (1-2) when No. 4 A.A. is isoleucine (I), each of No. 1 A.A. and No. "ii" (-2) A.A. may be an arbitrary amino acid;
   (1-3) when No. 4 A.A. is leucine (L), each of No. 1 A.A. and No. "ii" (-2) A.A. may be an arbitrary amino acid;
   (1-4) when No. 4 A.A. is methionine (M), each of No. 1 A.A. and No. "ii" (-2) A.A. may be an arbitrary amino acid;
   (1-5) when No. 4 A.A. is asparagine (N), each of No. 1 A.A. and No. "ii" (-2) A.A. may be an arbitrary amino acid;
   (1-6) when No. 4 A.A. is proline (P), each of No. 1 A.A. and No. "ii" (-2) A.A. may be an arbitrary amino acid;
   (1-7) when No. 4 A.A. is serine (S), each of No. 1 A.A. and No. "ii" (-2) A.A. may be an arbitrary amino acid;
   (1-8) when No. 4 A.A. is threonine (T), each of No. 1 A.A. and No. "ii" (-2) A.A. may be an arbitrary amino acid; and
   (1-9) when No. 4 A.A. is valine (V), each of No. 1 A.A. and No. "ii" (-2) A.A. may be an arbitrary amino acid.
[3] The protein according to [1], wherein the combination of the three amino acids of No. 1 A.A., No. 4 A.A., and No. "ii" (-2) A.A. is a combination corresponding to a target DNA base or target DNA base sequence, and the combination of amino acids is determined according to any one of the following definitions:
   (2-1) when the three amino acids, No. 1 A.A., No. 4 A.A., and No. "ii" (-2) A.A. are an arbitrary amino acid, glycine, and aspartic acid, respectively, the PPR motif selectively binds to G;
   (2-2) when the three amino acids, No. 1 A.A., No. 4 A.A., and No. "ii" (-2) A.A., are glutamic acid, glycine, and aspartic acid, respectively, the PPR motif selectively binds to G;
   (2-3) when the three amino acids, No. 1 A.A., No. 4 A.A., and No. "ii" (-2) A.A., are an arbitrary amino acid, glycine, and asparagine, respectively, the PPR motif selectively binds to A;
   (2-4) when the three amino acids, No. 1 A.A., No. 4 A.A., and No. "ii" (-2) A.A., are glutamic acid, glycine, and asparagine, respectively, the PPR motif selectively binds to A;
   (2-5) when the three amino acids, No. 1 A.A., No. 4 A.A., and No. "ii" (-2) A.A., are an arbitrary amino acid, glycine, and serine, respectively, the PPR motif selectively binds to A, and next binds to C;
   (2-6) when the three amino acids, No. 1 A.A., No. 4 A.A., and No. "ii" (-2) A.A., are an arbitrary amino acid, isoleucine, and an arbitrary amino acid, respectively, the PPR motif selectively binds to T and C;
   (2-7) when the three amino acids, No. 1 A.A., No. 4 A.A., and No. "ii" (-2) A.A., are an arbitrary amino acid, isoleucine, and asparagine, respectively, the PPR motif selectively binds to T, and next binds to C;
   (2-8) when the three amino acids, No. 1 A.A., No. 4 A.A., and No. "ii" (-2) A.A., are an arbitrary amino acid, leucine, and an arbitrary amino acid, respectively, the PPR motif selectively binds to T and C;
   (2-9) when the three amino acids, No. 1 A.A., No. 4 A.A., and No. "ii" (-2) A.A., are an arbitrary amino acid, leucine, and aspartic acid, respectively, the PPR motif selectively binds to C;
   (2-10) when the three amino acids, No. 1 A.A., No. 4 A.A., and No. "ii" (-2) A.A., are an arbitrary amino acid, leucine, and lysine, respectively, the PPR motif selectively binds to T;
   (2-11) when the three amino acids, No. 1 A.A., No. 4 A.A., and No. "ii" (-2) A.A., are an arbitrary amino acid, methionine, and an arbitrary amino acid, respectively, the PPR motif selectively binds to T;
   (2-12) when the three amino acids, No. 1 A.A., No. 4 A.A., and No. "ii" (-2) A.A., are an arbitrary amino acid, methionine, and aspartic acid, respectively, the PPR motif selectively binds to T;
   (2-13) when the three amino acids, No. 1 A.A., No. 4 A.A., and No. "ii" (-2) A.A., are isoleucine, methionine, and aspartic acid, respectively, the PPR motif selectively binds to T, and next binds to C;
   (2-14) when the three amino acids, No. 1 A.A., No. 4 A.A., and No. "ii" (-2) A.A., are an arbitrary amino acid, asparagine, and an arbitrary amino acid, respectively, the PPR motif selectively binds to C and T;
   (2-15) when the three amino acids, No. 1 A.A., No. 4 A.A., and No. "ii" (-2) A.A., are an arbitrary amino acid, asparagine, and aspartic acid, respectively, the PPR motif selectively binds to T;
   (2-16) when the three amino acids, No. 1 A.A., No. 4 A.A., and No. "ii" (-2) A.A., are phenylalanine, asparagine, and aspartic acid, respectively, the PPR motif selectively binds to T;
   (2-17) when the three amino acids, No. 1 A.A., No. 4 A.A., and No. "ii" (-2) A.A., are glycine, asparagine, and aspartic acid, respectively, the PPR motif selectively binds to T; (2-18) when the three amino acids, No. 1 A.A., No. 4 A.A., and No. "ii" (-2) A.A., are isoleucine, asparagine, and aspartic acid, respectively, the PPR motif selectively binds to T;
   (2-19) when the three amino acids, No. 1 A.A., No. 4 A.A., and No. "ii" (-2) A.A., are threonine, asparagine, and aspartic acid, respectively, the PPR motif selectively binds to T;
   (2-20) when the three amino acids, No. 1 A.A., No. 4 A.A., and No. "ii" (-2) A.A. are valine, asparagine, and aspartic acid, respectively, the PPR motif selectively binds to T, and next binds to C;
   (2-21) when the three amino acids, No. 1 A.A., No. 4 A.A., and No. "ii" (-2) A.A. are tyrosine, asparagine, and aspartic acid, respectively, the PPR motif selectively binds to T, and next binds to C;
   (2-22) when the three amino acids, No. 1 A.A., No. 4 A.A., and No. "ii" (-2) A.A., are an arbitrary amino acid, asparagine, and asparagine, respectively, the PPR motif selectively binds to C;
   (2-23) when the three amino acids, No. 1 A.A., No. 4 A.A., and No. "ii" (-2) A.A., are isoleucine, asparagine, and asparagine, respectively, the PPR motif selectively binds to C;
   (2-24) when the three amino acids, No. 1 A.A., No. 4 A.A., and No. "ii" (-2) A.A., are serine, asparagine, and asparagine, respectively, the PPR motif selectively binds to C;
   (2-25) when the three amino acids, No. 1 A.A., No. 4 A.A., and No. "ii" (-2) A.A., are valine, asparagine, and asparagine, respectively, the PPR motif selectively binds to C;
   (2-26) when the three amino acids, No. 1 A.A., No. 4 A.A., and No. "ii" (-2) A.A., are an arbitrary amino acid, asparagine, and serine, respectively, the PPR motif selectively binds to C;
   (2-27) when the three amino acids, No. 1 A.A., No. 4 A.A., and No. "ii" (-2) A.A., are valine, asparagine, and serine, respectively, the PPR motif selectively binds to C;
   (2-28) when the three amino acids, No. 1 A.A., No. 4 A.A., and No. "ii" (-2) A.A., are an arbitrary amino acid, asparagine, and threonine, respectively, the PPR motif selectively binds to C;
   (2-29) when the three amino acids, No. 1 A.A., No. 4 A.A., and No. "ii" (-2) A.A., are valine, asparagine, and threonine, respectively, the PPR motif selectively binds to C;
   (2-30) when the three amino acids, No. 1 A.A., No. 4 A.A., and No. "ii" (-2) A.A., are an arbitrary amino acid, asparagine, and tryptophan, respectively, the PPR motif selectively binds to C, and next binds to T;
   (2-31) when the three amino acids, No. 1 A.A., No. 4 A.A., and No. "ii" (-2) A.A., are isoleucine, asparagine, and tryptophan, respectively, the PPR motif selectively binds to T, and next binds to C;
   (2-32) when the three amino acids, No. 1 A.A., No. 4 A.A., and No. "ii" (-2) A.A., are an arbitrary amino acid, proline, and an arbitrary amino acid, respectively, the PPR motif selectively binds to T;
   (2-33) when the three amino acids, No. 1 A.A., No. 4 A.A., and No. "ii" (-2) A.A., are an arbitrary amino acid, proline, and aspartic acid, respectively, the PPR motif selectively binds to T;
   (2-34) when the three amino acids, No. 1 A.A., No. 4 A.A., and No. "ii" (-2) A.A., are phenylalanine, proline, and aspartic acid, respectively, the PPR motif selectively binds to T;
   (2-35) when the three amino acids, No. 1 A.A., No. 4 A.A., and No. "ii" (-2) A.A., are tyrosine, proline, and aspartic acid, respectively, the PPR motif selectively binds to T;
   (2-36) when the three amino acids, No. 1 A.A., No. 4 A.A., and No. "ii" (-2) A.A., are an arbitrary amino acid, serine, and an arbitrary amino acid, respectively, the PPR motif selectively binds to A and G;
   (2-37) when the three amino acids, No. 1 A.A., No. 4 A.A., and No. "ii" (-2) A.A., are an arbitrary amino acid, serine, and asparagine, respectively, the PPR motif selectively binds to A;
   (2-38) when the three amino acids, No. 1 A.A., No. 4 A.A., and No. "ii" (-2) A.A., are phenylalanine, serine, and asparagine, respectively, the PPR motif selectively binds to A;
   (2-39) when the three amino acids, No. 1 A.A., No. 4 A.A., and No. "ii" (-2) A.A., are valine, serine, and asparagine, respectively, the PPR motif selectively binds to A;
   (2-40) when the three amino acids, No. 1 A.A., No. 4 A.A., and No. "ii" (-2) A.A., are an arbitrary amino acid, threonine, and an arbitrary amino acid, respectively, the PPR motif selectively binds to A and G;
   (2-41) when the three amino acids, No. 1 A.A., No. 4 A.A., and No. "ii" (-2) A.A., are an arbitrary amino acid, threonine, and aspartic acid, respectively, the PPR motif selectively binds to G;
   (2-42) when the three amino acids, No. 1 A.A., No. 4 A.A., and No. "ii" (-2) A.A., are valine, threonine, and aspartic acid, respectively, the PPR motif selectively binds to G;
   (2-43) when the three amino acids, No. 1 A.A., No. 4 A.A., and No. "ii" (-2) A.A., are an arbitrary amino acid, threonine, and asparagine, respectively, the PPR motif selectively binds to A;
   (2-44) when the three amino acids, No. 1 A.A., No. 4 A.A., and No. "ii" (-2) A.A., are phenylalanine, threonine, and asparagine, respectively, the PPR motif selectively binds to A;
   (2-45) when the three amino acids, No. 1 A.A., No. 4 A.A., and No. "ii" (-2) A.A., are isoleucine, threonine, and asparagine, respectively, the PPR motif selectively binds to A; (2-46) when the three amino acids, No. 1 A.A., No. 4 A.A., and No. "ii" (-2) A.A., are valine, threonine, and asparagine, respectively, the PPR motif selectively binds to A;
   (2-47) when the three amino acids, No. 1 A.A., No. 4 A.A., and No. "ii" (-2) A.A., are an arbitrary amino acid, valine, and an arbitrary amino acid, respectively, the PPR motif binds with A, C, and T, but does not bind to G;
   (2-48) when the three amino acids, No. 1 A.A., No. 4 A.A., and No. "ii" (-2) A.A., are isoleucine, valine, and aspartic acid, respectively, the PPR motif selectively binds to C, and next binds to A;
   (2-49) when the three amino acids, No. 1 A.A., No. 4 A.A., and No. "ii" (-2) A.A., are an arbitrary amino acid, valine, and glycine, respectively, the PPR motif selectively binds to C; and
   (2-50) when the three amino acids, No. 1 A.A., No. 4 A.A., and No. "ii" (-2) A.A., are an arbitrary amino acid, valine, and threonine, respectively, the PPR motif selectively binds to T.
[4] The protein according to any one of [1] to [3], which contains 2 to 30 of the PPR motifs (Mₙ) defined in [1].
[5] The protein according to any one of [1] to [4], which satisfies at least one selected from the group consisting of the combination of (b) and (c), the combination of (d) and (e), (a), (g), and (h), preferably the protein according to anyone of [1] to [4], which satisfies at least one selected from the group consisting of the combination of (b) and (c), the combination of (d) and (e), (g), and (h).
[6] The protein according to [5], which satisfies the combination of (b) and (c), and satisfies at least one selected from the group consisting of the combination of (d) and (e), (a), (g), and (h), preferably the protein according to [5], which satisfies the combination of (b) and (c), and satisfies at least one selected from the group consisting of the combination of (d) and (e), (g), and (h).
[7] The protein according to [6], which satisfies the combination of (b) and (c), the combination of (d) and (e), (a), and (g), preferably the protein according to [6], which satisfies the combination of (b) and (c), the combination of (d) and (e), and (g).
[8] The protein according to any one of [1] to [7], which contains a plurality of PPR motifs, and satisfies any of the following (i) to (viii):
   (i) at least 40% of No. 7 A.A. consists of isoleucine (I);
   (ii) at least 36% of No. 9 A.A. consists of alanine (A);
   (iii) at least 37% of No. 10 A.A. consists of tyrosine (Y), phenylalanine (F), or tryptophan (W);
   (iv) at least 19% of No. 18 A.A. consists of lysine (K), arginine (R), or histidine (H);
   (v) at least 21% of No. 20 A.A. consists of glutamic acid (E) or aspartic acid (D);
   (vi) at least 9% of No. 29 A.A. consists of glutamic acid (E) or aspartic acid (D);
   (vii) at least 16% of No. 31 A.A. consists of isoleucine (I), leucine (L), or valine (V);
   (viii) at least 15% of No. 32 A.A. consists of lysine (K), arginine (R), or histidine (H), or
      the protein according to any one of [1] to [7], which contains a plurality of PPR motifs, and has a DNA-binding PPR motif content of 13% or higher.
[9] A protein consisting of:
   any one of the amino acid sequences of SEQ ID NOS: 7 to 214;
   any one amino acid sequence selected from the group consisting of the amino acid sequence of the 167 to 482 positions of SEQ ID NO: 291, the amino acid sequence of the 156 to 575 positions of SEQ ID NO: 292, the amino acid sequence of the 243 to 554 positions of SEQ ID NO: 293, the amino acid sequence of the 140 to 489 positions of SEQ ID NO: 294, the amino acid sequence of the 78 to 419 positions of SEQ ID NO: 295, the amino acid sequence of the 122 to 545 positions of SEQ ID NO: 296, the amino acid sequence of the 256 to 624 positions of SEQ ID NO: 297, the amino acid sequence of the 48 to 362 positions of SEQ ID NO: 298, the amino acid sequence of the 198 to 689 positions of SEQ ID NO: 299, the amino acid sequence of the 89 to 578 positions of SEQ ID NO: 300, the amino acid sequence of the 470 to 911 positions of SEQ ID NO: 301, the amino acid sequence of the 156 to 575 positions of SEQ ID NO: 302, the amino acid sequence of the 108 to 775 positions of SEQ ID NO: 303, the amino acid sequence of the 226 to 1137 positions of SEQ ID NO: 304, the amino acid sequence of the 145 to 496 positions of SEQ ID NO: 305, the amino acid sequence of the 104 to 538 positions of SEQ ID NO: 306, the amino acid sequence of the 151 to 502 positions of SEQ ID NO: 307, and the amino acid sequence of the 274 to 660 positions of SEQ ID NO: 308;
   any one of the amino acid sequences of SEQ ID NOS: 335 to 361; or
   any one of the amino acid sequences of SEQ ID NOS: 424 to 427.
[10] A complex consisting of
   a region consisting of
   the protein according to any one of [1] to [9], or a protein consisting of any one of the amino acid sequences of SEQ ID NOS: 291 to 308, or a part thereof;
   a protein consisting of any one of the amino acid sequences of SEQ ID NOS: 335 to 361; or
   a protein consisting of any one of the amino acid sequences of SEQ ID NOS: 424 to 427, and
   a functional region bound together.
[11] The complex according to [10], wherein the functional region is fused to the protein on the C-terminus side of the protein.
[12] The complex according to [10] or [11], wherein the functional region is a DNA-cleaving enzyme, or a nuclease domain thereof, or a transcription control domain, and the complex functions as a target sequence-specific DNA-cleaving enzyme or transcription control factor.
[13] The complex according to [12], wherein the DNA-cleaving enzyme is the nuclease domain of FokI (SEQ ID NO: 6).
[14] A method for designing a protein that binds to a DNA base or DNA having a specific base sequence, which comprises replacing one or two or more amino acids on the basis of any one selected from the group consisting of (a) to (h), preferably (b) to (h), defined in [1] in any of:
   a protein having any one amino acid sequence selected from the group consisting of the amino acid sequence of the 230 to 541 positions of SEQ ID NO: 1, the amino acid sequence of the 234 to 621 positions of SEQ ID NO: 2, the amino acid sequence of the 106 to 632 positions of SEQ ID NO: 3, the amino acid sequence of the 106 to 632 positions of SEQ ID NO: 4, and the amino acid sequence of the 256 to 624 positions of SEQ ID NO: 5;
   any one PPR motif selected from the group consisting of 9 PPR motifs of the protein consisting of the amino acid sequence of SEQ ID NO: 1, 11 PPR motifs of the protein consisting of the amino acid sequence of SEQ ID NO: 2, 15 PPR motifs of the protein consisting of the amino acid sequence of SEQ ID NO: 3, 10 PPR motifs of the protein consisting of the amino acid sequence of SEQ ID NO: 4, and 11 PPR motifs of the protein consisting of the amino acid sequence of SEQ ID NO: 5;
   a protein having any one amino acid sequence selected from the group consisting of the amino acid sequence of the 167 to 482 positions of SEQ ID NO: 291, the amino acid sequence of the 156 to 575 positions of SEQ ID NO: 292, the amino acid sequence of the 243 to 554 positions of SEQ ID NO: 293, the amino acid sequence of the 140 to 489 positions of SEQ ID NO: 294, the amino acid sequence of the 78 to 419 positions of SEQ ID NO: 295, the amino acid sequence of the 122 to 545 positions of SEQ ID NO: 296, the amino acid sequence of the 256 to 624 positions of SEQ ID NO: 297, the amino acid sequence of the 48 to 362 positions of SEQ ID NO: 298, the amino acid sequence of the 198 to 689 positions of SEQ ID NO: 299, the amino acid sequence of the 89 to 578 positions of SEQ ID NO: 300, the amino acid sequence of the 470 to 911 positions of SEQ ID NO: 301, the amino acid sequence of the 156 to 575 positions of SEQ ID NO: 302, the amino acid sequence of the 108 to 775 positions of SEQ ID NO: 303, the amino acid sequence of the 226 to 1137 positions of SEQ ID NO: 304, the amino acid sequence of the 145 to 496 positions of SEQ ID NO: 305, the amino acid sequence of the 104 to 538 positions of SEQ ID NO: 306, the amino acid sequence of the 151 to 502 positions of SEQ ID NO: 307, and the amino acid sequence of the 274 to 660 positions of SEQ ID NO: 308, and
   any one PPR motif selected from the group consisting of 9 PPR motifs of the protein consisting of the amino acid sequence SEQ ID NO: 291, 6 PPR motifs of the protein consisting of the amino acid sequence of SEQ ID NO: 292, 9 PPR motifs of the protein consisting of the amino acid sequence of SEQ ID NO: 293, 10 PPR motifs of the protein consisting of the amino acid sequence of SEQ ID NO: 294, 9 PPR motifs of the protein consisting of the amino acid sequence of SEQ ID NO: 295, 12 PPR motifs of the protein consisting of the amino acid sequence of SEQ ID NO: 296, 10 PPR motifs of the protein consisting of the amino acid sequence of SEQ ID NO: 297, 9 PPR motifs of the protein consisting of the amino acid sequence of SEQ ID NO: 298, 14 PPR motifs of the protein consisting of the amino acid sequence of SEQ ID NO: 299, 14 PPR motifs of the protein consisting of the amino acid sequence of SEQ ID NO: 300, 10 PPR motifs of the protein consisting of the amino acid sequence of SEQ ID NO: 301, 12 PPR motifs of the protein consisting of the amino acid sequence of SEQ ID NO: 302, 19 PPR motifs of the protein consisting of the amino acid sequence of SEQ ID NO: 303, 25 PPR motifs of the protein consisting of the amino acid sequence of SEQ ID NO: 304, 10 PPR motifs of the protein consisting of the amino acid sequence of SEQ ID NO: 305, 9 PPR motifs of the protein consisting of the amino acid sequence of SEQ ID NO: 306, 10 PPR motifs of the protein consisting of the amino acid sequence of SEQ ID NO: 307, and 11 PPR motifs of the protein consisting of the amino acid sequence of SEQ ID NO: 308.
[15] A method for designing a protein that binds to a DNA base or DNA having a specific base sequence, which comprises making the protein contain one or more PPR motifs having a structure of the following formula 1:
   [Chemical Formula 2]

   (Helix A)-X-(Helix B)-L (Formula 1)

   (wherein, in the formula 1:
   Helix A is a part that can form an α-helix structure;
   X does not exist, or is a part consisting of 1 to 9 amino acids;
   Helix B is a part that can form an α-helix structure; and
   L is a part consisting of 2 to 7 amino acids),
   wherein,
   under the following definitions:
   the first amino acid of Helix A is referred to as No. 1 amino acid (No. 1 A.A.), the fourth amino acid as No. 4 amino acid (No. 4 A.A.), and
      - when a next PPR motif (Mₙ₊₁) contiguously exists on the C-terminus side of the PPR motif (Mₙ) (when there is no amino acid insertion between the PPR motifs), the -2nd amino acid counted from the end (C-terminus side) of the amino acids constituting the PPR motif (Mₙ);
      - when a non-PPR motif consisting of 1 to 20 amino acids exists between the PPR motif (Mₙ) and the next PPR motif (Mₙ₊₁) on the C-terminus side, the amino acid locating upstream of the first amino acid of the next PPR motif (Mₙ₊₁) by 2 positions, i.e., the -2nd amino acid; or
      - when any next PPR motif (Mₙ₊₁) does not exist on the C-terminus side of the PPR motif (Mₙ), or 21 or more amino acids constituting a non-PPR motif exist between the PPR motif (Mₙ) and the next PPR motif (Mₙ₊₁) on the C-terminus side, the 2nd amino acid counted from the end (C-terminus side) of the amino acids constituting the PPR motif (Mₙ)
         is referred to as No. "ii" (-2) amino acid (No. "ii" (-2) A.A.),
   one PPR motif (Mₙ) contained in the protein is a PPR motif having a specific combination of amino acids corresponding to a target DNA base or target DNA base sequence as the three amino acids of No. 1 A.A., No. 4 A.A., and No. "ii" (-2) A.A, and satisfies at least one selected from the group consisting of the following conditions (a) to (h), preferably (b) to (h):
      (a) No. 7 A.A. of the PPR motif (Mₙ) is isoleucine (I);
      (b) No. 9 A.A. of the PPR motif (Mₙ) is alanine (A);
      (c) No. 10 A.A. of the PPR motif (Mₙ) is tyrosine (Y), phenylalanine (F), or tryptophan (W);
      (d) No. 18 A.A. of the PPR motif (Mₙ) is lysine (K), arginine (R), or histidine (H);
      (e) No. 20 A.A. of the PPR motif (Mₙ) is glutamic acid (E), or aspartic acid (D);
      (f) No. 29 A.A. of the PPR motif (Mₙ) is glutamic acid (E), or aspartic acid (D);
      (g) No. 31 A.A. of the PPR motif (Mₙ) is isoleucine (I), leucine (L), or valine (V); and
      (h) No. 32 A.A. of the PPR motif (Mₙ) is lysine (K), arginine (R), or histidine (H).
[16] The method according to [15], wherein the combination of the three amino acids of No. 1 A.A., No. 4 A.A., and No. "ii" (-2) A.A. is determined according to any one of the following definitions:
   (1-1) when No. 4 A.A. is glycine (G), No. 1 A.A. may be an arbitrary amino acid, and No. "ii" (-2) A.A. is aspartic acid (D), asparagine (N), or serine (S);
   (1-2) when No. 4 A.A. is isoleucine (I), each of No. 1 A.A. and No. "ii" (-2) A.A. may be an arbitrary amino acid;
   (1-3) when No. 4 A.A. is leucine (L), each of No. 1 A.A. and No. "ii" (-2) A.A. may be an arbitrary amino acid;
   (1-4) when No. 4 A.A. is methionine (M), each of No. 1 A.A. and No. "ii" (-2) A.A. may be an arbitrary amino acid;
   (1-5) when No. 4 A.A. is asparagine (N), each of No. 1 A.A. and No. "ii" (-2) A.A. may be an arbitrary amino acid;
   (1-6) when No. 4 A.A. is proline (P), each of No. 1 A.A. and No. "ii" (-2) A.A. may be an arbitrary amino acid;
   (1-7) when No. 4 A.A. is serine (S), each of No. 1 A.A. and No. "ii" (-2) A.A. may be an arbitrary amino acid;
   (1-8) when No. 4 A.A. is threonine (T), each of No. 1 A.A. and No. "ii" (-2) A.A. may be an arbitrary amino acid; and
   (1-9) when No. 4 A.A. is valine (V), each of No. 1 A.A. and No. "ii" (-2) A.A. may be an arbitrary amino acid.
[17] The method according to [15], wherein the combination of the three amino acids of No. 1 A.A., No. 4 A.A., and No. "ii" (-2) A.A. is determined according to any one of the following definitions:
   (2-1) when the three amino acids, No. 1 A.A., No. 4 A.A., and No. "ii" (-2) A.A. are an arbitrary amino acid, glycine, and aspartic acid, respectively, the PPR motif selectively binds to G;
   (2-2) when the three amino acids, No. 1 A.A., No. 4 A.A., and No. "ii" (-2) A.A., are glutamic acid, glycine, and aspartic acid, respectively, the PPR motif selectively binds to G;
   (2-3) when the three amino acids, No. 1 A.A., No. 4 A.A., and No. "ii" (-2) A.A., are an arbitrary amino acid, glycine, and asparagine, respectively, the PPR motif selectively binds to A;
   (2-4) when the three amino acids, No. 1 A.A., No. 4 A.A., and No. "ii" (-2) A.A., are glutamic acid, glycine, and asparagine, respectively, the PPR motif selectively binds to A;
   (2-5) when the three amino acids, No. 1 A.A., No. 4 A.A., and No. "ii" (-2) A.A., are an arbitrary amino acid, glycine, and serine, respectively, the PPR motif selectively binds to A, and next binds to C;
   (2-6) when the three amino acids, No. 1 A.A., No. 4 A.A., and No. "ii" (-2) A.A., are an arbitrary amino acid, isoleucine, and an arbitrary amino acid, respectively, the PPR motif selectively binds to T and C;
   (2-7) when the three amino acids, No. 1 A.A., No. 4 A.A., and No. "ii" (-2) A.A., are an arbitrary amino acid, isoleucine, and asparagine, respectively, the PPR motif selectively binds to T, and next binds to C;
   (2-8) when the three amino acids, No. 1 A.A., No. 4 A.A., and No. "ii" (-2) A.A., are an arbitrary amino acid, leucine, and an arbitrary amino acid, respectively, the PPR motif selectively binds to T and C;
   (2-9) when the three amino acids, No. 1 A.A., No. 4 A.A., and No. "ii" (-2) A.A., are an arbitrary amino acid, leucine, and aspartic acid, respectively, the PPR motif selectively binds to C;
   (2-10) when the three amino acids, No. 1 A.A., No. 4 A.A., and No. "ii" (-2) A.A., are an arbitrary amino acid, leucine, and lysine, respectively, the PPR motif selectively binds to T;
   (2-11) when the three amino acids, No. 1 A.A., No. 4 A.A., and No. "ii" (-2) A.A., are an arbitrary amino acid, methionine, and an arbitrary amino acid, respectively, the PPR motif selectively binds to T;
   (2-12) when the three amino acids, No. 1 A.A., No. 4 A.A., and No. "ii" (-2) A.A., are an arbitrary amino acid, methionine, and aspartic acid, respectively, the PPR motif selectively binds to T;
   (2-13) when the three amino acids, No. 1 A.A., No. 4 A.A., and No. "ii" (-2) A.A., are isoleucine, methionine, and aspartic acid, respectively, the PPR motif selectively binds to T, and next binds to C;
   (2-14) when the three amino acids, No. 1 A.A., No. 4 A.A., and No. "ii" (-2) A.A., are an arbitrary amino acid, asparagine, and an arbitrary amino acid, respectively, the PPR motif selectively binds to C and T;
   (2-15) when the three amino acids, No. 1 A.A., No. 4 A.A., and No. "ii" (-2) A.A., are an arbitrary amino acid, asparagine, and aspartic acid, respectively, the PPR motif selectively binds to T;
   (2-16) when the three amino acids, No. 1 A.A., No. 4 A.A., and No. "ii" (-2) A.A., are phenylalanine, asparagine, and aspartic acid, respectively, the PPR motif selectively binds to T;
   (2-17) when the three amino acids, No. 1 A.A., No. 4 A.A., and No. "ii" (-2) A.A., are glycine, asparagine, and aspartic acid, respectively, the PPR motif selectively binds to T; (2-18) when the three amino acids, No. 1 A.A., No. 4 A.A., and No. "ii" (-2) A.A., are isoleucine, asparagine, and aspartic acid, respectively, the PPR motif selectively binds to T;
   (2-19) when the three amino acids, No. 1 A.A., No. 4 A.A., and No. "ii" (-2) A.A., are threonine, asparagine, and aspartic acid, respectively, the PPR motif selectively binds to T;
   (2-20) when the three amino acids, No. 1 A.A., No. 4 A.A., and No. "ii" (-2) A.A. are valine, asparagine, and aspartic acid, respectively, the PPR motif selectively binds to T, and next binds to C;
   (2-21) when the three amino acids, No. 1 A.A., No. 4 A.A., and No. "ii" (-2) A.A. are tyrosine, asparagine, and aspartic acid, respectively, the PPR motif selectively binds to T, and next binds to C;
   (2-22) when the three amino acids, No. 1 A.A., No. 4 A.A., and No. "ii" (-2) A.A., are an arbitrary amino acid, asparagine, and asparagine, respectively, the PPR motif selectively binds to C;
   (2-23) when the three amino acids, No. 1 A.A., No. 4 A.A., and No. "ii" (-2) A.A., are isoleucine, asparagine, and asparagine, respectively, the PPR motif selectively binds to C;
   (2-24) when the three amino acids, No. 1 A.A., No. 4 A.A., and No. "ii" (-2) A.A., are serine, asparagine, and asparagine, respectively, the PPR motif selectively binds to C;
   (2-25) when the three amino acids, No. 1 A.A., No. 4 A.A., and No. "ii" (-2) A.A., are valine, asparagine, and asparagine, respectively, the PPR motif selectively binds to C;
   (2-26) when the three amino acids, No. 1 A.A., No. 4 A.A., and No. "ii" (-2) A.A., are an arbitrary amino acid, asparagine, and serine, respectively, the PPR motif selectively binds to C;
   (2-27) when the three amino acids, No. 1 A.A., No. 4 A.A., and No. "ii" (-2) A.A., are valine, asparagine, and serine, respectively, the PPR motif selectively binds to C;
   (2-28) when the three amino acids, No. 1 A.A., No. 4 A.A., and No. "ii" (-2) A.A., are an arbitrary amino acid, asparagine, and threonine, respectively, the PPR motif selectively binds to C;
   (2-29) when the three amino acids, No. 1 A.A., No. 4 A.A., and No. "ii" (-2) A.A., are valine, asparagine, and threonine, respectively, the PPR motif selectively binds to C;
   (2-30) when the three amino acids, No. 1 A.A., No. 4 A.A., and No. "ii" (-2) A.A., are an arbitrary amino acid, asparagine, and tryptophan, respectively, the PPR motif selectively binds to C, and next binds to T;
   (2-31) when the three amino acids, No. 1 A.A., No. 4 A.A., and No. "ii" (-2) A.A., are isoleucine, asparagine, and tryptophan, respectively, the PPR motif selectively binds to T, and next binds to C;
   (2-32) when the three amino acids, No. 1 A.A., No. 4 A.A., and No. "ii" (-2) A.A., are an arbitrary amino acid, proline, and an arbitrary amino acid, respectively, the PPR motif selectively binds to T;
   (2-33) when the three amino acids, No. 1 A.A., No. 4 A.A., and No. "ii" (-2) A.A., are an arbitrary amino acid, proline, and aspartic acid, respectively, the PPR motif selectively binds to T;
   (2-34) when the three amino acids, No. 1 A.A., No. 4 A.A., and No. "ii" (-2) A.A., are phenylalanine, proline, and aspartic acid, respectively, the PPR motif selectively binds to T;
   (2-35) when the three amino acids, No. 1 A.A., No. 4 A.A., and No. "ii" (-2) A.A., are tyrosine, proline, and aspartic acid, respectively, the PPR motif selectively binds to T;
   (2-36) when the three amino acids, No. 1 A.A., No. 4 A.A., and No. "ii" (-2) A.A., are an arbitrary amino acid, serine, and an arbitrary amino acid, respectively, the PPR motif selectively binds to A and G;
   (2-37) when the three amino acids, No. 1 A.A., No. 4 A.A., and No. "ii" (-2) A.A., are an arbitrary amino acid, serine, and asparagine, respectively, the PPR motif selectively binds to A;
   (2-38) when the three amino acids, No. 1 A.A., No. 4 A.A., and No. "ii" (-2) A.A., are phenylalanine, serine, and asparagine, respectively, the PPR motif selectively binds to A;
   (2-39) when the three amino acids, No. 1 A.A., No. 4 A.A., and No. "ii" (-2) A.A., are valine, serine, and asparagine, respectively, the PPR motif selectively binds to A;
   (2-40) when the three amino acids, No. 1 A.A., No. 4 A.A., and No. "ii" (-2) A.A., are an arbitrary amino acid, threonine, and an arbitrary amino acid, respectively, the PPR motif selectively binds to A and G;
   (2-41) when the three amino acids, No. 1 A.A., No. 4 A.A., and No. "ii" (-2) A.A., are an arbitrary amino acid, threonine, and aspartic acid, respectively, the PPR motif selectively binds to G;
   (2-42) when the three amino acids, No. 1 A.A., No. 4 A.A., and No. "ii" (-2) A.A., are valine, threonine, and aspartic acid, respectively, the PPR motif selectively binds to G;
   (2-43) when the three amino acids, No. 1 A.A., No. 4 A.A., and No. "ii" (-2) A.A., are an arbitrary amino acid, threonine, and asparagine, respectively, the PPR motif selectively binds to A;
   (2-44) when the three amino acids, No. 1 A.A., No. 4 A.A., and No. "ii" (-2) A.A., are phenylalanine, threonine, and asparagine, respectively, the PPR motif selectively binds to A;
   (2-45) when the three amino acids, No. 1 A.A., No. 4 A.A., and No. "ii" (-2) A.A., are isoleucine, threonine, and asparagine, respectively, the PPR motif selectively binds to A;
   (2-46) when the three amino acids, No. 1 A.A., No. 4 A.A., and No. "ii" (-2) A.A., are valine, threonine, and asparagine, respectively, the PPR motif selectively binds to A;
   (2-47) when the three amino acids, No. 1 A.A., No. 4 A.A., and No. "ii" (-2) A.A., are an arbitrary amino acid, valine, and an arbitrary amino acid, respectively, the PPR motif binds with A, C, and T, but does not bind to G;
   (2-48) when the three amino acids, No. 1 A.A., No. 4 A.A., and No. "ii" (-2) A.A., are isoleucine, valine, and aspartic acid, respectively, the PPR motif selectively binds to C, and next binds to A;
   (2-49) when the three amino acids, No. 1 A.A., No. 4 A.A., and No. "ii" (-2) A.A., are an arbitrary amino acid, valine, and glycine, respectively, the PPR motif selectively binds to C; and
   (2-50) when the three amino acids, No. 1 A.A., No. 4 A.A., and No. "ii" (-2) A.A., are an arbitrary amino acid, valine, and threonine, respectively, the PPR motif selectively binds to T.
[18] The method according to anyone of [15] to [17], wherein at least one selected from the group consisting of the combination of (b) and (c), the combination of (d) and (e), (a), (g), and (h), preferably at least one selected from the group consisting of the combination of (b) and (c), the combination of (d) and (e), (g), and (h), is satisfied.
[19] The method according to [18], wherein the combination of (b) and (c) is satisfied, and at least one selected from the group consisting of the combination of (d) and (e), (a), (g), and (h), preferably at least one selected from the group consisting of the combination of (d) and (e), (g), and (h), is satisfied.
[20] The method according to [19], wherein the combination of (b) and (c), the combination of (d) and (e), (a), and (g), preferably the combination of (b) and (c), the combination of (d) and (e), and (g), are satisfied.
[21] The method according to any one of [15] to [20], wherein the protein contains a plurality of PPR motifs, and the PPR motifs satisfy any of the following (i) to (viii):
   (i) at least 40% of No. 7 A.A. consists of isoleucine (I);
   (ii) at least 36% of No. 9 A.A. consists of alanine (A);
   (iii) at least 37% of No. 10 A.A. consists of tyrosine (Y);
   (iv) at least 19% of No. 18 A.A. consists of lysine (K), arginine (R), or histidine (H);
   (v) at least 21% of No. 20 A.A. consists of glutamic acid (E) or aspartic acid (D);
   (vi) at least 9% of No. 29 A.A. consists of glutamic acid (E) or aspartic acid (D);
   (vii) at least 16% of No. 31 A.A. consists of isoleucine (I); and
   (viii) at least 15% of No. 32 A.A. consists of lysine (K), arginine (R), or histidine (H), or
      the protein contains a plurality of PPR motifs, and has a DNA-binding PPR motif content of 13% or higher.
[22] A method for producing a protein, which comprises designing a protein by the method according to any one of [14] to [21], and producing the designed protein.
[23] A method for producing a complex, which comprises designing a protein by the method according to any one of [14] to [21], and binding a region consisting of the designed protein and a functional region to produce the complex.
[24] A method for editing a genome, which comprises using the complex according to any one of [10] to [13], or
   designing a protein by the method according to anyone of [14] to [21], binding a region consisting of the designed protein and a functional region to produce a complex, and using the produced complex (implementation in a human individual is excluded).
[25] A method for producing a cell containing a edited genome, which comprises editing a genome by the method according 23, and producing a cell containing the edited genome (implementation in a human individual is excluded).

### Effect of the Invention

According to the present invention, a PPR motif that can binds to a target DNA base, and a protein containing it can be provided. By arranging two or more PPR motifs, a protein that can binds to a target DNA having an arbitrary sequence or length can be provided. A nucleic acid (DNA or RNA) encoding such a protein, and a transformant using such a nucleic acid can also be provided.

According to the present invention, a complex having an activity to bind to a specific nucleic acid sequence and comprising a protein having a specific function (for example, cleavage, transcription, replication, restoration, synthesis, modification, etc. of DNA) can be prepared. With such a complex, genome editing utilizing a function of the functional region such as cleavage, transcription, replication, restoration, synthesis, modification, etc. of a target can be realized. By the genome editing, a cell or organism having a modified genome can be provided.

### Brief Description of the Drawings

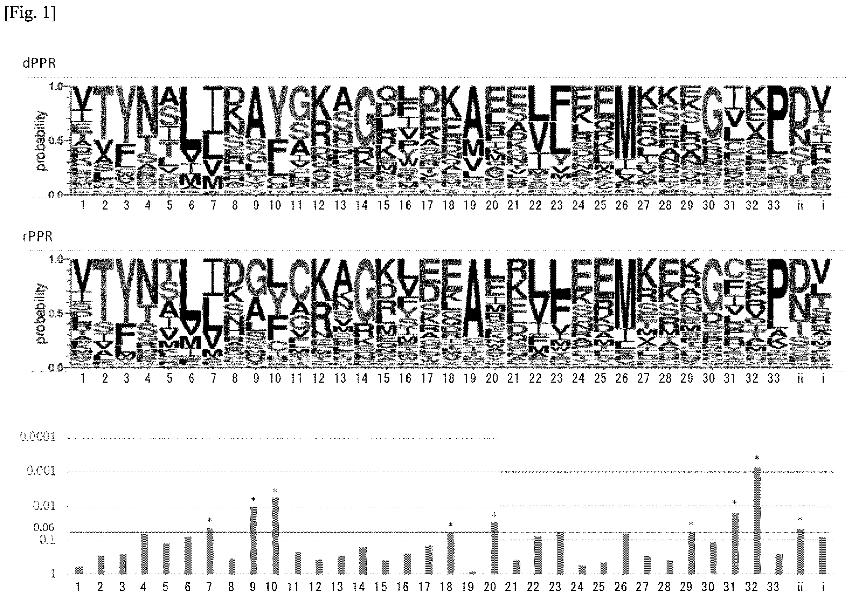

Fig. 1 shows identification of locations of the amino acids characterizing dPPR proteins. The upper part and the middle part show occurrence frequencies of amino acids of the PPR motifs at all the positions in 9 kinds of dPPR molecules and 5 known rPPR molecules, and the lower part shows the results of F test. The F test was used for comparison of the occurrence frequencies at a significance level of 5% (p < 0.06). According to the results of the F test, differences were observed in the amino acid frequencies for the residues of No. 7 amino acid (A. A.), No. 9 A.A., No. 10 A.A., No. 18 A.A., No. 20 A.A., No. 29 A.A., No. 31 A.A., No. 32 A.A., and No. ii A.A. However, No. ii A.A. was excluded, since it is a part involved in recognition of a DNA base.

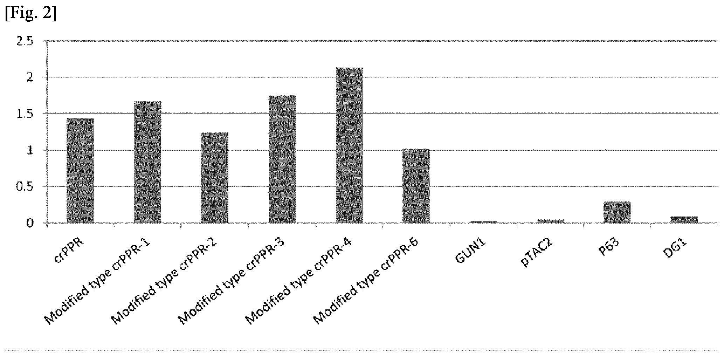

Fig. 2 shows comparison of DNA-binding powers of modified type crPPRs and naturally occurring dPPRs. The DNA binding ability was analyzed by DNA-protein pull-down assay (refer to Example 1). There were obtained results that DNA-binding powers of all the crPPRs and modified type crPPRs in which each dPPR motif-specific amino acid sequence was inserted were higher than those of GUN1, pTAC2, p63, and DG1, which are naturally occurring type dPPR molecules.

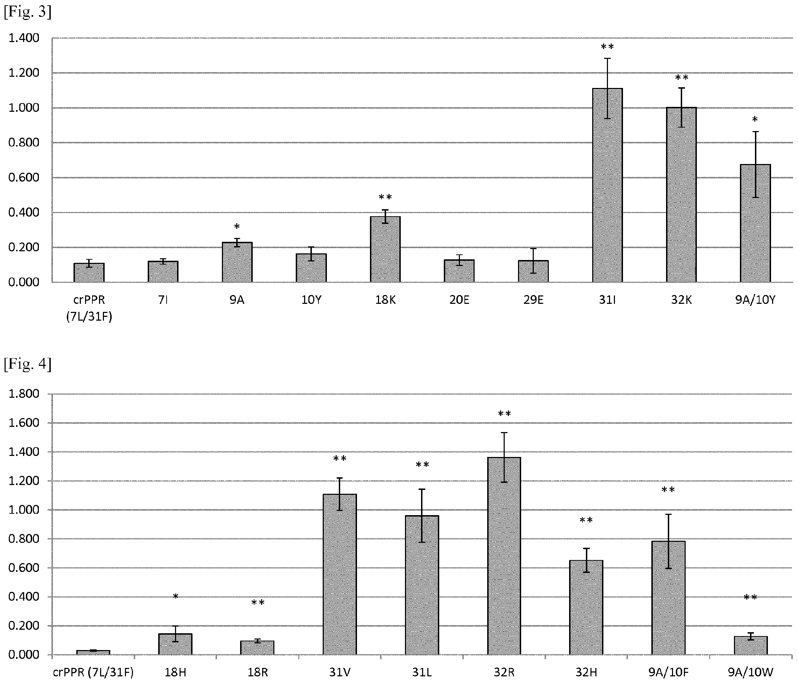

Fig. 3 shows comparison of DNA-binding powers of modified type rPPRs and crPPR (7L/31F). The powers were quantified by standardization in which luminescence intensity of each pulled-down protein was divided with luminescence intensity obtained with input 3%. As a result of the comparison of the DNA-binding powers of the modified type rPPRs and crPPR (7L/31F), significant differences were observed for modified type rPPRs introduced with of A.A. 9A, A.A. 18K, A.A. 311, A.A. 32K, and A.A. 9A/10Y. The vertical axis indicates DNA-binding power (pull down signal/input 3% signal), the introduced amino acid sequences are mentioned under the horizontal axis, * means p < 0.05, and ** means p < 0.01.
Fig. 4 shows comparison of the DNA-binding powers observed with replacing amino acids with those having similar characteristics. It was examined whether the effect can be obtained even when amino acids having similar characteristics are used for A.A. 18K, A.A. 311, A.A. 32K, and A.A. 9A/10Y. In this experiment, there were introduced histidine (H) and arginine (R), which are basic amino acids like K, for No. 18 A.A. and No. 32 A.A., valine (V) and leucine (L), which have a branched chain like I, for No. 31 A.A., and phenylalanine (F) and tryptophan (W), which have an aromatic group like Y, for No. 10 A.A. As a result of comparison of the DNA-binding powers of the modified type rPPRs and crPPR (7L/31F), significant differences were observed for all the modified type rPPRs. The vertical axis indicates DNA-binding ability (pull down signal/input 3% signal), the introduced amino acid sequences are mentioned under the horizontal axis, * means p < 0.05, and ** means p < 0.01.

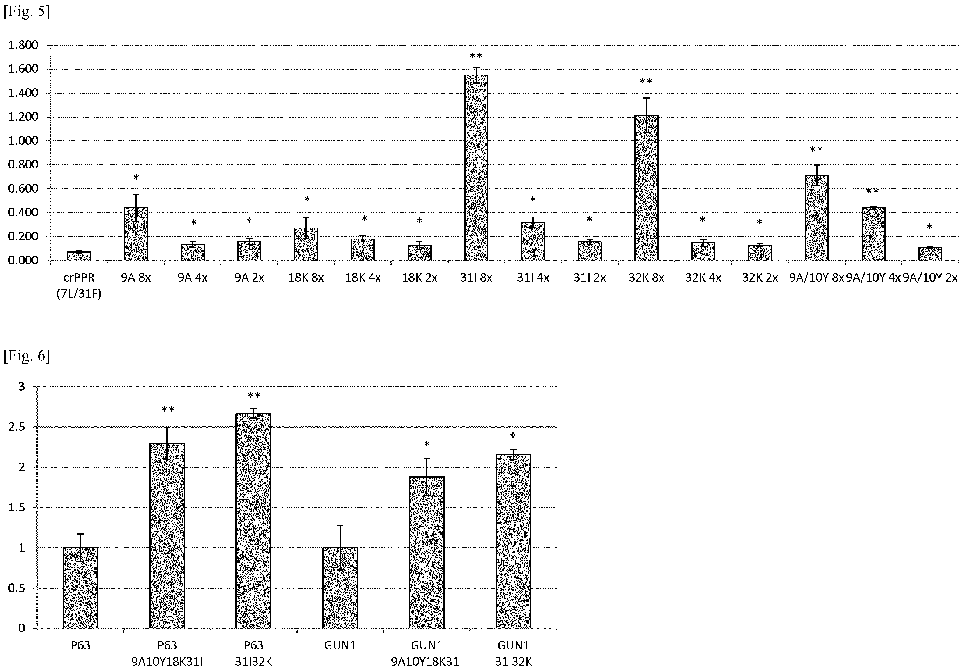

Fig. 5 shows comparison of the DNA-binding powers of the proteins having different contents of DNA-binding PPR motifs. In this experiment, there were analyzed DNA-binding powers of modified type rPPRs consisting of crPPR (7L/31F) in which 2 motifs (25% of the whole) or 4 motifs (50% of the whole) from the N-terminus were motifs having these amino acid sequences. Significant differences were observed for all the modified type rPPRs. The vertical axis indicates DNA-binding power (pull down signal/input 3% signal), the introduced amino acid sequences and contents thereof are mentioned under the horizontal axis, * means p < 0.05, and ** means p < 0.01.
Fig. 6 shows comparison of the DNA-binding powers of naturally occurring type dPPR proteins and modified type PPR proteins thereof. It was examined whether the DNA-binding ability of modified proteins of naturally occurring type dPPRs, P63 and GUN1, in which A.A. 9A/10Y/18K/31I, and A.A. 31I/32K were introduced into all the motifs thereof. The DNA-binding powers of all the P63 and GUN1 proteins introduced with any of the amino acid sequences were increased. The vertical axis indicates DNA-binding power (pull down signal/input 3% signal) calculated as relative value based on those of naturally occurring type dPPR proteins, the types of dPPR are mentioned under the horizontal axis, * means p < 0.05, and ** means p < 0.01.

### Modes for Carrying out the Invention

### [PPR motif and PPR protein]

The "PPR motif' referred to in the present invention means a polypeptide constituted with 30 to 38 amino acids and having an amino acid sequence that shows, when the amino acid sequence is analyzed with a protein domain search program on the web (for example, Pfam, Prosite, Uniprot, etc.), an E value not larger than a predetermined value (desirably E-03) obtained at PF01535 in the case of Pfam (http://pfam.sanger.ac.uk/), or PS51375 in the case of Prosite (http://www.expasy.org/prosite/), unless otherwise indicated. The PPR motifs in various proteins are also defined in the Uniprot database (http://www.uniprot.org).

Although the amino acid sequence of the PPR motif is not highly conserved in the PPR motif of the present invention, such a secondary structure of helix, loop, helix, and loop as shown by the following formula is conserved well.
[Chemical Formula 3]

(Helix A)-X-(Helix B)-L (Formula 1)

The position numbers of the amino acids constituting the PPR motif defined in the present invention are according to those defined in a paper of the inventors of the present invention (Kobayashi K, et al., Nucleic Acids Res., 40, 2712-2723 (2012)), and Patent document 4, unless especially indicated. That is, the position numbers of the amino acids constituting the PPR motif defined in the present invention are substantially the same as the amino acid numbers defined for PF01535 in Pfam, but correspond to numbers obtained by subtracting 2 from the amino acid numbers defined for PS51375 in Prosite (for example, position 1 according to the present invention is position 3 of PS51375), and also correspond to numbers obtained by subtracting 2 from the amino acid numbers of the PPR motif defined in Uniprot.

More precisely, in the present invention, the No. 1 amino acid is the first amino acid from which Helix A shown in the formula 1 starts. The No. 4 amino acid is the fourth amino acid counted from the No. 1 amino acid. As for "ii" (-2)nd amino acid,
- when a next PPR motif (Mₙ₊₁) contiguously exists on the C-terminus side of the PPR motif (Mₙ) (when there is no amino acid insertion between the PPR motifs, as in the cases of, for example, Motif Nos. 1, 2, 3,4, 6 and 7 in Fig. 4-1 (A) of Patent document 4), the -2nd amino acid counted from the end (C-terminus side) of the amino acids constituting the PPR motif (Mₙ) is referred to as No. "ii" (-2) amino acid;
- when a non-PPR motif (part that is not the PPR motif) consisting of 1 to 20 amino acids exists between the PPR motif (Mₙ) and the next PPR motif (Mₙ₊₁) on the C-terminus side (as in the cases of, for example, Motif Nos. 5 and 8 in Fig. 4-1 (A) of Patent document 4, and Motif Nos. 1, 2, 7 and 8 in Fig. 4-3 (D) of Patent document 4), the amino acid locating upstream of the first amino acid of the next PPR motif (Mₙ₊₁) by 2 positions, i.e., the -2nd amino acid, is referred to as No. "ii" (-2) amino acid (refer to Fig. 1 of Patent document 4); or
- when any next PPR motif (Mₙ₊₁) does not exist on the C-terminus side of the PPR motif (Mₙ) (as in the cases of, for example, Motif No. 9 in Fig. 4-1 (A) of Patent document 4, and Motif No. 11 in Fig. 4-1 (B) of Patent document 4), or 21 or more amino acids constituting a non-PPR motif exist between the PPR motif (Mₙ) and the next PPR motif (Mₙ₊₁) on the C-terminus side, the 2nd amino acid counted from the end (C-terminus side) of the amino acids constituting the PPR motif (Mₙ) is referred to as No. "ii" (-2) amino acid.

The positions of No. 31 A.A. and No. 32 A.A., which are amino acids contained in L of a certain PPR motif (Mₙ), may be determined on the basis of No. 1 amino acid of the next PPR motif (Mₙ₊₁) on the C-terminus side of that motif. Specifically, the No. 31 A.A. may be determined to be an amino acid locating upstream from the No. 1 amino acid of the next PPR motif (Mₙ₊₁) by 5 amino acids, and the No. 32 A.A. may be determined to be an amino acid locating upstream from the No. 1 amino acid of the next PPR motif (Mₙ₊₁) by 4 amino acids. When the next PPR motif (Mₙ₊₁) does not exist on the C-terminus side of the PPR motif (Mₙ), the 5th amino acid from the last amino acid (C-terminus side) among the amino acids constituting the PPR motif (Mₙ) is determined to be No. 31 A.A., and the amino acid locating upstream from the same by 4 amino acids is determined to be No. 32 A.A.

The "PPR protein" or "PPR molecule" referred to in the present invention means a PPR protein having one or more of the aforementioned PPR motifs, unless otherwise indicated. The term "protein" used in this specification means any substance consisting of a polypeptide (chain consisting of two or more amino acids bound through peptide bonds), and also includes those consisting of a comparatively low molecular weight polypeptide, unless otherwise indicated. The "amino acid" referred to in the present invention means a usual amino acid molecule, as well as an amino acid residue constituting a peptide chain. Which the term means will be apparent to those skilled in the art from the context.

Many PPR proteins exist in plants, and 500 proteins and about 5000 motifs can be found in *Arabidopsis thaliana.* PPR motifs and PPR proteins of various amino acid sequences also exist in many land plants such as rice, poplar, and selaginella. It is known that some PPR proteins are important factors for obtaining F1 seeds for hybrid vigor as fertility restoration factors that are involved in formation of pollen (male gamete). It has been clarified that some PPR proteins are involved in speciation, similarly in fertility restoration. It has also been clarified that almost all the PPR proteins act on RNA in mitochondria or chloroplasts.

It is known that, in animals, anomaly of the PPR protein identified as LRPPRC causes Leigh syndrome French Canadian (LSFC, Leigh's syndrome, subacute necrotizing encephalomyelopathy).

The term "selective" used for a property of a PPR motif for binding with a DNA base in the present invention means that a binding activity for any one base among the DNA bases is higher than binding activities for the other bases, unless otherwise indicated. Those skilled in the art can confirm this selectivity by planning an experiment, or it can also be obtained by calculation as described in the examples mentioned in Patent document 4.

The DNA base referred to in the present invention means a base of deoxyribonucleotide constituting DNA, and specifically, it means any of adenine (A), guanine (G), cytosine (C), and thymine (T), unless otherwise indicated. Although the PPR protein may have selectivity to a base in DNA, it does not bind to a nucleic acid monomer.

### [Information, novel dPPR protein, etc. provided by the present invention]

The present invention provides information about positions and types of amino acids important for binding with DNA, a method for designing a dPPR protein, a method for imparting a property of binding with a DNA base to a PPR protein, and a method for enhancing a property of a PPR protein for binding with DNA, which methods use the information, as well as a novel dPPR protein obtained by the aforementioned designing method, method for imparting the binding property, or method for enhancing the binding property. The origins of the dPPR protein provided by the present invention and the dPPR protein used in the present invention, and the methods for obtaining them are not particularly limited, and they may be, for example, naturally occurring dPPRs, modified naturally occurring dPPRs, dPPRs obtained by chemical synthesis, recombinant proteins of the foregoing, or the like, and they may also be fused proteins. Various dPPR proteins and embodiments using them fall within the scope of the present invention so long as they satisfy the requirements defined in the appended claims.

Designing a protein may be determining amino acid sequence of a protein according to the information provided by the present invention. Designing a protein may also be, in other words, producing a protein. The method for designing a protein, or the method for producing a protein includes the following steps:
the step of determining nucleotide sequence encoding a protein;
the step of preparing a polynucleotide having the nucleotide sequence; and
the step of preparing a transformant that is introduced with the polynucleotide, and can produce the protein.

The information about the positions of amino acids of PPR proteins important for base-selective or sequence-specific binding is disclosed in Patent documents 3 and 4. Further, according to the investigations of the inventors of the present invention, in addition to the aforementioned information, No. 7 amino acid (A.A.), No. 9 A.A., No. 10 A.A., No. 18 A.A., No. 20 A.A., No. 29 A.A., No. 31 A.A., No. 32 A.A., and No. ii A.A., preferably No. 9 A.A., No. 10 A.A., No. 18 A.A., No. 20 A.A., No. 29 A.A., No. 31 A.A., No. 32 A.A. and No. ii A.A., of the PPR motif (Mₙ) are important for binding with DNA. By paying attention to these, a property of binding with a DNA base can be imparted to PPR proteins, or a property of binding with DNA of PPR proteins can be enhanced. Since No. ii A.A. is a part involved in recognition of a DNA base, it may be excluded.

Whether a certain PPR protein has a property of binding with DNA, or degree of the binding ability of a certain PPR protein can be appropriately evaluated by those skilled in the art by planning an appropriate DNA-protein pull-down assay, or the like. As for specific experimental conditions and procedures, the sections of Examples of Patent document 4 and this specification can be referred to.

The ability of binding with DNA of the PPR protein obtained by the present invention is higher than the same of the modified PPR consisting of the consensus PPR (cPPR, also referred to as crPPR) reported in Non-patent document 15 (Coquille et al., 2014, An artificial PPR scaffold for programmable RNA recognition) cited below, of which A.A. 71 and A.A. 311 are replaced with leucine (L) and phenylalanine (F), respectively (crPPR (7L/31F)).

The ability of binding with DNA of the PPR protein obtained by the present invention is preferably higher than the same of existing DNA-binding PPRs, specifically, any one among the group consisting of p63 (SEQ ID NO: 1), GUN1 (SEQ ID NO: 2), pTac2 (SEQ ID NO: 3), DG1 (SEQ ID NO: 4), and GRP23 (SEQ ID NO: 5), more preferably higher than the abilities of binding with DNA of all of these proteins. The protein more preferably selectively binds with DNA among RNA and DNA having substantially the same sequences.

Impartation of a property of binding with DNA to a PPR protein and enhancement of a property of binding with DNA of a PPR protein can be achieved by, specifically, designing the PPR motif (Mₙ) of a base-selectively or base sequence-specifically bindable PPR protein so that it satisfies at least one condition selected from the group consisting of (a) to (h), preferably (b) to (h), mentioned below:
(a) No. 7 A.A. of the PPR motif (Mₙ) is isoleucine (I);
(b) No. 9 A.A. of the PPR motif (Mₙ) is alanine (A);
(c) No. 10 A.A. of the PPR motif (Mₙ) is tyrosine (Y), phenylalanine (F), or tryptophan (W);
(d) No. 18 A.A. of the PPR motif (Mₙ) is lysine (K), arginine (R), or histidine (H);
(e) No. 20 A.A. of the PPR motif (Mₙ) is glutamic acid (E), or aspartic acid (D);
(f) No. 29 A.A. of the PPR motif (Mₙ) is glutamic acid (E), or aspartic acid (D).
(g) No. 31 A.A. of the PPR motif (Mₙ) is isoleucine (I), leucine (L), or valine (V); and
(h) No. 32 A.A. of the PPR motif (Mₙ) is lysine (K), arginine (R), or histidine (H)

According to the investigations of the inventors of the present invention, when a DNA-binding ability of a certain PPR can be enhanced by using a specific amino acid at an appropriate position, the same effect can be obtained even if an amino acid having similar characteristics is used instead of the specific amino acid. It can be said that the amino acids of the following sets have similar characteristics: glycine and alanine (these have an alkyl chain), valine, leucine, and isoleucine (these have a branched alkyl chain), phenylalanine, tyrosine, and tryptophan (these have an aromatic group), lysine, arginine, and histidine (these have two amino groups, and are basic), aspartic acid and glutamic acid (these have two carboxyl groups and are acidic), asparagine and glutamine (these have amide group), serine and threonine (these have hydroxyl group), and cysteine and methionine (these contain sulfur).

According to the investigations of the inventors of the present invention, there are a tendency that A as No. 9 A.A. and Y as No. 10 A.A. are observed in the same motif, and a tendency that, when No. 18 A.A. is K, R, or H, No. 20 A.A. of the preceding motif is E or D. From this point of view, in one of preferred embodiments, the PPR motif (Mₙ) satisfies at least one selected from the group consisting of the combination of (b) and (c), the combination of (d) and (e), (a), (g), and (h), more preferably at least one selected from the group consisting of the combination of (b) and (c), the combination of (d) and (e), (g), and (h). In another preferred embodiment, the PPR motif (Mₙ) satisfies the combination of (b) and (c), and at least one selected from the group consisting of the combination of (d) and (e), (a), (g), and (h), more preferably the PPR motif (Mₙ) satisfies the combination of (b) and (c), and satisfies at least one selected from the group consisting of the combination of (d) and (e), (g), and (h). In still another preferred embodiment, the PPR motif (Mₙ) satisfies the combination of (b) and (c), the combination of (d) and (e), (a), and (g), more preferably the combination of (b) and (c), the combination of (d) and (e), and (g).

The PPR protein to be designed contains one or more PPR motifs (Mₙ), and it preferably contains 2 to 30, more preferably 5 to 25, still more preferably 9 to 15, of the motifs.

In the case of the protein containing two or more PPR motifs, if it is designed so that a certain part of the motifs satisfy the aforementioned conditions, a property of binding with a DNA base can be imparted to the PPR protein, or a property of binding with DNA of the PPR protein can be enhanced, even if all the contained motifs do not satisfy the requirements. For example, the protein containing two or more PPR motifs that satisfy any one of (i) to (viii) mentioned below (for example, any one, preferably any three, more preferably any five, further preferably all of them) constitutes one of the preferred embodiments of the present invention:
(i) at least 40%, preferably 44%, of No. 7 A.A. consists of isoleucine (I);
(ii) at least 36%, preferably 48%, of No. 9 A.A. consists of alanine (A);
(iii) at least 37%, preferably 49%, of No. 10 A.A. consists of tyrosine (Y);
(iv) at least 19% of No. 18 A.A. consists of lysine (K), arginine (R), or histidine (H);
(v) at least 21% of No. 20 A.A. consists of glutamic acid (E) or aspartic acid (D);
(vi) at least 9% of No. 29 A.A. consists of glutamic acid (E) or aspartic acid (D);
(vii) at least 16% of No. 31 A.A. consists of isoleucine (I); and
(viii) at least 15% of No. 32 A.A. is lysine (K), arginine (R), or histidine (H).

The ratios (%) mentioned above are calculated as [number of PPR motifs satisfying requirement]/[total number of PPR motifs contained in protein] x 100.

The PPR motif satisfying requirement is a DNA-binding PPR motif, and it refers to a PPR motif that satisfies at least one selected from the group consisting (b) to (h) mentioned above. More specifically, the ratio of DNA-binding PPR motif mentioned above may be referred to as "content of DNA-binding PPR motif', and calculated as [number of DNA-binding PPR motifs]/[(number of DNA-binding PPR motifs) + (number of PPR motifs that are not DNA-binding PPR motifs)] x 100. The PPR motif that is not a DNA-binding PPR motif refers to a PPR motif that does not satisfy all of (b) to (h) mentioned above, for example, crPPR (7L/31F).

According to the further investigations of the inventors of the present invention, in the case of a protein containing 8 PPR motifs, the DNA-binding ability thereof was significantly increased when it had a DNA-binding PPR motif content of 25% or higher, compared with a control protein of which DNA-binding PPR motif content is 0%, whereas significant increase of the DNA-binding ability was not observed for the protein of which DNA-binding PPR motif content was 12.5% compared with the control protein of which DNA-binding PPR motif content is 0%. Therefore, the PPR protein preferably contains two or more PPR motifs, and has a DNA-binding PPR motif content of 13% or higher, more preferably 15% or higher, further preferably 25% or higher, still further preferably 50% or higher, still further preferably 75% or more, still further preferably 100%.

Although the positions of DNA-binding PPRs in the protein containing two or more PPR motifs are not particularly limited, positions closer to the N-terminus are preferred. When the protein contains two or more PPR motifs, and the PPR motifs consist of two or more DNA-binding PPR motifs and PPR motifs that are not DNA-binding PPR motif, the DNA-binding PPR motifs may contiguously exist, or a PPR motif that is not DNA-binding PPR motif may exist between the DNA-binding PPR motifs, but it is considered that the DNA-binding PPR motifs preferably contiguously exist. For example, it is considered that, in the case of the protein containing 8 PPR motifs, it is preferred that 2 contiguous PPR motifs on the N-terminus side are DNA-binding PPR motifs, when the DNA-binding PPR motif content is 25%, it is preferred that 4 contiguous PPR motifs on the N-terminus side are DNA-binding PPR motifs, when the DNA-binding PPR motif content is 50%, and it is preferred that 6 contiguous PPR motifs on the N-terminus side are DNA-binding PPR motifs, when the DNA-binding PPR motif content is 75%.

The aforementioned method for imparting a property of binding with DNA to a PPR protein, or enhancing a property of binding with DNA of a PPR protein can be used not only for newly designing a DNA-binding PPR protein, but also for imparting a DNA-binding ability to an existing PPR protein, or increasing DNA-binding ability of an existing PPR protein.

The information about the positions and types of amino acids of PPR protein important for base-selective or sequence-specific binding described in Patent documents 3 and 4, which serves as the basis of the designing method of the present invention for imparting a property of binding with a DNA base to a PPR protein, or enhancing a property of binding with DNA of a PPR protein, is shown below.
(1-1) When No. 4 A.A. is glycine (G), No. 1 A.A. may be an arbitrary amino acid, No. "ii" (-2) A.A. is aspartic acid (D), asparagine (N), or serine (S), and the combination of No. 1 A.A., and No. "ii" (-2) A.A. may be, for example:
   - a combination of an arbitrary amino acid and aspartic acid (D) (*GD),
   - preferably a combination of glutamic acid (E) and aspartic acid (D) (EGD),
   - a combination of an arbitrary amino acid and asparagine (N) (*GN),
   - preferably a combination of glutamic acid (E) and asparagine (N) (EGN), or
   - a combination of an arbitrary amino acid and serine (S) (*GS);
(1-2) when No. 4 A.A. is isoleucine (I), each of No. 1 A.A. and No. "ii" (-2) A.A. may be an arbitrary amino acid, and the combination of No. 1 A.A., and No. "ii" (-2) A.A. may be, for example:
   - a combination of an arbitrary amino acid and asparagine (N) (*IN);
(1-3) when No. 4 A.A. is leucine (L), each of No. 1 A.A. and No. "ii" (-2) A.A. may be an arbitrary amino acid, and the combination of No. 1 A.A., and No. "ii" (-2) A.A. may be, for example:
   - a combination of an arbitrary amino acid and aspartic acid (D) (*LD), or
   - a combination of an arbitrary amino acid and lysine (K) (*LK);
(1-4) when No. 4 A.A. is methionine (M), each of No. 1 A.A. and No. "ii" (-2) A.A. may be an arbitrary amino acid, and the combination of No. 1 A.A., and No. "ii" (-2) A.A. may be, for example:
   - a combination of an arbitrary amino acid and aspartic acid (D) (*MD), or
   - a combination of isoleucine (I) and aspartic acid (D) (IMD);
(1-5) when No. 4 A.A. is asparagine (N), each of No. 1 A.A. and No. "ii" (-2) A.A. may be an arbitrary amino acid, and the combination of No. 1 A.A., and No. "ii" (-2) A.A. may be, for example:
   - a combination of an arbitrary amino acid and aspartic acid (D) (*ND),
   - a combination of any one of phenylalanine (F), glycine (G), isoleucine (I), threonine (T), valine (V) and tyrosines (Y), and aspartic acid (D) (FND, GND, IND, TND, VND, or YND),
   - a combination of an arbitrary amino acid and asparagine (N) (*NN),
   - a combination of any one of isoleucine (I), serine (S) and valine (V), and asparagine (N) (INN, SNN or VNN)
   - a combination of an arbitrary amino acid and serine (S) (*NS),
   - a combination of valine (V) and serine (S) (VNS),
   - a combination of an arbitrary amino acid and threonine (T) (*NT),
   - a combination of valine (V) and threonine (T) (VNT),
   - a combination of an arbitrary amino acid and tryptophan (W) (*NW), or
   - a combination of isoleucine (I) and tryptophan (W) (INW);
(1-6) when No. 4 A.A. is proline (P), each of No. 1 A.A. and No. "ii" (-2) A.A. may be an arbitrary amino acid, and the combination of No. 1 A.A., and No. "ii" (-2) A.A. may be, for example:
   - a combination of an arbitrary amino acid and aspartic acid (D) (*PD),
   - a combination of phenylalanine (F) and aspartic acid (D) (FPD), or
   - a combination of tyrosine (Y) and aspartic acid (D) (YPD);
(1-7) when No. 4 A.A. is serine (S), each of No. 1 A.A. and No. "ii" (-2) A.A. may be an arbitrary amino acid, and the combination of No. 1 A.A., and No. "ii" (-2) A.A. may be, for example:
   - a combination of an arbitrary amino acid and asparagine (N) (*SN),
   - a combination of phenylalanine (F) and asparagine (N) (FSN), or
   - a combination of valine (V) and asparagine (N) (VSN);
(1-8) when No. 4 A.A. is threonine (T), each of No. 1 A.A. and No. "ii" (-2) A.A. may be an arbitrary amino acid, and the combination of No. 1 A.A., and No. "ii" (-2) A.A. may be, for example:
   - a combination of an arbitrary amino acid and aspartic acid (D) (*TD),
   - a combination of valine (V) and aspartic acid (D) (VTD),
   - a combination of an arbitrary amino acid and asparagine (N) (*TN),
   - a combination of phenylalanine (F) and asparagine (N) (FTN),
   - a combination of isoleucine (I) and asparagine (N) (ITN), or
   - a combination of valine (V) and asparagine (N) (VTN); and
(1-9) when No. 4 A.A. is valine (V), each of No. 1 A.A. and No. "ii" (-2) A.A. may be an arbitrary amino acid, and the combination of No. 1 A.A., and No. "ii" (-2) A.A. may be, for example:
   - a combination of isoleucine (I) and aspartic acid (D) (IVD),
   - a combination of an arbitrary amino acid and glycine (G) (*VG), or
   - a combination of an arbitrary amino acid and threonine (T) (*VT).

More detailed information about the positions and types of amino acids important for base-selective or sequence-specific binding is shown below. The following explanations are made for DNA base-selective or DNA sequence-specific binding as examples, but those skilled in the art can understand that they can also appropriately apply to RNA base and RNA sequence.

The protein is a protein determined on the basis of the following definitions, and having a selective DNA base-binding property:
(2-1) when the three amino acids, No. 1 A.A., No. 4 A.A., and No. "ii" (-2) A.A., are an arbitrary amino acid, glycine, and aspartic acid, respectively, the PPR motif selectively binds to G;
(2-2) when the three amino acids, No. 1 A.A., No. 4 A.A., and No. "ii" (-2) A.A., are glutamic acid, glycine, and aspartic acid, respectively, the PPR motif selectively binds to G;
(2-3) when the three amino acids, No. 1 A.A., No. 4 A.A., and No. "ii" (-2) A.A., are an arbitrary amino acid, glycine, and asparagine, respectively, the PPR motif selectively binds to A;
(2-4) when the three amino acids, No. 1 A.A., No. 4 A.A., and No. "ii" (-2) A.A., are glutamic acid, glycine, and asparagine, respectively, the PPR motif selectively binds to A;
(2-5) when the three amino acids, No. 1 A.A., No. 4 A.A., and No. "ii" (-2) A.A., are an arbitrary amino acid, glycine, and serine, respectively, the PPR motif selectively binds to A, and next binds to C;
(2-6) when the three amino acids, No. 1 A.A., No. 4 A.A., and No. "ii" (-2) A.A., are an arbitrary amino acid, isoleucine, and an arbitrary amino acid, respectively, the PPR motif selectively binds to T and C;
(2-7) when the three amino acids, No. 1 A.A., No. 4 A.A., and No. "ii" (-2) A.A., are an arbitrary amino acid, isoleucine, and asparagine, respectively, the PPR motif selectively binds to T, and next binds to C;
(2-8) when the three amino acids, No. 1 A.A., No. 4 A.A., and No. "ii" (-2) A.A., are an arbitrary amino acid, leucine, and an arbitrary amino acid, respectively, the PPR motif selectively binds to T and C;
(2-9) when the three amino acids, No. 1 A.A., No. 4 A.A., and No. "ii" (-2) A.A., are an arbitrary amino acid, leucine, and aspartic acid, respectively, the PPR motif selectively binds to C;
(2-10) when the three amino acids, No. 1 A.A., No. 4 A.A., and No. "ii" (-2) A.A., are an arbitrary amino acid, leucine, and lysine, respectively, the PPR motif selectively binds to T;
(2-11) when the three amino acids, No. 1 A.A., No. 4 A.A., and No. "ii" (-2) A.A., are an arbitrary amino acid, methionine, and an arbitrary amino acid, respectively, the PPR motif selectively binds to T;
(2-12) when the three amino acids, No. 1 A.A., No. 4 A.A., and No. "ii" (-2) A.A., are an arbitrary amino acid, methionine, and aspartic acid, respectively, the PPR motif selectively binds to T;
(2-13) when the three amino acids, No. 1 A.A., No. 4 A.A., and No. "ii" (-2) A.A., are isoleucine, methionine, and aspartic acid, respectively, the PPR motif selectively binds to T, and next binds to C;
(2-14) when the three amino acids, No. 1 A.A., No. 4 A.A., and No. "ii" (-2) A.A., are an arbitrary amino acid, asparagine, and an arbitrary amino acid, respectively, the PPR motif selectively binds to C and T;
(2-15) when the three amino acids, No. 1 A.A., No. 4 A.A., and No. "ii" (-2) A.A., are an arbitrary amino acid, asparagine, and aspartic acid, respectively, the PPR motif selectively binds to T;
(2-16) when the three amino acids, No. 1 A.A., No. 4 A.A., and No. "ii" (-2) A.A., are phenylalanine, asparagine, and aspartic acid, respectively, the PPR motif selectively binds to T;
(2-17) when the three amino acids, No. 1 A.A., No. 4 A.A., and No. "ii" (-2) A.A., are glycine, asparagine, and aspartic acid, respectively, the PPR motif selectively binds to T;
(2-18) when the three amino acids, No. 1 A.A., No. 4 A.A., and No. "ii" (-2) A.A., are isoleucine, asparagine, and aspartic acid, respectively, the PPR motif selectively binds to T;
(2-19) when the three amino acids, No. 1 A.A., No. 4 A.A., and No. "ii" (-2) A.A., are threonine, asparagine, and aspartic acid, respectively, the PPR motif selectively binds to T;
(2-20) when the three amino acids, No. 1 A.A., No. 4 A.A., and No. "ii" (-2) A.A. are valine, asparagine, and aspartic acid, respectively, the PPR motif selectively binds to T, and next binds to C;
(2-21) when the three amino acids, No. 1 A.A., No. 4 A.A., and No. "ii" (-2) A.A. are tyrosine, asparagine, and aspartic acid, respectively, the PPR motif selectively binds to T, and next binds to C;
(2-22) when the three amino acids, No. 1 A.A., No. 4 A.A., and No. "ii" (-2) A.A., are an arbitrary amino acid, asparagine, and asparagine, respectively, the PPR motif selectively binds to C;
(2-23) when the three amino acids, No. 1 A.A., No. 4 A.A., and No. "ii" (-2) A.A., are isoleucine, asparagine, and asparagine, respectively, the PPR motif selectively binds to C;
(2-24) when the three amino acids, No. 1 A.A., No. 4 A.A., and No. "ii" (-2) A.A., are serine, asparagine, and asparagine, respectively, the PPR motif selectively binds to C;
(2-25) when the three amino acids, No. 1 A.A., No. 4 A.A., and No. "ii" (-2) A.A., are valine, asparagine, and asparagine, respectively, the PPR motif selectively binds to C;
(2-26) when the three amino acids, No. 1 A.A., No. 4 A.A., and No. "ii" (-2) A.A., are an arbitrary amino acid, asparagine, and serine, respectively, the PPR motif selectively binds to C;
(2-27) when the three amino acids, No. 1 A.A., No. 4 A.A., and No. "ii" (-2) A.A., are valine, asparagine, and serine, respectively, the PPR motif selectively binds to C;
(2-28) when the three amino acids, No. 1 A.A., No. 4 A.A., and No. "ii" (-2) A.A., are an arbitrary amino acid, asparagine, and threonine, respectively, the PPR motif selectively binds to C;
(2-29) when the three amino acids, No. 1 A.A., No. 4 A.A., and No. "ii" (-2) A.A., are valine, asparagine, and threonine, respectively, the PPR motif selectively binds to C;
(2-30) when the three amino acids, No. 1 A.A., No. 4 A.A., and No. "ii" (-2) A.A., are an arbitrary amino acid, asparagine, and tryptophan, respectively, the PPR motif selectively binds to C, and next binds to T;
(2-31) when the three amino acids, No. 1 A.A., No. 4 A.A., and No. "ii" (-2) A.A., are isoleucine, asparagine, and tryptophan, respectively, the PPR motif selectively binds to T, and next binds to C;
(2-32) when the three amino acids, No. 1 A.A., No. 4 A.A., and No. "ii" (-2) A.A., are an arbitrary amino acid, proline, and an arbitrary amino acid, respectively, the PPR motif selectively binds to T;
(2-33) when the three amino acids, No. 1 A.A., No. 4 A.A., and No. "ii" (-2) A.A., are an arbitrary amino acid, proline, and aspartic acid, respectively, the PPR motif selectively binds to T;
(2-34) when the three amino acids, No. 1 A.A., No. 4 A.A., and No. "ii" (-2) A.A., are phenylalanine, proline, and aspartic acid, respectively, the PPR motif selectively binds to T;
(2-35) when the three amino acids, No. 1 A.A., No. 4 A.A., and No. "ii" (-2) A.A., are tyrosine, proline, and aspartic acid, respectively, the PPR motif selectively binds to T;
(2-36) when the three amino acids, No. 1 A.A., No. 4 A.A., and No. "ii" (-2) A.A., are an arbitrary amino acid, serine, and an arbitrary amino acid, respectively, the PPR motif selectively binds to A and G;
(2-37) when the three amino acids, No. 1 A.A., No. 4 A.A., and No. "ii" (-2) A.A., are an arbitrary amino acid, serine, and asparagine, respectively, the PPR motif selectively binds to A;
(2-38) when the three amino acids, No. 1 A.A., No. 4 A.A., and No. "ii" (-2) A.A., are phenylalanine, serine, and asparagine, respectively, the PPR motif selectively binds to A;
(2-39) when the three amino acids, No. 1 A.A., No. 4 A.A., and No. "ii" (-2) A.A., are valine, serine, and asparagine, respectively, the PPR motif selectively binds to A;
(2-40) when the three amino acids, No. 1 A.A., No. 4 A.A., and No. "ii" (-2) A.A., are an arbitrary amino acid, threonine, and an arbitrary amino acid, respectively, the PPR motif selectively binds to A and G;
(2-41) when the three amino acids, No. 1 A.A., No. 4 A.A., and No. "ii" (-2) A.A., are an arbitrary amino acid, threonine, and aspartic acid, respectively, the PPR motif selectively binds to G;
(2-42) when the three amino acids, No. 1 A.A., No. 4 A.A., and No. "ii" (-2) A.A., are valine, threonine, and aspartic acid, respectively, the PPR motif selectively binds to G;
(2-43) when the three amino acids, No. 1 A.A., No. 4 A.A., and No. "ii" (-2) A.A., are an arbitrary amino acid, threonine, and asparagine, respectively, the PPR motif selectively binds to A;
(2-44) when the three amino acids, No. 1 A.A., No. 4 A.A., and No. "ii" (-2) A.A., are phenylalanine, threonine, and asparagine, respectively, the PPR motif selectively binds to A;
(2-45) when the three amino acids, No. 1 A.A., No. 4 A.A., and No. "ii" (-2) A.A., are isoleucine, threonine, and asparagine, respectively, the PPR motif selectively binds to A;
(2-46) when the three amino acids, No. 1 A.A., No. 4 A.A., and No. "ii" (-2) A.A., are valine, threonine, and asparagine, respectively, the PPR motif selectively binds to A;
(2-47) when the three amino acids, No. 1 A.A., No. 4 A.A., and No. "ii" (-2) A.A., are an arbitrary amino acid, valine, and an arbitrary amino acid, respectively, the PPR motif binds with A, C, and T, but does not bind to G;
(2-48) when the three amino acids, No. 1 A.A., No. 4 A.A., and No. "ii" (-2) A.A., are isoleucine, valine, and aspartic acid, respectively, the PPR motif selectively binds to C, and next binds to A;
(2-49) when the three amino acids, No. 1 A.A., No. 4 A.A., and No. "ii" (-2) A.A., are an arbitrary amino acid, valine, and glycine, respectively, the PPR motif selectively binds to C; and
(2-50) when the three amino acids, No. 1 A.A., No. 4 A.A., and No. "ii" (-2) A.A., are an arbitrary amino acid, valine, and threonine, respectively, the PPR motif selectively binds to T.

In the designing for base-selective or sequence-specific binding, amino acids other than those of the combination of the amino acids of No. 1 A.A., No. 4 A.A., and No. "ii" (-2) A.A. may be taken into consideration. For example, selection of the amino acids of No. 8 and No. 12 described in Patent document 2 mentioned above may be important for exhibiting a DNA-binding activity. According to the researches of the inventors of the present invention, the No. 8 amino acid of a certain PPR motif and the No. 12 amino acid of the same PPR motif may cooperate in binding with DNA. The No. 8 amino acid may be a basic amino acid, preferably lysine, or an acidic amino acid, preferably aspartic acid, and the No. 12 amino acid may be a basic amino acid, neutral amino acid, or hydrophobic amino acid.

When a target protein is designed, sequence information of the naturally occurring type PPR motifs of such DNA-binding PPR proteins as mentioned as SEQ ID NOS: 1 to 5, or crPPR motif shown as SEQ ID NO: 284 can be referred to for portions other than amino acids of the important positions in the PPR motifs. A target protein may also be designed by using a naturally occurring type sequence or existing sequence as a whole, and replacing only amino acids of the important positions.

Examples of naturally occurring type sequences and existing sequences usable for such design as described above are shown below.
- A protein consisting any one of the amino acid sequences of SEQ ID NOS: 1 to 5.
- A protein consisting any one of the amino acid sequences of SEQ ID NOS: 291 to 308.
- A protein having any one amino acid sequence selected from the group consisting of the amino acid sequence of the 230 to 541 positions of SEQ ID NO: 1, the amino acid sequence of the 234 to 621 positions of SEQ ID NO: 2, the amino acid sequence of the 106 to 632 positions of SEQ ID NO: 3, the amino acid sequence of the 106 to 632 positions of SEQ ID NO: 4, and the amino acid sequence of the 256 to 624 positions of SEQ ID NO: 5.
- Any one PPR motif selected from the group consisting of 9 PPR motifs of the protein consisting of the amino acid sequence of SEQ ID NO: 1, 11 PPR motifs of the protein consisting of the amino acid sequence of SEQ ID NO: 2, 15 PPR motifs of the protein consisting of the amino acid sequence of SEQ ID NO: 3, 10 PPR motifs of the protein consisting of the amino acid sequence of SEQ ID NO: 4, and 11 PPR motifs of the protein consisting of the amino acid sequence of SEQ ID NO: 5.
- A protein having any one amino acid sequence selected from the group consisting of the amino acid sequence of the 167 to 482 positions of SEQ ID NO: 291, the amino acid sequence of the 156 to 575 positions of SEQ ID NO: 292, the amino acid sequence of the 243 to 554 positions of SEQ ID NO: 293, the amino acid sequence of the 140 to 489 positions of SEQ ID NO: 294, the amino acid sequence of the 78 to 419 positions of SEQ ID NO: 295, the amino acid sequence of the 122 to 545 positions of SEQ ID NO: 296, the amino acid sequence of the 256 to 624 positions of SEQ ID NO: 297, the amino acid sequence of the 48 to 362 positions of SEQ ID NO: 298, the amino acid sequence of the 198 to 689 positions of SEQ ID NO: 299, the amino acid sequence of the 89 to 578 positions of SEQ ID NO: 300, the amino acid sequence of the 470 to 911 positions of SEQ ID NO: 301, the amino acid sequence of the 156 to 575 positions of SEQ ID NO: 302, the amino acid sequence of the 108 to 775 positions of SEQ ID NO: 303, the amino acid sequence of the 226 to 1137 positions of SEQ ID NO: 304, the amino acid sequence of the 145 to 496 positions of SEQ ID NO: 305, the amino acid sequence of the 104 to 538 positions of SEQ ID NO: 306, the amino acid sequence of the 151 to 502 positions of SEQ ID NO: 307, and the amino acid sequence of the 274 to 660 positions of SEQ ID NO: 308.
- Any one PPR motif selected from the group consisting of 9 PPR motifs of the protein consisting of the amino acid sequence SEQ ID NO: 291, 6 PPR motifs of the protein consisting of the amino acid sequence of SEQ ID NO: 292, 9 PPR motifs of the protein consisting of the amino acid sequence of SEQ ID NO: 293, 10 PPR motifs of the protein consisting of the amino acid sequence of SEQ ID NO: 294, 9 PPR motifs of the protein consisting of the amino acid sequence of SEQ ID NO: 295, 12 PPR motifs of the protein consisting of the amino acid sequence of SEQ ID NO: 296,10 PPR motifs of the protein consisting of the amino acid sequence of SEQ ID NO: 297,9 PPR motifs of the protein consisting of the amino acid sequence of SEQ ID NO: 298, 14 PPR motifs of the protein consisting of the amino acid sequence of SEQ ID NO: 299, 14 PPR motifs of the protein consisting of the amino acid sequence of SEQ ID NO: 300, 10 PPR motifs of the protein consisting of the amino acid sequence of SEQ ID NO: 301, 12 PPR motifs of the protein consisting of the amino acid sequence of SEQ ID NO: 302, 19 PPR motifs of the protein consisting of the amino acid sequence of SEQ ID NO: 303, 25 PPR motifs of the protein consisting of the amino acid sequence of SEQ ID NO: 304, 10 PPR motifs of the protein consisting of the amino acid sequence of SEQ ID NO: 305, 9 PPR motifs of the protein consisting of the amino acid sequence of SEQ ID NO: 306, 10 PPR motifs of the protein consisting of the amino acid sequence of SEQ ID NO: 307, and 11 PPR motifs of the protein consisting of the amino acid sequence of SEQ ID NO: 308.

The present invention provides a novel dPPR protein obtained by the method for designing a dPPR protein, method for imparting a property of binding with a DNA base to a PPR protein, or method of enhancing a property of binding with DNA of a PPR protein, which uses the information explained above. Examples of such a dPPR protein include those containing at least one PPR motif having any one of the amino acid sequences of SEQ ID NOS: 285 to 290. In a preferred embodiment, the protein may contain 2 or more, preferably 2 to 30, more preferably 5 to 25, further preferably 9 to 15, of PPR motifs having any one of the amino acid sequences of SEQ ID NOS: 285 to 290.

The present invention also provides the followings as a novel PPR motif or PPR protein.
- A PPR motif having any one of the amino acid sequences of SEQ ID NOS: 7 to 214.
- A PPR protein having any one amino acid sequence selected from the group consisting of the amino acid sequence of the 167 to 482 positions of SEQ ID NO: 291, the amino acid sequence of the 156 to 575 positions of SEQ ID NO: 292, the amino acid sequence of the 243 to 554 positions of SEQ ID NO: 293, the amino acid sequence of the 140 to 489 positions of SEQ ID NO: 294, the amino acid sequence of the 78 to 419 positions of SEQ ID NO: 295, the amino acid sequence of the 122 to 545 positions of SEQ ID NO: 296, the amino acid sequence of the 256 to 624 positions of SEQ ID NO: 297, the amino acid sequence of the 48 to 362 positions of SEQ ID NO: 298, the amino acid sequence of the 198 to 689 positions of SEQ ID NO: 299, the amino acid sequence of the 89 to 578 positions of SEQ ID NO: 300, the amino acid sequence of the 470 to 911 positions of SEQ ID NO: 301, the amino acid sequence of the 156 to 575 positions of SEQ ID NO: 302, the amino acid sequence of the 108 to 775 positions of SEQ ID NO: 303, the amino acid sequence of the 226 to 1137 positions of SEQ ID NO: 304, the amino acid sequence of the 145 to 496 positions of SEQ ID NO: 305, the amino acid sequence of the 104 to 538 positions of SEQ ID NO: 306, the amino acid sequence of the 151 to 502 positions of SEQ ID NO: 307, and the amino acid sequence of the 274 to 660 positions of SEQ ID NO: 308.
- A protein consisting of any one of the amino acid sequences of SEQ ID NOS: 335 to 361, and a motif contained in it.
- A protein consisting of any one of the amino acid sequences of SEQ ID NOS: 424 to 427, and a motif contained in it.

The existing p63 (SEQ ID NO: 1), GUN1 (SEQ ID NO: 2), pTac2 (SEQ ID NO: 3), DG1 (SEQ ID NO: 4), and GRP23 (SEQ ID NO: 5) themselves do not fall within the scope of the present invention. The proteins consisting of the amino acid sequence of SEQ ID NOS: 291 to 308 themselves (At1g10910, At1g26460, At3g15590, At3g59040, At5g10690, At5g24830, At5g67570, At3g42630, At5g42310, At1g12700, At1g30610, At2g35130, At2g41720, At3g18110, At3g53170, At4g21170, At5g48730, and At5g50280) also do not fall within the scope of the present invention.

### [Use of dPPR protein]

The dPPR protein provided by the present invention can be made into a complex by binding a functional region. The functional region generally refers to a part having such a function as a specific biological function exerted in a living body or cell, for example, enzymatic function, catalytic function, inhibitory function, promotion function, etc, or a function as a marker. Such a region consists of, for example, a protein, peptide, nucleic acid, physiologically active substance, or drug.

According to the present invention, by binding a functional region to the PPR protein, the target DNA sequence-binding function exerted by the PPR protein, and the function exerted by the functional region can be exhibited in combination. For example, if a protein having a DNA-cleaving function or a functional domain thereof (for example, nuclease domain of restriction enzyme FokI, SEQ ID NO: 6) is used as the functional region, the complex can function as an artificial DNA-cleaving enzyme.

In order to produce such a complex, methods generally available in this technical field can be used, and there are known a method of synthesizing such a complex as one protein molecule, a method of separately synthesizing two or more members of proteins, and then combining them to form a complex, and so forth.

In the case of the method of synthesizing a complex as one protein molecule, for example, a protein complex can be designed so as to comprise a PPR protein and a cleaving enzyme bound to the C-terminus or N-terminus of the PPR protein via an amino acid linker, an expression vector structure for expressing the protein complex can be constructed, and the target complex can be expressed from the structure. As such a preparation method, the method described in Japanese Patent Unexamined Publication (KOKAI) No. 2013-94148, and so forth can be used.

For binding the PPR protein and the functional region protein, any binding means known in this technical field may be used, including binding via an amino acid linker, binding utilizing specific affinity such as binding between avidin and biotin, binding utilizing another chemical linker, and so forth.

The functional region usable in the present invention refers to a region that can impart any one of various functions such as those for cleavage, transcription, replication, restoration, synthesis, or modification of DNA, and so forth. By choosing the sequence of the PPR motif to define a DNA base sequence as a target, which is the characteristic of the present invention, substantially any DNA sequence may be used as the target, and with such a target, genome editing utilizing the function of the functional region such as those for cleavage, transcription, replication, restoration, synthesis, or modification of DNA can be realized.

For example, when the function of the functional region is a DNA cleavage function, there is provided a complex comprising a PPR protein part prepared according to the present invention and a DNA cleavage region bound together. Such a complex can function as an artificial DNA-cleaving enzyme that recognizes a base sequence of DNA as a target by the PPR protein part, and then cleaves DNA by the DNA cleavage region.

An example of the functional region having a cleavage function usable for the present invention is a deoxyribonuclease (DNase), which functions as an endodeoxyribonuclease. As such a DNase, for example, endodeoxyribonucleases such as DNase A (e.g., bovine pancreatic ribonuclease A, PDB 2AAS), DNase H and DNase I, restriction enzymes derived from various bacteria (for example, FokI) and nuclease domains thereof can be used. Such a complex comprising a PPR protein and a functional region does not exist in the nature, and is novel.

When the function of the functional region is a transcription control function, there is provided a complex comprising a PPR protein part prepared according to the present invention and a DNA transcription control region bound together. Such a complex can function as an artificial transcription control factor, which recognizes a base sequence of DNA as a target by the PPR protein part, and then controls transcription of the target DNA.

The functional region having a transcription control function usable for the present invention may be a domain that activates transcription, or may be a domain that suppresses transcription. Examples of the transcription control domain include VP16, VP64, TA2, STAT-6, and p65. Such a complex comprising a PPR protein and a transcription control domain does not exist in the nature, and is novel.

Further, the complex obtainable according to the present invention may deliver a functional region in a living body or cell in a DNA sequence-specific manner, and allow it to function. It thereby makes it possible to perform modification or disruption in a DNA sequence-specific manner in a living body or cell, like protein complexes utilizing a zinc finger protein (Non-patent documents 1 and 2 mentioned above) or TAL effecter (Non-patent document 3 and Patent document 1 mentioned above), and thus it becomes possible to impart a novel function, i.e., function for cleavage of DNA and genome editing utilizing that function. Specifically, with a PPR protein comprising two or more PPR motifs that can bind with a specific base linked together, a specific DNA sequence can be recognized. Then, genome editing of the recognized DNA region can be realized by the functional region bound to the PPR protein using the function of the functional region.

Furthermore, by binding a drug to the PPR protein that binds to a DNA sequence in a DNA sequence-specific manner, the drug may be delivered to the neighborhood of the DNA sequence as the target. Therefore, the present invention provides a method for DNA sequence-specific delivery of a functional substance.

According to the present invention, the PPR protein shows high DNA-binding ability, and recognizes a specific base on DNA, and as a result, it can be expected to be used to introduce base polymorphism, or treat a disease or condition resulting from a base polymorphism, and in addition, it is considered that the combination of such a PPR protein with such another functional region as mentioned above contribute to modification or improvement of functions for realizing cleavage of DNA for genome editing.

Moreover, an exogenous DNA-cleaving enzyme can be fused to the C-terminus of the PPR protein. Alternatively, by improving binding DNA base selectivity of the PPR motif on the N-terminus side, a DNA sequence-specific DNA-cleaving enzyme can also be constituted. Moreover, such a complex to which a marker part such as GFP is bound can also be used for visualization of a desired DNA in vivo.

### Examples

### [Example 1: Collection of novel dPPR molecules]

As known dPPR proteins, there were only P63, GUN1, pTAC2, GRP23, and DG1 described in the prior patent (Patent document 4 mentioned above), and it was difficult to obtain information for generalizing and improving artificial nucleic acid-binding modules based on PPR technique. Therefore, it was then decided to perform screening for PPR proteins having a DNA-binding ability, and thereby increase variety of dPPR proteins. Although the genes of the dPPR molecules accidentally discovered so far contain introns, almost all the rPPR genes do not contain any intron. The total genome sequences of *Arabidopsis thaliana* as a model plant were analyzed on the basis of the fact mentioned above, and as a result, there were found 42 kinds of PPR genes containing two or more introns. In this example, the DNA-binding abilities of these 42 kinds of potential dPPR molecules were analyzed to attempt identification of novel dPPR molecules.

### (Experimental methods)

### 1. Construction of DPPR expression vector

From the Institute of Physical and Chemical Research (RIKEN), which holds cDNAs of *Arabidopsis thaliana,* genes of 10 kinds of the potential dPPRs were obtained. Gene synthesis of GENEWIZ was used for the remaining 32 kinds. The obtained regions corresponding to the PPR motifs of the 42 kinds of the obtained genes were introduced into an expression vector pEU-E01 for wheat cell-free protein synthesis (CellFree Science). Further, a gene encoding thioredoxin and a gene encoding a His-tag were inserted into each gene of potential dPPR molecule on the 5' end side and the 3' end side, respectively.

### 2. Synthesis of dPPR proteins

mRNAs of the potential dPPR molecules were obtained by using SP6 RNA Polymerase (Promega). The reaction conditions were determined according to the protocol described in the product information. The potential dPPR proteins were obtained by using WEPRO7240H (CellFree Science). The reaction conditions were determined according to the protocol described in the product information.

### 3. DNA-protein pull-down assay

To each potential dPPR protein, bovine thymus double-stranded DNA cellulose beads (Sigma-Aldrich, 2 mg), and a buffer (20 mM HEPES-KOH, pH 7.9, 60 mM NaCl, 12.5 mM MgCl₂, 0.3% Triton X-100) were added, and the reaction was allowed at 4°C for 1 hour. The beads were washed 3 times with a washing solution (10 mM Tris-HCl, pH 8.0, 300 mM NaCl, 0.3% Triton X-100), then a 5 x SDS-PAGE sample buffer was added to them, and they were heat-treated at 95°C for 5 minutes to elute the potential dPPR protein.

### 4. Western blotting

The protein was separated by using 10 to 20% acrylamide gel (ATTO), and transferred to a nitrocellulose membrane. As the transfer buffer, EzFastBlot (ATTO) was used. Blocking was performed with a 0.3% skim milk solution, and the reaction with 0.5 µg/ml of HRP-labeled anti-His-tag antibody (MBL) was allowed at room temperature for 1 hour. For the detection, Immobilon Chemiluminescent HRP Substrate (Millipore) was used. For the detection of the chemiluminescence, VersaDoc (BioRad) was used.

### (Results and discussion)

The DNA-binding powers of the potential dPPR proteins were compared with that of known rPPR OTP80 (Hammani et al., A Study of New Arabidopsis Chloroplast RNA Editing Mutants Reveals General Features of Editing Factors and Their Target Sites, The Plant Cell, Vol. 21:3686-3699, 2009) used as a negative control. The comparison with OTP80 was performed by using t-test performed for numerical values standardized by dividing luminescence intensity of each pulled down protein with that obtained with input 1% at 5% significance level (p < 0.06). As a result, significant differences were observed for 18 kinds of the potential dPPRs. These results revealed that these 18 kinds of PPR proteins are dPPR proteins. The sequences of the PPR motifs of the 18 kinds of dPPR proteins are shown in the following tables (mentioned in the order of 1, 2, 3 ...).

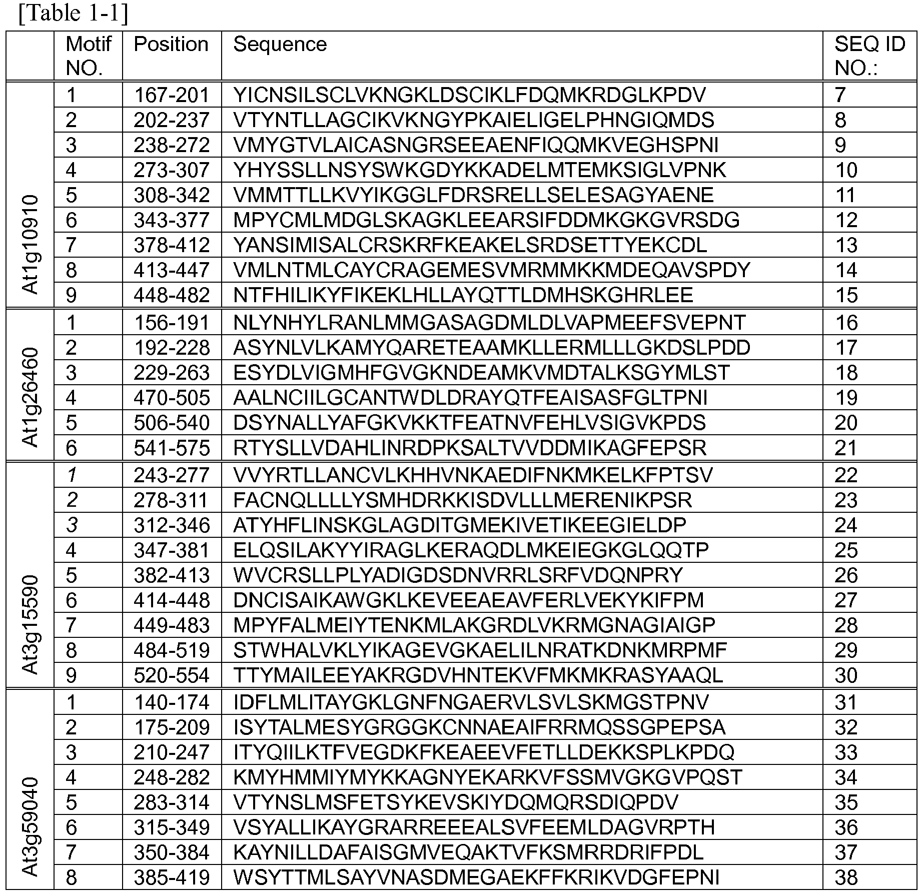

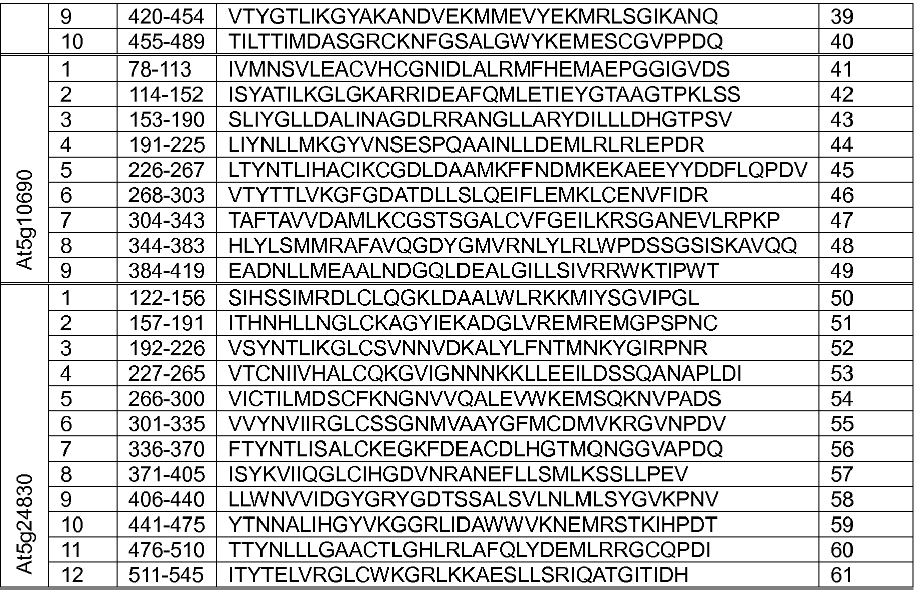

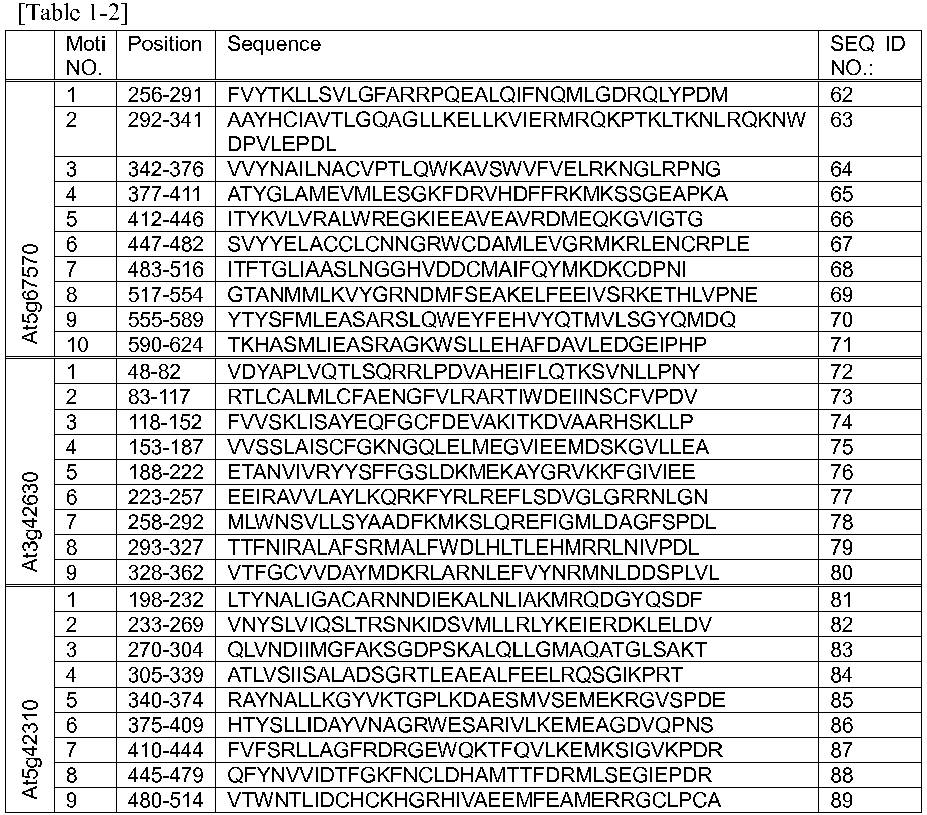

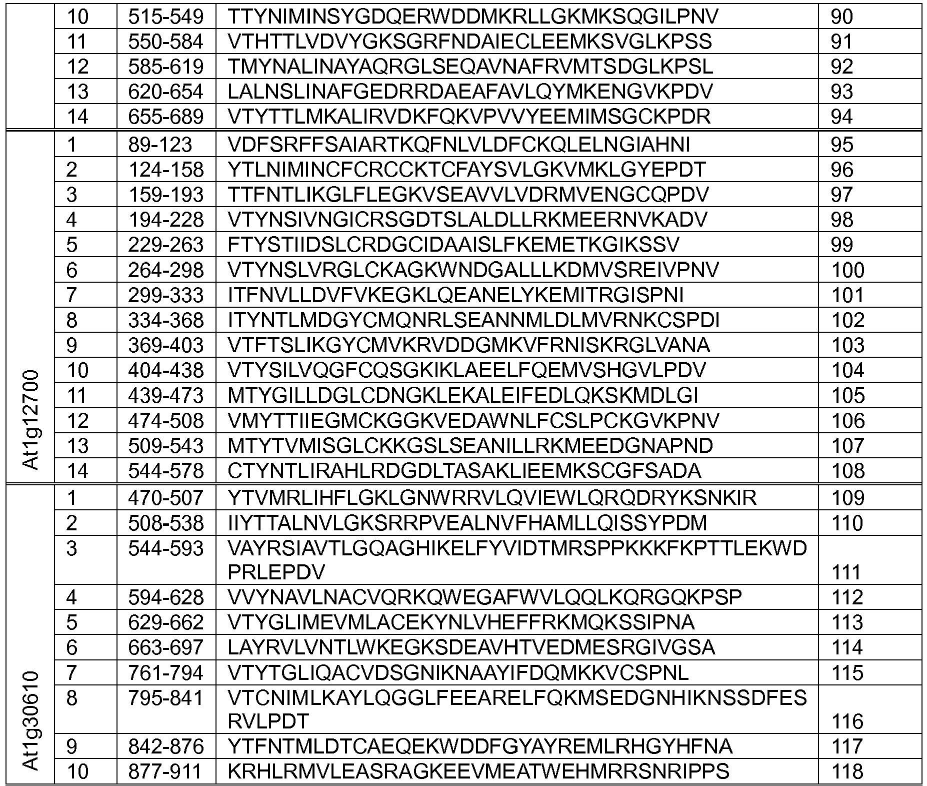

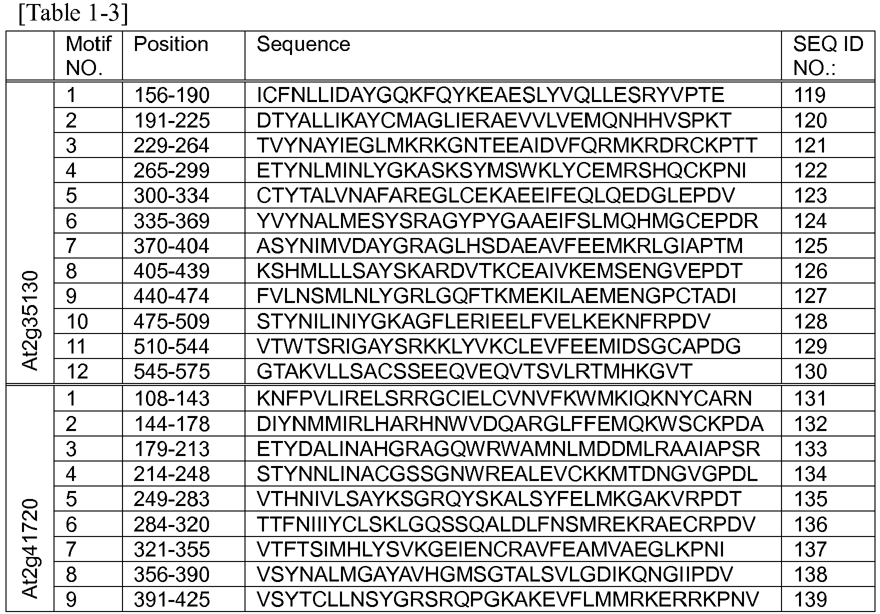

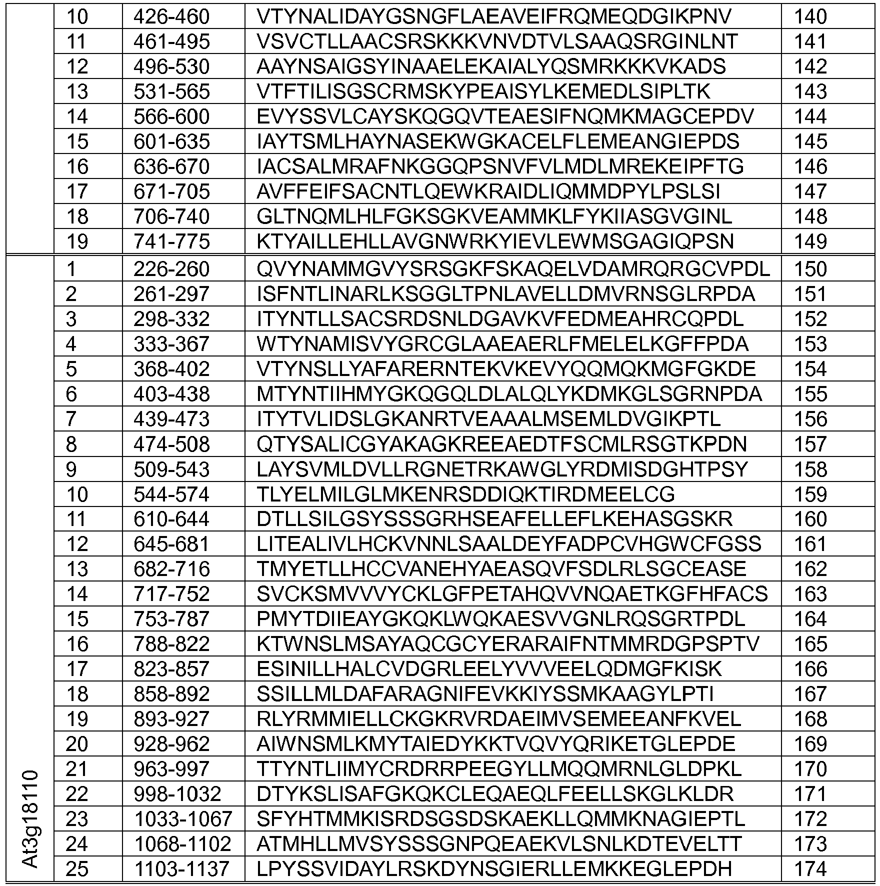

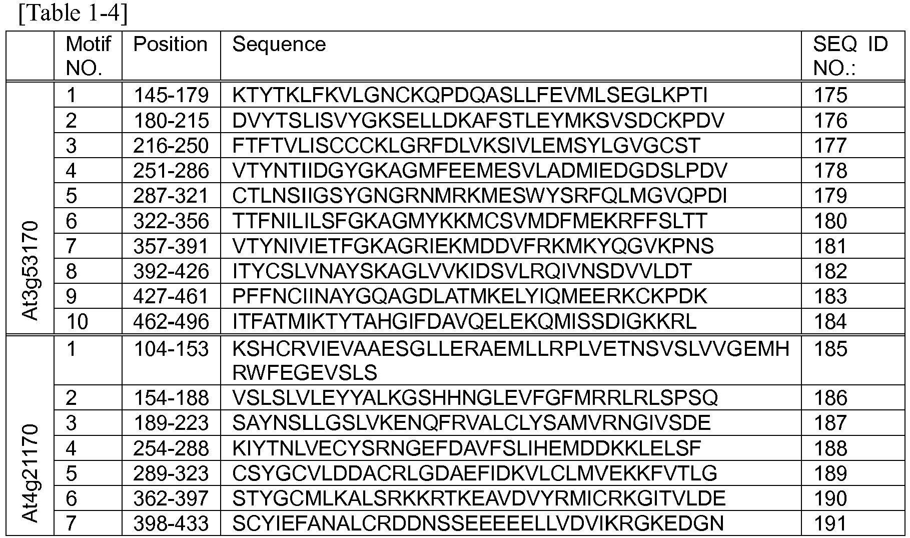

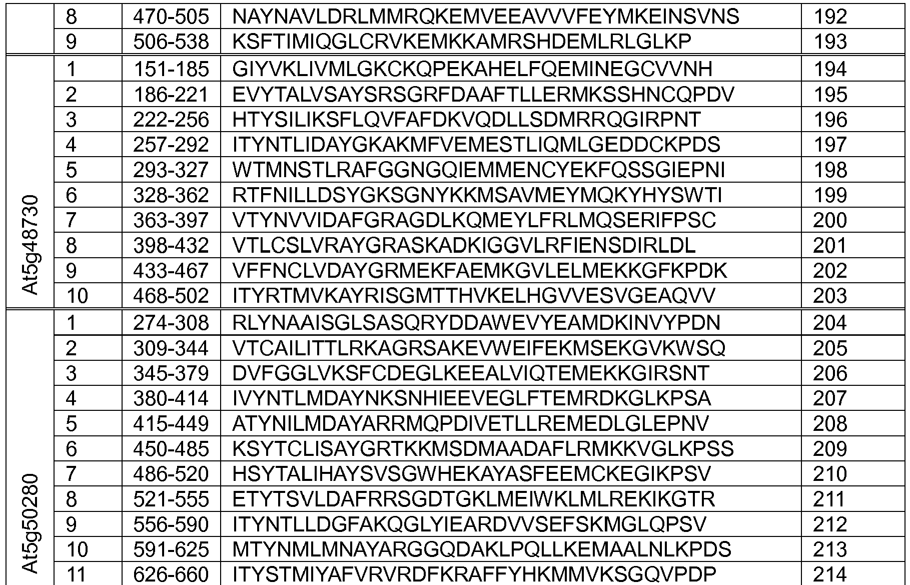

### [Example 2: Analysis of dPPR motif-specific amino acid sequences]

On the basis of the amino acid sequence information of the modules of the dPPR proteins identified in Example 1, dPPR motif-specific amino acid sequences were analyzed.

First, 9 kinds of the dPPR proteins were selected from the 18 kinds of dPPR proteins identified in Example 1 in order to approximately match the number of them with the number of motifs of rPPR proteins used in the F test. Specifically, on the basis of the numerical values obtained from the comparison of the DNA-binding power with that of OTP80 performed by the t-test, the dPPR proteins were classified into 3 groups of those showing the values of 0.05 to 0.01, 0.01 to 0.001, and < 0.001, and 3 kinds of proteins were randomly selected from each group to select 9 kinds of the proteins. The occurrence frequencies of amino acids in PPR motifs of the 9 kinds of dPPR molecules and the known 5 rPPR molecules mentioned in the following tables (mentioned in the order of 1, 2, 3 ...) were compared at every position to attempt identification of positions of amino acids characterizing the dPPR proteins. For the comparison, the F test was used at a significance level of 5% (p < 0.06).

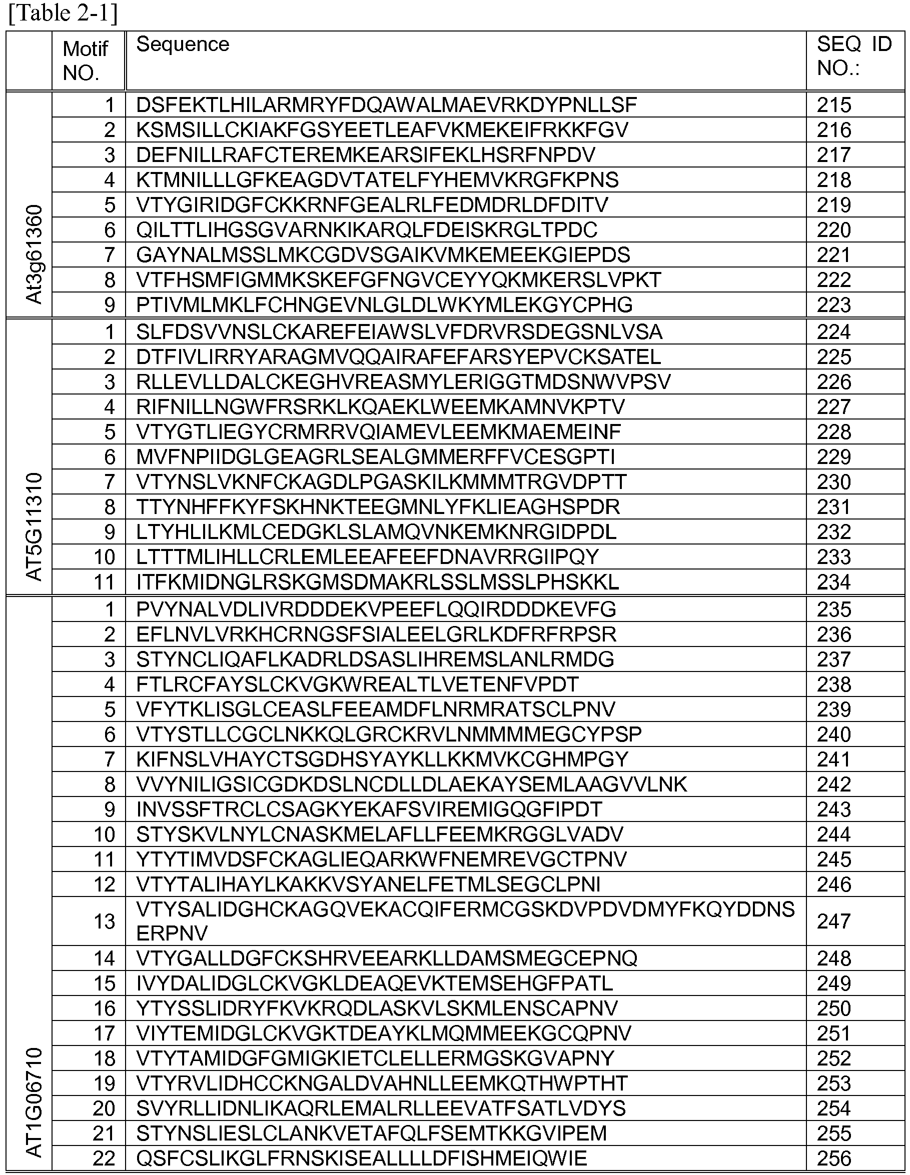

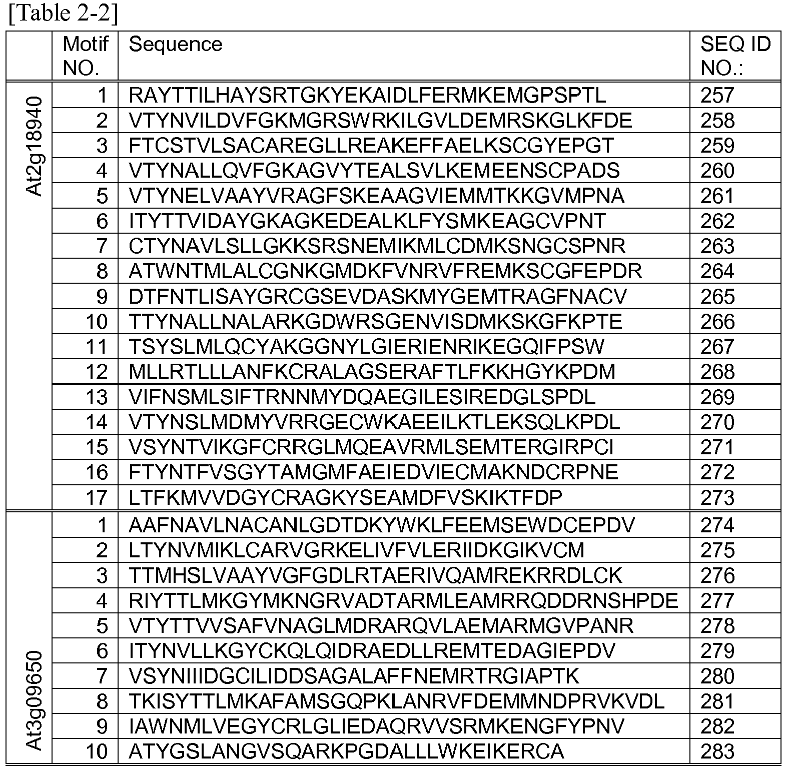

From the results of the F test (Fig. 1), there were observed differences in occurrence frequencies for the amino acids of the residues of No. 7 amino acid (A.A.), No. 9 A.A., No. 10 A.A., No. 18 A.A., No. 20 A.A., No. 29 A.A., No. 31 A.A., No. 32 A.A., and No. ii A.A. No. ii A.A. was excluded, since it is a part involved in recognition of a DNA base (Patent document 4 mentioned above).

Then, the occurrence frequencies of the amino acids at these positions were calculated, and amino acids that showed the largest positive differences between dPPR and rPPR were confirmed. As a result, it was found that occurrence frequencies of I as No. 7 A.A., A as No. 9 A.A., Y as No. 10 A.A., K as No. 18 A.A., E as No. 20 A.A., E as No. 29 A.A., I as No. 31 A.A., and K as No. 32 A.A. increased in the dPPR molecules. On the basis of these results, the aforementioned amino acids were determined as dPPR motif-specific amino acid sequences.

The contents (%) of the dPPR specific amino acids in the novel dPPR proteins (9 kinds of the proteins used for the data set) and known rPPRs are shown in the following table.

**[Table 3]**

| Novel dPPR proteins, known rPPR | | | | | Known dPPR | | | | |
|---|---|---|---|---|---|---|---|---|---|
| | Average (dPPR) | Average (rPPR) | Median | | **P63** | **GUN1** | **pTAC2** | **DG1** | **GRP23** |
| AA7I | 0.45 | 0.35 | 0.40 | | 0.33 | 0.64 | 0.47 | 0.10 | 0.36 |
| AA9A | 0.49 | 0.23 | 0.36 | | 0.11 | 0.45 | 0.47 | 0.40 | 0.27 |
| AA10Y | 0.50 | 0.25 | 0.37 | | 0.56 | 0.36 | 0.33 | 0.10 | 0.18 |
| AA18K | 0.29 | 0.09 | 0.19 | | 0.44 | 0.09 | 0.13 | 0.00 | 0.09 |
| AA20E | 0.25 | 0.16 | 0.21 | | 0.56 | 0.00 | 0.13 | 0.20 | 0.09 |
| AA29E | 0.12 | 0.06 | 0.09 | | 0.22 | 0.18 | 0.13 | 0.00 | 0.00 |
| AA31I | 0.23 | 0.10 | 0.16 | | 0.00 | 0.45 | 0.40 | 0.00 | 0.00 |
| AA32K | 0.22 | 0.09 | 0.15 | | 0.00 | 0.09 | 0.00 | 0.10 | 0.09 |

### [Example 3-1: Establishment of method for constructing artificial nucleic acid-binding module based on dPPR motif-specific amino acid sequences 1]

In this example, the DNA-binding abilities of modified type rPPRs introduced with the dPPR specific amino acid sequences were investigated in order to verify whether the DNA-binding abilities of PPR proteins are increased by the dPPR-specific amino acid sequences. As the base rPPR, the consensus PPR (cPPR) reported in Non-patent document 15 (Coquille et al., 2014, An artificial PPR scaffold for programmable RNA recognition) was used. cPPR is known as an RNA-binding protein (therefore, it may be referred to as crPPR), and it had not been known whether it binds with DNA. For the modification of crPPR, gene synthesis by Genewiz was used. The DNA-binding abilities of the modified type crPPRs were analyzed by the method used in Example 1. The target sequence of crPPR is AAAAAAAA.

Since there was a tendency that AA9A and AA10Y changed within the same motif, they were inserted in combination in this experiment. Since there was also a tendency that AA20E was introduced into a motif preceding that of AA18K, they were inserted in combination. When the contents were calculated from the data obtained from all the dPPRs (18 kinds also including the dPPR protein molecules other than those used for the data set), the content of AA10Y in a motif also having AA9A was 43.75%, and the content of AA18K in a motif next to a motif having AA20E was 41.3%. The sequences of cPPRs and the modified type PPR motifs prepared in this example are shown in the following table (mentioned in the order of 1, 2, 3 ...).

**[Table 4]**

| | | |
|---|---|---|
| crPPR | VTYTTLISGLGKAGRLEEALELFEEMKEKGIVPNV | SEQ ID NO.:284 |
| Modified crPPR-1 | VTYTTLISAYGKAGRLEEALELFEEMKEKGIVPNV | SEQ ID NO.:285 |
| Modified crPPR-2 | VTYTTLISGLGKAGRLEKAEELFEEMKEKGIVPNV | SEQ ID NO.:286 |
| Modified crPPR-3 | VTYTTLISGLGKAGRLEEALELFEEMKEKGIKPNV | SEQ ID NO.:287 |
| Modified crPPR-4 | VTYTTLISAYGKAGRLEKAEELFEEMKEKGIVPNV | SEQ ID NO.:288 |
| Modified crPPR-5 | VTYTTLISAYGKAGRLEEALELFEEMKEKGIKPNV | SEQ ID NO.:289 |
| Modified crPPR-6 | VTYTTLISAYGKAGRLEKAEELFEEMKEKGIKPNV | SEQ ID NO.:290 |

### (Results and discussion)

Comparison of the DNA-binding power was performed with values obtained by standardization by dividing luminescence intensity of each pulled-down protein with that obtained with input 3%. The results are shown in Fig. 2.

There were obtained results that the DNA-binding powers of crPPR and all the modified type crPPRs in which each dPPR motif-specific amino acid sequence was inserted were higher than those of GUN1, pTAC2, p63, and DG1, which are naturally occurring dPPR molecules. These results indicate that the dPPR motif-specific amino acid sequences found in this research and development relate to the DNA-binding ability of PPR protein.

On the basis of the above test results obtained in this example, it was discovered that a DNA-binding ability can be imparted to a PPR protein by inserting a dPPR motif-specific amino acid sequence.

### [Example 3-2: Establishment of method for constructing artificial nucleic acid-binding module based on dPPR motif-specific amino acid sequences 2]

The aforementioned cPPR (Non-patent document 15) has an RNA-binding property, but it has A.A. 71 and A.A. 311. Therefore, there was used a modified version thereof in which these amino acids are replaced with leucine (L) and phenylalanine (F), respectively, with reference to the occurrence frequencies of amino acids in rPPR. In this specification, this modified version is referred to as consensus RNA-binding PPR (7L/31F) (crPPR (7L/31F)). Since there was a tendency that AA9A and AA10Y changed within the same motif, one having them in combination was also examined (the ratio of AA10Y in a motif also having AA9A was 43.75%, when it was calculated from the data obtained from the 18 kinds of dPPRs including the dPPRs other than those used for the data set).

### (Experimental method)

### 1. Construction of modified type crPPR expression vector

For the genes of crPPR (7L/31F) and the modified versions of the same introduced with a modified type rPPR, the gene synthesis by GENEWIZ was used. Each of the obtained genes was introduced into the expression vector pEU-E01 for wheat cell-free protein synthesis (CellFree Science). A gene encoding thioredoxin and a gene encoding a His-tag were further inserted into the gene on the 5' and 3' end sides thereof, respectively.

### 2. Synthesis of dPPR proteins

mRNAs of the dPPR molecules were obtained by using SP6 RNA Polymerase (Promega). The reaction conditions were determined according to the protocol described in the product information. Proteins of PPRs were obtained by using WEPRO7240H (CellFree Science). The reaction conditions were determined according to the protocol described in the product information.

### 3. DNA-protein pull-down assay

To each of the modified type rPPRs and crPPR (7L/31F), bovine thymus double-stranded DNA cellulose beads (Sigma-Aldrich, 2 mg), and a buffer (20 mM HEPES-KOH, pH 7.9, 60 mM NaCl, 12.5 mM MgCl₂, 0.3% Triton X-100) were added, and the reaction was allowed at 4°C for 1 hour. The beads were washed 3 times with a washing solution (10 mM Tris-HCl, pH 8.0, 300 mM NaCl, 0.3% Triton X-100), a 5 x SDS-PAGE sample buffer was added to them, and they were heat-treated at 95°C for 5 minutes to perform elution.

### 4. Western blotting

Each protein was separated by using 5 to 20% acrylamide gel (Wako Pure Chemical Industries), and transferred to a nitrocellulose membrane. As the transfer buffer, AquaBlot High Efficiency Transfer Buffer (Wako Pure Chemical Industries) was used. Blocking was performed with a 5% skim milk solution, and then the reaction was allowed with 1 µg/ml of HRP-labeled anti-His-tag antibody (Wako Pure Chemical Industries) at room temperature for 1 hour. For the detection, Immunostar Zeta (Wako Pure Chemical Industries) was used. For the detection of the chemiluminescence, Amersham Imager 600 (GE Healthcare) and LAS-4000 (Fuji Photo Film) were used.

### (Results and discussion)

The DNA-binding power was represented with a value obtained by standardization in which luminescence intensity of each pulled-down protein was divided with luminescence intensity at input 3%. Comparison of the DNA-binding powers of the modified type rPPRs and CrPPR (7L/31F) was performed by t-test at 5% significance level (p < 0.06). As a result, significant differences were observed for the modified type rPPRs introduced with A.A. 9A, A.A. 18K, A.A. 311, A.A. 32K, and A.A. 9A/10Y (Fig. 3). These results revealed that a DNA-binding ability can be imparted to PPR by introducing these amino acid sequences.

The sequences of crPPR (7L/31F) and the modified type PPR motifs prepared in this example are shown in the following tables.

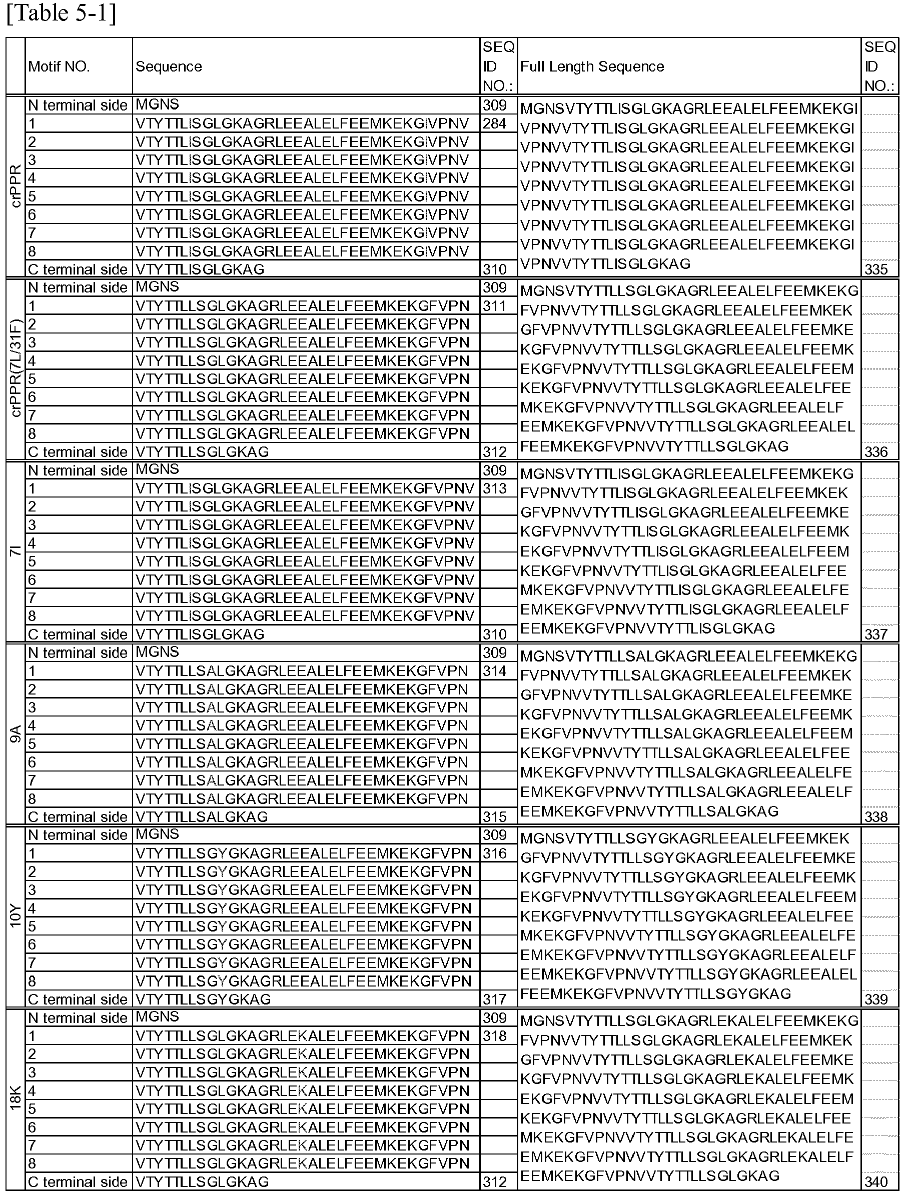

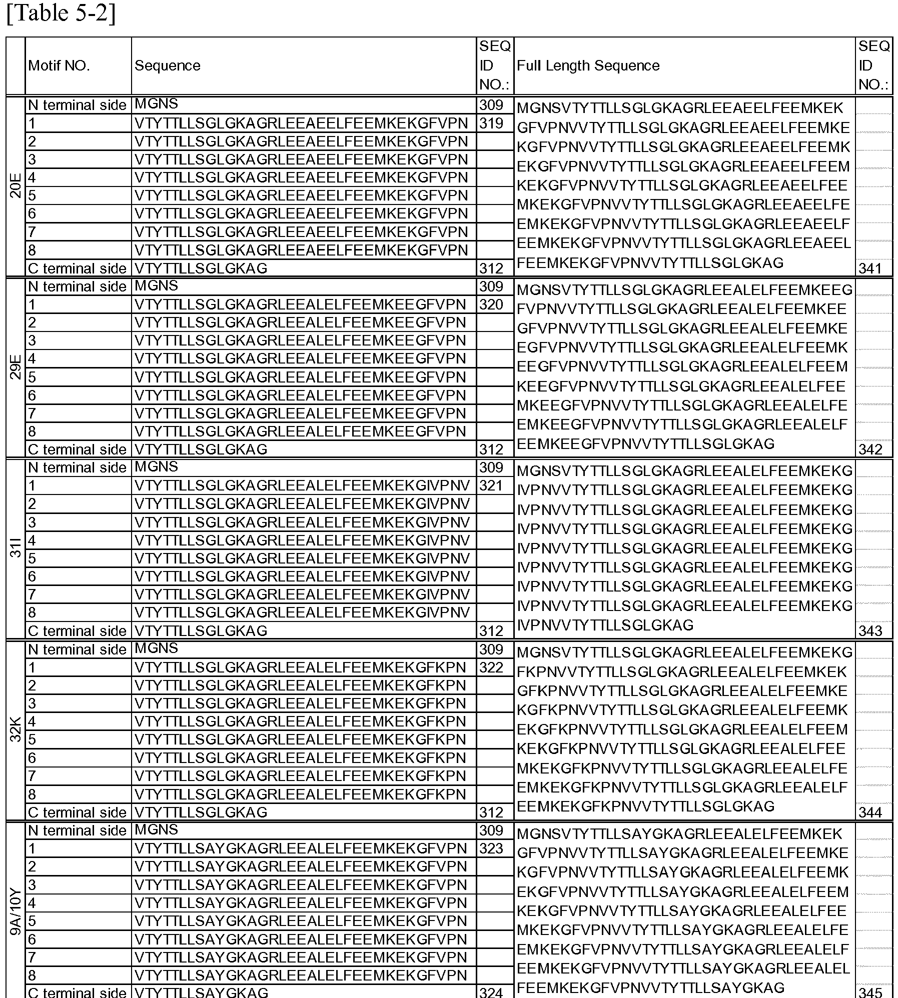

### [Example 4: Evaluation of amino acids having similar characteristics]

It was examined whether the effect would also be obtained even when amino acids having similar characteristics are used for A.A. 18K, A.A. 311, A.A. 32K, and A.A.9A/10Y. In this experiment, there were used histidine (H) and arginine (R), which are basic amino acids like K, for No. 18 A.A. and No. 32 A.A., valine (V) and leucine (L), which have a branched chain like I, for No. 31 A.A., and phenylalanine (F) and tryptophan (W), which have an aromatic group like Y, for No. 10 A.A. The DNA-binding ability was evaluated by analysis performed in the same manner as that used in Example 3.

### (Results and discussion)

The DNA-binding powers of the modified type rPPRs and crPPR (7L/31F) were compared by t-test at a significance level of 5% (p < 0.06). As a result, significant difference was observed for all the modified type rPPRs (Fig. 4). These results revealed that even when amino acids having similar characteristics are used, a DNA-binding ability can be imparted.

The sequences of the modified type rPPR motifs prepared in this example are shown in the following table.

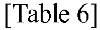

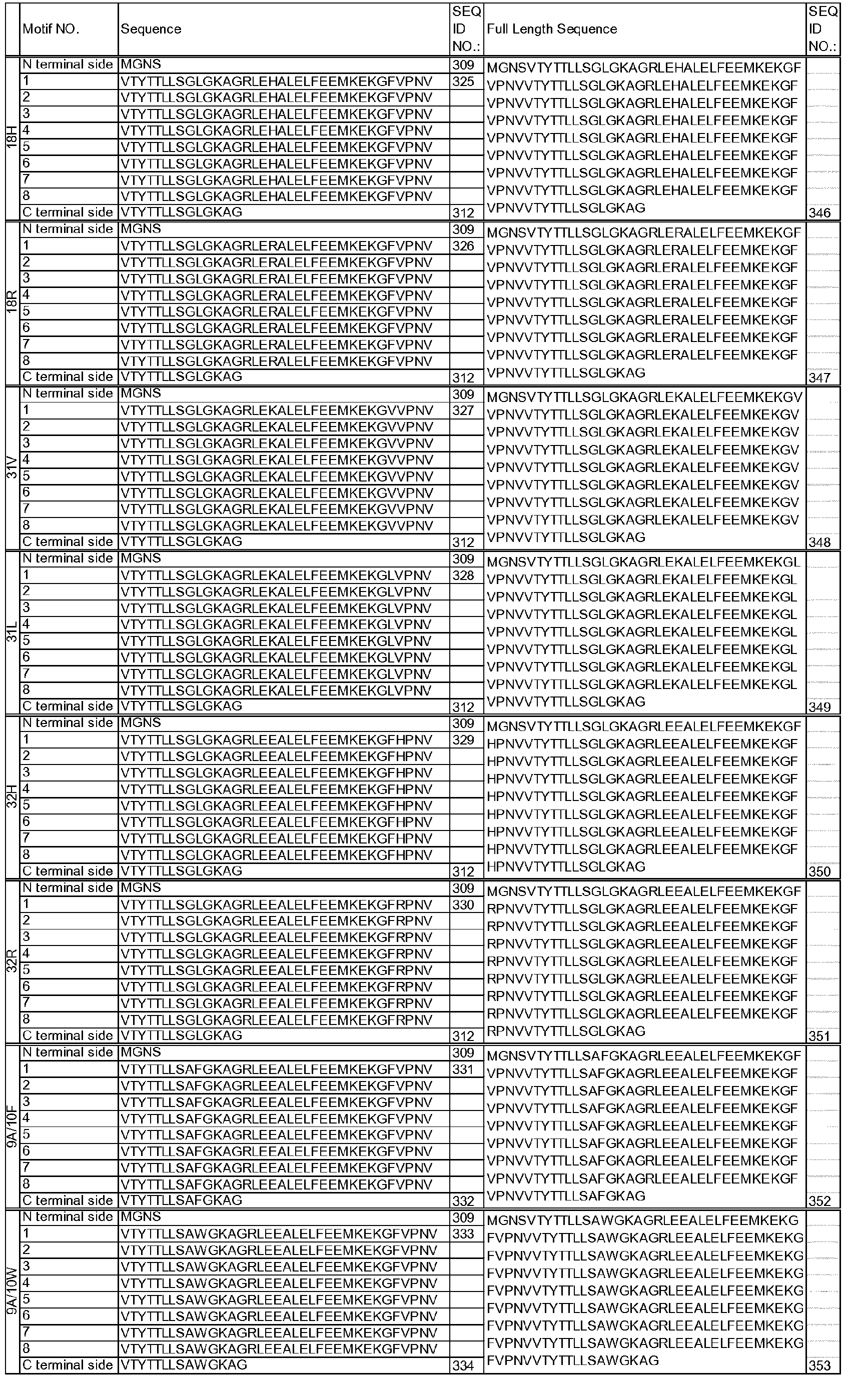

### [Example 5: Evaluation of contents of A.A. 9A, A.A. 18K, A.A. 31I, A.A. 32K, and A.A. 9A/10Y required for DNA-binding ability]

Contents (ratios) of A.A. 9A, A.A. 18K, A.A. 311, A.A. 32K, and A.A. 9A/10Y required for imparting a DNA-binding ability were examined. The content (ratio) referred to here is an amount (ratio) of motifs having the aforementioned amino acid sequences in PPR molecule. In this experiment, DNA-binding abilities of modified type rPPRs in which 2 motifs (25% of the whole) or 4 motifs (50% of the whole) of crPPR (7L/31F) on the N-terminus side were motifs having these amino acid sequences were analyzed. The DNA-binding ability was analyzed in the same manner as that used in Example 3.

### (Results and discussion)

The DNA-binding powers of the modified type rPPRs and crPPR (7L/31F) were compared by t-test at a significance level of 5% (p < 0.06). As a result, significant difference was observed for all the modified type rPPRs (Fig. 5). These results revealed that a DNA-binding ability can be imparted with a content of 2 or more (or 25% or more of the whole) of PPR motifs introduced with A.A. 9A, A.A. 18K, A.A. 311, A.A. 32K, and A.A. 9A/10Y.

The sequences of the modified type rPPR motifs prepared in this example are shown in the following table.

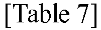

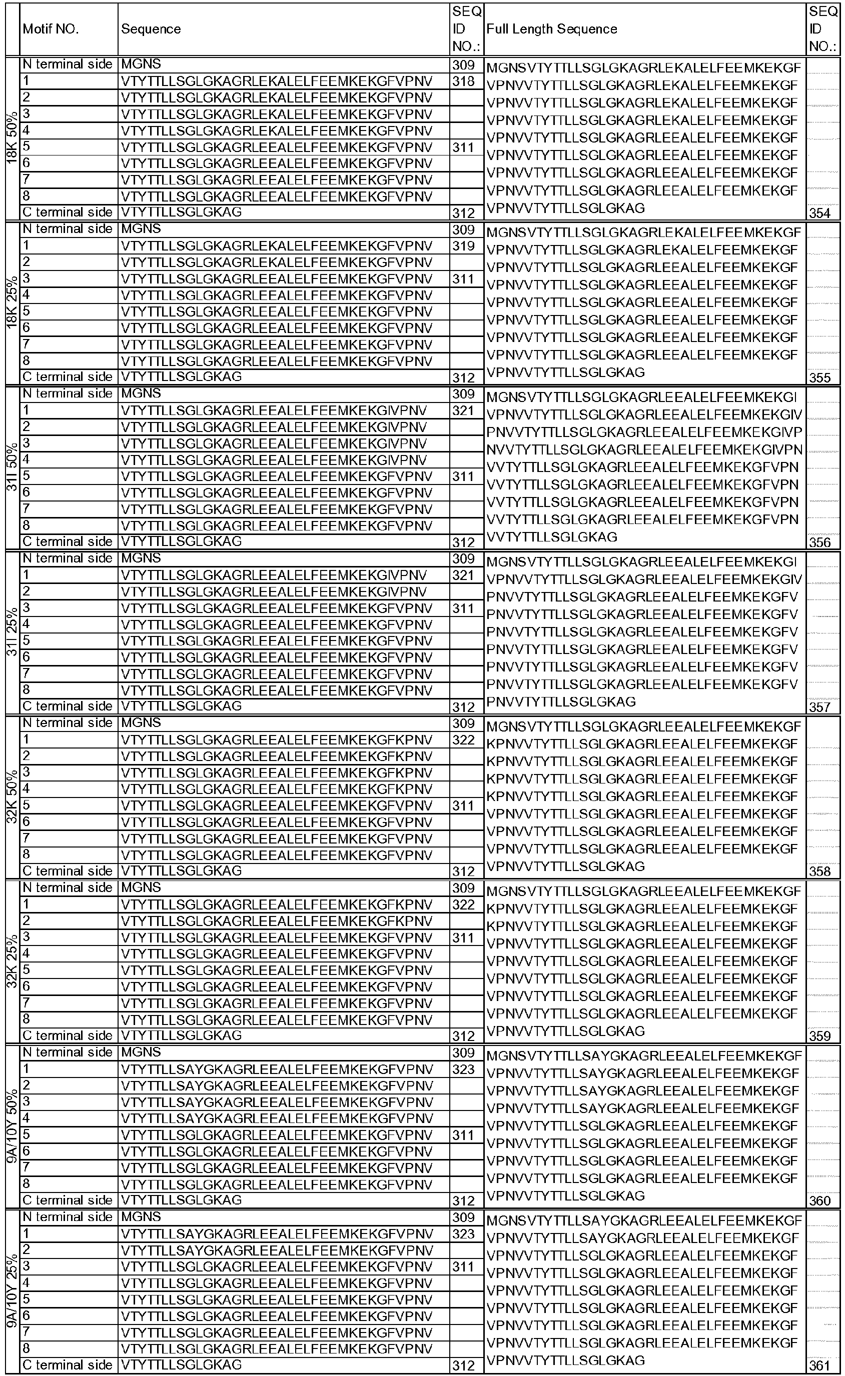

### [Example 6: Evaluation of generality of amino acid sequences capable of imparting DNA-binding ability]

All the above examinations were performed by using crPPR (7L/31F). Therefore, it was examined whether a DNA-binding ability can also be imparted to other PPRs by introducing A.A 9A, A.A. 18K, A.A. 311, A.A. 32K, and A.A. 9A/10Y. In this experiment, it was examined whether DNA-binding abilities of modified naturally occurring type dPPRs, P63 and GUN1, in which A.A. 9A/10Y/18K/31I, and A.A. 31I/32K were introduced into all the motifs thereof were increased. The DNA-binding ability was analyzed in the same manner as that used in Example 3. In this example, the positions of A.A. 311 and A.A. 32K in a motif were determined on the basis of the next motif. Specifically, the position of A.A. 311 was determined so as to be a position locating upstream from No. 1 amino acid of the next PPR motif by 5 amino acids, and the position of A.A.32K was determined so as to be a position locating upstream from No. 1 amino acid of the next PPR motif by 4 amino acids. In the case of the motif at the C-terminus (no next PPR motif), the amino acids of the 5th and 4th positions from the last amino acid (C-terminus side) among those constituting the motif were determined to be A.A. 311 and A.A. 32K, respectively.

### (Results and discussion)

The DNA-binding powers of modified type and naturally occurring type dPPRs were compared by t-test at a significance level of 5% (p < 0.06). As a result, DNA-binding powers of P63 and GUN1 introduced with any of the amino acid sequences were increased (Fig. 6). These results revealed that the impartation of DNA-binding ability by introduction of A.A. 9A, A.A. 18K, A.A. 311, A.A. 32K, and A.A. 9A/10Y is also effective for PPR proteins other than crPPR (7L/31F).

The sequences of the modified type rPPR motifs prepared by this example are shown in the following tables.

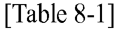

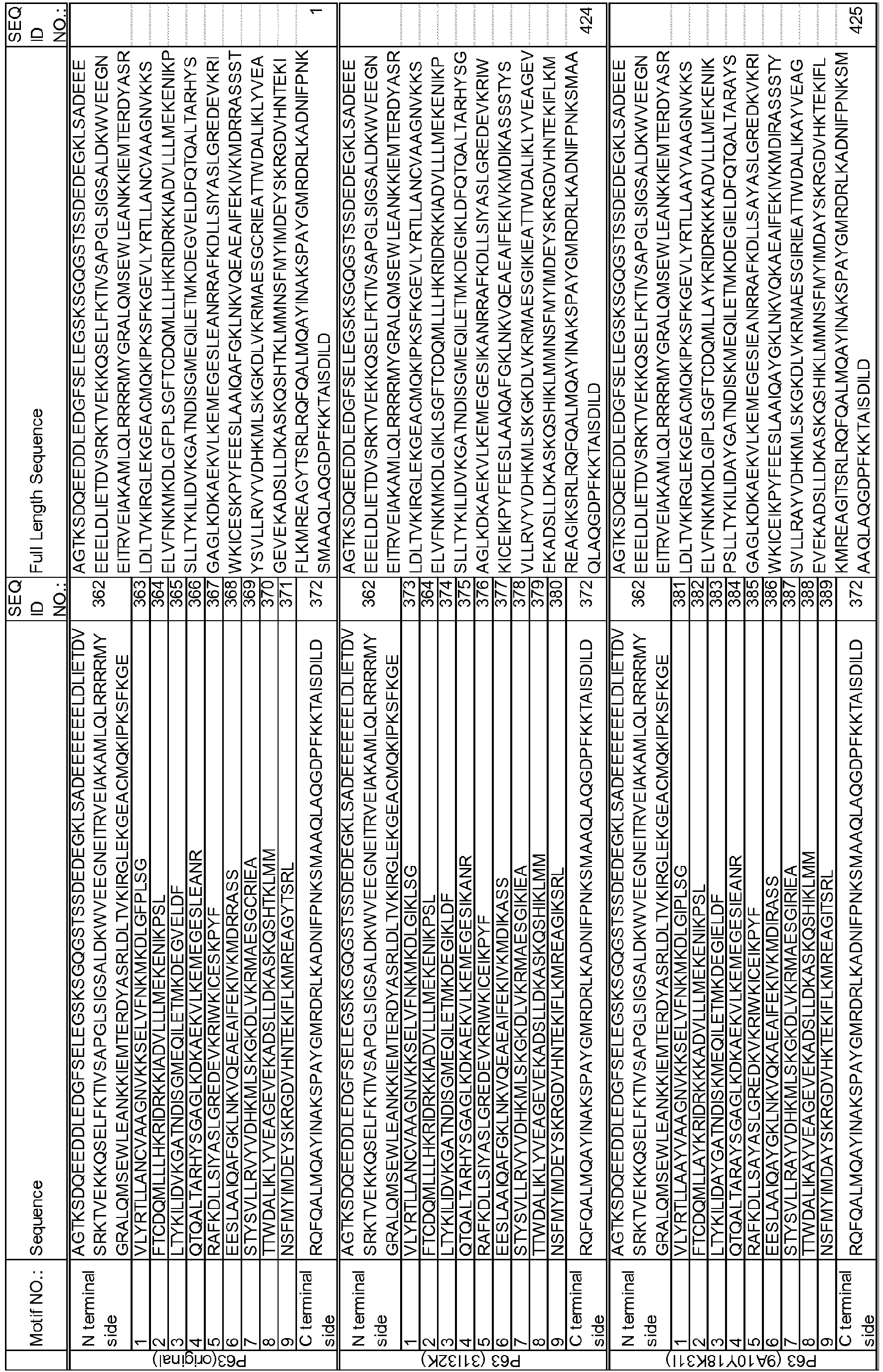

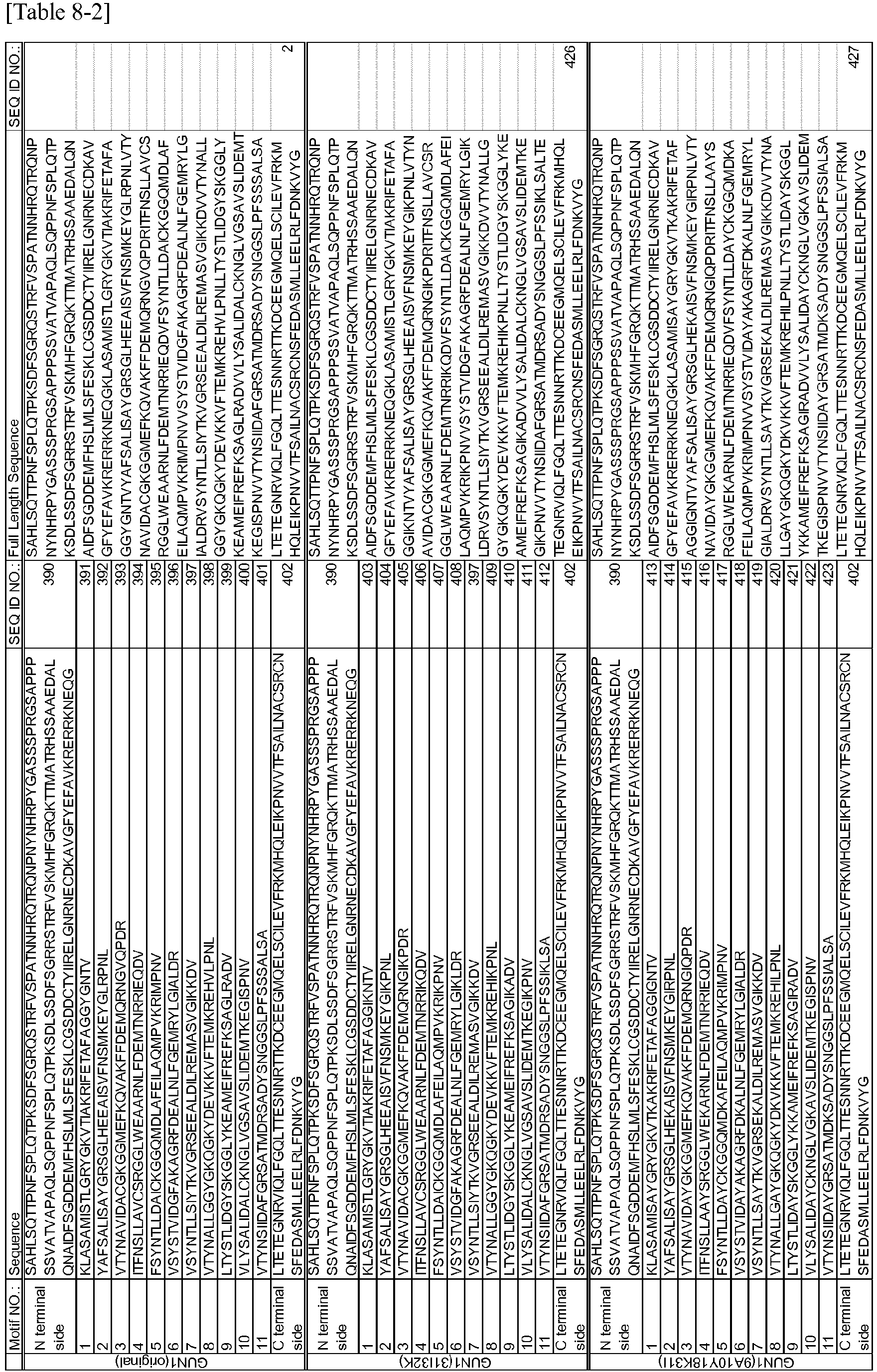

### [Reference cited in the section of Examples]

Non-patent-document 15: Coquille et al., 2014, An artificial PPR scaffold for programmable RNA recognition http://www.nature.com/ncomms/2014/141217/ncomms6729/abs/ncomms6729.html

### Sequence Listing Free Text

SEQ ID NO: 1, p63 protein
SEQ ID NO: 2, GUN1 protein
SEQ ID NO: 3, pTac2 protein
SEQ ID NO: 4, DG1 protein
SEQ ID NO: 5, GRP23 protein
SEQ ID NO: 6, FokI nuclease domain
SEQ ID NOS: 7 to 214, dPPRs
SEQ ID NOS: 215 to 283, known rPPRs
SEQ ID NO: 284, crPPR
SEQ ID NO: 285, modified type crPPR-1
SEQ ID NO: 286, modified type crPPR-2
SEQ ID NO: 287, modified type crPPR-3
SEQ ID NO: 288, modified type crPPR-4
SEQ ID NO: 289, modified type crPPR-5
SEQ ID NO: 290, modified type crPPR-6
SEQ ID NOS: 291 to 308, At1g10910, At1g26460, At3g15590, At3g59040, At5g10690, At5g24830, At5g67570, At3g42630, At5g42310, At1g12700, At1g30610, At2g35130, At2g41720, At3g18110, At3g53170, At4g21170, At5g48730, At5g50280
SEQ ID NO: 309, crPPR N terminal side
SEQ ID NO: 310, crPPR C terminal side
SEQ ID NOS: 311 to 334, modified type rPPR motifs or C terminal sides
SEQ ID NOS: 335 to 361, modified-type rPPR proteins (full length)
SEQ ID NOS: 362 to 423, N/C terminal sides, or motifs of original/modified type of p63 or GUN1
SEQ ID NOS: 424 to 427, modified-type p63 or GUN1 proteins (full length)

## Claims

1. A protein that can bind in a DNA base-selective manner or a DNA base sequence-specific manner, which contains one or more PPR motifs having a structure of the following formula 1:
[Chemical Formula 1]
(Helix A)-X-(Helix B)-L (Formula 1)
(wherein, in the formula 1:
Helix A is a part that can form an α-helix structure;
X does not exist, or is a part consisting of 1 to 9 amino acids;
Helix B is a part that can form an α-helix structure; and
L is a part consisting of 2 to 7 amino acids),
wherein,
under the following definitions:
the first amino acid of Helix A is referred to as No. 1 amino acid (No. 1 A.A.), the fourth amino acid as No. 4 amino acid (No. 4 A.A.), and
- when a next PPR motif (Mₙ₊₁) contiguously exists on the C-terminus side of the PPR motif (Mₙ) (when there is no amino acid insertion between the PPR motifs), the -2nd amino acid counted from the end (C-terminus side) of the amino acids constituting the PPR motif (Mₙ);
- when a non-PPR motif consisting of 1 to 20 amino acids exists between the PPR motif (Mₙ) and the next PPR motif (Mₙ₊₁) on the C-terminus side, the amino acid locating upstream of the first amino acid of the next PPR motif (Mₙ₊₁) by 2 positions, i.e., the -2nd amino acid; or
- when any next PPR motif (Mₙ₊₁) does not exist on the C-terminus side of the PPR motif (Mₙ), or 21 or more amino acids constituting a non-PPR motif exist between the PPR motif (Mₙ) and the next PPR motif (Mₙ₊₁) on the C-terminus side, the 2nd amino acid counted from the end (C-terminus side) of the amino acids constituting the PPR motif (Mₙ)
is referred to as No. "ii" (-2) amino acid (No. "ii" (-2) A.A.),
one PPR motif (Mₙ) contained in the protein is a PPR motif having a specific combination of amino acids corresponding to a target DNA base or target DNA base sequence as the three amino acids of No. 1 A.A., No. 4 A.A., and No. "ii" (-2) A.A, and the protein satisfies at least one selected from the group consisting of the following conditions (b) to (h):
(b) No. 9 A.A. of the PPR motif (Mₙ) is alanine (A);
(c) No. 10 A.A. of the PPR motif (Mₙ) is tyrosine (Y), phenylalanine (F), or tryptophan (W);
(d) No. 18 A.A. of the PPR motif (Mₙ) is lysine (K), arginine (R), or histidine (H);
(e) No. 20 A.A. of the PPR motif (Mₙ) is glutamic acid (E), or aspartic acid (D);
(f) No. 29 A.A. of the PPR motif (Mₙ) is glutamic acid (E), or aspartic acid (D);
(g) No. 31 A.A. of the PPR motif (Mₙ) is isoleucine (I), leucine (L), or valine (V); and
(h) No. 32 A.A. of the PPR motif (Mₙ) is lysine (K), arginine (R), or histidine (H) (provided that a protein consisting of any one of the amino acid sequences of SEQ ID NOS: 1 to 5 and SEQ ID NOS: 291 to 308 is excluded).

2. The protein according to claim 1, wherein the combination of the three amino acids of No. 1 A.A., No. 4 A.A., and No. "ii" (-2) A.A. is a combination corresponding to a target DNA base or target DNA base sequence, and the combination of amino acids is determined according to any one of the following definitions:
(1-1) when No. 4 A.A. is glycine (G), No. 1 A.A. may be an arbitrary amino acid, and No. "ii" (-2) A.A. is aspartic acid (D), asparagine (N), or serine (S);
(1-2) when No. 4 A.A. is isoleucine (I), each of No. 1 A.A. and No. "ii" (-2) A.A. may be an arbitrary amino acid;
(1-3) when No. 4 A.A. is leucine (L), each of No. 1 A.A. and No. "ii" (-2) A.A. may be an arbitrary amino acid;
(1-4) when No. 4 A.A. is methionine (M), each of No. 1 A.A. and No. "ii" (-2) A.A. may be an arbitrary amino acid;
(1-5) when No. 4 A.A. is asparagine (N), each of No. 1 A.A. and No. "ii" (-2) A.A. may be an arbitrary amino acid;
(1-6) when No. 4 A.A. is proline (P), each of No. 1 A.A. and No. "ii" (-2) A.A. may be an arbitrary amino acid;
(1-7) when No. 4 A.A. is serine (S), each of No. 1 A.A. and No. "ii" (-2) A.A. may be an arbitrary amino acid;
(1-8) when No. 4 A.A. is threonine (T), each of No. 1 A.A. and No. "ii" (-2) A.A. may be an arbitrary amino acid; and
(1-9) when No. 4 A.A. is valine (V), each of No. 1 A.A. and No. "ii" (-2) A.A. may be an arbitrary amino acid.

3. The protein according to claim 1, wherein the combination of the three amino acids of No. 1 A.A., No. 4 A.A., and No. "ii" (-2) A.A. is a combination corresponding to a target DNA base or target DNA base sequence, and the combination of amino acids is determined according to any one of the following definitions:
(2-1) when the three amino acids, No. 1 A.A., No. 4 A.A., and No. "ii" (-2) A.A. are an arbitrary amino acid, glycine, and aspartic acid, respectively, the PPR motif selectively binds to G;
(2-2) when the three amino acids, No. 1 A.A., No. 4 A.A., and No. "ii" (-2) A.A., are glutamic acid, glycine, and aspartic acid, respectively, the PPR motif selectively binds to G;
(2-3) when the three amino acids, No. 1 A.A., No. 4 A.A., and No. "ii" (-2) A.A., are an arbitrary amino acid, glycine, and asparagine, respectively, the PPR motif selectively binds to A;
(2-4) when the three amino acids, No. 1 A.A., No. 4 A.A., and No. "ii" (-2) A.A., are glutamic acid, glycine, and asparagine, respectively, the PPR motif selectively binds to A;
(2-5) when the three amino acids, No. 1 A.A., No. 4 A.A., and No. "ii" (-2) A.A., are an arbitrary amino acid, glycine, and serine, respectively, the PPR motif selectively binds to A, and next binds to C;
(2-6) when the three amino acids, No. 1 A.A., No. 4 A.A., and No. "ii" (-2) A.A., are an arbitrary amino acid, isoleucine, and an arbitrary amino acid, respectively, the PPR motif selectively binds to T and C;
(2-7) when the three amino acids, No. 1 A.A., No. 4 A.A., and No. "ii" (-2) A.A., are an arbitrary amino acid, isoleucine, and asparagine, respectively, the PPR motif selectively binds to T, and next binds to C;
(2-8) when the three amino acids, No. 1 A.A., No. 4 A.A., and No. "ii" (-2) A.A., are an arbitrary amino acid, leucine, and an arbitrary amino acid, respectively, the PPR motif selectively binds to T and C;
(2-9) when the three amino acids, No. 1 A.A., No. 4 A.A., and No. "ii" (-2) A.A., are an arbitrary amino acid, leucine, and aspartic acid, respectively, the PPR motif selectively binds to C;
(2-10) when the three amino acids, No. 1 A.A., No. 4 A.A., and No. "ii" (-2) A.A., are an arbitrary amino acid, leucine, and lysine, respectively, the PPR motif selectively binds to T;
(2-11) when the three amino acids, No. 1 A.A., No. 4 A.A., and No. "ii" (-2) A.A., are an arbitrary amino acid, methionine, and an arbitrary amino acid, respectively, the PPR motif selectively binds to T;
(2-12) when the three amino acids, No. 1 A.A., No. 4 A.A., and No. "ii" (-2) A.A., are an arbitrary amino acid, methionine, and aspartic acid, respectively, the PPR motif selectively binds to T;
(2-13) when the three amino acids, No. 1 A.A., No. 4 A.A., and No. "ii" (-2) A.A., are isoleucine, methionine, and aspartic acid, respectively, the PPR motif selectively binds to T, and next binds to C;
(2-14) when the three amino acids, No. 1 A.A., No. 4 A.A., and No. "ii" (-2) A.A., are an arbitrary amino acid, asparagine, and an arbitrary amino acid, respectively, the PPR motif selectively binds to C and T;
(2-15) when the three amino acids, No. 1 A.A., No. 4 A.A., and No. "ii" (-2) A.A., are an arbitrary amino acid, asparagine, and aspartic acid, respectively, the PPR motif selectively binds to T;
(2-16) when the three amino acids, No. 1 A.A., No. 4 A.A., and No. "ii" (-2) A.A., are phenylalanine, asparagine, and aspartic acid, respectively, the PPR motif selectively binds to T;
(2-17) when the three amino acids, No. 1 A.A., No. 4 A.A., and No. "ii" (-2) A.A., are glycine, asparagine, and aspartic acid, respectively, the PPR motif selectively binds to T;
(2-18) when the three amino acids, No. 1 A.A., No. 4 A.A., and No. "ii" (-2) A.A., are isoleucine, asparagine, and aspartic acid, respectively, the PPR motif selectively binds to T;
(2-19) when the three amino acids, No. 1 A.A., No. 4 A.A., and No. "ii" (-2) A.A., are threonine, asparagine, and aspartic acid, respectively, the PPR motif selectively binds to T;
(2-20) when the three amino acids, No. 1 A.A., No. 4 A.A., and No. "ii" (-2) A.A. are valine, asparagine, and aspartic acid, respectively, the PPR motif selectively binds to T, and next binds to C;
(2-21) when the three amino acids, No. 1 A.A., No. 4 A.A., and No. "ii" (-2) A.A. are tyrosine, asparagine, and aspartic acid, respectively, the PPR motif selectively binds to T, and next binds to C;
(2-22) when the three amino acids, No. 1 A.A., No. 4 A.A., and No. "ii" (-2) A.A., are an arbitrary amino acid, asparagine, and asparagine, respectively, the PPR motif selectively binds to C;
(2-23) when the three amino acids, No. 1 A.A., No. 4 A.A., and No. "ii" (-2) A.A., are isoleucine, asparagine, and asparagine, respectively, the PPR motif selectively binds to C;
(2-24) when the three amino acids, No. 1 A.A., No. 4 A.A., and No. "ii" (-2) A.A., are serine, asparagine, and asparagine, respectively, the PPR motif selectively binds to C;
(2-25) when the three amino acids, No. 1 A.A., No. 4 A.A., and No. "ii" (-2) A.A., are valine, asparagine, and asparagine, respectively, the PPR motif selectively binds to C;
(2-26) when the three amino acids, No. 1 A.A., No. 4 A.A., and No. "ii" (-2) A.A., are an arbitrary amino acid, asparagine, and serine, respectively, the PPR motif selectively binds to C;
(2-27) when the three amino acids, No. 1 A.A., No. 4 A.A., and No. "ii" (-2) A.A., are valine, asparagine, and serine, respectively, the PPR motif selectively binds to C;
(2-28) when the three amino acids, No. 1 A.A., No. 4 A.A., and No. "ii" (-2) A.A., are an arbitrary amino acid, asparagine, and threonine, respectively, the PPR motif selectively binds to C;
(2-29) when the three amino acids, No. 1 A.A., No. 4 A.A., and No. "ii" (-2) A.A., are valine, asparagine, and threonine, respectively, the PPR motif selectively binds to C;
(2-30) when the three amino acids, No. 1 A.A., No. 4 A.A., and No. "ii" (-2) A.A., are an arbitrary amino acid, asparagine, and tryptophan, respectively, the PPR motif selectively binds to C, and next binds to T;
(2-31) when the three amino acids, No. 1 A.A., No. 4 A.A., and No. "ii" (-2) A.A., are isoleucine, asparagine, and tryptophan, respectively, the PPR motif selectively binds to T, and next binds to C;
(2-32) when the three amino acids, No. 1 A.A., No. 4 A.A., and No. "ii" (-2) A.A., are an arbitrary amino acid, proline, and an arbitrary amino acid, respectively, the PPR motif selectively binds to T;
(2-33) when the three amino acids, No. 1 A.A., No. 4 A.A., and No. "ii" (-2) A.A., are an arbitrary amino acid, proline, and aspartic acid, respectively, the PPR motif selectively binds to T;
(2-34) when the three amino acids, No. 1 A.A., No. 4 A.A., and No. "ii" (-2) A.A., are phenylalanine, proline, and aspartic acid, respectively, the PPR motif selectively binds to T;
(2-35) when the three amino acids, No. 1 A.A., No. 4 A.A., and No. "ii" (-2) A.A., are tyrosine, proline, and aspartic acid, respectively, the PPR motif selectively binds to T;
(2-36) when the three amino acids, No. 1 A.A., No. 4 A.A., and No. "ii" (-2) A.A., are an arbitrary amino acid, serine, and an arbitrary amino acid, respectively, the PPR motif selectively binds to A and G;
(2-37) when the three amino acids, No. 1 A.A., No. 4 A.A., and No. "ii" (-2) A.A., are an arbitrary amino acid, serine, and asparagine, respectively, the PPR motif selectively binds to A;
(2-38) when the three amino acids, No. 1 A.A., No. 4 A.A., and No. "ii" (-2) A.A., are phenylalanine, serine, and asparagine, respectively, the PPR motif selectively binds to A;
(2-39) when the three amino acids, No. 1 A.A., No. 4 A.A., and No. "ii" (-2) A.A., are valine, serine, and asparagine, respectively, the PPR motif selectively binds to A;
(2-40) when the three amino acids, No. 1 A.A., No. 4 A.A., and No. "ii" (-2) A.A., are an arbitrary amino acid, threonine, and an arbitrary amino acid, respectively, the PPR motif selectively binds to A and G;
(2-41) when the three amino acids, No. 1 A.A., No. 4 A.A., and No. "ii" (-2) A.A., are an arbitrary amino acid, threonine, and aspartic acid, respectively, the PPR motif selectively binds to G;
(2-42) when the three amino acids, No. 1 A.A., No. 4 A.A., and No. "ii" (-2) A.A., are valine, threonine, and aspartic acid, respectively, the PPR motif selectively binds to G;
(2-43) when the three amino acids, No. 1 A.A., No. 4 A.A., and No. "ii" (-2) A.A., are an arbitrary amino acid, threonine, and asparagine, respectively, the PPR motif selectively binds to A;
(2-44) when the three amino acids, No. 1 A.A., No. 4 A.A., and No. "ii" (-2) A.A., are phenylalanine, threonine, and asparagine, respectively, the PPR motif selectively binds to A;
(2-45) when the three amino acids, No. 1 A.A., No. 4 A.A., and No. "ii" (-2) A.A., are isoleucine, threonine, and asparagine, respectively, the PPR motif selectively binds to A;
(2-46) when the three amino acids, No. 1 A.A., No. 4 A.A., and No. "ii" (-2) A.A., are valine, threonine, and asparagine, respectively, the PPR motif selectively binds to A;
(2-47) when the three amino acids, No. 1 A.A., No. 4 A.A., and No. "ii" (-2) A.A., are an arbitrary amino acid, valine, and an arbitrary amino acid, respectively, the PPR motif binds with A, C, and T, but does not bind to G;
(2-48) when the three amino acids, No. 1 A.A., No. 4 A.A., and No. "ii" (-2) A.A., are isoleucine, valine, and aspartic acid, respectively, the PPR motif selectively binds to C, and next binds to A;
(2-49) when the three amino acids, No. 1 A.A., No. 4 A.A., and No. "ii" (-2) A.A., are an arbitrary amino acid, valine, and glycine, respectively, the PPR motif selectively binds to C; and
(2-50) when the three amino acids, No. 1 A.A., No. 4 A.A., and No. "ii" (-2) A.A., are an arbitrary amino acid, valine, and threonine, respectively, the PPR motif selectively binds to T.

4. The protein according to any one of claims 1 to 3, which contains 2 to 30 of the PPR motifs (Mₙ) defined in claim 1.

5. The protein according to any one of claims 1 to 4, which satisfies at least one selected from the group consisting of the combination of (b) and (c), the combination of (d) and (e), (g), and (h).

6. The protein according to claim 5, which satisfies the combination of (b) and (c), and satisfies at least one selected from the group consisting of the combination of (d) and (e), (g), and (h).

7. The protein according to claim 6, which satisfies the combination of (b) and (c), the combination of (d) and (e), and (g).

8. The protein according to any one of claims 1 to 7, which contains a plurality of PPR motifs, and has a DNA-binding PPR motif content of 13% or higher.

9. A protein consisting of:
any one of the amino acid sequences of SEQ ID NOS: 7 to 214;
any one amino acid sequence selected from the group consisting of the amino acid sequence of the 167 to 482 positions of SEQ ID NO: 291, the amino acid sequence of the 156 to 575 positions of SEQ ID NO: 292, the amino acid sequence of the 243 to 554 positions of SEQ ID NO: 293, the amino acid sequence of the 140 to 489 positions of SEQ ID NO: 294, the amino acid sequence of the 78 to 419 positions of SEQ ID NO: 295, the amino acid sequence of the 122 to 545 positions of SEQ ID NO: 296, the amino acid sequence of the 256 to 624 positions of SEQ ID NO: 297, the amino acid sequence of the 48 to 362 positions of SEQ ID NO: 298, the amino acid sequence of the 198 to 689 positions of SEQ ID NO: 299, the amino acid sequence of the 89 to 578 positions of SEQ ID NO: 300, the amino acid sequence of the 470 to 911 positions of SEQ ID NO: 301, the amino acid sequence of the 156 to 575 positions of SEQ ID NO: 302, the amino acid sequence of the 108 to 775 positions of SEQ ID NO: 303, the amino acid sequence of the 226 to 1137 positions of SEQ ID NO: 304, the amino acid sequence of the 145 to 496 positions of SEQ ID NO: 305, the amino acid sequence of the 104 to 538 positions of SEQ ID NO: 306, the amino acid sequence of the 151 to 502 positions of SEQ ID NO: 307, and the amino acid sequence of the 274 to 660 positions of SEQ ID NO: 308;
any one of the amino acid sequences of SEQ ID NOS: 335 to 361; or
any one of the amino acid sequences of SEQ ID NOS: 424 to 427.

10. A complex consisting of
a region consisting of
the protein according to any one of claims 1 to 9, or a protein consisting of any one of the amino acid sequences of SEQ ID NOS: 291 to 308, or a part thereof;
a protein consisting of any one of the amino acid sequences of SEQ ID NOS: 335 to 361; or
a protein consisting of any one of the amino acid sequences of SEQ ID NOS: 424 to 427, and
a functional region bound together.

11. The complex according to claim 10, wherein the functional region is fused to the protein on the C-terminus side of the protein.

12. The complex according to claim 10 or 11, wherein the functional region is a DNA-cleaving enzyme, or a nuclease domain thereof, or a transcription control domain, and the complex functions as a target sequence-specific DNA-cleaving enzyme or transcription control factor.

13. The complex according to claim 12, wherein the DNA-cleaving enzyme is the nuclease domain of FokI (SEQ ID NO: 6).

14. A method for designing a protein that binds to a DNA base or DNA having a specific base sequence, which comprises replacing one or two or more amino acids on the basis of any selected from the group consisting of (b) to (h) defined in claim 1 in any of:
a protein having any one amino acid sequence selected from the group consisting of the amino acid sequence of the 230 to 541 positions of SEQ ID NO: 1, the amino acid sequence of the 234 to 621 positions of SEQ ID NO: 2, the amino acid sequence of the 106 to 632 positions of SEQ ID NO: 3, the amino acid sequence of the 106 to 632 positions of SEQ ID NO: 4, and the amino acid sequence of the 256 to 624 positions of SEQ ID NO: 5;
any one PPR motif selected from the group consisting of 9 PPR motifs of the protein consisting of the amino acid sequence of SEQ ID NO: 1, 11 PPR motifs of the protein consisting of the amino acid sequence of SEQ ID NO: 2, 15 PPR motifs of the protein consisting of the amino acid sequence of SEQ ID NO: 3, 10 PPR motifs of the protein consisting of the amino acid sequence of SEQ ID NO: 4, and 11 PPR motifs of the protein consisting of the amino acid sequence of SEQ ID NO: 5;
a protein having any one amino acid sequence selected from the group consisting of the amino acid sequence of the 167 to 482 positions of SEQ ID NO: 291, the amino acid sequence of the 156 to 575 positions of SEQ ID NO: 292, the amino acid sequence of the 243 to 554 positions of SEQ ID NO: 293, the amino acid sequence of the 140 to 489 positions of SEQ ID NO: 294, the amino acid sequence of the 78 to 419 positions of SEQ ID NO: 295, the amino acid sequence of the 122 to 545 positions of SEQ ID NO: 296, the amino acid sequence of the 256 to 624 positions of SEQ ID NO: 297, the amino acid sequence of the 48 to 362 positions of SEQ ID NO: 298, the amino acid sequence of the 198 to 689 positions of SEQ ID NO: 299, the amino acid sequence of the 89 to 578 positions of SEQ ID NO: 300, the amino acid sequence of the 470 to 911 positions of SEQ ID NO: 301, the amino acid sequence of the 156 to 575 positions of SEQ ID NO: 302, the amino acid sequence of the 108 to 775 positions of SEQ ID NO: 303, the amino acid sequence of the 226 to 1137 positions of SEQ ID NO: 304, the amino acid sequence of the 145 to 496 positions of SEQ ID NO: 305, the amino acid sequence of the 104 to 538 positions of SEQ ID NO: 306, the amino acid sequence of the 151 to 502 positions of SEQ ID NO: 307, and the amino acid sequence of the 274 to 660 positions of SEQ ID NO: 308, and
any one PPR motif selected from the group consisting of 9 PPR motifs of the protein consisting of the amino acid sequence SEQ ID NO: 291, 6 PPR motifs of the protein consisting of the amino acid sequence of SEQ ID NO: 292, 9 PPR motifs of the protein consisting of the amino acid sequence of SEQ ID NO: 293, 10 PPR motifs of the protein consisting of the amino acid sequence of SEQ ID NO: 294, 9 PPR motifs of the protein consisting of the amino acid sequence of SEQ ID NO: 295, 12 PPR motifs of the protein consisting of the amino acid sequence of SEQ ID NO: 296, 10 PPR motifs of the protein consisting of the amino acid sequence of SEQ ID NO: 297, 9 PPR motifs of the protein consisting of the amino acid sequence of SEQ ID NO: 298, 14 PPR motifs of the protein consisting of the amino acid sequence of SEQ ID NO: 299, 14 PPR motifs of the protein consisting of the amino acid sequence of SEQ ID NO: 300, 10 PPR motifs of the protein consisting of the amino acid sequence of SEQ ID NO: 301, 12 PPR motifs of the protein consisting of the amino acid sequence of SEQ ID NO: 302, 19 PPR motifs of the protein consisting of the amino acid sequence of SEQ ID NO: 303, 25 PPR motifs of the protein consisting of the amino acid sequence of SEQ ID NO: 304, 10 PPR motifs of the protein consisting of the amino acid sequence of SEQ ID NO: 305, 9 PPR motifs of the protein consisting of the amino acid sequence of SEQ ID NO: 306, 10 PPR motifs of the protein consisting of the amino acid sequence of SEQ ID NO: 307, and 11 PPR motifs of the protein consisting of the amino acid sequence of SEQ ID NO: 308.

15. A method for designing a protein that binds to a DNA base or DNA having a specific base sequence, which comprises making the protein contain one or more PPR motifs having a structure of the following formula 1:
[Chemical Formula 2]
(Helix A)-X-(Helix B)-L (Formula 1)
(wherein, in the formula 1:
Helix A is a part that can form an α-helix structure;
X does not exist, or is a part consisting of 1 to 9 amino acids;
Helix B is a part that can form an α-helix structure; and
L is a part consisting of 2 to 7 amino acids),
wherein,
under the following definitions:
the first amino acid of Helix A is referred to as No. 1 amino acid (No. 1 A.A.), the fourth amino acid as No. 4 amino acid (No. 4 A.A.), and
- when a next PPR motif (Mₙ₊₁) contiguously exists on the C-terminus side of the PPR motif (Mₙ) (when there is no amino acid insertion between the PPR motifs), the -2nd amino acid counted from the end (C-terminus side) of the amino acids constituting the PPR motif (Mₙ);
- when a non-PPR motif consisting of 1 to 20 amino acids exists between the PPR motif (Mₙ) and the next PPR motif (Mₙ₊₁) on the C-terminus side, the amino acid locating upstream of the first amino acid of the next PPR motif (Mₙ₊₁) by 2 positions, i.e., the -2nd amino acid; or
- when any next PPR motif (Mₙ₊₁) does not exist on the C-terminus side of the PPR motif (Mₙ), or 21 or more amino acids constituting a non-PPR motif exist between the PPR motif (Mₙ) and the next PPR motif (Mₙ₊₁) on the C-terminus side, the 2nd amino acid counted from the end (C-terminus side) of the amino acids constituting the PPR motif (Mₙ)
is referred to as No. "ii" (-2) amino acid (No. "ii" (-2) A.A.),
one PPR motif (Mₙ) contained in the protein is a PPR motif having a specific combination of amino acids corresponding to a target DNA base or target DNA base sequence as the three amino acids of No. 1 A.A., No. 4 A.A., and No. "ii" (-2) A.A, and satisfies at least one selected from the group consisting of the following conditions (b) to (h):
(b) No. 9 A.A. of the PPR motif (Mₙ) is alanine (A);
(c) No. 10 A.A. of the PPR motif (Mₙ) is tyrosine (Y), phenylalanine (F), or tryptophan (W);
(d) No. 18 A.A. of the PPR motif (Mₙ) is lysine (K), arginine (R), or histidine (H);
(e) No. 20 A.A. of the PPR motif (Mₙ) is glutamic acid (E), or aspartic acid (D);
(f) No. 29 A.A. of the PPR motif (Mₙ) is glutamic acid (E), or aspartic acid (D);
(g) No. 31 A.A. of the PPR motif (Mₙ) is isoleucine (I), leucine (L), or valine (V); and
(h) No. 32 A.A. of the PPR motif (Mₙ) is lysine (K), arginine (R), or histidine (H).

16. The method according to claim 15, wherein the combination of the three amino acids of No. 1 A.A., No. 4 A.A., and No. "ii" (-2) A.A. is determined according to any one of the following definitions:
(1-1) when No. 4 A.A. is glycine (G), No. 1 A.A. may be an arbitrary amino acid, and No. "ii" (-2) A.A. is aspartic acid (D), asparagine (N), or serine (S);
(1-2) when No. 4 A.A. is isoleucine (I), each of No. 1 A.A. and No. "ii" (-2) A.A. may be an arbitrary amino acid;
(1-3) when No. 4 A.A. is leucine (L), each of No. 1 A.A. and No. "ii" (-2) A.A. may be an arbitrary amino acid;
(1-4) when No. 4 A.A. is methionine (M), each of No. 1 A.A. and No. "ii" (-2) A.A. may be an arbitrary amino acid;
(1-5) when No. 4 A.A. is asparagine (N), each of No. 1 A.A. and No. "ii" (-2) A.A. may be an arbitrary amino acid;
(1-6) when No. 4 A.A. is proline (P), each of No. 1 A.A. and No. "ii" (-2) A.A. may be an arbitrary amino acid;
(1-7) when No. 4 A.A. is serine (S), each of No. 1 A.A. and No. "ii" (-2) A.A. may be an arbitrary amino acid;
(1-8) when No. 4 A.A. is threonine (T), each of No. 1 A.A. and No. "ii" (-2) A.A. may be an arbitrary amino acid; and
(1-9) when No. 4 A.A. is valine (V), each of No. 1 A.A. and No. "ii" (-2) A.A. may be an arbitrary amino acid.

17. The method according to claim 15, wherein the combination of the three amino acids of No. 1 A.A., No. 4 A.A., and No. "ii" (-2) A.A. is determined according to any one of the following definitions:
(2-1) when the three amino acids, No. 1 A.A., No. 4 A.A., and No. "ii" (-2) A.A. are an arbitrary amino acid, glycine, and aspartic acid, respectively, the PPR motif selectively binds to G;
(2-2) when the three amino acids, No. 1 A.A., No. 4 A.A., and No. "ii" (-2) A.A., are glutamic acid, glycine, and aspartic acid, respectively, the PPR motif selectively binds to G;
(2-3) when the three amino acids, No. 1 A.A., No. 4 A.A., and No. "ii" (-2) A.A., are an arbitrary amino acid, glycine, and asparagine, respectively, the PPR motif selectively binds to A;
(2-4) when the three amino acids, No. 1 A.A., No. 4 A.A., and No. "ii" (-2) A.A., are glutamic acid, glycine, and asparagine, respectively, the PPR motif selectively binds to A;
(2-5) when the three amino acids, No. 1 A.A., No. 4 A.A., and No. "ii" (-2) A.A., are an arbitrary amino acid, glycine, and serine, respectively, the PPR motif selectively binds to A, and next binds to C;
(2-6) when the three amino acids, No. 1 A.A., No. 4 A.A., and No. "ii" (-2) A.A., are an arbitrary amino acid, isoleucine, and an arbitrary amino acid, respectively, the PPR motif selectively binds to T and C;
(2-7) when the three amino acids, No. 1 A.A., No. 4 A.A., and No. "ii" (-2) A.A., are an arbitrary amino acid, isoleucine, and asparagine, respectively, the PPR motif selectively binds to T, and next binds to C;
(2-8) when the three amino acids, No. 1 A.A., No. 4 A.A., and No. "ii" (-2) A.A., are an arbitrary amino acid, leucine, and an arbitrary amino acid, respectively, the PPR motif selectively binds to T and C;
(2-9) when the three amino acids, No. 1 A.A., No. 4 A.A., and No. "ii" (-2) A.A., are an arbitrary amino acid, leucine, and aspartic acid, respectively, the PPR motif selectively binds to C;
(2-10) when the three amino acids, No. 1 A.A., No. 4 A.A., and No. "ii" (-2) A.A., are an arbitrary amino acid, leucine, and lysine, respectively, the PPR motif selectively binds to T;
(2-11) when the three amino acids, No. 1 A.A., No. 4 A.A., and No. "ii" (-2) A.A., are an arbitrary amino acid, methionine, and an arbitrary amino acid, respectively, the PPR motif selectively binds to T;
(2-12) when the three amino acids, No. 1 A.A., No. 4 A.A., and No. "ii" (-2) A.A., are an arbitrary amino acid, methionine, and aspartic acid, respectively, the PPR motif selectively binds to T;
(2-13) when the three amino acids, No. 1 A.A., No. 4 A.A., and No. "ii" (-2) A.A., are isoleucine, methionine, and aspartic acid, respectively, the PPR motif selectively binds to T, and next binds to C;
(2-14) when the three amino acids, No. 1 A.A., No. 4 A.A., and No. "ii" (-2) A.A., are an arbitrary amino acid, asparagine, and an arbitrary amino acid, respectively, the PPR motif selectively binds to C and T;
(2-15) when the three amino acids, No. 1 A.A., No. 4 A.A., and No. "ii" (-2) A.A., are an arbitrary amino acid, asparagine, and aspartic acid, respectively, the PPR motif selectively binds to T;
(2-16) when the three amino acids, No. 1 A.A., No. 4 A.A., and No. "ii" (-2) A.A., are phenylalanine, asparagine, and aspartic acid, respectively, the PPR motif selectively binds to T;
(2-17) when the three amino acids, No. 1 A.A., No. 4 A.A., and No. "ii" (-2) A.A., are glycine, asparagine, and aspartic acid, respectively, the PPR motif selectively binds to T;
(2-18) when the three amino acids, No. 1 A.A., No. 4 A.A., and No. "ii" (-2) A.A., are isoleucine, asparagine, and aspartic acid, respectively, the PPR motif selectively binds to T;
(2-19) when the three amino acids, No. 1 A.A., No. 4 A.A., and No. "ii" (-2) A.A., are threonine, asparagine, and aspartic acid, respectively, the PPR motif selectively binds to T;
(2-20) when the three amino acids, No. 1 A.A., No. 4 A.A., and No. "ii" (-2) A.A. are valine, asparagine, and aspartic acid, respectively, the PPR motif selectively binds to T, and next binds to C;
(2-21) when the three amino acids, No. 1 A.A., No. 4 A.A., and No. "ii" (-2) A.A. are tyrosine, asparagine, and aspartic acid, respectively, the PPR motif selectively binds to T, and next binds to C;
(2-22) when the three amino acids, No. 1 A.A., No. 4 A.A., and No. "ii" (-2) A.A., are an arbitrary amino acid, asparagine, and asparagine, respectively, the PPR motif selectively binds to C;
(2-23) when the three amino acids, No. 1 A.A., No. 4 A.A., and No. "ii" (-2) A.A., are isoleucine, asparagine, and asparagine, respectively, the PPR motif selectively binds to C;
(2-24) when the three amino acids, No. 1 A.A., No. 4 A.A., and No. "ii" (-2) A.A., are serine, asparagine, and asparagine, respectively, the PPR motif selectively binds to C;
(2-25) when the three amino acids, No. 1 A.A., No. 4 A.A., and No. "ii" (-2) A.A., are valine, asparagine, and asparagine, respectively, the PPR motif selectively binds to C;
(2-26) when the three amino acids, No. 1 A.A., No. 4 A.A., and No. "ii" (-2) A.A., are an arbitrary amino acid, asparagine, and serine, respectively, the PPR motif selectively binds to C;
(2-27) when the three amino acids, No. 1 A.A., No. 4 A.A., and No. "ii" (-2) A.A., are valine, asparagine, and serine, respectively, the PPR motif selectively binds to C;
(2-28) when the three amino acids, No. 1 A.A., No. 4 A.A., and No. "ii" (-2) A.A., are an arbitrary amino acid, asparagine, and threonine, respectively, the PPR motif selectively binds to C;
(2-29) when the three amino acids, No. 1 A.A., No. 4 A.A., and No. "ii" (-2) A.A., are valine, asparagine, and threonine, respectively, the PPR motif selectively binds to C;
(2-30) when the three amino acids, No. 1 A.A., No. 4 A.A., and No. "ii" (-2) A.A., are an arbitrary amino acid, asparagine, and tryptophan, respectively, the PPR motif selectively binds to C, and next binds to T;
(2-31) when the three amino acids, No. 1 A.A., No. 4 A.A., and No. "ii" (-2) A.A., are isoleucine, asparagine, and tryptophan, respectively, the PPR motif selectively binds to T, and next binds to C;
(2-32) when the three amino acids, No. 1 A.A., No. 4 A.A., and No. "ii" (-2) A.A., are an arbitrary amino acid, proline, and an arbitrary amino acid, respectively, the PPR motif selectively binds to T;
(2-33) when the three amino acids, No. 1 A.A., No. 4 A.A., and No. "ii" (-2) A.A., are an arbitrary amino acid, proline, and aspartic acid, respectively, the PPR motif selectively binds to T;
(2-34) when the three amino acids, No. 1 A.A., No. 4 A.A., and No. "ii" (-2) A.A., are phenylalanine, proline, and aspartic acid, respectively, the PPR motif selectively binds to T;
(2-35) when the three amino acids, No. 1 A.A., No. 4 A.A., and No. "ii" (-2) A.A., are tyrosine, proline, and aspartic acid, respectively, the PPR motif selectively binds to T;
(2-36) when the three amino acids, No. 1 A.A., No. 4 A.A., and No. "ii" (-2) A.A., are an arbitrary amino acid, serine, and an arbitrary amino acid, respectively, the PPR motif selectively binds to A and G;
(2-37) when the three amino acids, No. 1 A.A., No. 4 A.A., and No. "ii" (-2) A.A., are an arbitrary amino acid, serine, and asparagine, respectively, the PPR motif selectively binds to A;
(2-38) when the three amino acids, No. 1 A.A., No. 4 A.A., and No. "ii" (-2) A.A., are phenylalanine, serine, and asparagine, respectively, the PPR motif selectively binds to A;
(2-39) when the three amino acids, No. 1 A.A., No. 4 A.A., and No. "ii" (-2) A.A., are valine, serine, and asparagine, respectively, the PPR motif selectively binds to A;
(2-40) when the three amino acids, No. 1 A.A., No. 4 A.A., and No. "ii" (-2) A.A., are an arbitrary amino acid, threonine, and an arbitrary amino acid, respectively, the PPR motif selectively binds to A and G;
(2-41) when the three amino acids, No. 1 A.A., No. 4 A.A., and No. "ii" (-2) A.A., are an arbitrary amino acid, threonine, and aspartic acid, respectively, the PPR motif selectively binds to G;
(2-42) when the three amino acids, No. 1 A.A., No. 4 A.A., and No. "ii" (-2) A.A., are valine, threonine, and aspartic acid, respectively, the PPR motif selectively binds to G;
(2-43) when the three amino acids, No. 1 A.A., No. 4 A.A., and No. "ii" (-2) A.A., are an arbitrary amino acid, threonine, and asparagine, respectively, the PPR motif selectively binds to A;
(2-44) when the three amino acids, No. 1 A.A., No. 4 A.A., and No. "ii" (-2) A.A., are phenylalanine, threonine, and asparagine, respectively, the PPR motif selectively binds to A;
(2-45) when the three amino acids, No. 1 A.A., No. 4 A.A., and No. "ii" (-2) A.A., are isoleucine, threonine, and asparagine, respectively, the PPR motif selectively binds to A;
(2-46) when the three amino acids, No. 1 A.A., No. 4 A.A., and No. "ii" (-2) A.A., are valine, threonine, and asparagine, respectively, the PPR motif selectively binds to A;
(2-47) when the three amino acids, No. 1 A.A., No. 4 A.A., and No. "ii" (-2) A.A., are an arbitrary amino acid, valine, and an arbitrary amino acid, respectively, the PPR motif binds with A, C, and T, but does not bind to G;
(2-48) when the three amino acids, No. 1 A.A., No. 4 A.A., and No. "ii" (-2) A.A., are isoleucine, valine, and aspartic acid, respectively, the PPR motif selectively binds to C, and next binds to A;
(2-49) when the three amino acids, No. 1 A.A., No. 4 A.A., and No. "ii" (-2) A.A., are an arbitrary amino acid, valine, and glycine, respectively, the PPR motif selectively binds to C; and
(2-50) when the three amino acids, No. 1 A.A., No. 4 A.A., and No. "ii" (-2) A.A., are an arbitrary amino acid, valine, and threonine, respectively, the PPR motif selectively binds to T.

18. The method according to any one of claims 15 to 17, wherein at least one selected from the group consisting of the combination of (b) and (c), the combination of (d) and (e), (g), and (h) is satisfied.

19. The method according to claim 18, wherein the combination of (b) and (c) is satisfied, and at least one selected from the group consisting of the combination of (d) and (e), (g), and (h) is satisfied.

20. The method according to claim 19, wherein the combination of (b) and (c), the combination of (d) and (e), and (g) are satisfied.

21. The method according to any one of claims 15 to 20, wherein the protein contains a plurality of PPR motifs, and has a DNA-binding PPR motif content of 13% or higher.

22. A method for producing a protein, which comprises designing a protein by the method according to any one of claims 14 to 21, and producing the designed protein.

23. A method for producing a complex, which comprises designing a protein by the method according to any one of claims 14 to 21, and binding a region consisting of the designed protein and a functional region to produce the complex.

24. A method for editing a genome, which comprises
using the complex according to any one of claims 10 to 13, or
designing a protein by the method according to anyone of claims 14 to 21, binding a region consisting of the designed protein and a functional region to produce a complex, and using the produced complex provided that implementation in a human individual is excluded.

25. A method for producing a cell containing a edited genome, which comprises editing a genome by the method according to claim 23, and producing a cell containing the edited genome provided that implementation in a human individual is excluded.


SEQUENCE LISTING

<110>  Wako Pure Chemical Industries, Ltd.
       Kyushu University

<120>  DNA-binding protein using PPR motif, and use thereof

<130>  171504H

<150>  JP 2016-157698
<151>  2016-08-10

<160>  427

<170>  PatentIn version 3.5

<210>  1
<211>  596
<212>  PRT
<213>  Arabidopsis thaliana

<400>  1

Met Phe Ala Leu Ser Lys Val Leu Arg Arg Thr Gln Arg Leu Arg Leu
1               5                   10                  15

Gly Ala Cys Ser Ala Val Phe Ser Lys Asp Ile Gln Leu Gly Gly Glu
            20                  25                  30

Arg Ser Phe Asp Ser Asn Ser Ile Ala Ser Thr Lys Arg Glu Ala Val
        35                  40                  45

Pro Arg Phe Tyr Glu Ile Ser Ser Leu Ser Asn Arg Ala Leu Ser Ser
    50                  55                  60

Ser Ala Gly Thr Lys Ser Asp Gln Glu Glu Asp Asp Leu Glu Asp Gly
65                  70                  75                  80

Phe Ser Glu Leu Glu Gly Ser Lys Ser Gly Gln Gly Ser Thr Ser Ser
                85                  90                  95

Asp Glu Asp Glu Gly Lys Leu Ser Ala Asp Glu Glu Glu Glu Glu Glu
            100                 105                 110

Leu Asp Leu Ile Glu Thr Asp Val Ser Arg Lys Thr Val Glu Lys Lys
        115                 120                 125

Gln Ser Glu Leu Phe Lys Thr Ile Val Ser Ala Pro Gly Leu Ser Ile
    130                 135                 140

Gly Ser Ala Leu Asp Lys Trp Val Glu Glu Gly Asn Glu Ile Thr Arg
145                 150                 155                 160

Val Glu Ile Ala Lys Ala Met Leu Gln Leu Arg Arg Arg Arg Met Tyr
                165                 170                 175

Gly Arg Ala Leu Gln Met Ser Glu Trp Leu Glu Ala Asn Lys Lys Ile
            180                 185                 190

Glu Met Thr Glu Arg Asp Tyr Ala Ser Arg Leu Asp Leu Thr Val Lys
        195                 200                 205

Ile Arg Gly Leu Glu Lys Gly Glu Ala Cys Met Gln Lys Ile Pro Lys
    210                 215                 220

Ser Phe Lys Gly Glu Val Leu Tyr Arg Thr Leu Leu Ala Asn Cys Val
225                 230                 235                 240

Ala Ala Gly Asn Val Lys Lys Ser Glu Leu Val Phe Asn Lys Met Lys
                245                 250                 255

Asp Leu Gly Phe Pro Leu Ser Gly Phe Thr Cys Asp Gln Met Leu Leu
            260                 265                 270

Leu His Lys Arg Ile Asp Arg Lys Lys Ile Ala Asp Val Leu Leu Leu
        275                 280                 285

Met Glu Lys Glu Asn Ile Lys Pro Ser Leu Leu Thr Tyr Lys Ile Leu
    290                 295                 300

Ile Asp Val Lys Gly Ala Thr Asn Asp Ile Ser Gly Met Glu Gln Ile
305                 310                 315                 320

Leu Glu Thr Met Lys Asp Glu Gly Val Glu Leu Asp Phe Gln Thr Gln
                325                 330                 335

Ala Leu Thr Ala Arg His Tyr Ser Gly Ala Gly Leu Lys Asp Lys Ala
            340                 345                 350

Glu Lys Val Leu Lys Glu Met Glu Gly Glu Ser Leu Glu Ala Asn Arg
        355                 360                 365

Arg Ala Phe Lys Asp Leu Leu Ser Ile Tyr Ala Ser Leu Gly Arg Glu
    370                 375                 380

Asp Glu Val Lys Arg Ile Trp Lys Ile Cys Glu Ser Lys Pro Tyr Phe
385                 390                 395                 400

Glu Glu Ser Leu Ala Ala Ile Gln Ala Phe Gly Lys Leu Asn Lys Val
                405                 410                 415

Gln Glu Ala Glu Ala Ile Phe Glu Lys Ile Val Lys Met Asp Arg Arg
            420                 425                 430

Ala Ser Ser Ser Thr Tyr Ser Val Leu Leu Arg Val Tyr Val Asp His
        435                 440                 445

Lys Met Leu Ser Lys Gly Lys Asp Leu Val Lys Arg Met Ala Glu Ser
    450                 455                 460

Gly Cys Arg Ile Glu Ala Thr Thr Trp Asp Ala Leu Ile Lys Leu Tyr
465                 470                 475                 480

Val Glu Ala Gly Glu Val Glu Lys Ala Asp Ser Leu Leu Asp Lys Ala
                485                 490                 495

Ser Lys Gln Ser His Thr Lys Leu Met Met Asn Ser Phe Met Tyr Ile
            500                 505                 510

Met Asp Glu Tyr Ser Lys Arg Gly Asp Val His Asn Thr Glu Lys Ile
        515                 520                 525

Phe Leu Lys Met Arg Glu Ala Gly Tyr Thr Ser Arg Leu Arg Gln Phe
    530                 535                 540

Gln Ala Leu Met Gln Ala Tyr Ile Asn Ala Lys Ser Pro Ala Tyr Gly
545                 550                 555                 560

Met Arg Asp Arg Leu Lys Ala Asp Asn Ile Phe Pro Asn Lys Ser Met
                565                 570                 575

Ala Ala Gln Leu Ala Gln Gly Asp Pro Phe Lys Lys Thr Ala Ile Ser
            580                 585                 590

Asp Ile Leu Asp
        595

<210>  2
<211>  918
<212>  PRT
<213>  Arabidopsis thaliana

<400>  2

Met Ala Ser Thr Pro Pro His Trp Val Thr Thr Thr Asn Asn His Arg
1               5                   10                  15

Pro Trp Leu Pro Gln Arg Pro Arg Pro Gly Arg Ser Val Thr Ser Ala
            20                  25                  30

Pro Pro Ser Ser Ser Ala Ser Val Ser Ser Ala His Leu Ser Gln Thr
        35                  40                  45

Thr Pro Asn Phe Ser Pro Leu Gln Thr Pro Lys Ser Asp Phe Ser Gly
    50                  55                  60

Arg Gln Ser Thr Arg Phe Val Ser Pro Ala Thr Asn Asn His Arg Gln
65                  70                  75                  80

Thr Arg Gln Asn Pro Asn Tyr Asn His Arg Pro Tyr Gly Ala Ser Ser
                85                  90                  95

Ser Pro Arg Gly Ser Ala Pro Pro Pro Ser Ser Val Ala Thr Val Ala
            100                 105                 110

Pro Ala Gln Leu Ser Gln Pro Pro Asn Phe Ser Pro Leu Gln Thr Pro
        115                 120                 125

Lys Ser Asp Leu Ser Ser Asp Phe Ser Gly Arg Arg Ser Thr Arg Phe
    130                 135                 140

Val Ser Lys Met His Phe Gly Arg Gln Lys Thr Thr Met Ala Thr Arg
145                 150                 155                 160

His Ser Ser Ala Ala Glu Asp Ala Leu Gln Asn Ala Ile Asp Phe Ser
                165                 170                 175

Gly Asp Asp Glu Met Phe His Ser Leu Met Leu Ser Phe Glu Ser Lys
            180                 185                 190

Leu Cys Gly Ser Asp Asp Cys Thr Tyr Ile Ile Arg Glu Leu Gly Asn
        195                 200                 205

Arg Asn Glu Cys Asp Lys Ala Val Gly Phe Tyr Glu Phe Ala Val Lys
    210                 215                 220

Arg Glu Arg Arg Lys Asn Glu Gln Gly Lys Leu Ala Ser Ala Met Ile
225                 230                 235                 240

Ser Thr Leu Gly Arg Tyr Gly Lys Val Thr Ile Ala Lys Arg Ile Phe
                245                 250                 255

Glu Thr Ala Phe Ala Gly Gly Tyr Gly Asn Thr Val Tyr Ala Phe Ser
            260                 265                 270

Ala Leu Ile Ser Ala Tyr Gly Arg Ser Gly Leu His Glu Glu Ala Ile
        275                 280                 285

Ser Val Phe Asn Ser Met Lys Glu Tyr Gly Leu Arg Pro Asn Leu Val
    290                 295                 300

Thr Tyr Asn Ala Val Ile Asp Ala Cys Gly Lys Gly Gly Met Glu Phe
305                 310                 315                 320

Lys Gln Val Ala Lys Phe Phe Asp Glu Met Gln Arg Asn Gly Val Gln
                325                 330                 335

Pro Asp Arg Ile Thr Phe Asn Ser Leu Leu Ala Val Cys Ser Arg Gly
            340                 345                 350

Gly Leu Trp Glu Ala Ala Arg Asn Leu Phe Asp Glu Met Thr Asn Arg
        355                 360                 365

Arg Ile Glu Gln Asp Val Phe Ser Tyr Asn Thr Leu Leu Asp Ala Ile
    370                 375                 380

Cys Lys Gly Gly Gln Met Asp Leu Ala Phe Glu Ile Leu Ala Gln Met
385                 390                 395                 400

Pro Val Lys Arg Ile Met Pro Asn Val Val Ser Tyr Ser Thr Val Ile
                405                 410                 415

Asp Gly Phe Ala Lys Ala Gly Arg Phe Asp Glu Ala Leu Asn Leu Phe
            420                 425                 430

Gly Glu Met Arg Tyr Leu Gly Ile Ala Leu Asp Arg Val Ser Tyr Asn
        435                 440                 445

Thr Leu Leu Ser Ile Tyr Thr Lys Val Gly Arg Ser Glu Glu Ala Leu
    450                 455                 460

Asp Ile Leu Arg Glu Met Ala Ser Val Gly Ile Lys Lys Asp Val Val
465                 470                 475                 480

Thr Tyr Asn Ala Leu Leu Gly Gly Tyr Gly Lys Gln Gly Lys Tyr Asp
                485                 490                 495

Glu Val Lys Lys Val Phe Thr Glu Met Lys Arg Glu His Val Leu Pro
            500                 505                 510

Asn Leu Leu Thr Tyr Ser Thr Leu Ile Asp Gly Tyr Ser Lys Gly Gly
        515                 520                 525

Leu Tyr Lys Glu Ala Met Glu Ile Phe Arg Glu Phe Lys Ser Ala Gly

530                 535                 540

Leu Arg Ala Asp Val Val Leu Tyr Ser Ala Leu Ile Asp Ala Leu Cys
545                 550                 555                 560

Lys Asn Gly Leu Val Gly Ser Ala Val Ser Leu Ile Asp Glu Met Thr
                565                 570                 575

Lys Glu Gly Ile Ser Pro Asn Val Val Thr Tyr Asn Ser Ile Ile Asp
            580                 585                 590

Ala Phe Gly Arg Ser Ala Thr Met Asp Arg Ser Ala Asp Tyr Ser Asn
        595                 600                 605

Gly Gly Ser Leu Pro Phe Ser Ser Ser Ala Leu Ser Ala Leu Thr Glu
    610                 615                 620

Thr Glu Gly Asn Arg Val Ile Gln Leu Phe Gly Gln Leu Thr Thr Glu
625                 630                 635                 640

Ser Asn Asn Arg Thr Thr Lys Asp Cys Glu Glu Gly Met Gln Glu Leu
                645                 650                 655

Ser Cys Ile Leu Glu Val Phe Arg Lys Met His Gln Leu Glu Ile Lys
            660                 665                 670

Pro Asn Val Val Thr Phe Ser Ala Ile Leu Asn Ala Cys Ser Arg Cys
        675                 680                 685

Asn Ser Phe Glu Asp Ala Ser Met Leu Leu Glu Glu Leu Arg Leu Phe
    690                 695                 700

Asp Asn Lys Val Tyr Gly Val Val His Gly Leu Leu Met Gly Gln Arg
705                 710                 715                 720

Glu Asn Val Trp Leu Gln Ala Gln Ser Leu Phe Asp Lys Val Asn Glu
                725                 730                 735

Met Asp Gly Ser Thr Ala Ser Ala Phe Tyr Asn Ala Leu Thr Asp Met
            740                 745                 750

Leu Trp His Phe Gly Gln Lys Arg Gly Ala Glu Leu Val Ala Leu Glu
        755                 760                 765

Gly Arg Ser Arg Gln Val Trp Glu Asn Val Trp Ser Asp Ser Cys Leu
    770                 775                 780

Asp Leu His Leu Met Ser Ser Gly Ala Ala Arg Ala Met Val His Ala
785                 790                 795                 800

Trp Leu Leu Asn Ile Arg Ser Ile Val Tyr Glu Gly His Glu Leu Pro
                805                 810                 815

Lys Val Leu Ser Ile Leu Thr Gly Trp Gly Lys His Ser Lys Val Val
            820                 825                 830

Gly Asp Gly Ala Leu Arg Arg Ala Val Glu Val Leu Leu Arg Gly Met
        835                 840                 845

Asp Ala Pro Phe His Leu Ser Lys Cys Asn Met Gly Arg Phe Thr Ser
    850                 855                 860

Ser Gly Ser Val Val Ala Thr Trp Leu Arg Glu Ser Ala Thr Leu Lys
865                 870                 875                 880

Leu Leu Ile Leu His Asp His Ile Thr Thr Ala Thr Ala Thr Thr Thr
                885                 890                 895

Thr Met Lys Ser Thr Asp Gln Gln Gln Arg Lys Gln Thr Ser Phe Ala
            900                 905                 910

Leu Gln Pro Leu Leu Leu
        915

<210>  3
<211>  862
<212>  PRT
<213>  Arabidopsis thaliana

<400>  3

Met Asn Leu Ala Ile Pro Asn Pro Asn Ser His His Leu Ser Phe Leu
1               5                   10                  15

Ile Gln Asn Ser Ser Phe Ile Gly Asn Arg Arg Phe Ala Asp Gly Asn
            20                  25                  30

Arg Leu Arg Phe Leu Ser Gly Gly Asn Arg Lys Pro Cys Ser Phe Ser
        35                  40                  45

Gly Lys Ile Lys Ala Lys Thr Lys Asp Leu Val Leu Gly Asn Pro Ser
    50                  55                  60

Val Ser Val Glu Lys Gly Lys Tyr Ser Tyr Asp Val Glu Ser Leu Ile
65                  70                  75                  80

Asn Lys Leu Ser Ser Leu Pro Pro Arg Gly Ser Ile Ala Arg Cys Leu
                85                  90                  95

Asp Ile Phe Lys Asn Lys Leu Ser Leu Asn Asp Phe Ala Leu Val Phe
            100                 105                 110

Lys Glu Phe Ala Gly Arg Gly Asp Trp Gln Arg Ser Leu Arg Leu Phe
        115                 120                 125

Lys Tyr Met Gln Arg Gln Ile Trp Cys Lys Pro Asn Glu His Ile Tyr
    130                 135                 140

Thr Ile Met Ile Ser Leu Leu Gly Arg Glu Gly Leu Leu Asp Lys Cys
145                 150                 155                 160

Leu Glu Val Phe Asp Glu Met Pro Ser Gln Gly Val Ser Arg Ser Val
                165                 170                 175

Phe Ser Tyr Thr Ala Leu Ile Asn Ala Tyr Gly Arg Asn Gly Arg Tyr
            180                 185                 190

Glu Thr Ser Leu Glu Leu Leu Asp Arg Met Lys Asn Glu Lys Ile Ser
        195                 200                 205

Pro Ser Ile Leu Thr Tyr Asn Thr Val Ile Asn Ala Cys Ala Arg Gly
    210                 215                 220

Gly Leu Asp Trp Glu Gly Leu Leu Gly Leu Phe Ala Glu Met Arg His
225                 230                 235                 240

Glu Gly Ile Gln Pro Asp Ile Val Thr Tyr Asn Thr Leu Leu Ser Ala
                245                 250                 255

Cys Ala Ile Arg Gly Leu Gly Asp Glu Ala Glu Met Val Phe Arg Thr
            260                 265                 270

Met Asn Asp Gly Gly Ile Val Pro Asp Leu Thr Thr Tyr Ser His Leu
        275                 280                 285

Val Glu Thr Phe Gly Lys Leu Arg Arg Leu Glu Lys Val Cys Asp Leu
    290                 295                 300

Leu Gly Glu Met Ala Ser Gly Gly Ser Leu Pro Asp Ile Thr Ser Tyr
305                 310                 315                 320

Asn Val Leu Leu Glu Ala Tyr Ala Lys Ser Gly Ser Ile Lys Glu Ala
                325                 330                 335

Met Gly Val Phe His Gln Met Gln Ala Ala Gly Cys Thr Pro Asn Ala
            340                 345                 350

Asn Thr Tyr Ser Val Leu Leu Asn Leu Phe Gly Gln Ser Gly Arg Tyr
        355                 360                 365

Asp Asp Val Arg Gln Leu Phe Leu Glu Met Lys Ser Ser Asn Thr Asp
    370                 375                 380

Pro Asp Ala Ala Thr Tyr Asn Ile Leu Ile Glu Val Phe Gly Glu Gly
385                 390                 395                 400

Gly Tyr Phe Lys Glu Val Val Thr Leu Phe His Asp Met Val Glu Glu
                405                 410                 415

Asn Ile Glu Pro Asp Met Glu Thr Tyr Glu Gly Ile Ile Phe Ala Cys
            420                 425                 430

Gly Lys Gly Gly Leu His Glu Asp Ala Arg Lys Ile Leu Gln Tyr Met
        435                 440                 445

Thr Ala Asn Asp Ile Val Pro Ser Ser Lys Ala Tyr Thr Gly Val Ile
    450                 455                 460

Glu Ala Phe Gly Gln Ala Ala Leu Tyr Glu Glu Ala Leu Val Ala Phe
465                 470                 475                 480

Asn Thr Met His Glu Val Gly Ser Asn Pro Ser Ile Glu Thr Phe His
                485                 490                 495

Ser Leu Leu Tyr Ser Phe Ala Arg Gly Gly Leu Val Lys Glu Ser Glu
            500                 505                 510

Ala Ile Leu Ser Arg Leu Val Asp Ser Gly Ile Pro Arg Asn Arg Asp
        515                 520                 525

Thr Phe Asn Ala Gln Ile Glu Ala Tyr Lys Gln Gly Gly Lys Phe Glu
    530                 535                 540

Glu Ala Val Lys Thr Tyr Val Asp Met Glu Lys Ser Arg Cys Asp Pro
545                 550                 555                 560

Asp Glu Arg Thr Leu Glu Ala Val Leu Ser Val Tyr Ser Phe Ala Arg
                565                 570                 575

Leu Val Asp Glu Cys Arg Glu Gln Phe Glu Glu Met Lys Ala Ser Asp
            580                 585                 590

Ile Leu Pro Ser Ile Met Cys Tyr Cys Met Met Leu Ala Val Tyr Gly
        595                 600                 605

Lys Thr Glu Arg Trp Asp Asp Val Asn Glu Leu Leu Glu Glu Met Leu
    610                 615                 620

Ser Asn Arg Val Ser Asn Ile His Gln Val Ile Gly Gln Met Ile Lys
625                 630                 635                 640

Gly Asp Tyr Asp Asp Asp Ser Asn Trp Gln Ile Val Glu Tyr Val Leu
                645                 650                 655

Asp Lys Leu Asn Ser Glu Gly Cys Gly Leu Gly Ile Arg Phe Tyr Asn
            660                 665                 670

Ala Leu Leu Asp Ala Leu Trp Trp Leu Gly Gln Lys Glu Arg Ala Ala
        675                 680                 685

Arg Val Leu Asn Glu Ala Thr Lys Arg Gly Leu Phe Pro Glu Leu Phe
    690                 695                 700

Arg Lys Asn Lys Leu Val Trp Ser Val Asp Val His Arg Met Ser Glu
705                 710                 715                 720

Gly Gly Met Tyr Thr Ala Leu Ser Val Trp Leu Asn Asp Ile Asn Asp
                725                 730                 735

Met Leu Leu Lys Gly Asp Leu Pro Gln Leu Ala Val Val Val Ser Val
            740                 745                 750

Arg Gly Gln Leu Glu Lys Ser Ser Ala Ala Arg Glu Ser Pro Ile Ala
        755                 760                 765

Lys Ala Ala Phe Ser Phe Leu Gln Asp His Val Ser Ser Ser Phe Ser
    770                 775                 780

Phe Thr Gly Trp Asn Gly Gly Arg Ile Met Cys Gln Arg Ser Gln Leu
785                 790                 795                 800

Lys Gln Leu Leu Ser Thr Lys Glu Pro Thr Ser Glu Glu Ser Glu Asn
                805                 810                 815

Lys Asn Leu Val Ala Leu Ala Asn Ser Pro Ile Phe Ala Ala Gly Thr
            820                 825                 830

Arg Ala Ser Thr Ser Ser Asp Thr Asn His Ser Gly Asn Pro Thr Gln

835                 840                 845

Arg Arg Thr Arg Thr Lys Lys Glu Leu Ala Gly Ser Thr Ala
    850                 855                 860

<210>  4
<211>  798
<212>  PRT
<213>  Arabidopsis thaliana

<400>  4

Met Asp Ala Ser Val Val Arg Phe Ser Gln Ser Pro Ala Arg Val Pro
1               5                   10                  15

Pro Glu Phe Glu Pro Asp Met Glu Lys Ile Lys Arg Arg Leu Leu Lys
            20                  25                  30

Tyr Gly Val Asp Pro Thr Pro Lys Ile Leu Asn Asn Leu Arg Lys Lys
        35                  40                  45

Glu Ile Gln Lys His Asn Arg Arg Thr Lys Arg Glu Thr Glu Ser Glu
    50                  55                  60

Ala Glu Val Tyr Thr Glu Ala Gln Lys Gln Ser Met Glu Glu Glu Ala
65                  70                  75                  80

Arg Phe Gln Thr Leu Arg Arg Glu Tyr Lys Gln Phe Thr Arg Ser Ile
                85                  90                  95

Ser Gly Lys Arg Gly Gly Asp Val Gly Leu Met Val Gly Asn Pro Trp
            100                 105                 110

Glu Gly Ile Glu Arg Val Lys Leu Lys Glu Leu Val Ser Gly Val Arg
        115                 120                 125

Arg Glu Glu Val Ser Ala Gly Glu Leu Lys Lys Glu Asn Leu Lys Glu
    130                 135                 140

Leu Lys Lys Ile Leu Glu Lys Asp Leu Arg Trp Val Leu Asp Asp Asp
145                 150                 155                 160

Val Asp Val Glu Glu Phe Asp Leu Asp Lys Glu Phe Asp Pro Ala Lys
                165                 170                 175

Arg Trp Arg Asn Glu Gly Glu Ala Val Arg Val Leu Val Asp Arg Leu
            180                 185                 190

Ser Gly Arg Glu Ile Asn Glu Lys His Trp Lys Phe Val Arg Met Met

195                 200                 205

Asn Gln Ser Gly Leu Gln Phe Thr Glu Asp Gln Met Leu Lys Ile Val
    210                 215                 220

Asp Arg Leu Gly Arg Lys Gln Ser Trp Lys Gln Ala Ser Ala Val Val
225                 230                 235                 240

His Trp Val Tyr Ser Asp Lys Lys Arg Lys His Leu Arg Ser Arg Phe
                245                 250                 255

Val Tyr Thr Lys Leu Leu Ser Val Leu Gly Phe Ala Arg Arg Pro Gln
            260                 265                 270

Glu Ala Leu Gln Ile Phe Asn Gln Met Leu Gly Asp Arg Gln Leu Tyr
        275                 280                 285

Pro Asp Met Ala Ala Tyr His Cys Ile Ala Val Thr Leu Gly Gln Ala
    290                 295                 300

Gly Leu Leu Lys Glu Leu Leu Lys Val Ile Glu Arg Met Arg Gln Lys
305                 310                 315                 320

Pro Thr Lys Leu Thr Lys Asn Leu Arg Gln Lys Asn Trp Asp Pro Val
                325                 330                 335

Leu Glu Pro Asp Leu Val Val Tyr Asn Ala Ile Leu Asn Ala Cys Val
            340                 345                 350

Pro Thr Leu Gln Trp Lys Ala Val Ser Trp Val Phe Val Glu Leu Arg
        355                 360                 365

Lys Asn Gly Leu Arg Pro Asn Gly Ala Thr Tyr Gly Leu Ala Met Glu
    370                 375                 380

Val Met Leu Glu Ser Gly Lys Phe Asp Arg Val His Asp Phe Phe Arg
385                 390                 395                 400

Lys Met Lys Ser Ser Gly Glu Ala Pro Lys Ala Ile Thr Tyr Lys Val
                405                 410                 415

Leu Val Arg Ala Leu Trp Arg Glu Gly Lys Ile Glu Glu Ala Val Glu
            420                 425                 430

Ala Val Arg Asp Met Glu Gln Lys Gly Val Ile Gly Thr Gly Ser Val
        435                 440                 445

Tyr Tyr Glu Leu Ala Cys Cys Leu Cys Asn Asn Gly Arg Trp Cys Asp
    450                 455                 460

Ala Met Leu Glu Val Gly Arg Met Lys Arg Leu Glu Asn Cys Arg Pro
465                 470                 475                 480

Leu Glu Ile Thr Phe Thr Gly Leu Ile Ala Ala Ser Leu Asn Gly Gly
                485                 490                 495

His Val Asp Asp Cys Met Ala Ile Phe Gln Tyr Met Lys Asp Lys Cys
            500                 505                 510

Asp Pro Asn Ile Gly Thr Ala Asn Met Met Leu Lys Val Tyr Gly Arg
        515                 520                 525

Asn Asp Met Phe Ser Glu Ala Lys Glu Leu Phe Glu Glu Ile Val Ser
    530                 535                 540

Arg Lys Glu Thr His Leu Val Pro Asn Glu Tyr Thr Tyr Ser Phe Met
545                 550                 555                 560

Leu Glu Ala Ser Ala Arg Ser Leu Gln Trp Glu Tyr Phe Glu His Val
                565                 570                 575

Tyr Gln Thr Met Val Leu Ser Gly Tyr Gln Met Asp Gln Thr Lys His
            580                 585                 590

Ala Ser Met Leu Ile Glu Ala Ser Arg Ala Gly Lys Trp Ser Leu Leu
        595                 600                 605

Glu His Ala Phe Asp Ala Val Leu Glu Asp Gly Glu Ile Pro His Pro
    610                 615                 620

Leu Phe Phe Thr Glu Leu Leu Cys His Ala Thr Ala Lys Gly Asp Phe
625                 630                 635                 640

Gln Arg Ala Ile Thr Leu Ile Asn Thr Val Ala Leu Ala Ser Phe Gln
                645                 650                 655

Ile Ser Glu Glu Glu Trp Thr Asp Leu Phe Glu Glu His Gln Asp Trp
            660                 665                 670

Leu Thr Gln Asp Asn Leu His Lys Leu Ser Asp His Leu Ile Glu Cys
        675                 680                 685

Asp Tyr Val Ser Glu Pro Thr Val Ser Asn Leu Ser Lys Ser Leu Lys
    690                 695                 700

Ser Arg Cys Gly Ser Ser Ser Ser Ser Ala Gln Pro Leu Leu Ala Val
705                 710                 715                 720

Asp Val Thr Thr Gln Ser Gln Gly Glu Lys Pro Glu Glu Asp Leu Leu
                725                 730                 735

Leu Gln Asp Thr Thr Met Glu Asp Asp Asn Ser Ala Asn Gly Glu Ala
            740                 745                 750

Trp Glu Phe Thr Glu Thr Glu Leu Glu Thr Leu Gly Leu Glu Glu Leu
        755                 760                 765

Glu Ile Asp Asp Asp Glu Glu Ser Ser Asp Ser Asp Ser Leu Ser Val
    770                 775                 780

Tyr Asp Ile Leu Lys Glu Trp Glu Glu Ser Ser Lys Lys Glu
785                 790                 795

<210>  5
<211>  913
<212>  PRT
<213>  Arabidopsis thaliana

<400>  5

Met Ser Leu Ser His Leu Leu Arg Arg Leu Cys Thr Thr Thr Thr Thr
1               5                   10                  15

Thr Arg Ser Pro Leu Ser Ile Ser Phe Leu His Gln Arg Ile His Asn
            20                  25                  30

Ile Ser Leu Ser Pro Ala Asn Glu Asp Pro Glu Thr Thr Thr Gly Asn
        35                  40                  45

Asn Gln Asp Ser Glu Lys Tyr Pro Asn Leu Asn Pro Ile Pro Asn Asp
    50                  55                  60

Pro Ser Gln Phe Gln Ile Pro Gln Asn His Thr Pro Pro Ile Pro Tyr
65                  70                  75                  80

Pro Pro Ile Pro His Arg Thr Met Ala Phe Ser Ser Ala Glu Glu Ala
                85                  90                  95

Ala Ala Glu Arg Arg Arg Lys Arg Arg Leu Arg Ile Glu Pro Pro
            100                 105                 110

Leu His Ala Leu Arg Arg Asp Pro Ser Ala Pro Pro Pro Lys Arg Asp
        115                 120                 125

Pro Asn Ala Pro Arg Leu Pro Asp Ser Thr Ser Ala Leu Val Gly Gln
    130                 135                 140

Arg Leu Asn Leu His Asn Arg Val Gln Ser Leu Ile Arg Ala Ser Asp
145                 150                 155                 160

Leu Asp Ala Ala Ser Lys Leu Ala Arg Gln Ser Val Phe Ser Asn Thr
                165                 170                 175

Arg Pro Thr Val Phe Thr Cys Asn Ala Ile Ile Ala Ala Met Tyr Arg
            180                 185                 190

Ala Lys Arg Tyr Ser Glu Ser Ile Ser Leu Phe Gln Tyr Phe Phe Lys
        195                 200                 205

Gln Ser Asn Ile Val Pro Asn Val Val Ser Tyr Asn Gln Ile Ile Asn
    210                 215                 220

Ala His Cys Asp Glu Gly Asn Val Asp Glu Ala Leu Glu Val Tyr Arg
225                 230                 235                 240

His Ile Leu Ala Asn Ala Pro Phe Ala Pro Ser Ser Val Thr Tyr Arg
                245                 250                 255

His Leu Thr Lys Gly Leu Val Gln Ala Gly Arg Ile Gly Asp Ala Ala
            260                 265                 270

Ser Leu Leu Arg Glu Met Leu Ser Lys Gly Gln Ala Ala Asp Ser Thr
        275                 280                 285

Val Tyr Asn Asn Leu Ile Arg Gly Tyr Leu Asp Leu Gly Asp Phe Asp
    290                 295                 300

Lys Ala Val Glu Phe Phe Asp Glu Leu Lys Ser Lys Cys Thr Val Tyr
305                 310                 315                 320

Asp Gly Ile Val Asn Ala Thr Phe Met Glu Tyr Trp Phe Glu Lys Gly
                325                 330                 335

Asn Asp Lys Glu Ala Met Glu Ser Tyr Arg Ser Leu Leu Asp Lys Lys
            340                 345                 350

Phe Arg Met His Pro Pro Thr Gly Asn Val Leu Leu Glu Val Phe Leu
        355                 360                 365

Lys Phe Gly Lys Lys Asp Glu Ala Trp Ala Leu Phe Asn Glu Met Leu
    370                 375                 380

Asp Asn His Ala Pro Pro Asn Ile Leu Ser Val Asn Ser Asp Thr Val
385                 390                 395                 400

Gly Ile Met Val Asn Glu Cys Phe Lys Met Gly Glu Phe Ser Glu Ala
                405                 410                 415

Ile Asn Thr Phe Lys Lys Val Gly Ser Lys Val Thr Ser Lys Pro Phe
            420                 425                 430

Val Met Asp Tyr Leu Gly Tyr Cys Asn Ile Val Thr Arg Phe Cys Glu
        435                 440                 445

Gln Gly Met Leu Thr Glu Ala Glu Arg Phe Phe Ala Glu Gly Val Ser
    450                 455                 460

Arg Ser Leu Pro Ala Asp Ala Pro Ser His Arg Ala Met Ile Asp Ala
465                 470                 475                 480

Tyr Leu Lys Ala Glu Arg Ile Asp Asp Ala Val Lys Met Leu Asp Arg
                485                 490                 495

Met Val Asp Val Asn Leu Arg Val Val Ala Asp Phe Gly Ala Arg Val
            500                 505                 510

Phe Gly Glu Leu Ile Lys Asn Gly Lys Leu Thr Glu Ser Ala Glu Val
        515                 520                 525

Leu Thr Lys Met Gly Glu Arg Glu Pro Lys Pro Asp Pro Ser Ile Tyr
    530                 535                 540

Asp Val Val Val Arg Gly Leu Cys Asp Gly Asp Ala Leu Asp Gln Ala
545                 550                 555                 560

Lys Asp Ile Val Gly Glu Met Ile Arg His Asn Val Gly Val Thr Thr
                565                 570                 575

Val Leu Arg Glu Phe Ile Ile Glu Val Phe Glu Lys Ala Gly Arg Arg
            580                 585                 590

Glu Glu Ile Glu Lys Ile Leu Asn Ser Val Ala Arg Pro Val Arg Asn
        595                 600                 605

Ala Gly Gln Ser Gly Asn Thr Pro Pro Arg Val Pro Ala Val Phe Gly
    610                 615                 620

Thr Thr Pro Ala Ala Pro Gln Gln Pro Arg Asp Arg Ala Pro Trp Thr

625                 630                 635                 640

Ser Gln Gly Val Val His Ser Asn Ser Gly Trp Ala Asn Gly Thr Ala
                645                 650                 655

Gly Gln Thr Ala Gly Gly Ala Tyr Lys Ala Asn Asn Gly Gln Asn Pro
            660                 665                 670

Ser Trp Ser Asn Thr Ser Asp Asn Gln Gln Gln Ser Trp Ser Asn
        675                 680                 685

Gln Thr Ala Gly Gln Gln Pro Pro Ser Trp Ser Arg Gln Ala Pro Gly
    690                 695                 700

Tyr Gln Gln Gln Gln Ser Trp Ser Gln Gln Ser Gly Trp Ser Ser Pro
705                 710                 715                 720

Ser Gly His Gln Gln Ser Trp Thr Asn Gln Thr Ala Gly Gln Gln Gln
                725                 730                 735

Pro Trp Ala Asn Gln Thr Pro Gly Gln Gln Gln Trp Ala Asn Gln
            740                 745                 750

Thr Pro Gly Gln Gln Gln Gln Leu Ala Asn Gln Thr Pro Gly Gln Gln
        755                 760                 765

Gln Gln Trp Ala Asn Gln Thr Pro Gly Gln Gln Gln Gln Trp Ala Asn
    770                 775                 780

Gln Asn Asn Gly His Gln Gln Pro Trp Ala Asn Gln Asn Thr Gly His
785                 790                 795                 800

Gln Gln Ser Trp Ala Asn Gln Thr Pro Ser Gln Gln Gln Pro Trp Ala
                805                 810                 815

Asn Gln Thr Thr Gly Gln Gln Gln Gly Trp Gly Asn Gln Thr Thr Gly
            820                 825                 830

Gln Gln Gln Gln Trp Ala Asn Gln Thr Ala Gly Gln Gln Ser Gly Trp
        835                 840                 845

Thr Ala Gln Gln Gln Trp Ser Asn Gln Thr Ala Ser His Gln Gln Ser
    850                 855                 860

Gln Trp Leu Asn Pro Val Pro Gly Glu Val Ala Asn Gln Thr Pro Trp
865                 870                 875                 880

Ser Asn Ser Val Asp Ser His Leu Pro Gln Gln Gln Glu Pro Gly Pro
                885                 890                 895

Ser His Glu Cys Gln Glu Thr Gln Glu Lys Lys Val Val Glu Leu Arg
            900                 905                 910

Asn

<210>  6
<211>  196
<212>  PRT
<213>  Flabovacterium okeianocoites

<400>  6

Ala Leu Val Lys Ser Glu Leu Glu Glu Lys Lys Ser Glu Leu Arg His
1               5                   10                  15

Lys Leu Lys Tyr Val Pro His Glu Tyr Ile Glu Leu Ile Glu Ile Ala
            20                  25                  30

Arg Asn Ser Thr Gln Asp Arg Ile Leu Glu Met Lys Val Met Glu Phe
        35                  40                  45

Phe Met Lys Val Tyr Gly Tyr Arg Gly Lys His Leu Gly Gly Ser Arg
    50                  55                  60

Lys Pro Asp Gly Ala Ile Tyr Thr Val Gly Ser Pro Ile Asp Tyr Gly
65                  70                  75                  80

Val Ile Val Asp Thr Lys Ala Tyr Ser Gly Gly Tyr Asn Leu Pro Ile
                85                  90                  95

Gly Gln Ala Asp Glu Met Gln Arg Tyr Val Glu Glu Asn Gln Thr Arg
            100                 105                 110

Asn Lys His Ile Asn Pro Asn Glu Trp Trp Lys Val Tyr Pro Ser Ser
        115                 120                 125

Val Thr Glu Phe Lys Phe Leu Phe Val Ser Gly His Phe Lys Gly Asn
    130                 135                 140

Tyr Lys Ala Gln Leu Thr Arg Leu Asn His Ile Thr Asn Cys Asn Gly
145                 150                 155                 160

Ala Val Leu Ser Val Glu Glu Leu Leu Ile Gly Gly Glu Met Ile Lys
                165                 170                 175

Ala Gly Thr Leu Thr Leu Glu Glu Val Arg Arg Lys Phe Asn Asn Gly
            180                 185                 190

Glu Ile Asn Phe
        195

<210>  7
<211>  35
<212>  PRT
<213>  Arabidopsis thaliana

<400>  7

Asn Val Tyr Ile Cys Asn Ser Ile Leu Ser Cys Leu Val Lys Asn Gly
1               5                   10                  15

Lys Leu Asp Ser Cys Ile Lys Leu Phe Asp Gln Met Lys Arg Asp Gly
            20                  25                  30

Leu Lys Pro
        35

<210>  8
<211>  36
<212>  PRT
<213>  Arabidopsis thaliana

<400>  8

Asp Val Val Thr Tyr Asn Thr Leu Leu Ala Gly Cys Ile Lys Val Lys
1               5                   10                  15

Asn Gly Tyr Pro Lys Ala Ile Glu Leu Ile Gly Glu Leu Pro His Asn
            20                  25                  30

Gly Ile Gln Met
        35

<210>  9
<211>  35
<212>  PRT
<213>  Arabidopsis thaliana

<400>  9

Asp Ser Val Met Tyr Gly Thr Val Leu Ala Ile Cys Ala Ser Asn Gly
1               5                   10                  15

Arg Ser Glu Glu Ala Glu Asn Phe Ile Gln Gln Met Lys Val Glu Gly
            20                  25                  30

His Ser Pro
        35

<210>  10
<211>  35
<212>  PRT
<213>  Arabidopsis thaliana

<400>  10

Asn Ile Tyr His Tyr Ser Ser Leu Leu Asn Ser Tyr Ser Trp Lys Gly
1               5                   10                  15

Asp Tyr Lys Lys Ala Asp Glu Leu Met Thr Glu Met Lys Ser Ile Gly
            20                  25                  30

Leu Val Pro
        35

<210>  11
<211>  35
<212>  PRT
<213>  Arabidopsis thaliana

<400>  11

Asn Lys Val Met Met Thr Thr Leu Leu Lys Val Tyr Ile Lys Gly Gly
1               5                   10                  15

Leu Phe Asp Arg Ser Arg Glu Leu Leu Ser Glu Leu Glu Ser Ala Gly
            20                  25                  30

Tyr Ala Glu
        35

<210>  12
<211>  35
<212>  PRT
<213>  Arabidopsis thaliana

<400>  12

Asn Glu Met Pro Tyr Cys Met Leu Met Asp Gly Leu Ser Lys Ala Gly
1               5                   10                  15

Lys Leu Glu Glu Ala Arg Ser Ile Phe Asp Asp Met Lys Gly Lys Gly
            20                  25                  30

Val Arg Ser
        35

<210>  13
<211>  31
<212>  PRT
<213>  Arabidopsis thaliana

<400>  13

Asp Gly Tyr Ala Asn Ser Ile Met Ile Ser Ala Leu Cys Arg Ser Lys
1               5                   10                  15

Arg Phe Lys Glu Ala Lys Glu Leu Ser Arg Asp Ser Glu Thr Thr
            20                  25                  30

<210>  14
<211>  35
<212>  PRT
<213>  Arabidopsis thaliana

<400>  14

Asp Leu Val Met Leu Asn Thr Met Leu Cys Ala Tyr Cys Arg Ala Gly
1               5                   10                  15

Glu Met Glu Ser Val Met Arg Met Met Lys Lys Met Asp Glu Gln Ala
            20                  25                  30

Val Ser Pro
        35

<210>  15
<211>  35
<212>  PRT
<213>  Arabidopsis thaliana

<400>  15

Asp Tyr Asn Thr Phe His Ile Leu Ile Lys Tyr Phe Ile Lys Glu Lys
1               5                   10                  15

Leu His Leu Leu Ala Tyr Gln Thr Thr Leu Asp Met His Ser Lys Gly
            20                  25                  30

His Arg Leu
        35

<210>  16
<211>  36
<212>  PRT
<213>  Arabidopsis thaliana

<400>  16

Asp Val Asn Leu Tyr Asn His Tyr Leu Arg Ala Asn Leu Met Met Gly
1               5                   10                  15

Ala Ser Ala Gly Asp Met Leu Asp Leu Val Ala Pro Met Glu Glu Phe
            20                  25                  30

Ser Val Glu Pro
        35

<210>  17
<211>  35
<212>  PRT
<213>  Arabidopsis thaliana

<400>  17

Asn Thr Ala Ser Tyr Asn Leu Val Leu Lys Ala Met Tyr Gln Ala Arg
1               5                   10                  15

Glu Thr Glu Ala Ala Met Lys Leu Leu Glu Arg Met Leu Leu Leu Gly
            20                  25                  30

Lys Asp Ser
        35

<210>  18
<211>  35
<212>  PRT
<213>  Arabidopsis thaliana

<400>  18

Asp Asp Glu Ser Tyr Asp Leu Val Ile Gly Met His Phe Gly Val Gly
1               5                   10                  15

Lys Asn Asp Glu Ala Met Lys Val Met Asp Thr Ala Leu Lys Ser Gly
            20                  25                  30

Tyr Met Leu
        35

<210>  19
<211>  36
<212>  PRT
<213>  Arabidopsis thaliana

<400>  19

Ser Val Ala Ala Leu Asn Cys Ile Ile Leu Gly Cys Ala Asn Thr Trp
1               5                   10                  15

Asp Leu Asp Arg Ala Tyr Gln Thr Phe Glu Ala Ile Ser Ala Ser Phe
            20                  25                  30

Gly Leu Thr Pro
        35

<210>  20

<211>  35
<212>  PRT
<213>  Arabidopsis thaliana

<400>  20

Asn Ile Asp Ser Tyr Asn Ala Leu Leu Tyr Ala Phe Gly Lys Val Lys
1               5                   10                  15

Lys Thr Phe Glu Ala Thr Asn Val Phe Glu His Leu Val Ser Ile Gly
            20                  25                  30

Val Lys Pro
        35

<210>  21
<211>  35
<212>  PRT
<213>  Arabidopsis thaliana

<400>  21

Asp Ser Arg Thr Tyr Ser Leu Leu Val Asp Ala His Leu Ile Asn Arg
1               5                   10                  15

Asp Pro Lys Ser Ala Leu Thr Val Val Asp Asp Met Ile Lys Ala Gly
            20                  25                  30

Phe Glu Pro
        35

<210>  22
<211>  35
<212>  PRT
<213>  Arabidopsis thaliana

<400>  22

Gly Glu Val Val Tyr Arg Thr Leu Leu Ala Asn Cys Val Leu Lys His
1               5                   10                  15

His Val Asn Lys Ala Glu Asp Ile Phe Asn Lys Met Lys Glu Leu Lys
            20                  25                  30

Phe Pro Thr
        35

<210>  23
<211>  34
<212>  PRT
<213>  Arabidopsis thaliana

<400>  23

Ser Val Phe Ala Cys Asn Gln Leu Leu Leu Leu Tyr Ser Met His Asp
1               5                   10                  15

Arg Lys Lys Ile Ser Asp Val Leu Leu Leu Met Glu Arg Glu Asn Ile
            20                  25                  30

Lys Pro

<210>  24
<211>  35
<212>  PRT
<213>  Arabidopsis thaliana

<400>  24

Ser Arg Ala Thr Tyr His Phe Leu Ile Asn Ser Lys Gly Leu Ala Gly
1               5                   10                  15

Asp Ile Thr Gly Met Glu Lys Ile Val Glu Thr Ile Lys Glu Glu Gly
            20                  25                  30

Ile Glu Leu
        35

<210>  25
<211>  35
<212>  PRT
<213>  Arabidopsis thaliana

<400>  25

Asp Pro Glu Leu Gln Ser Ile Leu Ala Lys Tyr Tyr Ile Arg Ala Gly
1               5                   10                  15

Leu Lys Glu Arg Ala Gln Asp Leu Met Lys Glu Ile Glu Gly Lys Gly
            20                  25                  30

Leu Gln Gln
        35

<210>  26
<211>  31
<212>  PRT
<213>  Arabidopsis thaliana

<400>  26

Thr Pro Trp Val Cys Arg Ser Leu Leu Pro Leu Tyr Ala Asp Ile Gly
1               5                   10                  15

Asp Ser Asp Asn Val Arg Arg Leu Ser Arg Phe Val Asp Gln Asn
            20                  25                  30

<210>  27
<211>  31
<212>  PRT
<213>  Arabidopsis thaliana

<400>  27

Arg Tyr Asp Asn Cys Ile Ser Ala Ile Lys Ala Trp Gly Lys Leu Lys
1               5                   10                  15

Glu Val Glu Glu Ala Glu Ala Val Phe Glu Arg Leu Val Glu Lys
            20                  25                  30

<210>  28
<211>  35
<212>  PRT
<213>  Arabidopsis thaliana

<400>  28

Pro Met Met Pro Tyr Phe Ala Leu Met Glu Ile Tyr Thr Glu Asn Lys
1               5                   10                  15

Met Leu Ala Lys Gly Arg Asp Leu Val Lys Arg Met Gly Asn Ala Gly
            20                  25                  30

Ile Ala Ile
        35

<210>  29
<211>  36
<212>  PRT
<213>  Arabidopsis thaliana

<400>  29

Gly Pro Ser Thr Trp His Ala Leu Val Lys Leu Tyr Ile Lys Ala Gly
1               5                   10                  15

Glu Val Gly Lys Ala Glu Leu Ile Leu Asn Arg Ala Thr Lys Asp Asn
            20                  25                  30

Lys Met Arg Pro
        35

<210>  30
<211>  35
<212>  PRT
<213>  Arabidopsis thaliana

<400>  30

Met Phe Thr Thr Tyr Met Ala Ile Leu Glu Glu Tyr Ala Lys Arg Gly

1               5                   10                  15

Asp Val His Asn Thr Glu Lys Val Phe Met Lys Met Lys Arg Ala Ser
            20                  25                  30

Tyr Ala Ala
        35

<210>  31
<211>  35
<212>  PRT
<213>  Arabidopsis thaliana

<400>  31

Ser Glu Ile Asp Phe Leu Met Leu Ile Thr Ala Tyr Gly Lys Leu Gly
1               5                   10                  15

Asn Phe Asn Gly Ala Glu Arg Val Leu Ser Val Leu Ser Lys Met Gly
            20                  25                  30

Ser Thr Pro
        35

<210>  32
<211>  35
<212>  PRT
<213>  Arabidopsis thaliana

<400>  32

Asn Val Ile Ser Tyr Thr Ala Leu Met Glu Ser Tyr Gly Arg Gly Gly
1               5                   10                  15

Lys Cys Asn Asn Ala Glu Ala Ile Phe Arg Arg Met Gln Ser Ser Gly
            20                  25                  30

Pro Glu Pro
        35

<210>  33
<211>  35
<212>  PRT
<213>  Arabidopsis thaliana

<400>  33

Ser Ala Ile Thr Tyr Gln Ile Ile Leu Lys Thr Phe Val Glu Gly Asp
1               5                   10                  15

Lys Phe Lys Glu Ala Glu Glu Val Phe Glu Thr Leu Leu Asp Glu Lys
            20                  25                  30

Lys Ser Pro
        35

<210>  34
<211>  35
<212>  PRT
<213>  Arabidopsis thaliana

<400>  34

Asp Gln Lys Met Tyr His Met Met Ile Tyr Met Tyr Lys Lys Ala Gly
1               5                   10                  15

Asn Tyr Glu Lys Ala Arg Lys Val Phe Ser Ser Met Val Gly Lys Gly
            20                  25                  30

Val Pro Gln
        35

<210>  35
<211>  32
<212>  PRT
<213>  Arabidopsis thaliana

<400>  35

Ser Thr Val Thr Tyr Asn Ser Leu Met Ser Phe Glu Thr Ser Tyr Lys
1               5                   10                  15

Glu Val Ser Lys Ile Tyr Asp Gln Met Gln Arg Ser Asp Ile Gln Pro
            20                  25                  30

<210>  36
<211>  35
<212>  PRT
<213>  Arabidopsis thaliana

<400>  36

Asp Val Val Ser Tyr Ala Leu Leu Ile Lys Ala Tyr Gly Arg Ala Arg
1               5                   10                  15

Arg Glu Glu Glu Ala Leu Ser Val Phe Glu Glu Met Leu Asp Ala Gly
            20                  25                  30

Val Arg Pro
        35

<210>  37
<211>  35
<212>  PRT
<213>  Arabidopsis thaliana

<400>  37

Thr His Lys Ala Tyr Asn Ile Leu Leu Asp Ala Phe Ala Ile Ser Gly
1               5                   10                  15

Met Val Glu Gln Ala Lys Thr Val Phe Lys Ser Met Arg Arg Asp Arg
            20                  25                  30

Ile Phe Pro
        35

<210>  38
<211>  35
<212>  PRT
<213>  Arabidopsis thaliana

<400>  38

Asp Leu Trp Ser Tyr Thr Thr Met Leu Ser Ala Tyr Val Asn Ala Ser
1               5                   10                  15

Asp Met Glu Gly Ala Glu Lys Phe Phe Lys Arg Ile Lys Val Asp Gly
            20                  25                  30

Phe Glu Pro
        35

<210>  39
<211>  35
<212>  PRT
<213>  Arabidopsis thaliana

<400>  39

Asn Ile Val Thr Tyr Gly Thr Leu Ile Lys Gly Tyr Ala Lys Ala Asn
1               5                   10                  15

Asp Val Glu Lys Met Met Glu Val Tyr Glu Lys Met Arg Leu Ser Gly
            20                  25                  30

Ile Lys Ala
        35

<210>  40
<211>  35
<212>  PRT
<213>  Arabidopsis thaliana

<400>  40

Asn Gln Thr Ile Leu Thr Thr Ile Met Asp Ala Ser Gly Arg Cys Lys
1               5                   10                  15

Asn Phe Gly Ser Ala Leu Gly Trp Tyr Lys Glu Met Glu Ser Cys Gly
                20                  25                  30

Val Pro Pro
        35

<210>  41
<211>  35
<212>  PRT
<213>  Arabidopsis thaliana

<400>  41

Asn Thr Ile Val Met Asn Ser Val Leu Glu Ala Cys Val His Cys Gly
1               5                   10                  15

Asn Ile Asp Leu Ala Leu Arg Met Phe His Glu Met Ala Glu Pro Gly
                20                  25                  30

Gly Ile Gly
        35

<210>  42
<211>  31
<212>  PRT
<213>  Arabidopsis thaliana

<400>  42

Asp Ser Ile Ser Tyr Ala Thr Ile Leu Lys Gly Leu Gly Lys Ala Arg
1               5                   10                  15

Arg Ile Asp Glu Ala Phe Gln Met Leu Glu Thr Ile Glu Tyr Gly
                20                  25                  30

<210>  43
<211>  31
<212>  PRT
<213>  Arabidopsis thaliana

<400>  43

Ser Ser Ser Leu Ile Tyr Gly Leu Leu Asp Ala Leu Ile Asn Ala Gly
1               5                   10                  15

Asp Leu Arg Arg Ala Asn Gly Leu Leu Ala Arg Tyr Asp Ile Leu
                20                  25                  30

<210>  44
<211>  35
<212>  PRT
<213>  Arabidopsis thaliana

<400>  44

Ser Val Leu Ile Tyr Asn Leu Leu Met Lys Gly Tyr Val Asn Ser Glu
1               5                   10                  15

Ser Pro Gln Ala Ala Ile Asn Leu Leu Asp Glu Met Leu Arg Leu Arg
            20                  25                  30

Leu Glu Pro
        35

<210>  45
<211>  31
<212>  PRT
<213>  Arabidopsis thaliana

<400>  45

Asp Arg Leu Thr Tyr Asn Thr Leu Ile His Ala Cys Ile Lys Cys Gly
1               5                   10                  15

Asp Leu Asp Ala Ala Met Lys Phe Phe Asn Asp Met Lys Glu Lys
            20                  25                  30

<210>  46
<211>  31
<212>  PRT
<213>  Arabidopsis thaliana

<400>  46

Asp Val Val Thr Tyr Thr Thr Leu Val Lys Gly Phe Gly Asp Ala Thr
1               5                   10                  15

Asp Leu Leu Ser Leu Gln Glu Ile Phe Leu Glu Met Lys Leu Cys
            20                  25                  30

<210>  47
<211>  36
<212>  PRT
<213>  Arabidopsis thaliana

<400>  47

Asp Arg Thr Ala Phe Thr Ala Val Val Asp Ala Met Leu Lys Cys Gly
1               5                   10                  15

Ser Thr Ser Gly Ala Leu Cys Val Phe Gly Glu Ile Leu Lys Arg Ser
            20                  25                  30

Gly Ala Asn Glu
        35

<210>  48

<211>  35
<212>  PRT
<213>  Arabidopsis thaliana

<400>  48

Lys Pro His Leu Tyr Leu Ser Met Met Arg Ala Phe Ala Val Gln Gly
1               5                   10                  15

Asp Tyr Gly Met Val Arg Asn Leu Tyr Leu Arg Leu Trp Pro Asp Ser
            20                  25                  30

Ser Gly Ser
        35

<210>  49
<211>  36
<212>  PRT
<213>  Arabidopsis thaliana

<400>  49

Gln Gln Glu Ala Asp Asn Leu Leu Met Glu Ala Ala Leu Asn Asp Gly
1               5                   10                  15

Gln Leu Asp Glu Ala Leu Gly Ile Leu Leu Ser Ile Val Arg Arg Trp
            20                  25                  30

Lys Thr Ile Pro
        35

<210>  50
<211>  35
<212>  PRT
<213>  Arabidopsis thaliana

<400>  50

Cys Leu Ser Ile His Ser Ser Ile Met Arg Asp Leu Cys Leu Gln Gly
1               5                   10                  15

Lys Leu Asp Ala Ala Leu Trp Leu Arg Lys Lys Met Ile Tyr Ser Gly
            20                  25                  30

Val Ile Pro
        35

<210>  51
<211>  35
<212>  PRT
<213>  Arabidopsis thaliana

<400>  51

Gly Leu Ile Thr His Asn His Leu Leu Asn Gly Leu Cys Lys Ala Gly
1               5                   10                  15

Tyr Ile Glu Lys Ala Asp Gly Leu Val Arg Glu Met Arg Glu Met Gly
            20                  25                  30

Pro Ser Pro
        35

<210>  52
<211>  35
<212>  PRT
<213>  Arabidopsis thaliana

<400>  52

Asn Cys Val Ser Tyr Asn Thr Leu Ile Lys Gly Leu Cys Ser Val Asn
1               5                   10                  15

Asn Val Asp Lys Ala Leu Tyr Leu Phe Asn Thr Met Asn Lys Tyr Gly
            20                  25                  30

Ile Arg Pro
        35

<210>  53
<211>  32
<212>  PRT
<213>  Arabidopsis thaliana

<400>  53

Asn Arg Val Thr Cys Asn Ile Ile Val His Ala Leu Cys Gln Lys Gly
1               5                   10                  15

Val Ile Gly Asn Asn Asn Lys Lys Leu Leu Glu Glu Ile Leu Asp Ser
            20                  25                  30

<210>  54
<211>  35
<212>  PRT
<213>  Arabidopsis thaliana

<400>  54

Asp Ile Val Ile Cys Thr Ile Leu Met Asp Ser Cys Phe Lys Asn Gly
1               5                   10                  15

Asn Val Val Gln Ala Leu Glu Val Trp Lys Glu Met Ser Gln Lys Asn
            20                  25                  30

Val Pro Ala
        35

<210>  55
<211>  35
<212>  PRT
<213>  Arabidopsis thaliana

<400>  55

Asp Ser Val Val Tyr Asn Val Ile Ile Arg Gly Leu Cys Ser Ser Gly
1               5                   10                  15

Asn Met Val Ala Ala Tyr Gly Phe Met Cys Asp Met Val Lys Arg Gly
            20                  25                  30

Val Asn Pro
        35

<210>  56
<211>  35
<212>  PRT
<213>  Arabidopsis thaliana

<400>  56

Asp Val Phe Thr Tyr Asn Thr Leu Ile Ser Ala Leu Cys Lys Glu Gly
1               5                   10                  15

Lys Phe Asp Glu Ala Cys Asp Leu His Gly Thr Met Gln Asn Gly Gly
            20                  25                  30

Val Ala Pro
        35

<210>  57
<211>  35
<212>  PRT
<213>  Arabidopsis thaliana

<400>  57

Asp Gln Ile Ser Tyr Lys Val Ile Ile Gln Gly Leu Cys Ile His Gly
1               5                   10                  15

Asp Val Asn Arg Ala Asn Glu Phe Leu Leu Ser Met Leu Lys Ser Ser
            20                  25                  30

Leu Leu Pro
        35

<210>  58
<211>  35
<212>  PRT
<213>  Arabidopsis thaliana

<400>  58

Glu Val Leu Leu Trp Asn Val Val Ile Asp Gly Tyr Gly Arg Tyr Gly
1               5                   10                  15

Asp Thr Ser Ser Ala Leu Ser Val Leu Asn Leu Met Leu Ser Tyr Gly
            20                  25                  30

Val Lys Pro
        35

<210>  59
<211>  35
<212>  PRT
<213>  Arabidopsis thaliana

<400>  59

Asn Val Tyr Thr Asn Asn Ala Leu Ile His Gly Tyr Val Lys Gly Gly
1               5                   10                  15

Arg Leu Ile Asp Ala Trp Trp Val Lys Asn Glu Met Arg Ser Thr Lys
            20                  25                  30

Ile His Pro
        35

<210>  60
<211>  35
<212>  PRT
<213>  Arabidopsis thaliana

<400>  60

Asp Thr Thr Thr Tyr Asn Leu Leu Leu Gly Ala Ala Cys Thr Leu Gly
1               5                   10                  15

His Leu Arg Leu Ala Phe Gln Leu Tyr Asp Glu Met Leu Arg Arg Gly
            20                  25                  30

Cys Gln Pro
        35

<210>  61
<211>  35
<212>  PRT
<213>  Arabidopsis thaliana

<400>  61

Asp Ile Ile Thr Tyr Thr Glu Leu Val Arg Gly Leu Cys Trp Lys Gly
1               5                   10                  15

Arg Leu Lys Lys Ala Glu Ser Leu Leu Ser Arg Ile Gln Ala Thr Gly
            20                  25                  30

Ile Thr Ile
        35

<210>  62
<211>  31
<212>  PRT
<213>  Arabidopsis thaliana

<400>  62

Ser Arg Phe Val Tyr Thr Lys Leu Leu Ser Val Leu Gly Phe Ala Arg
1               5                   10                  15

Arg Pro Gln Glu Ala Leu Gln Ile Phe Asn Gln Met Leu Gly Asp
            20                  25                  30

<210>  63
<211>  35
<212>  PRT
<213>  Arabidopsis thaliana

<400>  63

Asp Met Ala Ala Tyr His Cys Ile Ala Val Thr Leu Gly Gln Ala Gly
1               5                   10                  15

Leu Leu Lys Glu Leu Leu Lys Val Ile Glu Arg Met Arg Gln Lys Pro
            20                  25                  30

Thr Lys Leu
        35

<210>  64
<211>  35
<212>  PRT
<213>  Arabidopsis thaliana

<400>  64

Asp Leu Val Val Tyr Asn Ala Ile Leu Asn Ala Cys Val Pro Thr Leu
1               5                   10                  15

Gln Trp Lys Ala Val Ser Trp Val Phe Val Glu Leu Arg Lys Asn Gly
            20                  25                  30

Leu Arg Pro
        35

<210>  65

<211>  35
<212>  PRT
<213>  Arabidopsis thaliana

<400>  65

Asn Gly Ala Thr Tyr Gly Leu Ala Met Glu Val Met Leu Glu Ser Gly
1               5                   10                  15

Lys Phe Asp Arg Val His Asp Phe Phe Arg Lys Met Lys Ser Ser Gly
            20                  25                  30

Glu Ala Pro
        35

<210>  66
<211>  35
<212>  PRT
<213>  Arabidopsis thaliana

<400>  66

Lys Ala Ile Thr Tyr Lys Val Leu Val Arg Ala Leu Trp Arg Glu Gly
1               5                   10                  15

Lys Ile Glu Glu Ala Val Glu Ala Val Arg Asp Met Glu Gln Lys Gly
            20                  25                  30

Val Ile Gly
        35

<210>  67
<211>  36
<212>  PRT
<213>  Arabidopsis thaliana

<400>  67

Thr Gly Ser Val Tyr Tyr Glu Leu Ala Cys Cys Leu Cys Asn Asn Gly
1               5                   10                  15

Arg Trp Cys Asp Ala Met Leu Glu Val Gly Arg Met Lys Arg Leu Glu
            20                  25                  30

Asn Cys Arg Pro
        35

<210>  68
<211>  31
<212>  PRT
<213>  Arabidopsis thaliana

<400>  68

Leu Glu Ile Thr Phe Thr Gly Leu Ile Ala Ala Ser Leu Asn Gly Gly
1               5                   10                  15

His Val Asp Asp Cys Met Ala Ile Phe Gln Tyr Met Lys Asp Lys
            20                  25                  30

<210>  69
<211>  31
<212>  PRT
<213>  Arabidopsis thaliana

<400>  69

Asn Ile Gly Thr Ala Asn Met Met Leu Lys Val Tyr Gly Arg Asn Asp
1               5                   10                  15

Met Phe Ser Glu Ala Lys Glu Leu Phe Glu Glu Ile Val Ser Arg
            20                  25                  30

<210>  70
<211>  35
<212>  PRT
<213>  Arabidopsis thaliana

<400>  70

Asn Glu Tyr Thr Tyr Ser Phe Met Leu Glu Ala Ser Ala Arg Ser Leu
1               5                   10                  15

Gln Trp Glu Tyr Phe Glu His Val Tyr Gln Thr Met Val Leu Ser Gly
            20                  25                  30

Tyr Gln Met
        35

<210>  71
<211>  35
<212>  PRT
<213>  Arabidopsis thaliana

<400>  71

Asp Gln Thr Lys His Ala Ser Met Leu Ile Glu Ala Ser Arg Ala Gly
1               5                   10                  15

Lys Trp Ser Leu Leu Glu His Ala Phe Asp Ala Val Leu Glu Asp Gly
            20                  25                  30

Glu Ile Pro
        35

<210>  72
<211>  35

<212>  PRT
<213>  Arabidopsis thaliana

<400>  72

Gln Ile Val Asp Tyr Ala Pro Leu Val Gln Thr Leu Ser Gln Arg Arg
1               5                   10                  15

Leu Pro Asp Val Ala His Glu Ile Phe Leu Gln Thr Lys Ser Val Asn
            20                  25                  30

Leu Leu Pro
        35

<210>  73
<211>  35
<212>  PRT
<213>  Arabidopsis thaliana

<400>  73

Asn Tyr Arg Thr Leu Cys Ala Leu Met Leu Cys Phe Ala Glu Asn Gly
1               5                   10                  15

Phe Val Leu Arg Ala Arg Thr Ile Trp Asp Glu Ile Ile Asn Ser Cys
            20                  25                  30

Phe Val Pro
        35

<210>  74
<211>  35
<212>  PRT
<213>  Arabidopsis thaliana

<400>  74

Asp Val Phe Val Val Ser Lys Leu Ile Ser Ala Tyr Glu Gln Phe Gly
1               5                   10                  15

Cys Phe Asp Glu Val Ala Lys Ile Thr Lys Asp Val Ala Ala Arg His
            20                  25                  30

Ser Lys Leu
        35

<210>  75
<211>  35
<212>  PRT
<213>  Arabidopsis thaliana

<400>  75

Leu Pro Val Val Ser Ser Leu Ala Ile Ser Cys Phe Gly Lys Asn Gly

1               5                   10                  15

Gln Leu Glu Leu Met Glu Gly Val Ile Glu Glu Met Asp Ser Lys Gly
            20                  25                  30

Val Leu Leu
        35

<210>  76
<211>  35
<212>  PRT
<213>  Arabidopsis thaliana

<400>  76

Glu Ala Glu Thr Ala Asn Val Ile Val Arg Tyr Tyr Ser Phe Phe Gly
1               5                   10                  15

Ser Leu Asp Lys Met Glu Lys Ala Tyr Gly Arg Val Lys Lys Phe Gly
            20                  25                  30

Ile Val Ile
        35

<210>  77
<211>  35
<212>  PRT
<213>  Arabidopsis thaliana

<400>  77

Glu Glu Glu Glu Ile Arg Ala Val Val Leu Ala Tyr Leu Lys Gln Arg
1               5                   10                  15

Lys Phe Tyr Arg Leu Arg Glu Phe Leu Ser Asp Val Gly Leu Gly Arg
            20                  25                  30

Arg Asn Leu
        35

<210>  78
<211>  35
<212>  PRT
<213>  Arabidopsis thaliana

<400>  78

Gly Asn Met Leu Trp Asn Ser Val Leu Leu Ser Tyr Ala Ala Asp Phe
1               5                   10                  15

Lys Met Lys Ser Leu Gln Arg Glu Phe Ile Gly Met Leu Asp Ala Gly
            20                  25                  30

Phe Ser Pro
        35

<210>  79
<211>  35
<212>  PRT
<213>  Arabidopsis thaliana

<400>  79

Asp Leu Thr Thr Phe Asn Ile Arg Ala Leu Ala Phe Ser Arg Met Ala
1               5                   10                  15

Leu Phe Trp Asp Leu His Leu Thr Leu Glu His Met Arg Arg Leu Asn
            20                  25                  30

Ile Val Pro
        35

<210>  80
<211>  35
<212>  PRT
<213>  Arabidopsis thaliana

<400>  80

Asp Leu Val Thr Phe Gly Cys Val Val Asp Ala Tyr Met Asp Lys Arg
1               5                   10                  15

Leu Ala Arg Asn Leu Glu Phe Val Tyr Asn Arg Met Asn Leu Asp Asp
            20                  25                  30

Ser Pro Leu
        35

<210>  81
<211>  35
<212>  PRT
<213>  Arabidopsis thaliana

<400>  81

Thr Pro Leu Thr Tyr Asn Ala Leu Ile Gly Ala Cys Ala Arg Asn Asn
1               5                   10                  15

Asp Ile Glu Lys Ala Leu Asn Leu Ile Ala Lys Met Arg Gln Asp Gly
            20                  25                  30

Tyr Gln Ser
        35

<210>  82

<211>  37
<212>  PRT
<213>  Arabidopsis thaliana

<400>  82

Asp Phe Val Asn Tyr Ser Leu Val Ile Gln Ser Leu Thr Arg Ser Asn
1               5                   10                  15

Lys Ile Asp Ser Val Met Leu Leu Arg Leu Tyr Lys Glu Ile Glu Arg
            20                  25                  30

Asp Lys Leu Glu Leu
        35

<210>  83
<211>  35
<212>  PRT
<213>  Arabidopsis thaliana

<400>  83

Asp Val Gln Leu Val Asn Asp Ile Ile Met Gly Phe Ala Lys Ser Gly
1               5                   10                  15

Asp Pro Ser Lys Ala Leu Gln Leu Leu Gly Met Ala Gln Ala Thr Gly
            20                  25                  30

Leu Ser Ala
        35

<210>  84
<211>  35
<212>  PRT
<213>  Arabidopsis thaliana

<400>  84

Lys Thr Ala Thr Leu Val Ser Ile Ile Ser Ala Leu Ala Asp Ser Gly
1               5                   10                  15

Arg Thr Leu Glu Ala Glu Ala Leu Phe Glu Glu Leu Arg Gln Ser Gly
            20                  25                  30

Ile Lys Pro
        35

<210>  85
<211>  35
<212>  PRT
<213>  Arabidopsis thaliana

<400>  85

Arg Thr Arg Ala Tyr Asn Ala Leu Leu Lys Gly Tyr Val Lys Thr Gly
1               5                   10                  15

Pro Leu Lys Asp Ala Glu Ser Met Val Ser Glu Met Glu Lys Arg Gly
            20                  25                  30

Val Ser Pro
        35

<210>  86
<211>  35
<212>  PRT
<213>  Arabidopsis thaliana

<400>  86

Asp Glu His Thr Tyr Ser Leu Leu Ile Asp Ala Tyr Val Asn Ala Gly
1               5                   10                  15

Arg Trp Glu Ser Ala Arg Ile Val Leu Lys Glu Met Glu Ala Gly Asp
            20                  25                  30

Val Gln Pro
        35

<210>  87
<211>  35
<212>  PRT
<213>  Arabidopsis thaliana

<400>  87

Asn Ser Phe Val Phe Ser Arg Leu Leu Ala Gly Phe Arg Asp Arg Gly
1               5                   10                  15

Glu Trp Gln Lys Thr Phe Gln Val Leu Lys Glu Met Lys Ser Ile Gly
            20                  25                  30

Val Lys Pro
        35

<210>  88
<211>  35
<212>  PRT
<213>  Arabidopsis thaliana

<400>  88

Asp Arg Gln Phe Tyr Asn Val Val Ile Asp Thr Phe Gly Lys Phe Asn
1               5                   10                  15

Cys Leu Asp His Ala Met Thr Thr Phe Asp Arg Met Leu Ser Glu Gly
            20                  25                  30

Ile Glu Pro
        35

<210>  89
<211>  35
<212>  PRT
<213>  Arabidopsis thaliana

<400>  89

Asp Arg Val Thr Trp Asn Thr Leu Ile Asp Cys His Cys Lys His Gly
1               5                   10                  15

Arg His Ile Val Ala Glu Glu Met Phe Glu Ala Met Glu Arg Arg Gly
            20                  25                  30

Cys Leu Pro
        35

<210>  90
<211>  35
<212>  PRT
<213>  Arabidopsis thaliana

<400>  90

Cys Ala Thr Thr Tyr Asn Ile Met Ile Asn Ser Tyr Gly Asp Gln Glu
1               5                   10                  15

Arg Trp Asp Asp Met Lys Arg Leu Leu Gly Lys Met Lys Ser Gln Gly
            20                  25                  30

Ile Leu Pro
        35

<210>  91
<211>  35
<212>  PRT
<213>  Arabidopsis thaliana

<400>  91

Asn Val Val Thr His Thr Thr Leu Val Asp Val Tyr Gly Lys Ser Gly
1               5                   10                  15

Arg Phe Asn Asp Ala Ile Glu Cys Leu Glu Glu Met Lys Ser Val Gly
            20                  25                  30

Leu Lys Pro
        35

<210>  92
<211>  35
<212>  PRT
<213>  Arabidopsis thaliana

<400>  92

Ser Ser Thr Met Tyr Asn Ala Leu Ile Asn Ala Tyr Ala Gln Arg Gly
1               5                   10                  15

Leu Ser Glu Gln Ala Val Asn Ala Phe Arg Val Met Thr Ser Asp Gly
            20                  25                  30

Leu Lys Pro
        35

<210>  93
<211>  35
<212>  PRT
<213>  Arabidopsis thaliana

<400>  93

Ser Leu Leu Ala Leu Asn Ser Leu Ile Asn Ala Phe Gly Glu Asp Arg
1               5                   10                  15

Arg Asp Ala Glu Ala Phe Ala Val Leu Gln Tyr Met Lys Glu Asn Gly
            20                  25                  30

Val Lys Pro
        35

<210>  94
<211>  35
<212>  PRT
<213>  Arabidopsis thaliana

<400>  94

Asp Val Val Thr Tyr Thr Thr Leu Met Lys Ala Leu Ile Arg Val Asp
1               5                   10                  15

Lys Phe Gln Lys Val Pro Val Val Tyr Glu Glu Met Ile Met Ser Gly
            20                  25                  30

Cys Lys Pro
        35

<210>  95
<211>  35
<212>  PRT
<213>  Arabidopsis thaliana

<400>  95

Ser Leu Val Asp Phe Ser Arg Phe Phe Ser Ala Ile Ala Arg Thr Lys
1               5                   10                  15

Gln Phe Asn Leu Val Leu Asp Phe Cys Lys Gln Leu Glu Leu Asn Gly
            20                  25                  30

Ile Ala His
        35

<210>  96
<211>  35
<212>  PRT
<213>  Arabidopsis thaliana

<400>  96

Asn Ile Tyr Thr Leu Asn Ile Met Ile Asn Cys Phe Cys Arg Cys Cys
1               5                   10                  15

Lys Thr Cys Phe Ala Tyr Ser Val Leu Gly Lys Val Met Lys Leu Gly
            20                  25                  30

Tyr Glu Pro
        35

<210>  97
<211>  35
<212>  PRT
<213>  Arabidopsis thaliana

<400>  97

Asp Thr Thr Thr Phe Asn Thr Leu Ile Lys Gly Leu Phe Leu Glu Gly
1               5                   10                  15

Lys Val Ser Glu Ala Val Val Leu Val Asp Arg Met Val Glu Asn Gly
            20                  25                  30

Cys Gln Pro
        35

<210>  98
<211>  35
<212>  PRT
<213>  Arabidopsis thaliana

<400>  98

Asp Val Val Thr Tyr Asn Ser Ile Val Asn Gly Ile Cys Arg Ser Gly
1               5                   10                  15

Asp Thr Ser Leu Ala Leu Asp Leu Leu Arg Lys Met Glu Glu Arg Asn

20                  25                  30

Val Lys Ala
        35

<210>  99
<211>  35
<212>  PRT
<213>  Arabidopsis thaliana

<400>  99

Asp Val Phe Thr Tyr Ser Thr Ile Ile Asp Ser Leu Cys Arg Asp Gly
1               5                   10                  15

Cys Ile Asp Ala Ala Ile Ser Leu Phe Lys Glu Met Glu Thr Lys Gly
            20                  25                  30

Ile Lys Ser
        35

<210>  100
<211>  35
<212>  PRT
<213>  Arabidopsis thaliana

<400>  100

Ser Val Val Thr Tyr Asn Ser Leu Val Arg Gly Leu Cys Lys Ala Gly
1               5                   10                  15

Lys Trp Asn Asp Gly Ala Leu Leu Leu Lys Asp Met Val Ser Arg Glu
            20                  25                  30

Ile Val Pro
        35

<210>  101
<211>  35
<212>  PRT
<213>  Arabidopsis thaliana

<400>  101

Asn Val Ile Thr Phe Asn Val Leu Leu Asp Val Phe Val Lys Glu Gly
1               5                   10                  15

Lys Leu Gln Glu Ala Asn Glu Leu Tyr Lys Glu Met Ile Thr Arg Gly
            20                  25                  30

Ile Ser Pro
        35

<210>  102
<211>  35
<212>  PRT
<213>  Arabidopsis thaliana

<400>  102

Asn Ile Ile Thr Tyr Asn Thr Leu Met Asp Gly Tyr Cys Met Gln Asn
1               5                   10                  15

Arg Leu Ser Glu Ala Asn Asn Met Leu Asp Leu Met Val Arg Asn Lys
            20                  25                  30

Cys Ser Pro
        35

<210>  103
<211>  35
<212>  PRT
<213>  Arabidopsis thaliana

<400>  103

Asp Ile Val Thr Phe Thr Ser Leu Ile Lys Gly Tyr Cys Met Val Lys
1               5                   10                  15

Arg Val Asp Asp Gly Met Lys Val Phe Arg Asn Ile Ser Lys Arg Gly
            20                  25                  30

Leu Val Ala
        35

<210>  104
<211>  35
<212>  PRT
<213>  Arabidopsis thaliana

<400>  104

Asn Ala Val Thr Tyr Ser Ile Leu Val Gln Gly Phe Cys Gln Ser Gly
1               5                   10                  15

Lys Ile Lys Leu Ala Glu Glu Leu Phe Gln Glu Met Val Ser His Gly
            20                  25                  30

Val Leu Pro
        35

<210>  105
<211>  35
<212>  PRT
<213>  Arabidopsis thaliana

<400>  105

Asp Val Met Thr Tyr Gly Ile Leu Leu Asp Gly Leu Cys Asp Asn Gly
1               5                   10                  15

Lys Leu Glu Lys Ala Leu Glu Ile Phe Glu Asp Leu Gln Lys Ser Lys
            20                  25                  30

Met Asp Leu
        35

<210>  106
<211>  35
<212>  PRT
<213>  Arabidopsis thaliana

<400>  106

Gly Ile Val Met Tyr Thr Thr Ile Ile Glu Gly Met Cys Lys Gly Gly
1               5                   10                  15

Lys Val Glu Asp Ala Trp Asn Leu Phe Cys Ser Leu Pro Cys Lys Gly
            20                  25                  30

Val Lys Pro
        35

<210>  107
<211>  35
<212>  PRT
<213>  Arabidopsis thaliana

<400>  107

Asn Val Met Thr Tyr Thr Val Met Ile Ser Gly Leu Cys Lys Lys Gly
1               5                   10                  15

Ser Leu Ser Glu Ala Asn Ile Leu Leu Arg Lys Met Glu Glu Asp Gly
            20                  25                  30

Asn Ala Pro
        35

<210>  108
<211>  35
<212>  PRT
<213>  Arabidopsis thaliana

<400>  108

Asn Asp Cys Thr Tyr Asn Thr Leu Ile Arg Ala His Leu Arg Asp Gly
1               5                   10                  15

Asp Leu Thr Ala Ser Ala Lys Leu Ile Glu Glu Met Lys Ser Cys Gly
            20                  25                  30

Phe Ser Ala
        35

<210>  109
<211>  35
<212>  PRT
<213>  Arabidopsis thaliana

<400>  109

Thr Asp Tyr Thr Val Met Arg Leu Ile His Phe Leu Gly Lys Leu Gly
1               5                   10                  15

Asn Trp Arg Arg Val Leu Gln Val Ile Glu Trp Leu Gln Arg Gln Asp
            20                  25                  30

Arg Tyr Lys
        35

<210>  110
<211>  31
<212>  PRT
<213>  Arabidopsis thaliana

<400>  110

Ile Arg Ile Ile Tyr Thr Thr Ala Leu Asn Val Leu Gly Lys Ser Arg
1               5                   10                  15

Arg Pro Val Glu Ala Leu Asn Val Phe His Ala Met Leu Leu Gln
            20                  25                  30

<210>  111
<211>  31
<212>  PRT
<213>  Arabidopsis thaliana

<400>  111

Asp Met Val Ala Tyr Arg Ser Ile Ala Val Thr Leu Gly Gln Ala Gly
1               5                   10                  15

His Ile Lys Glu Leu Phe Tyr Val Ile Asp Thr Met Arg Ser Pro
            20                  25                  30

<210>  112
<211>  35
<212>  PRT
<213>  Arabidopsis thaliana

<400>  112

Asp Val Val Val Tyr Asn Ala Val Leu Asn Ala Cys Val Gln Arg Lys
1               5                   10                  15

Gln Trp Glu Gly Ala Phe Trp Val Leu Gln Gln Leu Lys Gln Arg Gly
            20                  25                  30

Gln Lys Pro
        35

<210>  113
<211>  31
<212>  PRT
<213>  Arabidopsis thaliana

<400>  113

Ser Pro Val Thr Tyr Gly Leu Ile Met Glu Val Met Leu Ala Cys Glu
1               5                   10                  15

Lys Tyr Asn Leu Val His Glu Phe Phe Arg Lys Met Gln Lys Ser
            20                  25                  30

<210>  114
<211>  35
<212>  PRT
<213>  Arabidopsis thaliana

<400>  114

Asn Ala Leu Ala Tyr Arg Val Leu Val Asn Thr Leu Trp Lys Glu Gly
1               5                   10                  15

Lys Ser Asp Glu Ala Val His Thr Val Glu Asp Met Glu Ser Arg Gly
            20                  25                  30

Ile Val Gly
        35

<210>  115
<211>  31
<212>  PRT
<213>  Arabidopsis thaliana

<400>  115

Leu Val Val Thr Tyr Thr Gly Leu Ile Gln Ala Cys Val Asp Ser Gly
1               5                   10                  15

Asn Ile Lys Asn Ala Ala Tyr Ile Phe Asp Gln Met Lys Lys Val
            20                  25                  30

<210>  116

<211>  35
<212>  PRT
<213>  Arabidopsis thaliana

<400>  116

Asn Leu Val Thr Cys Asn Ile Met Leu Lys Ala Tyr Leu Gln Gly Gly
1               5                   10                  15

Leu Phe Glu Glu Ala Arg Glu Leu Phe Gln Lys Met Ser Glu Asp Gly
            20                  25                  30

Asn His Ile
        35

<210>  117
<211>  35
<212>  PRT
<213>  Arabidopsis thaliana

<400>  117

Asp Thr Tyr Thr Phe Asn Thr Met Leu Asp Thr Cys Ala Glu Gln Glu
1               5                   10                  15

Lys Trp Asp Asp Phe Gly Tyr Ala Tyr Arg Glu Met Leu Arg His Gly
            20                  25                  30

Tyr His Phe
        35

<210>  118
<211>  35
<212>  PRT
<213>  Arabidopsis thaliana

<400>  118

Asn Ala Lys Arg His Leu Arg Met Val Leu Glu Ala Ser Arg Ala Gly
1               5                   10                  15

Lys Glu Glu Val Met Glu Ala Thr Trp Glu His Met Arg Arg Ser Asn
            20                  25                  30

Arg Ile Pro
        35

<210>  119
<211>  35
<212>  PRT
<213>  Arabidopsis thaliana

<400>  119

Asp Val Ile Cys Phe Asn Leu Leu Ile Asp Ala Tyr Gly Gln Lys Phe
1               5                   10                  15

Gln Tyr Lys Glu Ala Glu Ser Leu Tyr Val Gln Leu Leu Glu Ser Arg
            20                  25                  30

Tyr Val Pro
        35

<210>  120
<211>  35
<212>  PRT
<213>  Arabidopsis thaliana

<400>  120

Thr Glu Asp Thr Tyr Ala Leu Leu Ile Lys Ala Tyr Cys Met Ala Gly
1               5                   10                  15

Leu Ile Glu Arg Ala Glu Val Val Leu Val Glu Met Gln Asn His His
            20                  25                  30

Val Ser Pro
        35

<210>  121
<211>  36
<212>  PRT
<213>  Arabidopsis thaliana

<400>  121

Gly Val Thr Val Tyr Asn Ala Tyr Ile Glu Gly Leu Met Lys Arg Lys
1               5                   10                  15

Gly Asn Thr Glu Glu Ala Ile Asp Val Phe Gln Arg Met Lys Arg Asp
            20                  25                  30

Arg Cys Lys Pro
        35

<210>  122
<211>  35
<212>  PRT
<213>  Arabidopsis thaliana

<400>  122

Thr Thr Glu Thr Tyr Asn Leu Met Ile Asn Leu Tyr Gly Lys Ala Ser
1               5                   10                  15

Lys Ser Tyr Met Ser Trp Lys Leu Tyr Cys Glu Met Arg Ser His Gln
            20                  25                  30

Cys Lys Pro
        35

<210>  123
<211>  35
<212>  PRT
<213>  Arabidopsis thaliana

<400>  123

Asn Ile Cys Thr Tyr Thr Ala Leu Val Asn Ala Phe Ala Arg Glu Gly
1               5                   10                  15

Leu Cys Glu Lys Ala Glu Glu Ile Phe Glu Gln Leu Gln Glu Asp Gly
            20                  25                  30

Leu Glu Pro
        35

<210>  124
<211>  35
<212>  PRT
<213>  Arabidopsis thaliana

<400>  124

Asp Val Tyr Val Tyr Asn Ala Leu Met Glu Ser Tyr Ser Arg Ala Gly
1               5                   10                  15

Tyr Pro Tyr Gly Ala Ala Glu Ile Phe Ser Leu Met Gln His Met Gly
            20                  25                  30

Cys Glu Pro
        35

<210>  125
<211>  35
<212>  PRT
<213>  Arabidopsis thaliana

<400>  125

Asp Arg Ala Ser Tyr Asn Ile Met Val Asp Ala Tyr Gly Arg Ala Gly
1               5                   10                  15

Leu His Ser Asp Ala Glu Ala Val Phe Glu Glu Met Lys Arg Leu Gly
            20                  25                  30

Ile Ala Pro
        35

<210>  126
<211>  35
<212>  PRT
<213>  Arabidopsis thaliana

<400>  126

Thr Met Lys Ser His Met Leu Leu Leu Ser Ala Tyr Ser Lys Ala Arg
1               5                   10                  15

Asp Val Thr Lys Cys Glu Ala Ile Val Lys Glu Met Ser Glu Asn Gly
            20                  25                  30

Val Glu Pro
        35

<210>  127
<211>  35
<212>  PRT
<213>  Arabidopsis thaliana

<400>  127

Asp Thr Phe Val Leu Asn Ser Met Leu Asn Leu Tyr Gly Arg Leu Gly
1               5                   10                  15

Gln Phe Thr Lys Met Glu Lys Ile Leu Ala Glu Met Glu Asn Gly Pro
            20                  25                  30

Cys Thr Ala
        35

<210>  128
<211>  35
<212>  PRT
<213>  Arabidopsis thaliana

<400>  128

Asp Ile Ser Thr Tyr Asn Ile Leu Ile Asn Ile Tyr Gly Lys Ala Gly
1               5                   10                  15

Phe Leu Glu Arg Ile Glu Glu Leu Phe Val Glu Leu Lys Glu Lys Asn
            20                  25                  30

Phe Arg Pro
        35

<210>  129
<211>  35
<212>  PRT
<213>  Arabidopsis thaliana

<400>  129

Asp Val Val Thr Trp Thr Ser Arg Ile Gly Ala Tyr Ser Arg Lys Lys
1               5                   10                  15

Leu Tyr Val Lys Cys Leu Glu Val Phe Glu Glu Met Ile Asp Ser Gly
            20                  25                  30

Cys Ala Pro
        35

<210>  130
<211>  31
<212>  PRT
<213>  Arabidopsis thaliana

<400>  130

Asp Gly Gly Thr Ala Lys Val Leu Leu Ser Ala Cys Ser Ser Glu Glu
1               5                   10                  15

Gln Val Glu Gln Val Thr Ser Val Leu Arg Thr Met His Lys Gly
            20                  25                  30

<210>  131
<211>  31
<212>  PRT
<213>  Arabidopsis thaliana

<400>  131

Ala Arg Lys Asn Phe Pro Val Leu Ile Arg Glu Leu Ser Arg Arg Gly
1               5                   10                  15

Cys Ile Glu Leu Cys Val Asn Val Phe Lys Trp Met Lys Ile Gln
            20                  25                  30

<210>  132
<211>  35
<212>  PRT
<213>  Arabidopsis thaliana

<400>  132

Arg Asn Asp Ile Tyr Asn Met Met Ile Arg Leu His Ala Arg His Asn
1               5                   10                  15

Trp Val Asp Gln Ala Arg Gly Leu Phe Phe Glu Met Gln Lys Trp Ser
            20                  25                  30

Cys Lys Pro
        35

<210>  133

<211>  35
<212>  PRT
<213>  Arabidopsis thaliana

<400>  133

Asp Ala Glu Thr Tyr Asp Ala Leu Ile Asn Ala His Gly Arg Ala Gly
1               5                   10                  15

Gln Trp Arg Trp Ala Met Asn Leu Met Asp Asp Met Leu Arg Ala Ala
            20                  25                  30

Ile Ala Pro
        35

<210>  134
<211>  35
<212>  PRT
<213>  Arabidopsis thaliana

<400>  134

Ser Arg Ser Thr Tyr Asn Asn Leu Ile Asn Ala Cys Gly Ser Ser Gly
1               5                   10                  15

Asn Trp Arg Glu Ala Leu Glu Val Cys Lys Lys Met Thr Asp Asn Gly
            20                  25                  30

Val Gly Pro
        35

<210>  135
<211>  35
<212>  PRT
<213>  Arabidopsis thaliana

<400>  135

Asp Leu Val Thr His Asn Ile Val Leu Ser Ala Tyr Lys Ser Gly Arg
1               5                   10                  15

Gln Tyr Ser Lys Ala Leu Ser Tyr Phe Glu Leu Met Lys Gly Ala Lys
            20                  25                  30

Val Arg Pro
        35

<210>  136
<211>  35
<212>  PRT
<213>  Arabidopsis thaliana

<400>  136

Asp Thr Thr Thr Phe Asn Ile Ile Ile Tyr Cys Leu Ser Lys Leu Gly
1               5                   10                  15

Gln Ser Ser Gln Ala Leu Asp Leu Phe Asn Ser Met Arg Glu Lys Arg
            20                  25                  30

Ala Glu Cys
        35

<210>  137
<211>  35
<212>  PRT
<213>  Arabidopsis thaliana

<400>  137

Asp Val Val Thr Phe Thr Ser Ile Met His Leu Tyr Ser Val Lys Gly
1               5                   10                  15

Glu Ile Glu Asn Cys Arg Ala Val Phe Glu Ala Met Val Ala Glu Gly
            20                  25                  30

Leu Lys Pro
        35

<210>  138
<211>  35
<212>  PRT
<213>  Arabidopsis thaliana

<400>  138

Asn Ile Val Ser Tyr Asn Ala Leu Met Gly Ala Tyr Ala Val His Gly
1               5                   10                  15

Met Ser Gly Thr Ala Leu Ser Val Leu Gly Asp Ile Lys Gln Asn Gly
            20                  25                  30

Ile Ile Pro
        35

<210>  139
<211>  35
<212>  PRT
<213>  Arabidopsis thaliana

<400>  139

Asp Val Val Ser Tyr Thr Cys Leu Leu Asn Ser Tyr Gly Arg Ser Arg
1               5                   10                  15

Gln Pro Gly Lys Ala Lys Glu Val Phe Leu Met Met Arg Lys Glu Arg
            20                  25                  30

Arg Lys Pro
        35

<210>  140
<211>  35
<212>  PRT
<213>  Arabidopsis thaliana

<400>  140

Asn Val Val Thr Tyr Asn Ala Leu Ile Asp Ala Tyr Gly Ser Asn Gly
1               5                   10                  15

Phe Leu Ala Glu Ala Val Glu Ile Phe Arg Gln Met Glu Gln Asp Gly
            20                  25                  30

Ile Lys Pro
        35

<210>  141
<211>  35
<212>  PRT
<213>  Arabidopsis thaliana

<400>  141

Asn Val Val Ser Val Cys Thr Leu Leu Ala Ala Cys Ser Arg Ser Lys
1               5                   10                  15

Lys Lys Val Asn Val Asp Thr Val Leu Ser Ala Ala Gln Ser Arg Gly
            20                  25                  30

Ile Asn Leu
        35

<210>  142
<211>  35
<212>  PRT
<213>  Arabidopsis thaliana

<400>  142

Asn Thr Ala Ala Tyr Asn Ser Ala Ile Gly Ser Tyr Ile Asn Ala Ala
1               5                   10                  15

Glu Leu Glu Lys Ala Ile Ala Leu Tyr Gln Ser Met Arg Lys Lys Lys
            20                  25                  30

Val Lys Ala
        35

<210>  143
<211>  35
<212>  PRT
<213>  Arabidopsis thaliana

<400>  143

Asp Ser Val Thr Phe Thr Ile Leu Ile Ser Gly Ser Cys Arg Met Ser
1               5                   10                  15

Lys Tyr Pro Glu Ala Ile Ser Tyr Leu Lys Glu Met Glu Asp Leu Ser
            20                  25                  30

Ile Pro Leu
        35

<210>  144
<211>  35
<212>  PRT
<213>  Arabidopsis thaliana

<400>  144

Thr Lys Glu Val Tyr Ser Ser Val Leu Cys Ala Tyr Ser Lys Gln Gly
1               5                   10                  15

Gln Val Thr Glu Ala Glu Ser Ile Phe Asn Gln Met Lys Met Ala Gly
            20                  25                  30

Cys Glu Pro
        35

<210>  145
<211>  35
<212>  PRT
<213>  Arabidopsis thaliana

<400>  145

Asp Val Ile Ala Tyr Thr Ser Met Leu His Ala Tyr Asn Ala Ser Glu
1               5                   10                  15

Lys Trp Gly Lys Ala Cys Glu Leu Phe Leu Glu Met Glu Ala Asn Gly
            20                  25                  30

Ile Glu Pro
        35

<210>  146
<211>  35
<212>  PRT
<213>  Arabidopsis thaliana

<400>  146

Asp Ser Ile Ala Cys Ser Ala Leu Met Arg Ala Phe Asn Lys Gly Gly
1               5                   10                  15

Gln Pro Ser Asn Val Phe Val Leu Met Asp Leu Met Arg Glu Lys Glu
            20                  25                  30

Ile Pro Phe
        35

<210>  147
<211>  31
<212>  PRT
<213>  Arabidopsis thaliana

<400>  147

Thr Gly Ala Val Phe Phe Glu Ile Phe Ser Ala Cys Asn Thr Leu Gln
1               5                   10                  15

Glu Trp Lys Arg Ala Ile Asp Leu Ile Gln Met Met Asp Pro Tyr
            20                  25                  30

<210>  148
<211>  35
<212>  PRT
<213>  Arabidopsis thaliana

<400>  148

Ser Ile Gly Leu Thr Asn Gln Met Leu His Leu Phe Gly Lys Ser Gly
1               5                   10                  15

Lys Val Glu Ala Met Met Lys Leu Phe Tyr Lys Ile Ile Ala Ser Gly
            20                  25                  30

Val Gly Ile
        35

<210>  149
<211>  35
<212>  PRT
<213>  Arabidopsis thaliana

<400>  149

Asn Leu Lys Thr Tyr Ala Ile Leu Leu Glu His Leu Leu Ala Val Gly
1               5                   10                  15

Asn Trp Arg Lys Tyr Ile Glu Val Leu Glu Trp Met Ser Gly Ala Gly
            20                  25                  30

Ile Gln Pro

        35

<210>  150
<211>  35
<212>  PRT
<213>  Arabidopsis thaliana

<400>  150

Asp Arg Ser Phe Tyr His Thr Met Met Lys Ile Ser Arg Asp Ser Gly
1               5                   10                  15

Ser Asp Ser Lys Ala Glu Lys Leu Leu Gln Met Met Lys Asn Ala Gly
            20                  25                  30

Ile Glu Pro
        35

<210>  151
<211>  35
<212>  PRT
<213>  Arabidopsis thaliana

<400>  151

Thr Leu Ala Thr Met His Leu Leu Met Val Ser Tyr Ser Ser Ser Gly
1               5                   10                  15

Asn Pro Gln Glu Ala Glu Lys Val Leu Ser Asn Leu Lys Asp Thr Glu
            20                  25                  30

Val Glu Leu
        35

<210>  152
<211>  35
<212>  PRT
<213>  Arabidopsis thaliana

<400>  152

Thr Thr Leu Pro Tyr Ser Ser Val Ile Asp Ala Tyr Leu Arg Ser Lys
1               5                   10                  15

Asp Tyr Asn Ser Gly Ile Glu Arg Leu Leu Glu Met Lys Lys Glu Gly
            20                  25                  30

Leu Glu Pro
        35

<210>  153
<211>  35
<212>  PRT

<213>  Arabidopsis thaliana

<400>  153

Arg Val Gln Val Tyr Asn Ala Met Met Gly Val Tyr Ser Arg Ser Gly
1               5                   10                  15

Lys Phe Ser Lys Ala Gln Glu Leu Val Asp Ala Met Arg Gln Arg Gly
            20                  25                  30

Cys Val Pro
        35

<210>  154
<211>  37
<212>  PRT
<213>  Arabidopsis thaliana

<400>  154

Asp Leu Ile Ser Phe Asn Thr Leu Ile Asn Ala Arg Leu Lys Ser Gly
1               5                   10                  15

Gly Leu Thr Pro Asn Leu Ala Val Glu Leu Leu Asp Met Val Arg Asn
            20                  25                  30

Ser Gly Leu Arg Pro
        35

<210>  155
<211>  35
<212>  PRT
<213>  Arabidopsis thaliana

<400>  155

Asp Ala Ile Thr Tyr Asn Thr Leu Leu Ser Ala Cys Ser Arg Asp Ser
1               5                   10                  15

Asn Leu Asp Gly Ala Val Lys Val Phe Glu Asp Met Glu Ala His Arg
            20                  25                  30

Cys Gln Pro
        35

<210>  156
<211>  35
<212>  PRT
<213>  Arabidopsis thaliana

<400>  156

Asp Leu Trp Thr Tyr Asn Ala Met Ile Ser Val Tyr Gly Arg Cys Gly
1               5                   10                  15

Leu Ala Ala Glu Ala Glu Arg Leu Phe Met Glu Leu Glu Leu Lys Gly
                20                  25                  30

Phe Phe Pro
        35

<210>  157
<211>  35
<212>  PRT
<213>  Arabidopsis thaliana

<400>  157

Asp Ala Val Thr Tyr Asn Ser Leu Leu Tyr Ala Phe Ala Arg Glu Arg
1               5                   10                  15

Asn Thr Glu Lys Val Lys Glu Val Tyr Gln Gln Met Gln Lys Met Gly
                20                  25                  30

Phe Gly Lys
        35

<210>  158
<211>  31
<212>  PRT
<213>  Arabidopsis thaliana

<400>  158

Asp Glu Met Thr Tyr Asn Thr Ile Ile His Met Tyr Gly Lys Gln Gly
1               5                   10                  15

Gln Leu Asp Leu Ala Leu Gln Leu Tyr Lys Asp Met Lys Gly Leu
                20                  25                  30

<210>  159
<211>  35
<212>  PRT
<213>  Arabidopsis thaliana

<400>  159

Asp Ala Ile Thr Tyr Thr Val Leu Ile Asp Ser Leu Gly Lys Ala Asn
1               5                   10                  15

Arg Thr Val Glu Ala Ala Ala Leu Met Ser Glu Met Leu Asp Val Gly
                20                  25                  30

Ile Lys Pro
        35

<210>  160
<211>  35
<212>  PRT
<213>  Arabidopsis thaliana

<400>  160

Thr Leu Gln Thr Tyr Ser Ala Leu Ile Cys Gly Tyr Ala Lys Ala Gly
1               5                   10                  15

Lys Arg Glu Glu Ala Glu Asp Thr Phe Ser Cys Met Leu Arg Ser Gly
            20                  25                  30

Thr Lys Pro
        35

<210>  161
<211>  35
<212>  PRT
<213>  Arabidopsis thaliana

<400>  161

Asp Asn Leu Ala Tyr Ser Val Met Leu Asp Val Leu Leu Arg Gly Asn
1               5                   10                  15

Glu Thr Arg Lys Ala Trp Gly Leu Tyr Arg Asp Met Ile Ser Asp Gly
            20                  25                  30

His Thr Pro
        35

<210>  162
<211>  31
<212>  PRT
<213>  Arabidopsis thaliana

<400>  162

Ser Tyr Thr Leu Tyr Glu Leu Met Ile Leu Gly Leu Met Lys Glu Asn
1               5                   10                  15

Arg Ser Asp Asp Ile Gln Lys Thr Ile Arg Asp Met Glu Glu Leu
            20                  25                  30

<210>  163
<211>  31
<212>  PRT
<213>  Arabidopsis thaliana

<400>  163

Glu Asn Asp Thr Leu Leu Ser Ile Leu Gly Ser Tyr Ser Ser Ser Gly
1               5                   10                  15

Arg His Ser Glu Ala Phe Glu Leu Leu Glu Phe Leu Lys Glu His
            20                  25                  30

<210>  164
<211>  36
<212>  PRT
<213>  Arabidopsis thaliana

<400>  164

Lys Arg Leu Ile Thr Glu Ala Leu Ile Val Leu His Cys Lys Val Asn
1               5                   10                  15

Asn Leu Ser Ala Ala Leu Asp Glu Tyr Phe Ala Asp Pro Cys Val His
            20                  25                  30

Gly Trp Cys Phe
        35

<210>  165
<211>  35
<212>  PRT
<213>  Arabidopsis thaliana

<400>  165

Ser Ser Thr Met Tyr Glu Thr Leu Leu His Cys Cys Val Ala Asn Glu
1               5                   10                  15

His Tyr Ala Glu Ala Ser Gln Val Phe Ser Asp Leu Arg Leu Ser Gly
            20                  25                  30

Cys Glu Ala
        35

<210>  166
<211>  35
<212>  PRT
<213>  Arabidopsis thaliana

<400>  166

Ser Glu Ser Val Cys Lys Ser Met Val Val Val Tyr Cys Lys Leu Gly
1               5                   10                  15

Phe Pro Glu Thr Ala His Gln Val Val Asn Gln Ala Glu Thr Lys Gly
            20                  25                  30

Phe His Phe
        35

<210>  167

<211>  35
<212>  PRT
<213>  Arabidopsis thaliana

<400>  167

Cys Ser Pro Met Tyr Thr Asp Ile Ile Glu Ala Tyr Gly Lys Gln Lys
1               5                   10                  15

Leu Trp Gln Lys Ala Glu Ser Val Val Gly Asn Leu Arg Gln Ser Gly
            20                  25                  30

Arg Thr Pro
        35

<210>  168
<211>  35
<212>  PRT
<213>  Arabidopsis thaliana

<400>  168

Asp Leu Lys Thr Trp Asn Ser Leu Met Ser Ala Tyr Ala Gln Cys Gly
1               5                   10                  15

Cys Tyr Glu Arg Ala Arg Ala Ile Phe Asn Thr Met Met Arg Asp Gly
            20                  25                  30

Pro Ser Pro
        35

<210>  169
<211>  35
<212>  PRT
<213>  Arabidopsis thaliana

<400>  169

Thr Val Glu Ser Ile Asn Ile Leu Leu His Ala Leu Cys Val Asp Gly
1               5                   10                  15

Arg Leu Glu Glu Leu Tyr Val Val Val Glu Glu Leu Gln Asp Met Gly
            20                  25                  30

Phe Lys Ile
        35

<210>  170
<211>  35
<212>  PRT
<213>  Arabidopsis thaliana

<400>  170

Ser Lys Ser Ser Ile Leu Leu Met Leu Asp Ala Phe Ala Arg Ala Gly
1               5                   10                  15

Asn Ile Phe Glu Val Lys Lys Ile Tyr Ser Ser Met Lys Ala Ala Gly
            20                  25                  30

Tyr Leu Pro
        35

<210>  171
<211>  35
<212>  PRT
<213>  Arabidopsis thaliana

<400>  171

Thr Ile Arg Leu Tyr Arg Met Met Ile Glu Leu Leu Cys Lys Gly Lys
1               5                   10                  15

Arg Val Arg Asp Ala Glu Ile Met Val Ser Glu Met Glu Glu Ala Asn
            20                  25                  30

Phe Lys Val
        35

<210>  172
<211>  35
<212>  PRT
<213>  Arabidopsis thaliana

<400>  172

Glu Leu Ala Ile Trp Asn Ser Met Leu Lys Met Tyr Thr Ala Ile Glu
1               5                   10                  15

Asp Tyr Lys Lys Thr Val Gln Val Tyr Gln Arg Ile Lys Glu Thr Gly
            20                  25                  30

Leu Glu Pro
        35

<210>  173
<211>  35
<212>  PRT
<213>  Arabidopsis thaliana

<400>  173

Asp Glu Thr Thr Tyr Asn Thr Leu Ile Ile Met Tyr Cys Arg Asp Arg
1               5                   10                  15

Arg Pro Glu Glu Gly Tyr Leu Leu Met Gln Gln Met Arg Asn Leu Gly
            20                  25                  30

Leu Asp Pro
        35

<210>  174
<211>  35
<212>  PRT
<213>  Arabidopsis thaliana

<400>  174

Lys Leu Asp Thr Tyr Lys Ser Leu Ile Ser Ala Phe Gly Lys Gln Lys
1               5                   10                  15

Cys Leu Glu Gln Ala Glu Gln Leu Phe Glu Glu Leu Leu Ser Lys Gly
            20                  25                  30

Leu Lys Leu
        35

<210>  175
<211>  35
<212>  PRT
<213>  Arabidopsis thaliana

<400>  175

Arg Cys Lys Thr Tyr Thr Lys Leu Phe Lys Val Leu Gly Asn Cys Lys
1               5                   10                  15

Gln Pro Asp Gln Ala Ser Leu Leu Phe Glu Val Met Leu Ser Glu Gly
            20                  25                  30

Leu Lys Pro
        35

<210>  176
<211>  31
<212>  PRT
<213>  Arabidopsis thaliana

<400>  176

Thr Ile Asp Val Tyr Thr Ser Leu Ile Ser Val Tyr Gly Lys Ser Glu
1               5                   10                  15

Leu Leu Asp Lys Ala Phe Ser Thr Leu Glu Tyr Met Lys Ser Val
            20                  25                  30

<210>  177
<211>  35
<212>  PRT
<213>  Arabidopsis thaliana

<400>  177

Asp Val Phe Thr Phe Thr Val Leu Ile Ser Cys Cys Cys Lys Leu Gly
1               5                   10                  15

Arg Phe Asp Leu Val Lys Ser Ile Val Leu Glu Met Ser Tyr Leu Gly
            20                  25                  30

Val Gly Cys
        35

<210>  178
<211>  35
<212>  PRT
<213>  Arabidopsis thaliana

<400>  178

Ser Thr Val Thr Tyr Asn Thr Ile Ile Asp Gly Tyr Gly Lys Ala Gly
1               5                   10                  15

Met Phe Glu Glu Met Glu Ser Val Leu Ala Asp Met Ile Glu Asp Gly
            20                  25                  30

Asp Ser Leu
        35

<210>  179
<211>  35
<212>  PRT
<213>  Arabidopsis thaliana

<400>  179

Asp Val Cys Thr Leu Asn Ser Ile Ile Gly Ser Tyr Gly Asn Gly Arg
1               5                   10                  15

Asn Met Arg Lys Met Glu Ser Trp Tyr Ser Arg Phe Gln Leu Met Gly
            20                  25                  30

Val Gln Pro
        35

<210>  180
<211>  35
<212>  PRT
<213>  Arabidopsis thaliana

<400>  180

Asp Ile Thr Thr Phe Asn Ile Leu Ile Leu Ser Phe Gly Lys Ala Gly
1               5                   10                  15

Met Tyr Lys Lys Met Cys Ser Val Met Asp Phe Met Glu Lys Arg Phe
            20                  25                  30

Phe Ser Leu
        35

<210>  181
<211>  35
<212>  PRT
<213>  Arabidopsis thaliana

<400>  181

Thr Thr Val Thr Tyr Asn Ile Val Ile Glu Thr Phe Gly Lys Ala Gly
1               5                   10                  15

Arg Ile Glu Lys Met Asp Asp Val Phe Arg Lys Met Lys Tyr Gln Gly
            20                  25                  30

Val Lys Pro
        35

<210>  182
<211>  35
<212>  PRT
<213>  Arabidopsis thaliana

<400>  182

Asn Ser Ile Thr Tyr Cys Ser Leu Val Asn Ala Tyr Ser Lys Ala Gly
1               5                   10                  15

Leu Val Val Lys Ile Asp Ser Val Leu Arg Gln Ile Val Asn Ser Asp
            20                  25                  30

Val Val Leu
        35

<210>  183
<211>  35
<212>  PRT
<213>  Arabidopsis thaliana

<400>  183

Asp Thr Pro Phe Phe Asn Cys Ile Ile Asn Ala Tyr Gly Gln Ala Gly
1               5                   10                  15

Asp Leu Ala Thr Met Lys Glu Leu Tyr Ile Gln Met Glu Glu Arg Lys
            20                  25                  30

Cys Lys Pro

        35

<210>  184
<211>  35
<212>  PRT
<213>  Arabidopsis thaliana

<400>  184

Asp Lys Ile Thr Phe Ala Thr Met Ile Lys Thr Tyr Thr Ala His Gly
1               5                   10                  15

Ile Phe Asp Ala Val Gln Glu Leu Glu Lys Gln Met Ile Ser Ser Gly
            20                  25                  30

Glu Asn Leu
        35

<210>  185
<211>  35
<212>  PRT
<213>  Arabidopsis thaliana

<400>  185

Leu Ser Val Ser Leu Ser Leu Val Leu Glu Tyr Tyr Ala Leu Lys Gly
1               5                   10                  15

Ser His His Asn Gly Leu Glu Val Phe Gly Phe Met Arg Arg Leu Arg
            20                  25                  30

Leu Ser Pro
        35

<210>  186
<211>  35
<212>  PRT
<213>  Arabidopsis thaliana

<400>  186

Ser Gln Ser Ala Tyr Asn Ser Leu Leu Gly Ser Leu Val Lys Glu Asn
1               5                   10                  15

Gln Phe Arg Val Ala Leu Cys Leu Tyr Ser Ala Met Val Arg Asn Gly
            20                  25                  30

Ile Val Ser
        35

<210>  187
<211>  26
<212>  PRT

<213>  Arabidopsis thaliana

<400>  187

Asp Glu Leu Thr Trp Asp Leu Ile Ala Gln Ile Leu Cys Glu Gln Gly
1               5                   10                  15

Arg Ser Lys Ser Val Phe Lys Leu Met Glu
            20                  25

<210>  188
<211>  35
<212>  PRT
<213>  Arabidopsis thaliana

<400>  188

Ser Cys Lys Ile Tyr Thr Asn Leu Val Glu Cys Tyr Ser Arg Asn Gly
1               5                   10                  15

Glu Phe Asp Ala Val Phe Ser Leu Ile His Glu Met Asp Asp Lys Lys
            20                  25                  30

Leu Glu Leu
        35

<210>  189
<211>  35
<212>  PRT
<213>  Arabidopsis thaliana

<400>  189

Ser Phe Cys Ser Tyr Gly Cys Val Leu Asp Asp Ala Cys Arg Leu Gly
1               5                   10                  15

Asp Ala Glu Phe Ile Asp Lys Val Leu Cys Leu Met Val Glu Lys Lys
            20                  25                  30

Phe Val Thr
        35

<210>  190
<211>  35
<212>  PRT
<213>  Arabidopsis thaliana

<400>  190

Asp Ser Ala Val Asn Asp Lys Ile Ile Glu Arg Leu Cys Asp Met Gly
1               5                   10                  15

Lys Thr Phe Ala Ser Glu Met Leu Phe Arg Lys Ala Cys Asn Gly Glu
            20                  25                  30

Thr Val Arg
        35

<210>  191
<211>  35
<212>  PRT
<213>  Arabidopsis thaliana

<400>  191

Trp Asp Ser Thr Tyr Gly Cys Met Leu Lys Ala Leu Ser Arg Lys Lys
1               5                   10                  15

Arg Thr Lys Glu Ala Val Asp Val Tyr Arg Met Ile Cys Arg Lys Gly
            20                  25                  30

Ile Thr Val
        35

<210>  192
<211>  36
<212>  PRT
<213>  Arabidopsis thaliana

<400>  192

Asp Glu Ser Cys Tyr Ile Glu Phe Ala Asn Ala Leu Cys Arg Asp Asp
1               5                   10                  15

Asn Ser Ser Glu Glu Glu Glu Glu Leu Leu Val Asp Val Ile Lys Arg
            20                  25                  30

Gly Phe Val Pro
        35

<210>  193
<211>  35
<212>  PRT
<213>  Arabidopsis thaliana

<400>  193

Cys Thr His Lys Leu Ser Glu Val Leu Ala Ser Met Cys Arg Lys Arg
1               5                   10                  15

Arg Trp Lys Ser Ala Glu Lys Leu Leu Asp Ser Val Met Glu Met Glu
            20                  25                  30

Val Tyr Phe
        35

<210>  194
<211>  35
<212>  PRT
<213>  Arabidopsis thaliana

<400>  194

Asn Val Gly Ile Tyr Val Lys Leu Ile Val Met Leu Gly Lys Cys Lys
1               5                   10                  15

Gln Pro Glu Lys Ala His Glu Leu Phe Gln Glu Met Ile Asn Glu Gly
            20                  25                  30

Cys Val Val
        35

<210>  195
<211>  31
<212>  PRT
<213>  Arabidopsis thaliana

<400>  195

Asn His Glu Val Tyr Thr Ala Leu Val Ser Ala Tyr Ser Arg Ser Gly
1               5                   10                  15

Arg Phe Asp Ala Ala Phe Thr Leu Leu Glu Arg Met Lys Ser Ser
            20                  25                  30

<210>  196
<211>  35
<212>  PRT
<213>  Arabidopsis thaliana

<400>  196

Asp Val His Thr Tyr Ser Ile Leu Ile Lys Ser Phe Leu Gln Val Phe
1               5                   10                  15

Ala Phe Asp Lys Val Gln Asp Leu Leu Ser Asp Met Arg Arg Gln Gly
            20                  25                  30

Ile Arg Pro
        35

<210>  197
<211>  36
<212>  PRT
<213>  Arabidopsis thaliana

<400>  197

Asn Thr Ile Thr Tyr Asn Thr Leu Ile Asp Ala Tyr Gly Lys Ala Lys
1               5                   10                  15

Met Phe Val Glu Met Glu Ser Thr Leu Ile Gln Met Leu Gly Glu Asp
            20                  25                  30

Asp Cys Lys Pro
        35

<210>  198
<211>  35
<212>  PRT
<213>  Arabidopsis thaliana

<400>  198

Asp Ser Trp Thr Met Asn Ser Thr Leu Arg Ala Phe Gly Gly Asn Gly
1               5                   10                  15

Gln Ile Glu Met Met Glu Asn Cys Tyr Glu Lys Phe Gln Ser Ser Gly
            20                  25                  30

Ile Glu Pro
        35

<210>  199
<211>  35
<212>  PRT
<213>  Arabidopsis thaliana

<400>  199

Asn Ile Arg Thr Phe Asn Ile Leu Leu Asp Ser Tyr Gly Lys Ser Gly
1               5                   10                  15

Asn Tyr Lys Lys Met Ser Ala Val Met Glu Tyr Met Gln Lys Tyr His
            20                  25                  30

Tyr Ser Trp
        35

<210>  200
<211>  35
<212>  PRT
<213>  Arabidopsis thaliana

<400>  200

Thr Ile Val Thr Tyr Asn Val Val Ile Asp Ala Phe Gly Arg Ala Gly
1               5                   10                  15

Asp Leu Lys Gln Met Glu Tyr Leu Phe Arg Leu Met Gln Ser Glu Arg
            20                  25                  30

Ile Phe Pro

        35

<210>  201
<211>  35
<212>  PRT
<213>  Arabidopsis thaliana

<400>  201

Ser Cys Val Thr Leu Cys Ser Leu Val Arg Ala Tyr Gly Arg Ala Ser
1               5                   10                  15

Lys Ala Asp Lys Ile Gly Gly Val Leu Arg Phe Ile Glu Asn Ser Asp
            20                  25                  30

Ile Arg Leu
        35

<210>  202
<211>  35
<212>  PRT
<213>  Arabidopsis thaliana

<400>  202

Asp Leu Val Phe Phe Asn Cys Leu Val Asp Ala Tyr Gly Arg Met Glu
1               5                   10                  15

Lys Phe Ala Glu Met Lys Gly Val Leu Glu Leu Met Glu Lys Lys Gly
            20                  25                  30

Phe Lys Pro
        35

<210>  203
<211>  35
<212>  PRT
<213>  Arabidopsis thaliana

<400>  203

Asp Lys Ile Thr Tyr Arg Thr Met Val Lys Ala Tyr Arg Ile Ser Gly
1               5                   10                  15

Met Thr Thr His Val Lys Glu Leu His Gly Val Val Glu Ser Val Gly
            20                  25                  30

Glu Ala Gln
        35

<210>  204
<211>  35
<212>  PRT

<213>  Arabidopsis thaliana

<400>  204

Asp Val Arg Leu Tyr Asn Ala Ala Ile Ser Gly Leu Ser Ala Ser Gln
1               5                   10                  15

Arg Tyr Asp Asp Ala Trp Glu Val Tyr Glu Ala Met Asp Lys Ile Asn
            20                  25                  30

Val Tyr Pro
        35

<210>  205
<211>  36
<212>  PRT
<213>  Arabidopsis thaliana

<400>  205

Asp Asn Val Thr Cys Ala Ile Leu Ile Thr Thr Leu Arg Lys Ala Gly
1               5                   10                  15

Arg Ser Ala Lys Glu Val Trp Glu Ile Phe Glu Lys Met Ser Glu Lys
            20                  25                  30

Gly Val Lys Trp
        35

<210>  206
<211>  35
<212>  PRT
<213>  Arabidopsis thaliana

<400>  206

Ser Gln Asp Val Phe Gly Gly Leu Val Lys Ser Phe Cys Asp Glu Gly
1               5                   10                  15

Leu Lys Glu Glu Ala Leu Val Ile Gln Thr Glu Met Glu Lys Lys Gly
            20                  25                  30

Ile Arg Ser
        35

<210>  207
<211>  35
<212>  PRT
<213>  Arabidopsis thaliana

<400>  207

Asn Thr Ile Val Tyr Asn Thr Leu Met Asp Ala Tyr Asn Lys Ser Asn
1               5                   10                  15

His Ile Glu Glu Val Glu Gly Leu Phe Thr Glu Met Arg Asp Lys Gly
            20                  25                  30

Leu Lys Pro
        35

<210>  208
<211>  35
<212>  PRT
<213>  Arabidopsis thaliana

<400>  208

Ser Ala Ala Thr Tyr Asn Ile Leu Met Asp Ala Tyr Ala Arg Arg Met
1               5                   10                  15

Gln Pro Asp Ile Val Glu Thr Leu Leu Arg Glu Met Glu Asp Leu Gly
            20                  25                  30

Leu Glu Pro
        35

<210>  209
<211>  36
<212>  PRT
<213>  Arabidopsis thaliana

<400>  209

Asn Val Lys Ser Tyr Thr Cys Leu Ile Ser Ala Tyr Gly Arg Thr Lys
1               5                   10                  15

Lys Met Ser Asp Met Ala Ala Asp Ala Phe Leu Arg Met Lys Lys Val
            20                  25                  30

Gly Leu Lys Pro
        35

<210>  210
<211>  35
<212>  PRT
<213>  Arabidopsis thaliana

<400>  210

Ser Ser His Ser Tyr Thr Ala Leu Ile His Ala Tyr Ser Val Ser Gly
1               5                   10                  15

Trp His Glu Lys Ala Tyr Ala Ser Phe Glu Glu Met Cys Lys Glu Gly
            20                  25                  30

Ile Lys Pro
        35

<210>  211
<211>  35
<212>  PRT
<213>  Arabidopsis thaliana

<400>  211

Ser Val Glu Thr Tyr Thr Ser Val Leu Asp Ala Phe Arg Arg Ser Gly
1               5                   10                  15

Asp Thr Gly Lys Leu Met Glu Ile Trp Lys Leu Met Leu Arg Glu Lys
            20                  25                  30

Ile Lys Gly
        35

<210>  212
<211>  35
<212>  PRT
<213>  Arabidopsis thaliana

<400>  212

Thr Arg Ile Thr Tyr Asn Thr Leu Leu Asp Gly Phe Ala Lys Gln Gly
1               5                   10                  15

Leu Tyr Ile Glu Ala Arg Asp Val Val Ser Glu Phe Ser Lys Met Gly
            20                  25                  30

Leu Gln Pro
        35

<210>  213
<211>  35
<212>  PRT
<213>  Arabidopsis thaliana

<400>  213

Ser Val Met Thr Tyr Asn Met Leu Met Asn Ala Tyr Ala Arg Gly Gly
1               5                   10                  15

Gln Asp Ala Lys Leu Pro Gln Leu Leu Lys Glu Met Ala Ala Leu Asn
            20                  25                  30

Leu Lys Pro
        35

<210>  214
<211>  35

<212>  PRT
<213>  Arabidopsis thaliana

<400>  214

Asp Ser Ile Thr Tyr Ser Thr Met Ile Tyr Ala Phe Val Arg Val Arg
1               5                   10                  15

Asp Phe Lys Arg Ala Phe Phe Tyr His Lys Met Met Val Lys Ser Gly
            20                  25                  30

Gln Val Pro
        35

<210>  215
<211>  31
<212>  PRT
<213>  Arabidopsis thaliana

<400>  215

Thr Ser Asp Ser Phe Glu Lys Thr Leu His Ile Leu Ala Arg Met Arg
1               5                   10                  15

Tyr Phe Asp Gln Ala Trp Ala Leu Met Ala Glu Val Arg Lys Asp
            20                  25                  30

<210>  216
<211>  35
<212>  PRT
<213>  Arabidopsis thaliana

<400>  216

Ser Phe Lys Ser Met Ser Ile Leu Leu Cys Lys Ile Ala Lys Phe Gly
1               5                   10                  15

Ser Tyr Glu Glu Thr Leu Glu Ala Phe Val Lys Met Glu Lys Glu Ile
            20                  25                  30

Phe Arg Lys
        35

<210>  217
<211>  31
<212>  PRT
<213>  Arabidopsis thaliana

<400>  217

Gly Val Asp Glu Phe Asn Ile Leu Leu Arg Ala Phe Cys Thr Glu Arg
1               5                   10                  15

Glu Met Lys Glu Ala Arg Ser Ile Phe Glu Lys Leu His Ser Arg

20                  25                  30

<210>  218
<211>  35
<212>  PRT
<213>  Arabidopsis thaliana

<400>  218

Asp Val Lys Thr Met Asn Ile Leu Leu Leu Gly Phe Lys Glu Ala Gly
1               5                   10                  15

Asp Val Thr Ala Thr Glu Leu Phe Tyr His Glu Met Val Lys Arg Gly
            20                  25                  30

Phe Lys Pro
        35

<210>  219
<211>  35
<212>  PRT
<213>  Arabidopsis thaliana

<400>  219

Asn Ser Val Thr Tyr Gly Ile Arg Ile Asp Gly Phe Cys Lys Lys Arg
1               5                   10                  15

Asn Phe Gly Glu Ala Leu Arg Leu Phe Glu Asp Met Asp Arg Leu Asp
            20                  25                  30

Phe Asp Ile
        35

<210>  220
<211>  35
<212>  PRT
<213>  Arabidopsis thaliana

<400>  220

Thr Val Gln Ile Leu Thr Thr Leu Ile His Gly Ser Gly Val Ala Arg
1               5                   10                  15

Asn Lys Ile Lys Ala Arg Gln Leu Phe Asp Glu Ile Ser Lys Arg Gly
            20                  25                  30

Leu Thr Pro
        35

<210>  221
<211>  35
<212>  PRT

<213>  Arabidopsis thaliana

<400>  221

Asp Cys Gly Ala Tyr Asn Ala Leu Met Ser Ser Leu Met Lys Cys Gly
1               5                   10                  15

Asp Val Ser Gly Ala Ile Lys Val Met Lys Glu Met Glu Glu Lys Gly
            20                  25                  30

Ile Glu Pro
        35

<210>  222
<211>  37
<212>  PRT
<213>  Arabidopsis thaliana

<400>  222

Asp Ser Val Thr Phe His Ser Met Phe Ile Gly Met Met Lys Ser Lys
1               5                   10                  15

Glu Phe Gly Phe Asn Gly Val Cys Glu Tyr Tyr Gln Lys Met Lys Glu
            20                  25                  30

Arg Ser Leu Val Pro
        35

<210>  223
<211>  35
<212>  PRT
<213>  Arabidopsis thaliana

<400>  223

Lys Thr Pro Thr Ile Val Met Leu Met Lys Leu Phe Cys His Asn Gly
1               5                   10                  15

Glu Val Asn Leu Gly Leu Asp Leu Trp Lys Tyr Met Leu Glu Lys Gly
            20                  25                  30

Tyr Cys Pro
        35

<210>  224
<211>  32
<212>  PRT
<213>  Arabidopsis thaliana

<400>  224

Ser Pro Ser Leu Phe Asp Ser Val Val Asn Ser Leu Cys Lys Ala Arg
1               5                   10                  15

Glu Phe Glu Ile Ala Trp Ser Leu Val Phe Asp Arg Val Arg Ser Asp
            20                  25                  30

<210>  225
<211>  31
<212>  PRT
<213>  Arabidopsis thaliana

<400>  225

Ser Ala Asp Thr Phe Ile Val Leu Ile Arg Arg Tyr Ala Arg Ala Gly
1               5                   10                  15

Met Val Gln Gln Ala Ile Arg Ala Phe Glu Phe Ala Arg Ser Tyr
            20                  25                  30

<210>  226
<211>  31
<212>  PRT
<213>  Arabidopsis thaliana

<400>  226

Glu Leu Arg Leu Leu Glu Val Leu Leu Asp Ala Leu Cys Lys Glu Gly
1               5                   10                  15

His Val Arg Glu Ala Ser Met Tyr Leu Glu Arg Ile Gly Gly Thr
            20                  25                  30

<210>  227
<211>  35
<212>  PRT
<213>  Arabidopsis thaliana

<400>  227

Ser Val Arg Ile Phe Asn Ile Leu Leu Asn Gly Trp Phe Arg Ser Arg
1               5                   10                  15

Lys Leu Lys Gln Ala Glu Lys Leu Trp Glu Glu Met Lys Ala Met Asn
            20                  25                  30

Val Lys Pro
        35

<210>  228
<211>  35
<212>  PRT
<213>  Arabidopsis thaliana

<400>  228

Thr Val Val Thr Tyr Gly Thr Leu Ile Glu Gly Tyr Cys Arg Met Arg

1               5                   10                  15

Arg Val Gln Ile Ala Met Glu Val Leu Glu Glu Met Lys Met Ala Glu
            20                  25                  30

Met Glu Ile
        35

<210>  229
<211>  35
<212>  PRT
<213>  Arabidopsis thaliana

<400>  229

Asn Phe Met Val Phe Asn Pro Ile Ile Asp Gly Leu Gly Glu Ala Gly
1               5                   10                  15

Arg Leu Ser Glu Ala Leu Gly Met Met Glu Arg Phe Phe Val Cys Glu
            20                  25                  30

Ser Gly Pro
        35

<210>  230
<211>  35
<212>  PRT
<213>  Arabidopsis thaliana

<400>  230

Thr Ile Val Thr Tyr Asn Ser Leu Val Lys Asn Phe Cys Lys Ala Gly
1               5                   10                  15

Asp Leu Pro Gly Ala Ser Lys Ile Leu Lys Met Met Met Thr Arg Gly
            20                  25                  30

Val Asp Pro
        35

<210>  231
<211>  35
<212>  PRT
<213>  Arabidopsis thaliana

<400>  231

Thr Thr Thr Thr Tyr Asn His Phe Phe Lys Tyr Phe Ser Lys His Asn
1               5                   10                  15

Lys Thr Glu Glu Gly Met Asn Leu Tyr Phe Lys Leu Ile Glu Ala Gly
            20                  25                  30

His Ser Pro
        35

<210>  232
<211>  35
<212>  PRT
<213>  Arabidopsis thaliana

<400>  232

Asp Arg Leu Thr Tyr His Leu Ile Leu Lys Met Leu Cys Glu Asp Gly
1               5                   10                  15

Lys Leu Ser Leu Ala Met Gln Val Asn Lys Glu Met Lys Asn Arg Gly
            20                  25                  30

Ile Asp Pro
        35

<210>  233
<211>  35
<212>  PRT
<213>  Arabidopsis thaliana

<400>  233

Asp Leu Leu Thr Thr Thr Met Leu Ile His Leu Leu Cys Arg Leu Glu
1               5                   10                  15

Met Leu Glu Glu Ala Phe Glu Glu Phe Asp Asn Ala Val Arg Arg Gly
            20                  25                  30

Ile Ile Pro
        35

<210>  234
<211>  35
<212>  PRT
<213>  Arabidopsis thaliana

<400>  234

Gln Tyr Ile Thr Phe Lys Met Ile Asp Asn Gly Leu Arg Ser Lys Gly
1               5                   10                  15

Met Ser Asp Met Ala Lys Arg Leu Ser Ser Leu Met Ser Ser Leu Pro
            20                  25                  30

His Ser Lys
        35

<210>  235

<211>  35
<212>  PRT
<213>  Arabidopsis thaliana

<400>  235

Thr Ala Pro Val Tyr Asn Ala Leu Val Asp Leu Ile Val Arg Asp Asp
1               5                   10                  15

Asp Glu Lys Val Pro Glu Glu Phe Leu Gln Gln Ile Arg Asp Asp Asp
            20                  25                  30

Lys Glu Val
        35

<210>  236
<211>  35
<212>  PRT
<213>  Arabidopsis thaliana

<400>  236

Phe Gly Glu Phe Leu Asn Val Leu Val Arg Lys His Cys Arg Asn Gly
1               5                   10                  15

Ser Phe Ser Ile Ala Leu Glu Glu Leu Gly Arg Leu Lys Asp Phe Arg
            20                  25                  30

Phe Arg Pro
        35

<210>  237
<211>  35
<212>  PRT
<213>  Arabidopsis thaliana

<400>  237

Ser Arg Ser Thr Tyr Asn Cys Leu Ile Gln Ala Phe Leu Lys Ala Asp
1               5                   10                  15

Arg Leu Asp Ser Ala Ser Leu Ile His Arg Glu Met Ser Leu Ala Asn
            20                  25                  30

Leu Arg Met
        35

<210>  238
<211>  31
<212>  PRT
<213>  Arabidopsis thaliana

<400>  238

Asp Gly Phe Thr Leu Arg Cys Phe Ala Tyr Ser Leu Cys Lys Val Gly
1               5                   10                  15

Lys Trp Arg Glu Ala Leu Thr Leu Val Glu Thr Glu Asn Phe Val
            20                  25                  30

<210>  239
<211>  35
<212>  PRT
<213>  Arabidopsis thaliana

<400>  239

Asp Thr Val Phe Tyr Thr Lys Leu Ile Ser Gly Leu Cys Glu Ala Ser
1               5                   10                  15

Leu Phe Glu Glu Ala Met Asp Phe Leu Asn Arg Met Arg Ala Thr Ser
            20                  25                  30

Cys Leu Pro
        35

<210>  240
<211>  35
<212>  PRT
<213>  Arabidopsis thaliana

<400>  240

Asn Val Val Thr Tyr Ser Thr Leu Leu Cys Gly Cys Leu Asn Lys Lys
1               5                   10                  15

Gln Leu Gly Arg Cys Lys Arg Val Leu Asn Met Met Met Met Glu Gly
            20                  25                  30

Cys Tyr Pro
        35

<210>  241
<211>  35
<212>  PRT
<213>  Arabidopsis thaliana

<400>  241

Ser Pro Lys Ile Phe Asn Ser Leu Val His Ala Tyr Cys Thr Ser Gly
1               5                   10                  15

Asp His Ser Tyr Ala Tyr Lys Leu Leu Lys Lys Met Val Lys Cys Gly
            20                  25                  30

His Met Pro
        35

<210>  242
<211>  41
<212>  PRT
<213>  Arabidopsis thaliana

<400>  242

Gly Tyr Val Val Tyr Asn Ile Leu Ile Gly Ser Ile Cys Gly Asp Lys
1               5                   10                  15

Asp Ser Leu Asn Cys Asp Leu Leu Asp Leu Ala Glu Lys Ala Tyr Ser
            20                  25                  30

Glu Met Leu Ala Ala Gly Val Val Leu
        35                  40

<210>  243
<211>  35
<212>  PRT
<213>  Arabidopsis thaliana

<400>  243

Asn Lys Ile Asn Val Ser Ser Phe Thr Arg Cys Leu Cys Ser Ala Gly
1               5                   10                  15

Lys Tyr Glu Lys Ala Phe Ser Val Ile Arg Glu Met Ile Gly Gln Gly
            20                  25                  30

Phe Ile Pro
        35

<210>  244
<211>  35
<212>  PRT
<213>  Arabidopsis thaliana

<400>  244

Asp Thr Ser Thr Tyr Ser Lys Val Leu Asn Tyr Leu Cys Asn Ala Ser
1               5                   10                  15

Lys Met Glu Leu Ala Phe Leu Leu Phe Glu Glu Met Lys Arg Gly Gly
            20                  25                  30

Leu Val Ala
        35

<210>  245
<211>  35
<212>  PRT
<213>  Arabidopsis thaliana

<400>  245

Asp Val Tyr Thr Tyr Thr Ile Met Val Asp Ser Phe Cys Lys Ala Gly
1               5                   10                  15

Leu Ile Glu Gln Ala Arg Lys Trp Phe Asn Glu Met Arg Glu Val Gly
            20                  25                  30

Cys Thr Pro
        35

<210>  246
<211>  35
<212>  PRT
<213>  Arabidopsis thaliana

<400>  246

Asn Val Val Thr Tyr Thr Ala Leu Ile His Ala Tyr Leu Lys Ala Lys
1               5                   10                  15

Lys Val Ser Tyr Ala Asn Glu Leu Phe Glu Thr Met Leu Ser Glu Gly
            20                  25                  30

Cys Leu Pro
        35

<210>  247
<211>  35
<212>  PRT
<213>  Arabidopsis thaliana

<400>  247

Asn Ile Val Thr Tyr Ser Ala Leu Ile Asp Gly His Cys Lys Ala Gly
1               5                   10                  15

Gln Val Glu Lys Ala Cys Gln Ile Phe Glu Arg Met Cys Gly Ser Lys
            20                  25                  30

Asp Val Pro
        35

<210>  248
<211>  35
<212>  PRT
<213>  Arabidopsis thaliana

<400>  248

Asn Val Val Thr Tyr Gly Ala Leu Leu Asp Gly Phe Cys Lys Ser His
1               5                   10                  15

Arg Val Glu Glu Ala Arg Lys Leu Leu Asp Ala Met Ser Met Glu Gly
            20                  25                  30

Cys Glu Pro
        35

<210>  249
<211>  35
<212>  PRT
<213>  Arabidopsis thaliana

<400>  249

Asn Gln Ile Val Tyr Asp Ala Leu Ile Asp Gly Leu Cys Lys Val Gly
1               5                   10                  15

Lys Leu Asp Glu Ala Gln Glu Val Lys Thr Glu Met Ser Glu His Gly
            20                  25                  30

Phe Pro Ala
        35

<210>  250
<211>  35
<212>  PRT
<213>  Arabidopsis thaliana

<400>  250

Thr Leu Tyr Thr Tyr Ser Ser Leu Ile Asp Arg Tyr Phe Lys Val Lys
1               5                   10                  15

Arg Gln Asp Leu Ala Ser Lys Val Leu Ser Lys Met Leu Glu Asn Ser
            20                  25                  30

Cys Ala Pro
        35

<210>  251
<211>  35
<212>  PRT
<213>  Arabidopsis thaliana

<400>  251

Asn Val Val Ile Tyr Thr Glu Met Ile Asp Gly Leu Cys Lys Val Gly
1               5                   10                  15

Lys Thr Asp Glu Ala Tyr Lys Leu Met Gln Met Met Glu Glu Lys Gly
            20                  25                  30

Cys Gln Pro

        35

<210>  252
<211>  35
<212>  PRT
<213>  Arabidopsis thaliana

<400>  252

Asn Val Val Thr Tyr Thr Ala Met Ile Asp Gly Phe Gly Met Ile Gly
1               5                   10                  15

Lys Ile Glu Thr Cys Leu Glu Leu Leu Glu Arg Met Gly Ser Lys Gly
            20                  25                  30

Val Ala Pro
        35

<210>  253
<211>  35
<212>  PRT
<213>  Arabidopsis thaliana

<400>  253

Asn Tyr Val Thr Tyr Arg Val Leu Ile Asp His Cys Cys Lys Asn Gly
1               5                   10                  15

Ala Leu Asp Val Ala His Asn Leu Leu Glu Glu Met Lys Gln Thr His
            20                  25                  30

Trp Pro Thr
        35

<210>  254
<211>  35
<212>  PRT
<213>  Arabidopsis thaliana

<400>  254

Phe Leu Ser Val Tyr Arg Leu Leu Ile Asp Asn Leu Ile Lys Ala Gln
1               5                   10                  15

Arg Leu Glu Met Ala Leu Arg Leu Leu Glu Glu Val Ala Thr Phe Ser
            20                  25                  30

Ala Thr Leu
        35

<210>  255
<211>  35
<212>  PRT

<213>  Arabidopsis thaliana

<400>  255

Tyr Ser Ser Thr Tyr Asn Ser Leu Ile Glu Ser Leu Cys Leu Ala Asn
1               5                   10                  15

Lys Val Glu Thr Ala Phe Gln Leu Phe Ser Glu Met Thr Lys Lys Gly
            20                  25                  30

Val Ile Pro
        35

<210>  256
<211>  35
<212>  PRT
<213>  Arabidopsis thaliana

<400>  256

Glu Met Gln Ser Phe Cys Ser Leu Ile Lys Gly Leu Phe Arg Asn Ser
1               5                   10                  15

Lys Ile Ser Glu Ala Leu Leu Leu Leu Asp Phe Ile Ser His Met Val
            20                  25                  30

Cys Pro Leu
        35

<210>  257
<211>  35
<212>  PRT
<213>  Arabidopsis thaliana

<400>  257

Asp Val Arg Ala Tyr Thr Thr Ile Leu His Ala Tyr Ser Arg Thr Gly
1               5                   10                  15

Lys Tyr Glu Lys Ala Ile Asp Leu Phe Glu Arg Met Lys Glu Met Gly
            20                  25                  30

Pro Ser Pro
        35

<210>  258
<211>  36
<212>  PRT
<213>  Arabidopsis thaliana

<400>  258

Thr Leu Val Thr Tyr Asn Val Ile Leu Asp Val Phe Gly Lys Met Gly
1               5                   10                  15

Arg Ser Trp Arg Lys Ile Leu Gly Val Leu Asp Glu Met Arg Ser Lys
            20                  25                  30

Gly Leu Lys Phe
        35

<210>  259
<211>  35
<212>  PRT
<213>  Arabidopsis thaliana

<400>  259

Asp Glu Phe Thr Cys Ser Thr Val Leu Ser Ala Cys Ala Arg Glu Gly
1               5                   10                  15

Leu Leu Arg Glu Ala Lys Glu Phe Phe Ala Glu Leu Lys Ser Cys Gly
            20                  25                  30

Tyr Glu Pro
        35

<210>  260
<211>  35
<212>  PRT
<213>  Arabidopsis thaliana

<400>  260

Gly Thr Val Thr Tyr Asn Ala Leu Leu Gln Val Phe Gly Lys Ala Gly
1               5                   10                  15

Val Tyr Thr Glu Ala Leu Ser Val Leu Lys Glu Met Glu Glu Asn Ser
            20                  25                  30

Cys Pro Ala
        35

<210>  261
<211>  35
<212>  PRT
<213>  Arabidopsis thaliana

<400>  261

Asp Ser Val Thr Tyr Asn Glu Leu Val Ala Ala Tyr Val Arg Ala Gly
1               5                   10                  15

Phe Ser Lys Glu Ala Ala Gly Val Ile Glu Met Met Thr Lys Lys Gly
            20                  25                  30

Val Met Pro
        35

<210>  262
<211>  35
<212>  PRT
<213>  Arabidopsis thaliana

<400>  262

Asn Ala Ile Thr Tyr Thr Thr Val Ile Asp Ala Tyr Gly Lys Ala Gly
1               5                   10                  15

Lys Glu Asp Glu Ala Leu Lys Leu Phe Tyr Ser Met Lys Glu Ala Gly
            20                  25                  30

Cys Val Pro
        35

<210>  263
<211>  35
<212>  PRT
<213>  Arabidopsis thaliana

<400>  263

Asn Thr Cys Thr Tyr Asn Ala Val Leu Ser Leu Leu Gly Lys Lys Ser
1               5                   10                  15

Arg Ser Asn Glu Met Ile Lys Met Leu Cys Asp Met Lys Ser Asn Gly
            20                  25                  30

Cys Ser Pro
        35

<210>  264
<211>  35
<212>  PRT
<213>  Arabidopsis thaliana

<400>  264

Asn Arg Ala Thr Trp Asn Thr Met Leu Ala Leu Cys Gly Asn Lys Gly
1               5                   10                  15

Met Asp Lys Phe Val Asn Arg Val Phe Arg Glu Met Lys Ser Cys Gly
            20                  25                  30

Phe Glu Pro
        35

<210>  265
<211>  35

<212>  PRT
<213>  Arabidopsis thaliana

<400>  265

Asp Arg Asp Thr Phe Asn Thr Leu Ile Ser Ala Tyr Gly Arg Cys Gly
1               5                   10                  15

Ser Glu Val Asp Ala Ser Lys Met Tyr Gly Glu Met Thr Arg Ala Gly
            20                  25                  30

Phe Asn Ala
        35

<210>  266
<211>  35
<212>  PRT
<213>  Arabidopsis thaliana

<400>  266

Cys Val Thr Thr Tyr Asn Ala Leu Leu Asn Ala Leu Ala Arg Lys Gly
1               5                   10                  15

Asp Trp Arg Ser Gly Glu Asn Val Ile Ser Asp Met Lys Ser Lys Gly
            20                  25                  30

Phe Lys Pro
        35

<210>  267
<211>  35
<212>  PRT
<213>  Arabidopsis thaliana

<400>  267

Thr Glu Thr Ser Tyr Ser Leu Met Leu Gln Cys Tyr Ala Lys Gly Gly
1               5                   10                  15

Asn Tyr Leu Gly Ile Glu Arg Ile Glu Asn Arg Ile Lys Glu Gly Gln
            20                  25                  30

Ile Phe Pro
        35

<210>  268
<211>  35
<212>  PRT
<213>  Arabidopsis thaliana

<400>  268

Ser Trp Met Leu Leu Arg Thr Leu Leu Leu Ala Asn Phe Lys Cys Arg

1               5                   10                  15

Ala Leu Ala Gly Ser Glu Arg Ala Phe Thr Leu Phe Lys Lys His Gly
            20                  25                  30

Tyr Lys Pro
        35

<210>  269
<211>  35
<212>  PRT
<213>  Arabidopsis thaliana

<400>  269

Asp Met Val Ile Phe Asn Ser Met Leu Ser Ile Phe Thr Arg Asn Asn
1               5                   10                  15

Met Tyr Asp Gln Ala Glu Gly Ile Leu Glu Ser Ile Arg Glu Asp Gly
            20                  25                  30

Leu Ser Pro
        35

<210>  270
<211>  35
<212>  PRT
<213>  Arabidopsis thaliana

<400>  270

Asp Leu Val Thr Tyr Asn Ser Leu Met Asp Met Tyr Val Arg Arg Gly
1               5                   10                  15

Glu Cys Trp Lys Ala Glu Glu Ile Leu Lys Thr Leu Glu Lys Ser Gln
            20                  25                  30

Leu Lys Pro
        35

<210>  271
<211>  35
<212>  PRT
<213>  Arabidopsis thaliana

<400>  271

Asp Leu Val Ser Tyr Asn Thr Val Ile Lys Gly Phe Cys Arg Arg Gly
1               5                   10                  15

Leu Met Gln Glu Ala Val Arg Met Leu Ser Glu Met Thr Glu Arg Gly
            20                  25                  30

Ile Arg Pro
        35

<210>  272
<211>  35
<212>  PRT
<213>  Arabidopsis thaliana

<400>  272

Cys Ile Phe Thr Tyr Asn Thr Phe Val Ser Gly Tyr Thr Ala Met Gly
1               5                   10                  15

Met Phe Ala Glu Ile Glu Asp Val Ile Glu Cys Met Ala Lys Asn Asp
            20                  25                  30

Cys Arg Pro
        35

<210>  273
<211>  31
<212>  PRT
<213>  Arabidopsis thaliana

<400>  273

Asn Glu Leu Thr Phe Lys Met Val Val Asp Gly Tyr Cys Arg Ala Gly
1               5                   10                  15

Lys Tyr Ser Glu Ala Met Asp Phe Val Ser Lys Ile Lys Thr Phe
            20                  25                  30

<210>  274
<211>  35
<212>  PRT
<213>  Arabidopsis thaliana

<400>  274

Asp Thr Ala Ala Phe Asn Ala Val Leu Asn Ala Cys Ala Asn Leu Gly
1               5                   10                  15

Asp Thr Asp Lys Tyr Trp Lys Leu Phe Glu Glu Met Ser Glu Trp Asp
            20                  25                  30

Cys Glu Pro
        35

<210>  275
<211>  35
<212>  PRT
<213>  Arabidopsis thaliana

<400>  275

Asp Val Leu Thr Tyr Asn Val Met Ile Lys Leu Cys Ala Arg Val Gly
1               5                   10                  15

Arg Lys Glu Leu Ile Val Phe Val Leu Glu Arg Ile Ile Asp Lys Gly
            20                  25                  30

Ile Lys Val
        35

<210>  276
<211>  35
<212>  PRT
<213>  Arabidopsis thaliana

<400>  276

Cys Met Thr Thr Met His Ser Leu Val Ala Ala Tyr Val Gly Phe Gly
1               5                   10                  15

Asp Leu Arg Thr Ala Glu Arg Ile Val Gln Ala Met Arg Glu Lys Arg
            20                  25                  30

Arg Asp Leu
        35

<210>  277
<211>  31
<212>  PRT
<213>  Arabidopsis thaliana

<400>  277

Asp Ser Arg Ile Tyr Thr Thr Leu Met Lys Gly Tyr Met Lys Asn Gly
1               5                   10                  15

Arg Val Ala Asp Thr Ala Arg Met Leu Glu Ala Met Arg Arg Gln
            20                  25                  30

<210>  278
<211>  35
<212>  PRT
<213>  Arabidopsis thaliana

<400>  278

Asp Glu Val Thr Tyr Thr Thr Val Val Ser Ala Phe Val Asn Ala Gly
1               5                   10                  15

Leu Met Asp Arg Ala Arg Gln Val Leu Ala Glu Met Ala Arg Met Gly
            20                  25                  30

Val Pro Ala
        35

<210>  279
<211>  36
<212>  PRT
<213>  Arabidopsis thaliana

<400>  279

Asn Arg Ile Thr Tyr Asn Val Leu Leu Lys Gly Tyr Cys Lys Gln Leu
1               5                   10                  15

Gln Ile Asp Arg Ala Glu Asp Leu Leu Arg Glu Met Thr Glu Asp Ala
            20                  25                  30

Gly Ile Glu Pro
        35

<210>  280
<211>  35
<212>  PRT
<213>  Arabidopsis thaliana

<400>  280

Asp Val Val Ser Tyr Asn Ile Ile Ile Asp Gly Cys Ile Leu Ile Asp
1               5                   10                  15

Asp Ser Ala Gly Ala Leu Ala Phe Phe Asn Glu Met Arg Thr Arg Gly
            20                  25                  30

Ile Ala Pro
        35

<210>  281
<211>  31
<212>  PRT
<213>  Arabidopsis thaliana

<400>  281

Thr Lys Ile Ser Tyr Thr Thr Leu Met Lys Ala Phe Ala Met Ser Gly
1               5                   10                  15

Gln Pro Lys Leu Ala Asn Arg Val Phe Asp Glu Met Met Asn Asp
            20                  25                  30

<210>  282
<211>  35
<212>  PRT
<213>  Arabidopsis thaliana

<400>  282

Asp Leu Ile Ala Trp Asn Met Leu Val Glu Gly Tyr Cys Arg Leu Gly
1               5                   10                  15

Leu Ile Glu Asp Ala Gln Arg Val Val Ser Arg Met Lys Glu Asn Gly
            20                  25                  30

Phe Tyr Pro
        35

<210>  283
<211>  31
<212>  PRT
<213>  Arabidopsis thaliana

<400>  283

Asn Val Ala Thr Tyr Gly Ser Leu Ala Asn Gly Val Ser Gln Ala Arg
1               5                   10                  15

Lys Pro Gly Asp Ala Leu Leu Leu Trp Lys Glu Ile Lys Glu Arg
            20                  25                  30

<210>  284
<211>  35
<212>  PRT
<213>  Artificial Sequence

<220>
<223>  crPPR

<400>  284

Val Thr Tyr Thr Thr Leu Ile Ser Gly Leu Gly Lys Ala Gly Arg Leu
1               5                   10                  15

Glu Glu Ala Leu Glu Leu Phe Glu Glu Met Lys Glu Lys Gly Ile Val
            20                  25                  30

Pro Asn Val
        35

<210>  285
<211>  35
<212>  PRT
<213>  Artificial Sequence

<220>
<223>  MODIFIED TYPE crPPR-1

<400>  285

Val Thr Tyr Thr Thr Leu Ile Ser Ala Tyr Gly Lys Ala Gly Arg Leu
1               5                   10                  15

Glu Glu Ala Leu Glu Leu Phe Glu Glu Met Lys Glu Lys Gly Ile Val
            20                  25                  30

Pro Asn Val
        35

<210> 286
<211> 35
<212> PRT
<213> Artificial Sequence

<220>
<223> MODIFIED TYPE crPPR-2

<400> 286

Val Thr Tyr Thr Thr Leu Ile Ser Gly Leu Gly Lys Ala Gly Arg Leu
1               5                   10                  15

Glu Lys Ala Glu Glu Leu Phe Glu Glu Met Lys Glu Lys Gly Ile Val
            20                  25                  30

Pro Asn Val
        35

<210> 287
<211> 35
<212> PRT
<213> Artificial Sequence

<220>
<223> MODIFIED TYPE crPPR-3

<400> 287

Val Thr Tyr Thr Thr Leu Ile Ser Gly Leu Gly Lys Ala Gly Arg Leu
1               5                   10                  15

Glu Glu Ala Leu Glu Leu Phe Glu Glu Met Lys Glu Lys Gly Ile Lys
            20                  25                  30

Pro Asn Val
        35

<210> 288
<211> 35
<212> PRT
<213> Artificial Sequence

<220>
<223> MODIFIED TYPE crPPR-4

<400> 288

Val Thr Tyr Thr Thr Leu Ile Ser Ala Tyr Gly Lys Ala Gly Arg Leu
1               5                   10                  15

Glu Lys Ala Glu Glu Leu Phe Glu Glu Met Lys Glu Lys Gly Ile Val
            20                  25                  30

Pro Asn Val
        35

<210>  289
<211>  35
<212>  PRT
<213>  Artificial Sequence

<220>
<223>  MODIFIED TYPE crPPR-5

<400>  289

Val Thr Tyr Thr Thr Leu Ile Ser Ala Tyr Gly Lys Ala Gly Arg Leu
1               5                   10                  15

Glu Glu Ala Leu Glu Leu Phe Glu Glu Met Lys Glu Lys Gly Ile Lys
            20                  25                  30

Pro Asn Val
        35

<210>  290
<211>  35
<212>  PRT
<213>  Artificial Sequence

<220>
<223>  MODIFIED TYPE crPPR-6

<400>  290

Val Thr Tyr Thr Thr Leu Ile Ser Ala Tyr Gly Lys Ala Gly Arg Leu
1               5                   10                  15

Glu Lys Ala Glu Glu Leu Phe Glu Glu Met Lys Glu Lys Gly Ile Lys
            20                  25                  30

Pro Asn Val
        35

<210>  291
<211>  664
<212>  PRT
<213>  Arabidopsis thaliana

<400>  291

Met Glu Thr Pro Leu Leu Val Gly Leu Glu Leu Arg Cys Pro Pro His
1               5                   10                  15

Leu Phe Asn Thr His Ser Arg Pro Ser Ser Ser Leu Ser Ile Pro Ala
            20                  25                  30

Leu Ser Leu Arg Ile Leu Thr Pro Thr Ala Ala Thr Thr Ser Ser Ala
        35                  40                  45

Val Ile Glu Leu Pro Ala Asn Val Ala Glu Ala Pro Arg Ser Lys Arg
    50                  55                  60

His Ser Asn Ser Tyr Leu Ala Arg Lys Ser Ala Ile Ser Glu Val Gln
65                  70                  75                  80

Arg Ser Ser Asp Phe Leu Ser Ser Leu Gln Arg Leu Ala Thr Val Leu
                85                  90                  95

Lys Val Gln Asp Leu Asn Val Ile Leu Arg Asp Phe Gly Ile Ser Gly
            100                 105                 110

Arg Trp Gln Asp Leu Ile Gln Leu Phe Glu Trp Met Gln Gln His Gly
        115                 120                 125

Lys Ile Ser Val Ser Thr Tyr Ser Ser Cys Ile Lys Phe Val Gly Ala
    130                 135                 140

Lys Asn Val Ser Lys Ala Leu Glu Ile Tyr Gln Ser Ile Pro Asp Glu
145                 150                 155                 160

Ser Thr Lys Ile Asn Val Tyr Ile Cys Asn Ser Ile Leu Ser Cys Leu
                165                 170                 175

Val Lys Asn Gly Lys Leu Asp Ser Cys Ile Lys Leu Phe Asp Gln Met
            180                 185                 190

Lys Arg Asp Gly Leu Lys Pro Asp Val Val Thr Tyr Asn Thr Leu Leu
        195                 200                 205

Ala Gly Cys Ile Lys Val Lys Asn Gly Tyr Pro Lys Ala Ile Glu Leu
    210                 215                 220

Ile Gly Glu Leu Pro His Asn Gly Ile Gln Met Asp Ser Val Met Tyr
225                 230                 235                 240

Gly Thr Val Leu Ala Ile Cys Ala Ser Asn Gly Arg Ser Glu Glu Ala
                245                 250                 255

Glu Asn Phe Ile Gln Gln Met Lys Val Glu Gly His Ser Pro Asn Ile
            260                 265                 270

Tyr His Tyr Ser Ser Leu Leu Asn Ser Tyr Ser Trp Lys Gly Asp Tyr
        275                 280                 285

Lys Lys Ala Asp Glu Leu Met Thr Glu Met Lys Ser Ile Gly Leu Val
    290                 295                 300

Pro Asn Lys Val Met Met Thr Thr Leu Leu Lys Val Tyr Ile Lys Gly
305                 310                 315                 320

Gly Leu Phe Asp Arg Ser Arg Glu Leu Leu Ser Glu Leu Glu Ser Ala
                325                 330                 335

Gly Tyr Ala Glu Asn Glu Met Pro Tyr Cys Met Leu Met Asp Gly Leu
            340                 345                 350

Ser Lys Ala Gly Lys Leu Glu Glu Ala Arg Ser Ile Phe Asp Asp Met
        355                 360                 365

Lys Gly Lys Gly Val Arg Ser Asp Gly Tyr Ala Asn Ser Ile Met Ile
    370                 375                 380

Ser Ala Leu Cys Arg Ser Lys Arg Phe Lys Glu Ala Lys Glu Leu Ser
385                 390                 395                 400

Arg Asp Ser Glu Thr Thr Tyr Glu Lys Cys Asp Leu Val Met Leu Asn
                405                 410                 415

Thr Met Leu Cys Ala Tyr Cys Arg Ala Gly Glu Met Glu Ser Val Met
            420                 425                 430

Arg Met Met Lys Lys Met Asp Glu Gln Ala Val Ser Pro Asp Tyr Asn
        435                 440                 445

Thr Phe His Ile Leu Ile Lys Tyr Phe Ile Lys Glu Lys Leu His Leu
    450                 455                 460

Leu Ala Tyr Gln Thr Thr Leu Asp Met His Ser Lys Gly His Arg Leu
465                 470                 475                 480

Glu Glu Glu Leu Cys Ser Ser Leu Ile Tyr His Leu Gly Lys Ile Arg
                485                 490                 495

Ala Gln Ala Glu Ala Phe Ser Val Tyr Asn Met Leu Arg Tyr Ser Lys
            500                 505                 510

Arg Thr Ile Cys Lys Glu Leu His Glu Lys Ile Leu His Ile Leu Ile
        515                 520                 525

Gln Gly Asn Leu Leu Lys Asp Ala Tyr Ile Val Val Lys Asp Asn Ala
    530                 535                 540

Lys Met Ile Ser Gln Pro Thr Leu Lys Lys Phe Gly Arg Ala Phe Met
545                 550                 555                 560

Ile Ser Gly Asn Ile Asn Leu Val Asn Asp Val Leu Lys Val Leu His
                565                 570                 575

Gly Ser Gly His Lys Ile Asp Gln Val Gln Phe Glu Ile Ala Ile Ser
            580                 585                 590

Arg Tyr Ile Ser Gln Pro Asp Lys Lys Glu Leu Leu Leu Gln Leu Leu
        595                 600                 605

Gln Trp Met Pro Gly Gln Gly Tyr Val Val Asp Ser Ser Thr Arg Asn
    610                 615                 620

Leu Ile Leu Lys Asn Ser His Met Phe Gly Arg Leu Leu Ile Ala Glu
625                 630                 635                 640

Ile Leu Ser Lys His His Val Ala Ser Arg Pro Met Ile Lys Ser Arg
                645                 650                 655

Pro Glu Gln Lys Phe Arg Cys Lys
            660


<210>  292
<211>  630
<212>  PRT
<213>  Arabidopsis thaliana

<400>  292

Met Ala Ser His Leu Phe Thr Arg Ser Arg Ile Ser Leu Leu Lys Thr
1               5                   10                  15

Leu Lys Pro Asn Pro Phe Thr Ser Ala Ser Pro Ile Arg Ala Ile Ser
            20                  25                  30

Gly Thr Pro Phe Leu Ser Gln Asp Pro Leu Leu Ala Thr Glu Ser Thr
        35                  40                  45

Asp His Asp Pro Ser Asn His Gln Ser Thr Ser Thr Pro Leu Pro Pro
    50                  55                  60

Asn Pro Ala Thr Gly Ser Pro Leu Tyr Gln Glu Asn Trp Arg Ser Pro
65                  70                  75                  80

Ile Pro Asn Thr Pro Ser Phe Asn Gln Ser Leu Val Pro Leu Gly Phe
                85                  90                  95

Leu Asn Gln Ala Pro Ala Pro Arg Ile Arg Ala Leu Ser Glu Thr Leu
            100                 105                 110

Asp Met Asn Ser Leu Leu Asn Met Phe Ala Asp Trp Thr Ala Ser Gln
        115                 120                 125

Arg Trp Ser Asp Met Lys Gln Leu Phe Glu Val Trp Val Arg Ser Leu
    130                 135                 140

Asp Lys Asn Gly Lys Pro Asn Lys Pro Asp Val Asn Leu Tyr Asn His
145                 150                 155                 160

Tyr Leu Arg Ala Asn Leu Met Met Gly Ala Ser Ala Gly Asp Met Leu
                165                 170                 175

Asp Leu Val Ala Pro Met Glu Glu Phe Ser Val Glu Pro Asn Thr Ala
            180                 185                 190

Ser Tyr Asn Leu Val Leu Lys Ala Met Tyr Gln Ala Arg Glu Thr Glu
        195                 200                 205

Ala Ala Met Lys Leu Leu Glu Arg Met Leu Leu Leu Gly Lys Asp Ser
    210                 215                 220

Leu Pro Asp Asp Glu Ser Tyr Asp Leu Val Ile Gly Met His Phe Gly
225                 230                 235                 240

Val Gly Lys Asn Asp Glu Ala Met Lys Val Met Asp Thr Ala Leu Lys
                245                 250                 255

Ser Gly Tyr Met Leu Ser Thr Ser Val Phe Thr Glu Cys Val Arg Ser
            260                 265                 270

Cys Val Ala Lys Gly Arg Thr Asp Thr Leu Val Ser Ile Ile Glu Arg
        275                 280                 285

Cys Lys Ala Val Asp Arg Asn Lys Ser Leu Cys Pro Ser Trp Ile Leu
    290                 295                 300

Cys Asn Tyr Ile Ala Glu Val Ala Ile Gln Glu Asp Asn Ser Lys Leu

305                 310                 315                 320

Ala Phe Tyr Ala Phe Glu Phe Met Phe Lys Trp Ile Thr Arg Gly Glu
                325                 330                 335

Met Ala Arg Pro Ser Val Ile Phe Ser Val Asp Glu Gly Leu Val Val
            340                 345                 350

Ala Gly Leu Ala Ser Ala Ala Arg Thr Cys Ser Ser Ser Leu Val Glu
        355                 360                 365

Gly Ser Trp Thr Ile Leu Lys Gln Ser Leu Arg Gly Arg Lys Ala Ala
    370                 375                 380

Asn Pro Ala Ser Tyr Ile Ala Lys Ile Asn Ala Tyr Ala Ser Leu Gly
385                 390                 395                 400

Asn Leu Gln Lys Ala Phe Thr Ser Leu His Glu Leu Glu Ser Ala Tyr
                405                 410                 415

Ala Asp Ser Glu Lys Glu Val Val Glu Glu Met Leu Ser Pro Phe Thr
            420                 425                 430

Ser Leu Tyr Pro Leu Val Val Ala Cys Ser Lys Lys Gly Phe Glu Thr
        435                 440                 445

Leu Asp Glu Val Tyr Phe Gln Leu Glu Ser Leu Ser Gln Gly Asp Thr
    450                 455                 460

Pro Tyr Lys Ser Val Ala Ala Leu Asn Cys Ile Ile Leu Gly Cys Ala
465                 470                 475                 480

Asn Thr Trp Asp Leu Asp Arg Ala Tyr Gln Thr Phe Glu Ala Ile Ser
                485                 490                 495

Ala Ser Phe Gly Leu Thr Pro Asn Ile Asp Ser Tyr Asn Ala Leu Leu
            500                 505                 510

Tyr Ala Phe Gly Lys Val Lys Lys Thr Phe Glu Ala Thr Asn Val Phe
        515                 520                 525

Glu His Leu Val Ser Ile Gly Val Lys Pro Asp Ser Arg Thr Tyr Ser
    530                 535                 540

Leu Leu Val Asp Ala His Leu Ile Asn Arg Asp Pro Lys Ser Ala Leu
545                 550                 555                 560

Thr Val Val Asp Asp Met Ile Lys Ala Gly Phe Glu Pro Ser Arg Glu
                565                 570                 575

Thr Leu Lys Lys Leu Arg Arg Arg Cys Val Arg Glu Met Asp Asp Glu
            580                 585                 590

Asn Asp Asp Gln Val Glu Ala Leu Ala Lys Lys Phe Gln Ile Arg Met
        595                 600                 605

Gly Ser Glu Asn Arg Arg Asn Met Leu Phe Asn Ile Asp Tyr Ser Arg
    610                 615                 620

Gly Arg Ala Leu Asn Asn
625                 630

<210>  293
<211>  610
<212>  PRT
<213>  Arabidopsis thaliana

<400>  293

Met Tyr Ser Leu Ser Arg Ile Leu Gln Arg Ser Gln Arg Tyr Asn Phe
1               5                   10                  15

Ala Pro Ser Ser Phe Gly Ala Val Ser Lys Leu Glu Val Ser Ser Gly
            20                  25                  30

Gly Asp Lys Glu Arg Val Phe Lys Ser Phe Gly Leu Ile Tyr Ser Lys
        35                  40                  45

Pro Gln Gly Leu Val Arg Leu Tyr Ser Ala Arg Asp Val Phe Ser Arg
    50                  55                  60

Phe Phe Gly Ile His Lys Leu Ser Ser Ile Ala Asp Ala Lys Asp Lys
65                  70                  75                  80

Gly Asp Glu Val Val Arg Glu Glu Glu Leu Ser Glu Ser Glu Glu Ala
                85                  90                  95

Val Pro Val Ser Gly Asp Val Pro Glu Gly Val Val Asp Asp Asp Ser
            100                 105                 110

Leu Phe Glu Pro Glu Leu Gly Ser Asp Asn Asp Asp Leu Glu Ile Glu
        115                 120                 125

Glu Lys His Ser Lys Asp Gly Gly Lys Pro Thr Lys Lys Arg Gly Gln
    130                 135                 140

Ser Glu Leu Tyr Glu Ser Ile Val Ala Tyr Lys Ser Val Lys His Val
145                 150                 155                 160

Leu Glu Lys Trp Val Lys Glu Gly Lys Asp Leu Ser Gln Ala Glu Val
                165                 170                 175

Thr Leu Ala Ile His Asn Leu Arg Lys Arg Lys Ser Tyr Ala Met Cys
            180                 185                 190

Leu Gln Leu Trp Glu Trp Leu Gly Ala Asn Thr Gln Phe Glu Phe Thr
        195                 200                 205

Glu Ala Asn Tyr Ala Ser Gln Leu Asp Leu Val Ala Lys Val His Ser
    210                 215                 220

Leu Gln Lys Ala Glu Ile Phe Leu Lys Asp Ile Pro Glu Ser Ser Arg
225                 230                 235                 240

Gly Glu Val Val Tyr Arg Thr Leu Leu Ala Asn Cys Val Leu Lys His
                245                 250                 255

His Val Asn Lys Ala Glu Asp Ile Phe Asn Lys Met Lys Glu Leu Lys
            260                 265                 270

Phe Pro Thr Ser Val Phe Ala Cys Asn Gln Leu Leu Leu Leu Tyr Ser
        275                 280                 285

Met His Asp Arg Lys Lys Ile Ser Asp Val Leu Leu Leu Met Glu Arg
    290                 295                 300

Glu Asn Ile Lys Pro Ser Arg Ala Thr Tyr His Phe Leu Ile Asn Ser
305                 310                 315                 320

Lys Gly Leu Ala Gly Asp Ile Thr Gly Met Glu Lys Ile Val Glu Thr
                325                 330                 335

Ile Lys Glu Glu Gly Ile Glu Leu Asp Pro Glu Leu Gln Ser Ile Leu
            340                 345                 350

Ala Lys Tyr Tyr Ile Arg Ala Gly Leu Lys Glu Arg Ala Gln Asp Leu
        355                 360                 365

Met Lys Glu Ile Glu Gly Lys Gly Leu Gln Gln Thr Pro Trp Val Cys
    370                 375                 380

Arg Ser Leu Leu Pro Leu Tyr Ala Asp Ile Gly Asp Ser Asp Asn Val
385                 390                 395                 400

Arg Arg Leu Ser Arg Phe Val Asp Gln Asn Pro Arg Tyr Asp Asn Cys
                405                 410                 415

Ile Ser Ala Ile Lys Ala Trp Gly Lys Leu Lys Glu Val Glu Glu Ala
            420                 425                 430

Glu Ala Val Phe Glu Arg Leu Val Glu Lys Tyr Lys Ile Phe Pro Met
        435                 440                 445

Met Pro Tyr Phe Ala Leu Met Glu Ile Tyr Thr Glu Asn Lys Met Leu
    450                 455                 460

Ala Lys Gly Arg Asp Leu Val Lys Arg Met Gly Asn Ala Gly Ile Ala
465                 470                 475                 480

Ile Gly Pro Ser Thr Trp His Ala Leu Val Lys Leu Tyr Ile Lys Ala
                485                 490                 495

Gly Glu Val Gly Lys Ala Glu Leu Ile Leu Asn Arg Ala Thr Lys Asp
            500                 505                 510

Asn Lys Met Arg Pro Met Phe Thr Thr Tyr Met Ala Ile Leu Glu Glu
        515                 520                 525

Tyr Ala Lys Arg Gly Asp Val His Asn Thr Glu Lys Val Phe Met Lys
    530                 535                 540

Met Lys Arg Ala Ser Tyr Ala Ala Gln Leu Met Gln Tyr Glu Thr Val
545                 550                 555                 560

Leu Leu Ala Tyr Ile Asn Ala Lys Thr Pro Ala Tyr Gly Met Ile Glu
                565                 570                 575

Arg Met Lys Ala Asp Asn Val Phe Pro Asn Lys Ser Leu Ala Ala Lys
            580                 585                 590

Leu Ala Gln Val Asn Pro Phe Lys Lys Cys Pro Val Ser Val Leu Leu
        595                 600                 605

Asp Ile
    610

<210>  294
<211>  590
<212>  PRT
<213>  Arabidopsis thaliana

<400>  294

Met Phe Phe Ser Phe Arg Leu Leu Lys Val Leu Val Phe His Leu Gln
1               5                   10                  15

Ile Arg Pro Cys Val Leu Leu Cys Val Ser Gln Arg Lys Leu Gln Asn
            20                  25                  30

Asn Ile Ile Asn Val Gly Val Lys Ile Gln Asn Arg Phe Arg Val Val
        35                  40                  45

Cys Met Gly Met Leu Ala Pro Arg Lys Phe Leu Gln Lys Arg Arg Lys
    50                  55                  60

Met Glu Val Phe Lys Asp Ala Ala Asp Glu Thr Asp Gln Lys Arg Trp
65                  70                  75                  80

Arg Gly Leu Met Leu Glu Ile Glu Ser Thr Gly Ser Ala Val Pro Val
                85                  90                  95

Leu Arg Gln Tyr Lys Thr Asp Gly Asp Gln Gly Leu Pro Arg Asp Leu
            100                 105                 110

Val Leu Gly Thr Leu Val Arg Phe Lys Gln Leu Lys Lys Trp Asn Leu
        115                 120                 125

Val Ser Glu Ile Leu Glu Trp Leu Arg Tyr Gln Asn Trp Trp Asn Phe
    130                 135                 140

Ser Glu Ile Asp Phe Leu Met Leu Ile Thr Ala Tyr Gly Lys Leu Gly
145                 150                 155                 160

Asn Phe Asn Gly Ala Glu Arg Val Leu Ser Val Leu Ser Lys Met Gly
                165                 170                 175

Ser Thr Pro Asn Val Ile Ser Tyr Thr Ala Leu Met Glu Ser Tyr Gly
            180                 185                 190

Arg Gly Gly Lys Cys Asn Asn Ala Glu Ala Ile Phe Arg Arg Met Gln
        195                 200                 205

Ser Ser Gly Pro Glu Pro Ser Ala Ile Thr Tyr Gln Ile Ile Leu Lys
    210                 215                 220

Thr Phe Val Glu Gly Asp Lys Phe Lys Glu Ala Glu Glu Val Phe Glu
225                 230                 235                 240

Thr Leu Leu Asp Glu Lys Lys Ser Pro Leu Lys Pro Asp Gln Lys Met
                245                 250                 255

Tyr His Met Met Ile Tyr Met Tyr Lys Lys Ala Gly Asn Tyr Glu Lys
            260                 265                 270

Ala Arg Lys Val Phe Ser Ser Met Val Gly Lys Gly Val Pro Gln Ser
        275                 280                 285

Thr Val Thr Tyr Asn Ser Leu Met Ser Phe Glu Thr Ser Tyr Lys Glu
    290                 295                 300

Val Ser Lys Ile Tyr Asp Gln Met Gln Arg Ser Asp Ile Gln Pro Asp
305                 310                 315                 320

Val Val Ser Tyr Ala Leu Leu Ile Lys Ala Tyr Gly Arg Ala Arg Arg
                325                 330                 335

Glu Glu Glu Ala Leu Ser Val Phe Glu Glu Met Leu Asp Ala Gly Val
            340                 345                 350

Arg Pro Thr His Lys Ala Tyr Asn Ile Leu Leu Asp Ala Phe Ala Ile
        355                 360                 365

Ser Gly Met Val Glu Gln Ala Lys Thr Val Phe Lys Ser Met Arg Arg
    370                 375                 380

Asp Arg Ile Phe Pro Asp Leu Trp Ser Tyr Thr Thr Met Leu Ser Ala
385                 390                 395                 400

Tyr Val Asn Ala Ser Asp Met Glu Gly Ala Glu Lys Phe Phe Lys Arg
                405                 410                 415

Ile Lys Val Asp Gly Phe Glu Pro Asn Ile Val Thr Tyr Gly Thr Leu
            420                 425                 430

Ile Lys Gly Tyr Ala Lys Ala Asn Asp Val Glu Lys Met Met Glu Val
        435                 440                 445

Tyr Glu Lys Met Arg Leu Ser Gly Ile Lys Ala Asn Gln Thr Ile Leu
    450                 455                 460

Thr Thr Ile Met Asp Ala Ser Gly Arg Cys Lys Asn Phe Gly Ser Ala
465                 470                 475                 480

Leu Gly Trp Tyr Lys Glu Met Glu Ser Cys Gly Val Pro Pro Asp Gln
                485                 490                 495

Lys Ala Lys Asn Val Leu Leu Ser Leu Ala Ser Thr Gln Asp Glu Leu

500                 505                 510

Glu Glu Ala Lys Glu Leu Thr Gly Ile Arg Asn Glu Thr Ala Thr Ile
        515                 520                 525

Ile Ala Arg Val Tyr Gly Ser Asp Asp Asp Glu Glu Gly Val Glu Asp
    530                 535                 540

Ile Ser Ser Glu Ser Ser Asp Asp Glu Asp Glu Gly Asp Asp Asp Asp
545                 550                 555                 560

Asp Asp Ala Arg Glu Thr Val Leu Tyr Asp Lys Pro Gln Glu Gly Ser
                565                 570                 575

Leu Gly Tyr Gly Ser Leu Gln Thr Glu Glu Leu Val Gly Leu
            580                 585                 590

<210>  295
<211>  580
<212>  PRT
<213>  Arabidopsis thaliana

<400>  295

Met Asn Arg Ile Ser Ala Ile Ser Thr Leu Val Thr Pro Leu Pro Leu
1               5                   10                  15

Leu Pro Ser Cys Ser Phe Val Pro Thr Arg Arg Cys Tyr Pro Arg Arg
            20                  25                  30

Ala Thr Pro Tyr Ser Arg Arg Ile Asn Leu Lys Pro Leu Thr Ser Arg
        35                  40                  45

Ile Val Leu Leu Thr Arg Arg Arg Gln Leu Gly Gln Ile Val Glu Glu
    50                  55                  60

Val Glu Ala Ala Lys Lys Arg Tyr Gly Arg Leu Asn Thr Ile Val Met
65                  70                  75                  80

Asn Ser Val Leu Glu Ala Cys Val His Cys Gly Asn Ile Asp Leu Ala
                85                  90                  95

Leu Arg Met Phe His Glu Met Ala Glu Pro Gly Gly Ile Gly Val Asp
            100                 105                 110

Ser Ile Ser Tyr Ala Thr Ile Leu Lys Gly Leu Gly Lys Ala Arg Arg
        115                 120                 125

Ile Asp Glu Ala Phe Gln Met Leu Glu Thr Ile Glu Tyr Gly Thr Ala

130                 135                 140

Ala Gly Thr Pro Lys Leu Ser Ser Ser Leu Ile Tyr Gly Leu Leu Asp
145                 150                 155                 160

Ala Leu Ile Asn Ala Gly Asp Leu Arg Arg Ala Asn Gly Leu Leu Ala
                165                 170                 175

Arg Tyr Asp Ile Leu Leu Leu Asp His Gly Thr Pro Ser Val Leu Ile
            180                 185                 190

Tyr Asn Leu Leu Met Lys Gly Tyr Val Asn Ser Glu Ser Pro Gln Ala
        195                 200                 205

Ala Ile Asn Leu Leu Asp Glu Met Leu Arg Leu Arg Leu Glu Pro Asp
    210                 215                 220

Arg Leu Thr Tyr Asn Thr Leu Ile His Ala Cys Ile Lys Cys Gly Asp
225                 230                 235                 240

Leu Asp Ala Ala Met Lys Phe Phe Asn Asp Met Lys Glu Lys Ala Glu
                245                 250                 255

Glu Tyr Tyr Asp Asp Phe Leu Gln Pro Asp Val Val Thr Tyr Thr Thr
            260                 265                 270

Leu Val Lys Gly Phe Gly Asp Ala Thr Asp Leu Leu Ser Leu Gln Glu
        275                 280                 285

Ile Phe Leu Glu Met Lys Leu Cys Glu Asn Val Phe Ile Asp Arg Thr
    290                 295                 300

Ala Phe Thr Ala Val Val Asp Ala Met Leu Lys Cys Gly Ser Thr Ser
305                 310                 315                 320

Gly Ala Leu Cys Val Phe Gly Glu Ile Leu Lys Arg Ser Gly Ala Asn
                325                 330                 335

Glu Val Leu Arg Pro Lys Pro His Leu Tyr Leu Ser Met Met Arg Ala
            340                 345                 350

Phe Ala Val Gln Gly Asp Tyr Gly Met Val Arg Asn Leu Tyr Leu Arg
        355                 360                 365

Leu Trp Pro Asp Ser Ser Gly Ser Ile Ser Lys Ala Val Gln Gln Glu
    370                 375                 380

Ala Asp Asn Leu Leu Met Glu Ala Ala Leu Asn Asp Gly Gln Leu Asp
385                 390                 395                 400

Glu Ala Leu Gly Ile Leu Leu Ser Ile Val Arg Arg Trp Lys Thr Ile
                405                 410                 415

Pro Trp Thr Thr Ser Gly Gly Met Ala Ala Val Arg Leu Glu Thr Leu
            420                 425                 430

Leu Gly Phe Ser Lys Ser Ile Leu Arg Pro His Leu Leu Ser Lys Val
        435                 440                 445

Ile Pro Ser Glu Pro Ile Glu Ser Ile Met Ile Arg Phe Glu Ala Thr
    450                 455                 460

Arg Pro Leu Leu Gly Thr Leu Gln Leu Lys Asn Val Ala Met Arg Phe
465                 470                 475                 480

Phe Lys Glu Gln Val Val Pro Ile Val Asp Asp Arg Gly Ser Cys Ile
                485                 490                 495

Gly Leu Leu His Arg Glu Asp Cys Asn Asn Leu Asp Ala Pro Leu Val
            500                 505                 510

Ser Met Met Arg Ser Pro Pro Thr Cys Val Ser Thr Thr Thr Ser Ile
        515                 520                 525

Gly Arg Val Val Asp Leu Val Leu Glu Lys Lys Leu Lys Met Val Ile
    530                 535                 540

Val Val His Cys Gly Asn Phe Ser Gly Ser Gly Tyr Ser Ser Lys Ala
545                 550                 555                 560

Val Gly Ala Phe Thr Arg Ala Gln Leu Tyr Arg Leu Phe Glu Ser Glu
                565                 570                 575

Gln Lys Leu Leu
            580

<210>  296
<211>  593
<212>  PRT
<213>  Arabidopsis thaliana

<400>  296

Met Ala Leu Leu Ile Ser Cys Gly Glu Val Thr Ser Ser Gln Phe Thr
1               5                   10                  15

Val Phe Arg Leu Leu Asn Gln Ser Leu Asp Phe Val Ser Asp Asn Val
            20                  25                  30

Ser Arg Leu Leu Ala Pro Ile Phe Thr Asn Leu Arg Asp Phe Glu Met
        35                  40                  45

Arg Leu Ser Cys Ile Glu Arg Pro Pro Ser Ile Ser Gly Asn His Ser
    50                  55                  60

His Leu Cys Thr Glu Lys Trp Phe Ser Asp Gln Lys Asp Tyr Asp Gln
65                  70                  75                  80

Lys Glu Asp Pro Glu Ala Ile Phe Asn Val Leu Asp Tyr Ile Leu Lys
                85                  90                  95

Ser Ser Leu Asp Arg Leu Ala Ser Leu Arg Glu Ser Val Cys Gln Thr
            100                 105                 110

Lys Ser Phe Asp Tyr Asp Asp Cys Leu Ser Ile His Ser Ser Ile Met
        115                 120                 125

Arg Asp Leu Cys Leu Gln Gly Lys Leu Asp Ala Ala Leu Trp Leu Arg
    130                 135                 140

Lys Lys Met Ile Tyr Ser Gly Val Ile Pro Gly Leu Ile Thr His Asn
145                 150                 155                 160

His Leu Leu Asn Gly Leu Cys Lys Ala Gly Tyr Ile Glu Lys Ala Asp
                165                 170                 175

Gly Leu Val Arg Glu Met Arg Glu Met Gly Pro Ser Pro Asn Cys Val
            180                 185                 190

Ser Tyr Asn Thr Leu Ile Lys Gly Leu Cys Ser Val Asn Asn Val Asp
        195                 200                 205

Lys Ala Leu Tyr Leu Phe Asn Thr Met Asn Lys Tyr Gly Ile Arg Pro
    210                 215                 220

Asn Arg Val Thr Cys Asn Ile Ile Val His Ala Leu Cys Gln Lys Gly
225                 230                 235                 240

Val Ile Gly Asn Asn Asn Lys Lys Leu Leu Glu Glu Ile Leu Asp Ser
                245                 250                 255

Ser Gln Ala Asn Ala Pro Leu Asp Ile Val Ile Cys Thr Ile Leu Met
            260                 265                 270

Asp Ser Cys Phe Lys Asn Gly Asn Val Val Gln Ala Leu Glu Val Trp
        275                 280                 285

Lys Glu Met Ser Gln Lys Asn Val Pro Ala Asp Ser Val Val Tyr Asn
    290                 295                 300

Val Ile Ile Arg Gly Leu Cys Ser Ser Gly Asn Met Val Ala Ala Tyr
305                 310                 315                 320

Gly Phe Met Cys Asp Met Val Lys Arg Gly Val Asn Pro Asp Val Phe
                325                 330                 335

Thr Tyr Asn Thr Leu Ile Ser Ala Leu Cys Lys Glu Gly Lys Phe Asp
            340                 345                 350

Glu Ala Cys Asp Leu His Gly Thr Met Gln Asn Gly Gly Val Ala Pro
        355                 360                 365

Asp Gln Ile Ser Tyr Lys Val Ile Ile Gln Gly Leu Cys Ile His Gly
    370                 375                 380

Asp Val Asn Arg Ala Asn Glu Phe Leu Leu Ser Met Leu Lys Ser Ser
385                 390                 395                 400

Leu Leu Pro Glu Val Leu Leu Trp Asn Val Val Ile Asp Gly Tyr Gly
                405                 410                 415

Arg Tyr Gly Asp Thr Ser Ser Ala Leu Ser Val Leu Asn Leu Met Leu
            420                 425                 430

Ser Tyr Gly Val Lys Pro Asn Val Tyr Thr Asn Asn Ala Leu Ile His
        435                 440                 445

Gly Tyr Val Lys Gly Gly Arg Leu Ile Asp Ala Trp Trp Val Lys Asn
    450                 455                 460

Glu Met Arg Ser Thr Lys Ile His Pro Asp Thr Thr Thr Tyr Asn Leu
465                 470                 475                 480

Leu Leu Gly Ala Ala Cys Thr Leu Gly His Leu Arg Leu Ala Phe Gln
                485                 490                 495

Leu Tyr Asp Glu Met Leu Arg Arg Gly Cys Gln Pro Asp Ile Ile Thr
            500                 505                 510

Tyr Thr Glu Leu Val Arg Gly Leu Cys Trp Lys Gly Arg Leu Lys Lys
        515                 520                 525

Ala Glu Ser Leu Leu Ser Arg Ile Gln Ala Thr Gly Ile Thr Ile Asp
    530                 535                 540

His Val Pro Phe Leu Ile Leu Ala Lys Lys Tyr Thr Arg Leu Gln Arg
545                 550                 555                 560

Pro Gly Glu Ala Tyr Leu Val Tyr Lys Lys Trp Leu Ala Thr Arg Asn
                565                 570                 575

Arg Gly Val Ser Cys Pro Ser Ile Leu Asn His Met His Thr Glu Glu
            580                 585                 590

Gln

<210>  297
<211>  798
<212>  PRT
<213>  Arabidopsis thaliana

<400>  297

Met Asp Ala Ser Val Val Arg Phe Ser Gln Ser Pro Ala Arg Val Pro
1               5                   10                  15

Pro Glu Phe Glu Pro Asp Met Glu Lys Ile Lys Arg Arg Leu Leu Lys
            20                  25                  30

Tyr Gly Val Asp Pro Thr Pro Lys Ile Leu Asn Asn Leu Arg Lys Lys
        35                  40                  45

Glu Ile Gln Lys His Asn Arg Arg Thr Lys Arg Glu Thr Glu Ser Glu
    50                  55                  60

Ala Glu Val Tyr Thr Glu Ala Gln Lys Gln Ser Met Glu Glu Glu Ala
65                  70                  75                  80

Arg Phe Gln Thr Leu Arg Arg Glu Tyr Lys Gln Phe Thr Arg Ser Ile
                85                  90                  95

Ser Gly Lys Arg Gly Gly Asp Val Gly Leu Met Val Gly Asn Pro Trp
            100                 105                 110

Glu Gly Ile Glu Arg Val Lys Leu Lys Glu Leu Val Ser Gly Val Arg
        115                 120                 125

Arg Glu Glu Val Ser Ala Gly Glu Leu Lys Lys Glu Asn Leu Lys Glu
    130                 135                 140

Leu Lys Lys Ile Leu Glu Lys Asp Leu Arg Trp Val Leu Asp Asp Asp
145                 150                 155                 160

Val Asp Val Glu Glu Phe Asp Leu Asp Lys Glu Phe Asp Pro Ala Lys
                165                 170                 175

Arg Trp Arg Asn Glu Gly Glu Ala Val Arg Val Leu Val Asp Arg Leu
            180                 185                 190

Ser Gly Arg Glu Ile Asn Glu Lys His Trp Lys Phe Val Arg Met Met
        195                 200                 205

Asn Gln Ser Gly Leu Gln Phe Thr Glu Asp Gln Met Leu Lys Ile Val
    210                 215                 220

Asp Arg Leu Gly Arg Lys Gln Ser Trp Lys Gln Ala Ser Ala Val Val
225                 230                 235                 240

His Trp Val Tyr Ser Asp Lys Lys Arg Lys His Leu Arg Ser Arg Phe
                245                 250                 255

Val Tyr Thr Lys Leu Leu Ser Val Leu Gly Phe Ala Arg Arg Pro Gln
            260                 265                 270

Glu Ala Leu Gln Ile Phe Asn Gln Met Leu Gly Asp Arg Gln Leu Tyr
        275                 280                 285

Pro Asp Met Ala Ala Tyr His Cys Ile Ala Val Thr Leu Gly Gln Ala
    290                 295                 300

Gly Leu Leu Lys Glu Leu Leu Lys Val Ile Glu Arg Met Arg Gln Lys
305                 310                 315                 320

Pro Thr Lys Leu Thr Lys Asn Leu Arg Gln Lys Asn Trp Asp Pro Val
                325                 330                 335

Leu Glu Pro Asp Leu Val Val Tyr Asn Ala Ile Leu Asn Ala Cys Val
            340                 345                 350

Pro Thr Leu Gln Trp Lys Ala Val Ser Trp Val Phe Val Glu Leu Arg
        355                 360                 365

Lys Asn Gly Leu Arg Pro Asn Gly Ala Thr Tyr Gly Leu Ala Met Glu
    370                 375                 380

Val Met Leu Glu Ser Gly Lys Phe Asp Arg Val His Asp Phe Phe Arg

385                 390                 395                 400

Lys Met Lys Ser Ser Gly Glu Ala Pro Lys Ala Ile Thr Tyr Lys Val
                405                 410                 415

Leu Val Arg Ala Leu Trp Arg Glu Gly Lys Ile Glu Glu Ala Val Glu
            420                 425                 430

Ala Val Arg Asp Met Glu Gln Lys Gly Val Ile Gly Thr Gly Ser Val
        435                 440                 445

Tyr Tyr Glu Leu Ala Cys Cys Leu Cys Asn Asn Gly Arg Trp Cys Asp
    450                 455                 460

Ala Met Leu Glu Val Gly Arg Met Lys Arg Leu Glu Asn Cys Arg Pro
465                 470                 475                 480

Leu Glu Ile Thr Phe Thr Gly Leu Ile Ala Ala Ser Leu Asn Gly Gly
                485                 490                 495

His Val Asp Asp Cys Met Ala Ile Phe Gln Tyr Met Lys Asp Lys Cys
            500                 505                 510

Asp Pro Asn Ile Gly Thr Ala Asn Met Met Leu Lys Val Tyr Gly Arg
        515                 520                 525

Asn Asp Met Phe Ser Glu Ala Lys Glu Leu Phe Glu Glu Ile Val Ser
    530                 535                 540

Arg Lys Glu Thr His Leu Val Pro Asn Glu Tyr Thr Tyr Ser Phe Met
545                 550                 555                 560

Leu Glu Ala Ser Ala Arg Ser Leu Gln Trp Glu Tyr Phe Glu His Val
                565                 570                 575

Tyr Gln Thr Met Val Leu Ser Gly Tyr Gln Met Asp Gln Thr Lys His
            580                 585                 590

Ala Ser Met Leu Ile Glu Ala Ser Arg Ala Gly Lys Trp Ser Leu Leu
        595                 600                 605

Glu His Ala Phe Asp Ala Val Leu Glu Asp Gly Glu Ile Pro His Pro
    610                 615                 620

Leu Phe Phe Thr Glu Leu Leu Cys His Ala Thr Ala Lys Gly Asp Phe
625                 630                 635                 640

Gln Arg Ala Ile Thr Leu Ile Asn Thr Val Ala Leu Ala Ser Phe Gln
                645                 650                 655

Ile Ser Glu Glu Glu Trp Thr Asp Leu Phe Glu Glu His Gln Asp Trp
            660                 665                 670

Leu Thr Gln Asp Asn Leu His Lys Leu Ser Asp His Leu Ile Glu Cys
        675                 680                 685

Asp Tyr Val Ser Glu Pro Thr Val Ser Asn Leu Ser Lys Ser Leu Lys
    690                 695                 700

Ser Arg Cys Gly Ser Ser Ser Ser Ser Ala Gln Pro Leu Leu Ala Val
705                 710                 715                 720

Asp Val Thr Thr Gln Ser Gln Gly Glu Lys Pro Glu Glu Asp Leu Leu
                725                 730                 735

Leu Gln Asp Thr Thr Met Glu Asp Asp Asn Ser Ala Asn Gly Glu Ala
            740                 745                 750

Trp Glu Phe Thr Glu Thr Glu Leu Glu Thr Leu Gly Leu Glu Glu Leu
        755                 760                 765

Glu Ile Asp Asp Asp Glu Glu Ser Ser Asp Ser Asp Ser Leu Ser Val
    770                 775                 780

Tyr Asp Ile Leu Lys Glu Trp Glu Glu Ser Ser Lys Lys Glu
785                 790                 795

<210> 298
<211> 415
<212> PRT
<213> Arabidopsis thaliana

<400> 298

Met Leu Ser Leu Asn Leu Ser Leu Lys Pro Gln His Leu Lys Leu Leu
1               5                   10                  15

Ser Cys Tyr Thr Asp Ser Ser Ala Pro Ser Ile Ala Lys Lys Leu Ile
            20                  25                  30

Lys Glu Ser Lys Leu Ser Arg Asp Phe Ser Gln Lys Ile Gln Ile Val
        35                  40                  45

Asp Tyr Ala Pro Leu Val Gln Thr Leu Ser Gln Arg Arg Leu Pro Asp
    50                  55                  60

Val Ala His Glu Ile Phe Leu Gln Thr Lys Ser Val Asn Leu Leu Pro
65                  70                  75                  80

Asn Tyr Arg Thr Leu Cys Ala Leu Met Leu Cys Phe Ala Glu Asn Gly
                85                  90                  95

Phe Val Leu Arg Ala Arg Thr Ile Trp Asp Glu Ile Ile Asn Ser Cys
            100                 105                 110

Phe Val Pro Asp Val Phe Val Val Ser Lys Leu Ile Ser Ala Tyr Glu
        115                 120                 125

Gln Phe Gly Cys Phe Asp Glu Val Ala Lys Ile Thr Lys Asp Val Ala
    130                 135                 140

Ala Arg His Ser Lys Leu Leu Pro Val Val Ser Ser Leu Ala Ile Ser
145                 150                 155                 160

Cys Phe Gly Lys Asn Gly Gln Leu Glu Leu Met Glu Gly Val Ile Glu
                165                 170                 175

Glu Met Asp Ser Lys Gly Val Leu Leu Glu Ala Glu Thr Ala Asn Val
            180                 185                 190

Ile Val Arg Tyr Tyr Ser Phe Phe Gly Ser Leu Asp Lys Met Glu Lys
        195                 200                 205

Ala Tyr Gly Arg Val Lys Lys Phe Gly Ile Val Ile Glu Glu Glu Glu
    210                 215                 220

Ile Arg Ala Val Val Leu Ala Tyr Leu Lys Gln Arg Lys Phe Tyr Arg
225                 230                 235                 240

Leu Arg Glu Phe Leu Ser Asp Val Gly Leu Gly Arg Arg Asn Leu Gly
                245                 250                 255

Asn Met Leu Trp Asn Ser Val Leu Leu Ser Tyr Ala Ala Asp Phe Lys
            260                 265                 270

Met Lys Ser Leu Gln Arg Glu Phe Ile Gly Met Leu Asp Ala Gly Phe
        275                 280                 285

Ser Pro Asp Leu Thr Thr Phe Asn Ile Arg Ala Leu Ala Phe Ser Arg
    290                 295                 300

Met Ala Leu Phe Trp Asp Leu His Leu Thr Leu Glu His Met Arg Arg
305                 310                 315                 320

Leu Asn Ile Val Pro Asp Leu Val Thr Phe Gly Cys Val Val Asp Ala
                325                 330                 335

Tyr Met Asp Lys Arg Leu Ala Arg Asn Leu Glu Phe Val Tyr Asn Arg
            340                 345                 350

Met Asn Leu Asp Asp Ser Pro Leu Val Leu Thr Asp Pro Leu Ala Phe
        355                 360                 365

Glu Val Leu Gly Lys Gly Asp Phe His Leu Ser Ser Glu Ala Val Leu
    370                 375                 380

Glu Phe Ser Pro Arg Lys Asn Trp Thr Tyr Arg Lys Leu Ile Gly Val
385                 390                 395                 400

Tyr Leu Lys Lys Lys Leu Arg Arg Asp Gln Ile Phe Trp Asn Tyr
                405                 410                 415

<210>  299
<211>  709
<212>  PRT
<213>  Arabidopsis thaliana

<400>  299

Met Leu Leu Leu Gln Gln Pro Pro Leu Val Ser Thr Arg Phe His Ser
1               5                   10                  15

Leu Tyr Phe Leu Thr His His His His His His His Arg Phe Phe Gln
            20                  25                  30

Pro Pro Ile Ser Ala Phe Ser Ala Thr Thr Ser Ala Ser Leu Pro Ser
        35                  40                  45

Pro Ser Pro Ser Ser Ser Ser Ser Tyr Phe Ser Ser Trp Asn Gly Leu
    50                  55                  60

Asp Thr Asn Glu Glu Glu Asp Asn Glu Phe Ser Ser Glu Val His Arg
65                  70                  75                  80

Arg Tyr Asp Phe Ser Pro Leu Leu Lys Phe Leu Ser Arg Phe Gly Pro
                85                  90                  95

Val Glu Leu Ala Leu Asp Ser Glu Ser Glu Ser Glu Ala Ser Pro Glu
            100                 105                 110

Ser Leu Asn Pro Val Glu Phe Asp Leu Val Glu Ser Tyr Arg Ala Val
        115                 120                 125

Pro Ala Pro Tyr Trp His Ser Leu Ile Lys Ser Leu Thr Ser Ser Thr
    130                 135                 140

Ser Ser Leu Gly Leu Ala Tyr Ala Val Val Ser Trp Leu Gln Lys His
145                 150                 155                 160

Asn Leu Cys Phe Ser Tyr Glu Leu Leu Tyr Ser Ile Leu Ile His Ala
                165                 170                 175

Leu Gly Arg Ser Glu Lys Leu Tyr Glu Ala Phe Leu Leu Ser Gln Lys
            180                 185                 190

Gln Thr Leu Thr Pro Leu Thr Tyr Asn Ala Leu Ile Gly Ala Cys Ala
        195                 200                 205

Arg Asn Asn Asp Ile Glu Lys Ala Leu Asn Leu Ile Ala Lys Met Arg
    210                 215                 220

Gln Asp Gly Tyr Gln Ser Asp Phe Val Asn Tyr Ser Leu Val Ile Gln
225                 230                 235                 240

Ser Leu Thr Arg Ser Asn Lys Ile Asp Ser Val Met Leu Leu Arg Leu
                245                 250                 255

Tyr Lys Glu Ile Glu Arg Asp Lys Leu Glu Leu Asp Val Gln Leu Val
            260                 265                 270

Asn Asp Ile Ile Met Gly Phe Ala Lys Ser Gly Asp Pro Ser Lys Ala
        275                 280                 285

Leu Gln Leu Leu Gly Met Ala Gln Ala Thr Gly Leu Ser Ala Lys Thr
    290                 295                 300

Ala Thr Leu Val Ser Ile Ile Ser Ala Leu Ala Asp Ser Gly Arg Thr
305                 310                 315                 320

Leu Glu Ala Glu Ala Leu Phe Glu Glu Leu Arg Gln Ser Gly Ile Lys
                325                 330                 335

Pro Arg Thr Arg Ala Tyr Asn Ala Leu Leu Lys Gly Tyr Val Lys Thr
            340                 345                 350

Gly Pro Leu Lys Asp Ala Glu Ser Met Val Ser Glu Met Glu Lys Arg
        355                 360                 365

Gly Val Ser Pro Asp Glu His Thr Tyr Ser Leu Leu Ile Asp Ala Tyr
    370                 375                 380

Val Asn Ala Gly Arg Trp Glu Ser Ala Arg Ile Val Leu Lys Glu Met
385                 390                 395                 400

Glu Ala Gly Asp Val Gln Pro Asn Ser Phe Val Phe Ser Arg Leu Leu
                405                 410                 415

Ala Gly Phe Arg Asp Arg Gly Glu Trp Gln Lys Thr Phe Gln Val Leu
            420                 425                 430

Lys Glu Met Lys Ser Ile Gly Val Lys Pro Asp Arg Gln Phe Tyr Asn
        435                 440                 445

Val Val Ile Asp Thr Phe Gly Lys Phe Asn Cys Leu Asp His Ala Met
    450                 455                 460

Thr Thr Phe Asp Arg Met Leu Ser Glu Gly Ile Glu Pro Asp Arg Val
465                 470                 475                 480

Thr Trp Asn Thr Leu Ile Asp Cys His Cys Lys His Gly Arg His Ile
                485                 490                 495

Val Ala Glu Glu Met Phe Glu Ala Met Glu Arg Arg Gly Cys Leu Pro
            500                 505                 510

Cys Ala Thr Thr Tyr Asn Ile Met Ile Asn Ser Tyr Gly Asp Gln Glu
        515                 520                 525

Arg Trp Asp Asp Met Lys Arg Leu Leu Gly Lys Met Lys Ser Gln Gly
    530                 535                 540

Ile Leu Pro Asn Val Val Thr His Thr Thr Leu Val Asp Val Tyr Gly
545                 550                 555                 560

Lys Ser Gly Arg Phe Asn Asp Ala Ile Glu Cys Leu Glu Glu Met Lys
                565                 570                 575

Ser Val Gly Leu Lys Pro Ser Ser Thr Met Tyr Asn Ala Leu Ile Asn
            580                 585                 590

Ala Tyr Ala Gln Arg Gly Leu Ser Glu Gln Ala Val Asn Ala Phe Arg
        595                 600                 605

Val Met Thr Ser Asp Gly Leu Lys Pro Ser Leu Leu Ala Leu Asn Ser
    610                 615                 620

Leu Ile Asn Ala Phe Gly Glu Asp Arg Arg Asp Ala Glu Ala Phe Ala

625                 630                 635                 640

Val Leu Gln Tyr Met Lys Glu Asn Gly Val Lys Pro Asp Val Val Thr
                645                 650                 655

Tyr Thr Thr Leu Met Lys Ala Leu Ile Arg Val Asp Lys Phe Gln Lys
            660                 665                 670

Val Pro Val Val Tyr Glu Glu Met Ile Met Ser Gly Cys Lys Pro Asp
        675                 680                 685

Arg Lys Ala Arg Ser Met Leu Arg Ser Ala Leu Arg Tyr Met Lys Gln
    690                 695                 700

Thr Leu Arg Ala Ser
705

<210>  300
<211>  735
<212>  PRT
<213>  Arabidopsis thaliana

<400>  300

Met Met Ile Lys Arg Ser Ile Thr Thr Asn Met Lys Ala Leu Arg Leu
1               5                   10                  15

Ile Gln Pro His Leu Leu Lys Thr Gly Ser Leu Arg Thr Asp Leu Leu
            20                  25                  30

Cys Thr Ile Ser Ser Phe Phe Ser Ser Cys Glu Arg Asp Phe Ser Ser
        35                  40                  45

Ile Ser Asn Gly Asn Val Cys Phe Arg Glu Arg Leu Arg Ser Gly Ile
    50                  55                  60

Val Asp Ile Lys Lys Asp Asp Ala Ile Ala Leu Phe Gln Glu Met Ile
65                  70                  75                  80

Arg Ser Arg Pro Leu Pro Ser Leu Val Asp Phe Ser Arg Phe Phe Ser
                85                  90                  95

Ala Ile Ala Arg Thr Lys Gln Phe Asn Leu Val Leu Asp Phe Cys Lys
            100                 105                 110

Gln Leu Glu Leu Asn Gly Ile Ala His Asn Ile Tyr Thr Leu Asn Ile
        115                 120                 125

Met Ile Asn Cys Phe Cys Arg Cys Cys Lys Thr Cys Phe Ala Tyr Ser

130                 135                 140

Val Leu Gly Lys Val Met Lys Leu Gly Tyr Glu Pro Asp Thr Thr Thr
145                 150                 155                 160

Phe Asn Thr Leu Ile Lys Gly Leu Phe Leu Glu Gly Lys Val Ser Glu
                165                 170                 175

Ala Val Val Leu Val Asp Arg Met Val Glu Asn Gly Cys Gln Pro Asp
            180                 185                 190

Val Val Thr Tyr Asn Ser Ile Val Asn Gly Ile Cys Arg Ser Gly Asp
        195                 200                 205

Thr Ser Leu Ala Leu Asp Leu Leu Arg Lys Met Glu Glu Arg Asn Val
    210                 215                 220

Lys Ala Asp Val Phe Thr Tyr Ser Thr Ile Ile Asp Ser Leu Cys Arg
225                 230                 235                 240

Asp Gly Cys Ile Asp Ala Ala Ile Ser Leu Phe Lys Glu Met Glu Thr
                245                 250                 255

Lys Gly Ile Lys Ser Ser Val Val Thr Tyr Asn Ser Leu Val Arg Gly
            260                 265                 270

Leu Cys Lys Ala Gly Lys Trp Asn Asp Gly Ala Leu Leu Leu Lys Asp
        275                 280                 285

Met Val Ser Arg Glu Ile Val Pro Asn Val Ile Thr Phe Asn Val Leu
    290                 295                 300

Leu Asp Val Phe Val Lys Glu Gly Lys Leu Gln Glu Ala Asn Glu Leu
305                 310                 315                 320

Tyr Lys Glu Met Ile Thr Arg Gly Ile Ser Pro Asn Ile Ile Thr Tyr
                325                 330                 335

Asn Thr Leu Met Asp Gly Tyr Cys Met Gln Asn Arg Leu Ser Glu Ala
            340                 345                 350

Asn Asn Met Leu Asp Leu Met Val Arg Asn Lys Cys Ser Pro Asp Ile
        355                 360                 365

Val Thr Phe Thr Ser Leu Ile Lys Gly Tyr Cys Met Val Lys Arg Val
    370                 375                 380

Asp Asp Gly Met Lys Val Phe Arg Asn Ile Ser Lys Arg Gly Leu Val
385                 390                 395                 400

Ala Asn Ala Val Thr Tyr Ser Ile Leu Val Gln Gly Phe Cys Gln Ser
                405                 410                 415

Gly Lys Ile Lys Leu Ala Glu Glu Leu Phe Gln Glu Met Val Ser His
            420                 425                 430

Gly Val Leu Pro Asp Val Met Thr Tyr Gly Ile Leu Leu Asp Gly Leu
        435                 440                 445

Cys Asp Asn Gly Lys Leu Glu Lys Ala Leu Glu Ile Phe Glu Asp Leu
    450                 455                 460

Gln Lys Ser Lys Met Asp Leu Gly Ile Val Met Tyr Thr Thr Ile Ile
465                 470                 475                 480

Glu Gly Met Cys Lys Gly Gly Lys Val Glu Asp Ala Trp Asn Leu Phe
                485                 490                 495

Cys Ser Leu Pro Cys Lys Gly Val Lys Pro Asn Val Met Thr Tyr Thr
            500                 505                 510

Val Met Ile Ser Gly Leu Cys Lys Lys Gly Ser Leu Ser Glu Ala Asn
        515                 520                 525

Ile Leu Leu Arg Lys Met Glu Glu Asp Gly Asn Ala Pro Asn Asp Cys
    530                 535                 540

Thr Tyr Asn Thr Leu Ile Arg Ala His Leu Arg Asp Gly Asp Leu Thr
545                 550                 555                 560

Ala Ser Ala Lys Leu Ile Glu Glu Met Lys Ser Cys Gly Phe Ser Ala
                565                 570                 575

Asp Ala Ser Ser Ile Lys Met Val Ile Asp Met Leu Leu Ser Ala Met
            580                 585                 590

Lys Arg Leu Thr Leu Arg Tyr Cys Leu Ser Lys Gly Ser Lys Ser Arg
        595                 600                 605

Gln Asp Leu Leu Glu Leu Ser Gly Ser Glu Lys Ile Arg Leu Ser Ser
    610                 615                 620

Leu Thr Phe Val Lys Met Phe Pro Cys Asn Thr Ile Thr Thr Ser Leu
625                 630                 635                 640

Asn Val Asn Thr Ile Glu Ala Arg Gly Met Asn Ser Ala Glu Leu Asn
                645                 650                 655

Arg Asp Leu Arg Lys Leu Arg Arg Ser Ser Val Leu Lys Lys Phe Lys
            660                 665                 670

Asn Arg Asp Val Arg Val Leu Val Thr Asn Glu Leu Leu Thr Trp Gly
        675                 680                 685

Leu Glu Asp Ala Glu Cys Asp Leu Met Val Asp Leu Glu Leu Pro Thr
    690                 695                 700

Asp Ala Val His Tyr Ala His Arg Ala Gly Arg Met Arg Arg Pro Gly
705                 710                 715                 720

Arg Lys Met Thr Val Val Thr Val Cys Glu Glu Ser Gln Val Leu
                725                 730                 735

<210>  301
<211>  1006
<212>  PRT
<213>  Arabidopsis thaliana

<400>  301

Met Ala Val Thr Ile Ser Thr Asn Ala Phe Val Asn Ala Ser Leu Leu
1               5                   10                  15

Asp Glu Ser Arg Asn Ser Phe Trp Arg Pro Leu Phe His Gln Pro Tyr
            20                  25                  30

Tyr Asn Cys Arg Arg Val Val Arg Leu Asn Ser Arg Lys Leu Asn Ser
        35                  40                  45

Lys Val Met Phe Cys Leu Asn Leu Asn Thr Lys Glu Val Gly Leu Gln
    50                  55                  60

Lys Pro Gly Asp Lys Gly Phe Glu Phe Lys Pro Ser Phe Asp Gln Tyr
65                  70                  75                  80

Leu Gln Ile Met Glu Ser Val Lys Thr Ala Arg Lys Lys Lys Lys Phe
                85                  90                  95

Asp Arg Leu Lys Val Glu Glu Asp Asp Gly Gly Gly Gly Asn Gly Asp
            100                 105                 110

Ser Val Tyr Glu Val Lys Asp Met Lys Ile Lys Ser Gly Glu Leu Lys
        115                 120                 125

Asp Glu Thr Phe Arg Lys Arg Tyr Ser Arg Gln Glu Ile Val Ser Asp
    130                 135                 140

Lys Arg Asn Glu Arg Val Phe Lys Arg Asn Gly Glu Ile Glu Asn His
145                 150                 155                 160

Arg Val Ala Thr Asp Leu Lys Trp Ser Lys Ser Gly Glu Ser Ser Val
                165                 170                 175

Ala Leu Lys Leu Ser Lys Ser Gly Glu Ser Ser Val Thr Val Pro Glu
            180                 185                 190

Asp Glu Ser Phe Arg Lys Arg Tyr Ser Lys Gln Glu Tyr His Arg Ser
        195                 200                 205

Ser Asp Thr Ser Arg Gly Ile Glu Arg Gly Ser Arg Gly Asp Glu Leu
    210                 215                 220

Asp Leu Val Val Glu Glu Arg Arg Val Gln Arg Ile Ala Lys Asp Ala
225                 230                 235                 240

Arg Trp Ser Lys Ser Arg Glu Ser Ser Val Ala Val Lys Trp Ser Asn
                245                 250                 255

Ser Gly Glu Ser Ser Val Thr Met Pro Lys Asp Glu Ser Phe Arg Arg
            260                 265                 270

Arg Tyr Ser Lys Gln Glu His His Arg Ser Ser Asp Thr Ser Arg Gly
        275                 280                 285

Ile Ala Arg Gly Ser Lys Gly Asp Glu Leu Glu Leu Val Val Glu Glu
    290                 295                 300

Arg Arg Val Gln Arg Ile Ala Lys Asp Val Arg Trp Ser Lys Ser Asp
305                 310                 315                 320

Glu Ser Leu Val Pro Val Ser Glu Asp Glu Ser Phe Arg Arg Gly Asn
                325                 330                 335

Pro Lys Gln Glu Met Val Arg Tyr Gln Arg Val Ser Asp Thr Ser Arg
            340                 345                 350

Gly Ile Glu Arg Gly Ser Lys Gly Asp Gly Leu Asp Leu Leu Ala Glu
        355                 360                 365

Glu Arg Arg Ile Glu Arg Leu Ala Asn Glu Arg His Glu Ile Arg Ser
    370                 375                 380

Ser Lys Leu Ser Gly Thr Arg Arg Ile Gly Ala Lys Arg Asn Asp Asp
385                 390                 395                 400

Asp Asp Asp Ser Leu Phe Ala Met Glu Thr Pro Ala Phe Arg Phe Ser
                405                 410                 415

Asp Glu Ser Ser Asp Ile Val Asp Lys Pro Ala Thr Ser Arg Val Glu
            420                 425                 430

Met Glu Asp Arg Ile Glu Lys Leu Ala Lys Val Leu Asn Gly Ala Asp
        435                 440                 445

Ile Asn Met Pro Glu Trp Gln Phe Ser Lys Ala Ile Arg Ser Ala Lys
    450                 455                 460

Ile Arg Tyr Thr Asp Tyr Thr Val Met Arg Leu Ile His Phe Leu Gly
465                 470                 475                 480

Lys Leu Gly Asn Trp Arg Arg Val Leu Gln Val Ile Glu Trp Leu Gln
                485                 490                 495

Arg Gln Asp Arg Tyr Lys Ser Asn Lys Ile Arg Ile Ile Tyr Thr Thr
            500                 505                 510

Ala Leu Asn Val Leu Gly Lys Ser Arg Arg Pro Val Glu Ala Leu Asn
        515                 520                 525

Val Phe His Ala Met Leu Leu Gln Ile Ser Ser Tyr Pro Asp Met Val
    530                 535                 540

Ala Tyr Arg Ser Ile Ala Val Thr Leu Gly Gln Ala Gly His Ile Lys
545                 550                 555                 560

Glu Leu Phe Tyr Val Ile Asp Thr Met Arg Ser Pro Pro Lys Lys Lys
                565                 570                 575

Phe Lys Pro Thr Thr Leu Glu Lys Trp Asp Pro Arg Leu Glu Pro Asp
            580                 585                 590

Val Val Val Tyr Asn Ala Val Leu Asn Ala Cys Val Gln Arg Lys Gln
        595                 600                 605

Trp Glu Gly Ala Phe Trp Val Leu Gln Gln Leu Lys Gln Arg Gly Gln
    610                 615                 620

Lys Pro Ser Pro Val Thr Tyr Gly Leu Ile Met Glu Val Met Leu Ala

625                 630                 635                 640

Cys Glu Lys Tyr Asn Leu Val His Glu Phe Phe Arg Lys Met Gln Lys
                645                 650                 655

Ser Ser Ile Pro Asn Ala Leu Ala Tyr Arg Val Leu Val Asn Thr Leu
            660                 665                 670

Trp Lys Glu Gly Lys Ser Asp Glu Ala Val His Thr Val Glu Asp Met
        675                 680                 685

Glu Ser Arg Gly Ile Val Gly Ser Ala Ala Leu Tyr Tyr Asp Leu Ala
    690                 695                 700

Arg Cys Leu Cys Ser Ala Gly Arg Cys Asn Glu Gly Leu Asn Met Val
705                 710                 715                 720

Asn Phe Val Asn Pro Val Val Leu Lys Leu Ile Glu Asn Leu Ile Tyr
                725                 730                 735

Lys Ala Asp Leu Val His Thr Ile Gln Phe Gln Leu Lys Lys Ile Cys
            740                 745                 750

Arg Val Ala Asn Lys Pro Leu Val Val Thr Tyr Thr Gly Leu Ile Gln
        755                 760                 765

Ala Cys Val Asp Ser Gly Asn Ile Lys Asn Ala Ala Tyr Ile Phe Asp
    770                 775                 780

Gln Met Lys Lys Val Cys Ser Pro Asn Leu Val Thr Cys Asn Ile Met
785                 790                 795                 800

Leu Lys Ala Tyr Leu Gln Gly Gly Leu Phe Glu Glu Ala Arg Glu Leu
                805                 810                 815

Phe Gln Lys Met Ser Glu Asp Gly Asn His Ile Lys Asn Ser Ser Asp
            820                 825                 830

Phe Glu Ser Arg Val Leu Pro Asp Thr Tyr Thr Phe Asn Thr Met Leu
        835                 840                 845

Asp Thr Cys Ala Glu Gln Glu Lys Trp Asp Asp Phe Gly Tyr Ala Tyr
    850                 855                 860

Arg Glu Met Leu Arg His Gly Tyr His Phe Asn Ala Lys Arg His Leu
865                 870                 875                 880

Arg Met Val Leu Glu Ala Ser Arg Ala Gly Lys Glu Glu Val Met Glu
                885                 890                 895

Ala Thr Trp Glu His Met Arg Arg Ser Asn Arg Ile Pro Pro Ser Pro
            900                 905                 910

Leu Ile Lys Glu Arg Phe Phe Arg Lys Leu Glu Lys Gly Asp His Ile
        915                 920                 925

Ser Ala Ile Ser Ser Leu Ala Asp Leu Asn Gly Lys Ile Glu Glu Thr
    930                 935                 940

Glu Leu Arg Ala Phe Ser Thr Ser Ala Trp Ser Arg Val Leu Ser Arg
945                 950                 955                 960

Phe Glu Gln Asp Ser Val Leu Arg Leu Met Asp Asp Val Asn Arg Arg
                965                 970                 975

Leu Gly Ser Arg Ser Glu Ser Ser Asp Ser Val Leu Gly Asn Leu Leu
            980                 985                 990

Ser Ser Cys Lys Asp Tyr Leu Lys  Thr Arg Thr His Asn  Leu
        995                 1000                1005

<210>  302
<211>  613
<212>  PRT
<213>  Arabidopsis thaliana

<400>  302

Met Phe Val His Lys Leu Arg Cys Tyr Tyr Gly Phe Leu Leu Lys His
1               5                   10                  15

Phe Glu Asn Cys Leu Leu Trp Leu Val Ala Gly Asn Ala Leu Asn Cys
            20                  25                  30

Leu Phe Ile Asp Ser Ser Gly Phe Gln Arg Tyr Leu Gly Phe Gly Val
        35                  40                  45

Thr Asn Leu Asn Gly Ala Thr Val Lys Ser Tyr Lys Gln Glu Gly Phe
    50                  55                  60

Val Ile Asp Glu Arg Gly Lys Leu Lys Arg Phe Asn Arg Lys Lys Leu
65                  70                  75                  80

Ser Arg Lys Arg Cys Gly Ser Leu Arg Gly Arg Gly Trp Lys Tyr Gly
                85                  90                  95

Ser Gly Phe Val Asp Gly Ile Phe Pro Val Leu Ser Pro Ile Ala Gln
            100                 105                 110

Lys Ile Leu Ser Phe Ile Gln Lys Glu Thr Asp Pro Asp Lys Val Ala
        115                 120                 125

Asp Val Leu Gly Ala Leu Pro Ser Thr His Ala Ser Trp Asp Asp Leu
    130                 135                 140

Ile Asn Val Ser Val Gln Leu Arg Leu Asn Lys Lys Trp Asp Ser Ile
145                 150                 155                 160

Ile Leu Val Cys Glu Trp Ile Leu Arg Lys Ser Ser Phe Gln Pro Asp
                165                 170                 175

Val Ile Cys Phe Asn Leu Leu Ile Asp Ala Tyr Gly Gln Lys Phe Gln
            180                 185                 190

Tyr Lys Glu Ala Glu Ser Leu Tyr Val Gln Leu Leu Glu Ser Arg Tyr
        195                 200                 205

Val Pro Thr Glu Asp Thr Tyr Ala Leu Leu Ile Lys Ala Tyr Cys Met
    210                 215                 220

Ala Gly Leu Ile Glu Arg Ala Glu Val Val Leu Val Glu Met Gln Asn
225                 230                 235                 240

His His Val Ser Pro Lys Thr Ile Gly Val Thr Val Tyr Asn Ala Tyr
                245                 250                 255

Ile Glu Gly Leu Met Lys Arg Lys Gly Asn Thr Glu Glu Ala Ile Asp
            260                 265                 270

Val Phe Gln Arg Met Lys Arg Asp Arg Cys Lys Pro Thr Thr Glu Thr
        275                 280                 285

Tyr Asn Leu Met Ile Asn Leu Tyr Gly Lys Ala Ser Lys Ser Tyr Met
    290                 295                 300

Ser Trp Lys Leu Tyr Cys Glu Met Arg Ser His Gln Cys Lys Pro Asn
305                 310                 315                 320

Ile Cys Thr Tyr Thr Ala Leu Val Asn Ala Phe Ala Arg Glu Gly Leu
                325                 330                 335

Cys Glu Lys Ala Glu Glu Ile Phe Glu Gln Leu Gln Glu Asp Gly Leu
            340                 345                 350

Glu Pro Asp Val Tyr Val Tyr Asn Ala Leu Met Glu Ser Tyr Ser Arg
        355                 360                 365

Ala Gly Tyr Pro Tyr Gly Ala Ala Glu Ile Phe Ser Leu Met Gln His
    370                 375                 380

Met Gly Cys Glu Pro Asp Arg Ala Ser Tyr Asn Ile Met Val Asp Ala
385                 390                 395                 400

Tyr Gly Arg Ala Gly Leu His Ser Asp Ala Glu Ala Val Phe Glu Glu
                405                 410                 415

Met Lys Arg Leu Gly Ile Ala Pro Thr Met Lys Ser His Met Leu Leu
            420                 425                 430

Leu Ser Ala Tyr Ser Lys Ala Arg Asp Val Thr Lys Cys Glu Ala Ile
        435                 440                 445

Val Lys Glu Met Ser Glu Asn Gly Val Glu Pro Asp Thr Phe Val Leu
    450                 455                 460

Asn Ser Met Leu Asn Leu Tyr Gly Arg Leu Gly Gln Phe Thr Lys Met
465                 470                 475                 480

Glu Lys Ile Leu Ala Glu Met Glu Asn Gly Pro Cys Thr Ala Asp Ile
                485                 490                 495

Ser Thr Tyr Asn Ile Leu Ile Asn Ile Tyr Gly Lys Ala Gly Phe Leu
            500                 505                 510

Glu Arg Ile Glu Glu Leu Phe Val Glu Leu Lys Glu Lys Asn Phe Arg
        515                 520                 525

Pro Asp Val Val Thr Trp Thr Ser Arg Ile Gly Ala Tyr Ser Arg Lys
    530                 535                 540

Lys Leu Tyr Val Lys Cys Leu Glu Val Phe Glu Glu Met Ile Asp Ser
545                 550                 555                 560

Gly Cys Ala Pro Asp Gly Gly Thr Ala Lys Val Leu Leu Ser Ala Cys
                565                 570                 575

Ser Ser Glu Glu Gln Val Glu Gln Val Thr Ser Val Leu Arg Thr Met
            580                 585                 590

His Lys Gly Val Thr Val Ser Ser Leu Val Pro Lys Leu Met Ala Lys
        595                 600                 605

Ser Leu Thr Val Asn
    610

<210>  303
<211>  822
<212>  PRT
<213>  Arabidopsis thaliana

<400>  303

Met Ala Thr Val Thr Asn Phe Lys Leu Val Thr Pro Pro Glu Ser Ser
1               5                   10                  15

Arg Ala Asp Lys Pro Gly Ala Thr Lys Ala Ser Asp Ala Phe Gln Glu
            20                  25                  30

Lys Lys Ser Val Ser Val Asn Tyr Asp Arg Gly Glu His Glu Val Ser
        35                  40                  45

Val Asn Ile Gly Gly Leu Arg Lys Ala Asp Ile Pro Arg Arg Tyr Arg
    50                  55                  60

Ile Arg Val Glu Asn Asp Arg Phe Gln Lys Asp Trp Ser Val Ser Glu
65                  70                  75                  80

Val Val Asp Arg Leu Met Ala Leu Asn Arg Trp Glu Glu Val Asp Gly
                85                  90                  95

Val Leu Asn Ser Trp Val Gly Arg Phe Ala Arg Lys Asn Phe Pro Val
            100                 105                 110

Leu Ile Arg Glu Leu Ser Arg Arg Gly Cys Ile Glu Leu Cys Val Asn
        115                 120                 125

Val Phe Lys Trp Met Lys Ile Gln Lys Asn Tyr Cys Ala Arg Asn Asp
    130                 135                 140

Ile Tyr Asn Met Met Ile Arg Leu His Ala Arg His Asn Trp Val Asp
145                 150                 155                 160

Gln Ala Arg Gly Leu Phe Phe Glu Met Gln Lys Trp Ser Cys Lys Pro
                165                 170                 175

Asp Ala Glu Thr Tyr Asp Ala Leu Ile Asn Ala His Gly Arg Ala Gly
            180                 185                 190

Gln Trp Arg Trp Ala Met Asn Leu Met Asp Asp Met Leu Arg Ala Ala
        195                 200                 205

Ile Ala Pro Ser Arg Ser Thr Tyr Asn Asn Leu Ile Asn Ala Cys Gly
    210                 215                 220

Ser Ser Gly Asn Trp Arg Glu Ala Leu Glu Val Cys Lys Lys Met Thr
225                 230                 235                 240

Asp Asn Gly Val Gly Pro Asp Leu Val Thr His Asn Ile Val Leu Ser
                245                 250                 255

Ala Tyr Lys Ser Gly Arg Gln Tyr Ser Lys Ala Leu Ser Tyr Phe Glu
            260                 265                 270

Leu Met Lys Gly Ala Lys Val Arg Pro Asp Thr Thr Thr Phe Asn Ile
        275                 280                 285

Ile Ile Tyr Cys Leu Ser Lys Leu Gly Gln Ser Ser Gln Ala Leu Asp
    290                 295                 300

Leu Phe Asn Ser Met Arg Glu Lys Arg Ala Glu Cys Arg Pro Asp Val
305                 310                 315                 320

Val Thr Phe Thr Ser Ile Met His Leu Tyr Ser Val Lys Gly Glu Ile
                325                 330                 335

Glu Asn Cys Arg Ala Val Phe Glu Ala Met Val Ala Glu Gly Leu Lys
            340                 345                 350

Pro Asn Ile Val Ser Tyr Asn Ala Leu Met Gly Ala Tyr Ala Val His
        355                 360                 365

Gly Met Ser Gly Thr Ala Leu Ser Val Leu Gly Asp Ile Lys Gln Asn
    370                 375                 380

Gly Ile Ile Pro Asp Val Val Ser Tyr Thr Cys Leu Leu Asn Ser Tyr
385                 390                 395                 400

Gly Arg Ser Arg Gln Pro Gly Lys Ala Lys Glu Val Phe Leu Met Met
                405                 410                 415

Arg Lys Glu Arg Arg Lys Pro Asn Val Val Thr Tyr Asn Ala Leu Ile
            420                 425                 430

Asp Ala Tyr Gly Ser Asn Gly Phe Leu Ala Glu Ala Val Glu Ile Phe
        435                 440                 445

Arg Gln Met Glu Gln Asp Gly Ile Lys Pro Asn Val Val Ser Val Cys

450                 455                 460

Thr Leu Leu Ala Ala Cys Ser Arg Ser Lys Lys Lys Val Asn Val Asp
465                 470                 475                 480

Thr Val Leu Ser Ala Ala Gln Ser Arg Gly Ile Asn Leu Asn Thr Ala
                485                 490                 495

Ala Tyr Asn Ser Ala Ile Gly Ser Tyr Ile Asn Ala Ala Glu Leu Glu
            500                 505                 510

Lys Ala Ile Ala Leu Tyr Gln Ser Met Arg Lys Lys Lys Val Lys Ala
        515                 520                 525

Asp Ser Val Thr Phe Thr Ile Leu Ile Ser Gly Ser Cys Arg Met Ser
    530                 535                 540

Lys Tyr Pro Glu Ala Ile Ser Tyr Leu Lys Glu Met Glu Asp Leu Ser
545                 550                 555                 560

Ile Pro Leu Thr Lys Glu Val Tyr Ser Ser Val Leu Cys Ala Tyr Ser
                565                 570                 575

Lys Gln Gly Gln Val Thr Glu Ala Glu Ser Ile Phe Asn Gln Met Lys
            580                 585                 590

Met Ala Gly Cys Glu Pro Asp Val Ile Ala Tyr Thr Ser Met Leu His
        595                 600                 605

Ala Tyr Asn Ala Ser Glu Lys Trp Gly Lys Ala Cys Glu Leu Phe Leu
    610                 615                 620

Glu Met Glu Ala Asn Gly Ile Glu Pro Asp Ser Ile Ala Cys Ser Ala
625                 630                 635                 640

Leu Met Arg Ala Phe Asn Lys Gly Gly Gln Pro Ser Asn Val Phe Val
                645                 650                 655

Leu Met Asp Leu Met Arg Glu Lys Glu Ile Pro Phe Thr Gly Ala Val
            660                 665                 670

Phe Phe Glu Ile Phe Ser Ala Cys Asn Thr Leu Gln Glu Trp Lys Arg
        675                 680                 685

Ala Ile Asp Leu Ile Gln Met Met Asp Pro Tyr Leu Pro Ser Leu Ser
    690                 695                 700

Ile Gly Leu Thr Asn Gln Met Leu His Leu Phe Gly Lys Ser Gly Lys
705                 710                 715                 720

Val Glu Ala Met Met Lys Leu Phe Tyr Lys Ile Ile Ala Ser Gly Val
                725                 730                 735

Gly Ile Asn Leu Lys Thr Tyr Ala Ile Leu Leu Glu His Leu Leu Ala
            740                 745                 750

Val Gly Asn Trp Arg Lys Tyr Ile Glu Val Leu Glu Trp Met Ser Gly
        755                 760                 765

Ala Gly Ile Gln Pro Ser Asn Gln Met Tyr Arg Asp Ile Ile Ser Phe
    770                 775                 780

Gly Glu Arg Ser Ala Gly Ile Glu Phe Glu Pro Leu Ile Arg Gln Lys
785                 790                 795                 800

Leu Glu Ser Leu Arg Asn Lys Gly Glu Gly Leu Ile Pro Thr Phe Arg
                805                 810                 815

His Glu Gly Thr Leu Leu
            820

<210>  304
<211>  1440
<212>  PRT
<213>  Arabidopsis thaliana

<400>  304

Met Ala Val Ser Ala Gly Ala Leu Ala Phe Pro Ala Leu Ser Val Arg
1               5                   10                  15

Ala Thr Leu Asn Pro Glu Ile Lys Asp Glu Gln Ala Asn Ile Ser Ser
            20                  25                  30

Thr Thr Ser Ser Ser Gln Lys Phe Thr Tyr Ser Arg Ala Ser Pro Ala
        35                  40                  45

Val Arg Trp Pro His Leu Asn Leu Arg Glu Ile Tyr Asp Ser Thr Pro
    50                  55                  60

Ser Gln Thr Leu Ser Ser Pro Val Ser Pro Ile Ala Gly Thr Pro Asp
65                  70                  75                  80

Ser Gly Asp Val Val Asp Ser Ile Ala Ser Arg Glu Glu Gln Lys Thr
                85                  90                  95

Lys Asp Glu Thr Ala Val Ala Thr Arg Arg Arg Arg Val Lys Lys Met
            100                 105                 110

Asn Lys Val Ala Leu Ile Lys Ala Lys Asp Trp Arg Glu Arg Val Lys
        115                 120                 125

Phe Leu Thr Asp Lys Ile Leu Ser Leu Lys Ser Asn Gln Phe Val Ala
    130                 135                 140

Asp Ile Leu Asp Ala Arg Leu Val Gln Met Thr Pro Thr Asp Tyr Cys
145                 150                 155                 160

Phe Val Val Lys Ser Val Gly Gln Glu Ser Trp Gln Arg Ala Leu Glu
                165                 170                 175

Val Phe Glu Trp Leu Asn Leu Arg His Trp His Ser Pro Asn Ala Arg
            180                 185                 190

Met Val Ala Ala Ile Leu Gly Val Leu Gly Arg Trp Asn Gln Glu Ser
        195                 200                 205

Leu Ala Val Glu Ile Phe Thr Arg Ala Glu Pro Thr Val Gly Asp Arg
    210                 215                 220

Val Gln Val Tyr Asn Ala Met Met Gly Val Tyr Ser Arg Ser Gly Lys
225                 230                 235                 240

Phe Ser Lys Ala Gln Glu Leu Val Asp Ala Met Arg Gln Arg Gly Cys
                245                 250                 255

Val Pro Asp Leu Ile Ser Phe Asn Thr Leu Ile Asn Ala Arg Leu Lys
            260                 265                 270

Ser Gly Gly Leu Thr Pro Asn Leu Ala Val Glu Leu Leu Asp Met Val
        275                 280                 285

Arg Asn Ser Gly Leu Arg Pro Asp Ala Ile Thr Tyr Asn Thr Leu Leu
    290                 295                 300

Ser Ala Cys Ser Arg Asp Ser Asn Leu Asp Gly Ala Val Lys Val Phe
305                 310                 315                 320

Glu Asp Met Glu Ala His Arg Cys Gln Pro Asp Leu Trp Thr Tyr Asn
                325                 330                 335

Ala Met Ile Ser Val Tyr Gly Arg Cys Gly Leu Ala Ala Glu Ala Glu
            340                 345                 350

Arg Leu Phe Met Glu Leu Glu Leu Lys Gly Phe Phe Pro Asp Ala Val
        355                 360                 365

Thr Tyr Asn Ser Leu Leu Tyr Ala Phe Ala Arg Glu Arg Asn Thr Glu
    370                 375                 380

Lys Val Lys Glu Val Tyr Gln Gln Met Gln Lys Met Gly Phe Gly Lys
385                 390                 395                 400

Asp Glu Met Thr Tyr Asn Thr Ile Ile His Met Tyr Gly Lys Gln Gly
                405                 410                 415

Gln Leu Asp Leu Ala Leu Gln Leu Tyr Lys Asp Met Lys Gly Leu Ser
            420                 425                 430

Gly Arg Asn Pro Asp Ala Ile Thr Tyr Thr Val Leu Ile Asp Ser Leu
        435                 440                 445

Gly Lys Ala Asn Arg Thr Val Glu Ala Ala Ala Leu Met Ser Glu Met
    450                 455                 460

Leu Asp Val Gly Ile Lys Pro Thr Leu Gln Thr Tyr Ser Ala Leu Ile
465                 470                 475                 480

Cys Gly Tyr Ala Lys Ala Gly Lys Arg Glu Glu Ala Glu Asp Thr Phe
                485                 490                 495

Ser Cys Met Leu Arg Ser Gly Thr Lys Pro Asp Asn Leu Ala Tyr Ser
            500                 505                 510

Val Met Leu Asp Val Leu Leu Arg Gly Asn Glu Thr Arg Lys Ala Trp
        515                 520                 525

Gly Leu Tyr Arg Asp Met Ile Ser Asp Gly His Thr Pro Ser Tyr Thr
    530                 535                 540

Leu Tyr Glu Leu Met Ile Leu Gly Leu Met Lys Glu Asn Arg Ser Asp
545                 550                 555                 560

Asp Ile Gln Lys Thr Ile Arg Asp Met Glu Glu Leu Cys Gly Met Asn
                565                 570                 575

Pro Leu Glu Ile Ser Ser Val Leu Val Lys Gly Glu Cys Phe Asp Leu
            580                 585                 590

Ala Ala Arg Gln Leu Lys Val Ala Ile Thr Asn Gly Tyr Glu Leu Glu
        595                 600                 605

Asn Asp Thr Leu Leu Ser Ile Leu Gly Ser Tyr Ser Ser Ser Gly Arg
    610                 615                 620

His Ser Glu Ala Phe Glu Leu Leu Glu Phe Leu Lys Glu His Ala Ser
625                 630                 635                 640

Gly Ser Lys Arg Leu Ile Thr Glu Ala Leu Ile Val Leu His Cys Lys
                645                 650                 655

Val Asn Asn Leu Ser Ala Ala Leu Asp Glu Tyr Phe Ala Asp Pro Cys
            660                 665                 670

Val His Gly Trp Cys Phe Gly Ser Ser Thr Met Tyr Glu Thr Leu Leu
        675                 680                 685

His Cys Cys Val Ala Asn Glu His Tyr Ala Glu Ala Ser Gln Val Phe
    690                 695                 700

Ser Asp Leu Arg Leu Ser Gly Cys Glu Ala Ser Glu Ser Val Cys Lys
705                 710                 715                 720

Ser Met Val Val Val Tyr Cys Lys Leu Gly Phe Pro Glu Thr Ala His
                725                 730                 735

Gln Val Val Asn Gln Ala Glu Thr Lys Gly Phe His Phe Ala Cys Ser
            740                 745                 750

Pro Met Tyr Thr Asp Ile Ile Glu Ala Tyr Gly Lys Gln Lys Leu Trp
        755                 760                 765

Gln Lys Ala Glu Ser Val Val Gly Asn Leu Arg Gln Ser Gly Arg Thr
    770                 775                 780

Pro Asp Leu Lys Thr Trp Asn Ser Leu Met Ser Ala Tyr Ala Gln Cys
785                 790                 795                 800

Gly Cys Tyr Glu Arg Ala Arg Ala Ile Phe Asn Thr Met Met Arg Asp
                805                 810                 815

Gly Pro Ser Pro Thr Val Glu Ser Ile Asn Ile Leu Leu His Ala Leu
            820                 825                 830

Cys Val Asp Gly Arg Leu Glu Glu Leu Tyr Val Val Val Glu Glu Leu
        835                 840                 845

Gln Asp Met Gly Phe Lys Ile Ser Lys Ser Ser Ile Leu Leu Met Leu

850                 855                 860

Asp Ala Phe Ala Arg Ala Gly Asn Ile Phe Glu Val Lys Lys Ile Tyr
865                 870                 875                 880

Ser Ser Met Lys Ala Ala Gly Tyr Leu Pro Thr Ile Arg Leu Tyr Arg
                885                 890                 895

Met Met Ile Glu Leu Leu Cys Lys Gly Lys Arg Val Arg Asp Ala Glu
            900                 905                 910

Ile Met Val Ser Glu Met Glu Glu Ala Asn Phe Lys Val Glu Leu Ala
        915                 920                 925

Ile Trp Asn Ser Met Leu Lys Met Tyr Thr Ala Ile Glu Asp Tyr Lys
    930                 935                 940

Lys Thr Val Gln Val Tyr Gln Arg Ile Lys Glu Thr Gly Leu Glu Pro
945                 950                 955                 960

Asp Glu Thr Thr Tyr Asn Thr Leu Ile Ile Met Tyr Cys Arg Asp Arg
                965                 970                 975

Arg Pro Glu Glu Gly Tyr Leu Leu Met Gln Gln Met Arg Asn Leu Gly
            980                 985                 990

Leu Asp Pro Lys Leu Asp Thr Tyr  Lys Ser Leu Ile Ser  Ala Phe Gly
        995                 1000                1005

Lys Gln  Lys Cys Leu Glu Gln  Ala Glu Gln Leu Phe  Glu Glu Leu
    1010                1015                1020

Leu Ser  Lys Gly Leu Lys Leu  Asp Arg Ser Phe Tyr  His Thr Met
    1025                1030                1035

Met Lys  Ile Ser Arg Asp Ser  Gly Ser Asp Ser Lys  Ala Glu Lys
    1040                1045                1050

Leu Leu  Gln Met Met Lys Asn  Ala Gly Ile Glu Pro  Thr Leu Ala
    1055                1060                1065

Thr Met  His Leu Leu Met Val  Ser Tyr Ser Ser Ser  Gly Asn Pro
    1070                1075                1080

Gln Glu  Ala Glu Lys Val Leu  Ser Asn Leu Lys Asp  Thr Glu Val
    1085                1090                1095

Glu Leu Thr Thr Leu Pro Tyr Ser Ser Val Ile Asp Ala Tyr Leu
    1100                1105                1110

Arg Ser Lys Asp Tyr Asn Ser Gly Ile Glu Arg Leu Leu Glu Met
    1115                1120                1125

Lys Lys Glu Gly Leu Glu Pro Asp His Arg Ile Trp Thr Cys Phe
    1130                1135                1140

Val Arg Ala Ala Ser Phe Ser Lys Glu Lys Ile Glu Val Met Leu
    1145                1150                1155

Leu Leu Lys Ala Leu Glu Asp Ile Gly Phe Asp Leu Pro Ile Arg
    1160                1165                1170

Leu Leu Ala Gly Arg Pro Glu Leu Leu Val Ser Glu Val Asp Gly
    1175                1180                1185

Trp Phe Glu Lys Leu Lys Ser Ile Glu Asp Asn Ala Ala Leu Asn
    1190                1195                1200

Phe Val Asn Ala Leu Leu Asn Leu Leu Trp Ala Phe Glu Leu Arg
    1205                1210                1215

Ala Thr Ala Ser Trp Val Phe Gln Leu Gly Ile Lys Arg Gly Ile
    1220                1225                1230

Phe Ser Leu Asp Val Phe Arg Val Ala Asp Lys Asp Trp Gly Ala
    1235                1240                1245

Asp Phe Arg Arg Leu Ser Gly Gly Ala Ala Leu Val Ala Leu Thr
    1250                1255                1260

Leu Trp Leu Asp His Met Gln Asp Ala Ser Leu Glu Gly Tyr Pro
    1265                1270                1275

Glu Ser Pro Lys Ser Val Val Leu Ile Thr Gly Thr Ala Glu Tyr
    1280                1285                1290

Asn Gly Ile Ser Leu Asp Lys Thr Leu Lys Ala Cys Leu Trp Glu
    1295                1300                1305

Met Gly Ser Pro Phe Leu Pro Cys Lys Thr Arg Thr Gly Leu Leu
    1310                1315                1320

Val Ala Lys Ala His Ser Leu Arg Met Trp Leu Lys Asp Ser Pro
    1325                1330                1335

Phe Cys Phe Asp Leu Glu Leu Lys Asp Ser Val Ser Leu Pro Glu
    1340                1345                1350

Ser Asn Ser Met Asp Leu Ile Asp Gly Cys Phe Ile Arg Arg Gly
    1355                1360                1365

Leu Val Pro Ala Phe Asn His Ile Lys Glu Arg Leu Gly Gly Phe
    1370                1375                1380

Val Ser Pro Lys Lys Phe Ser Arg Leu Ala Leu Leu Pro Asp Glu
    1385                1390                1395

Met Arg Glu Arg Val Ile Lys Thr Asp Ile Glu Gly His Arg Gln
    1400                1405                1410

Lys Leu Glu Lys Met Lys Lys Lys Lys Met Gly Asn Glu Thr Asn
    1415                1420                1425

Gly Ile Asn Thr Arg Arg Lys Phe Val Arg Ser Lys
    1430                1435                1440

<210> 305
<211> 499
<212> PRT
<213> Arabidopsis thaliana

<400> 305

Met Ala Leu Ile Gln Asn Pro Val Gln Gly Thr Thr Ser Ala Tyr Ala
1               5                   10                  15

Asn Glu Ile Ala Gln Leu Gly Phe Ser Arg Ser Val Val Gln Gln His
            20                  25                  30

Ile Ser Ser Pro Val Tyr Phe Arg Cys Ile Pro Thr Ile Ser Ile Thr
        35                  40                  45

Pro Thr Met Cys Ser Thr Lys Val Pro Asn Glu Arg Thr Glu Lys Met
    50                  55                  60

Asn Ser Gly Leu Ile Ser Thr Arg His Gln Val Asp Pro Lys Lys Glu
65                  70                  75                  80

Leu Ser Arg Ile Leu Arg Thr Asp Ala Ala Val Lys Gly Ile Glu Arg
                85                  90                  95

Lys Ala Asn Ser Glu Lys Tyr Leu Thr Leu Trp Pro Lys Ala Val Leu
            100                 105                 110

Glu Ala Leu Asp Glu Ala Ile Lys Glu Asn Arg Trp Gln Ser Ala Leu
        115                 120                 125

Lys Ile Phe Asn Leu Leu Arg Lys Gln His Trp Tyr Glu Pro Arg Cys
    130                 135                 140

Lys Thr Tyr Thr Lys Leu Phe Lys Val Leu Gly Asn Cys Lys Gln Pro
145                 150                 155                 160

Asp Gln Ala Ser Leu Leu Phe Glu Val Met Leu Ser Glu Gly Leu Lys
                165                 170                 175

Pro Thr Ile Asp Val Tyr Thr Ser Leu Ile Ser Val Tyr Gly Lys Ser
            180                 185                 190

Glu Leu Leu Asp Lys Ala Phe Ser Thr Leu Glu Tyr Met Lys Ser Val
        195                 200                 205

Ser Asp Cys Lys Pro Asp Val Phe Thr Phe Thr Val Leu Ile Ser Cys
    210                 215                 220

Cys Cys Lys Leu Gly Arg Phe Asp Leu Val Lys Ser Ile Val Leu Glu
225                 230                 235                 240

Met Ser Tyr Leu Gly Val Gly Cys Ser Thr Val Thr Tyr Asn Thr Ile
                245                 250                 255

Ile Asp Gly Tyr Gly Lys Ala Gly Met Phe Glu Glu Met Glu Ser Val
            260                 265                 270

Leu Ala Asp Met Ile Glu Asp Gly Asp Ser Leu Pro Asp Val Cys Thr
        275                 280                 285

Leu Asn Ser Ile Ile Gly Ser Tyr Gly Asn Gly Arg Asn Met Arg Lys
    290                 295                 300

Met Glu Ser Trp Tyr Ser Arg Phe Gln Leu Met Gly Val Gln Pro Asp
305                 310                 315                 320

Ile Thr Thr Phe Asn Ile Leu Ile Leu Ser Phe Gly Lys Ala Gly Met
                325                 330                 335

Tyr Lys Lys Met Cys Ser Val Met Asp Phe Met Glu Lys Arg Phe Phe
            340                 345                 350

Ser Leu Thr Thr Val Thr Tyr Asn Ile Val Ile Glu Thr Phe Gly Lys
        355                 360                 365

Ala Gly Arg Ile Glu Lys Met Asp Asp Val Phe Arg Lys Met Lys Tyr
    370                 375                 380

Gln Gly Val Lys Pro Asn Ser Ile Thr Tyr Cys Ser Leu Val Asn Ala
385                 390                 395                 400

Tyr Ser Lys Ala Gly Leu Val Val Lys Ile Asp Ser Val Leu Arg Gln
                405                 410                 415

Ile Val Asn Ser Asp Val Val Leu Asp Thr Pro Phe Phe Asn Cys Ile
            420                 425                 430

Ile Asn Ala Tyr Gly Gln Ala Gly Asp Leu Ala Thr Met Lys Glu Leu
        435                 440                 445

Tyr Ile Gln Met Glu Glu Arg Lys Cys Lys Pro Asp Lys Ile Thr Phe
    450                 455                 460

Ala Thr Met Ile Lys Thr Tyr Thr Ala His Gly Ile Phe Asp Ala Val
465                 470                 475                 480

Gln Glu Leu Glu Lys Gln Met Ile Ser Ser Asp Ile Gly Lys Lys Arg
                485                 490                 495

Leu Thr Glu

<210>  306
<211>  551
<212>  PRT
<213>  Arabidopsis thaliana

<400>  306

Met Val Leu Ile His Thr Ser Val Gly Phe Phe Lys Arg Phe Ser Thr
1               5                   10                  15

Ser Ala Thr Pro Ser Thr Ser Ser Ala Ser Asp Trp Lys Thr Gln Gln
            20                  25                  30

Thr Leu Phe Arg Val Ala Thr Glu Ile Ser Ser Ile Leu Leu Gln Arg
        35                  40                  45

Arg Asn Trp Ile Thr His Leu Gln Tyr Val Lys Ser Lys Leu Pro Arg
    50                  55                  60

Ser Thr Leu Thr Ser Pro Val Phe Leu Gln Ile Leu Arg Glu Thr Arg
65                  70                  75                  80

Lys Cys Pro Lys Thr Thr Leu Asp Phe Phe Asp Phe Ala Lys Thr His
                85                  90                  95

Leu Arg Phe Glu Pro Asp Leu Lys Ser His Cys Arg Val Ile Glu Val
            100                 105                 110

Ala Ala Glu Ser Gly Leu Leu Glu Arg Ala Glu Met Leu Leu Arg Pro
        115                 120                 125

Leu Val Glu Thr Asn Ser Val Ser Leu Val Val Gly Glu Met His Arg
    130                 135                 140

Trp Phe Glu Gly Glu Val Ser Leu Ser Val Ser Leu Ser Leu Val Leu
145                 150                 155                 160

Glu Tyr Tyr Ala Leu Lys Gly Ser His His Asn Gly Leu Glu Val Phe
                165                 170                 175

Gly Phe Met Arg Arg Leu Arg Leu Ser Pro Ser Gln Ser Ala Tyr Asn
            180                 185                 190

Ser Leu Leu Gly Ser Leu Val Lys Glu Asn Gln Phe Arg Val Ala Leu
        195                 200                 205

Cys Leu Tyr Ser Ala Met Val Arg Asn Gly Ile Val Ser Asp Glu Leu
    210                 215                 220

Thr Trp Asp Leu Ile Ala Gln Ile Leu Cys Glu Gln Gly Arg Ser Lys
225                 230                 235                 240

Ser Val Phe Lys Leu Met Glu Thr Gly Val Glu Ser Cys Lys Ile Tyr
                245                 250                 255

Thr Asn Leu Val Glu Cys Tyr Ser Arg Asn Gly Glu Phe Asp Ala Val
            260                 265                 270

Phe Ser Leu Ile His Glu Met Asp Asp Lys Lys Leu Glu Leu Ser Phe
        275                 280                 285

Cys Ser Tyr Gly Cys Val Leu Asp Asp Ala Cys Arg Leu Gly Asp Ala
    290                 295                 300

Glu Phe Ile Asp Lys Val Leu Cys Leu Met Val Glu Lys Lys Phe Val
305                 310                 315                 320

Thr Leu Gly Asp Ser Ala Val Asn Asp Lys Ile Ile Glu Arg Leu Cys

                325                 330                 335

Asp Met Gly Lys Thr Phe Ala Ser Glu Met Leu Phe Arg Lys Ala Cys
            340                 345                 350

Asn Gly Glu Thr Val Arg Leu Trp Asp Ser Thr Tyr Gly Cys Met Leu
        355                 360                 365

Lys Ala Leu Ser Arg Lys Lys Arg Thr Lys Glu Ala Val Asp Val Tyr
    370                 375                 380

Arg Met Ile Cys Arg Lys Gly Ile Thr Val Leu Asp Glu Ser Cys Tyr
385                 390                 395                 400

Ile Glu Phe Ala Asn Ala Leu Cys Arg Asp Asp Asn Ser Ser Glu Glu
                405                 410                 415

Glu Glu Glu Leu Leu Val Asp Val Ile Lys Arg Gly Lys Glu Asp Gly
            420                 425                 430

Asn Pro Gln Arg Ser Phe Leu Ile Arg Leu Trp Lys Trp Arg Ser Gly
        435                 440                 445

Lys Leu Glu Lys Ala Leu Val Leu His Glu Lys Ile Lys Lys Met Lys
    450                 455                 460

Gly Ser Leu Asp Val Asn Ala Tyr Asn Ala Val Leu Asp Arg Leu Met
465                 470                 475                 480

Met Arg Gln Lys Glu Met Val Glu Glu Ala Val Val Val Phe Glu Tyr
                485                 490                 495

Met Lys Glu Ile Asn Ser Val Asn Ser Lys Ser Phe Thr Ile Met Ile
            500                 505                 510

Gln Gly Leu Cys Arg Val Lys Glu Met Lys Lys Ala Met Arg Ser His
        515                 520                 525

Asp Glu Met Leu Arg Leu Gly Leu Lys Pro Asp Leu Val Thr Tyr Lys
    530                 535                 540

Arg Leu Ile Leu Gly Phe Lys
545                 550

<210> 307
<211> 508
<212> PRT
<213> Arabidopsis thaliana

<400>  307

Met Val Ser Leu Ser Thr Ser Thr Ser His Ala Pro Pro Leu Pro Thr
1               5                   10                  15

Asn Arg Arg Thr Ala Glu Arg Thr Phe Thr Val Arg Cys Ile Ser Ile
            20                  25                  30

Ser Pro Arg Glu Pro Asn Tyr Ala Ile Thr Ser Asp Lys Ser Asn Asn
        35                  40                  45

Thr Ser Leu Ser Leu Arg Glu Thr Arg Gln Ser Lys Trp Leu Ile Asn
    50                  55                  60

Ala Glu Asp Val Asn Glu Arg Asp Ser Lys Glu Ile Lys Glu Asp Lys
65                  70                  75                  80

Asn Thr Lys Ile Ala Ser Arg Lys Ala Ile Ser Ile Ile Leu Arg Arg
                85                  90                  95

Glu Ala Thr Lys Ser Ile Ile Glu Lys Lys Lys Gly Ser Lys Lys Leu
            100                 105                 110

Leu Pro Arg Thr Val Leu Glu Ser Leu His Glu Arg Ile Thr Ala Leu
        115                 120                 125

Arg Trp Glu Ser Ala Ile Gln Val Phe Glu Leu Leu Arg Glu Gln Leu
    130                 135                 140

Trp Tyr Lys Pro Asn Val Gly Ile Tyr Val Lys Leu Ile Val Met Leu
145                 150                 155                 160

Gly Lys Cys Lys Gln Pro Glu Lys Ala His Glu Leu Phe Gln Glu Met
                165                 170                 175

Ile Asn Glu Gly Cys Val Val Asn His Glu Val Tyr Thr Ala Leu Val
            180                 185                 190

Ser Ala Tyr Ser Arg Ser Gly Arg Phe Asp Ala Ala Phe Thr Leu Leu
        195                 200                 205

Glu Arg Met Lys Ser Ser His Asn Cys Gln Pro Asp Val His Thr Tyr
    210                 215                 220

Ser Ile Leu Ile Lys Ser Phe Leu Gln Val Phe Ala Phe Asp Lys Val
225                 230                 235                 240

Gln Asp Leu Leu Ser Asp Met Arg Arg Gln Gly Ile Arg Pro Asn Thr
                245                 250                 255

Ile Thr Tyr Asn Thr Leu Ile Asp Ala Tyr Gly Lys Ala Lys Met Phe
            260                 265                 270

Val Glu Met Glu Ser Thr Leu Ile Gln Met Leu Gly Glu Asp Asp Cys
        275                 280                 285

Lys Pro Asp Ser Trp Thr Met Asn Ser Thr Leu Arg Ala Phe Gly Gly
    290                 295                 300

Asn Gly Gln Ile Glu Met Met Glu Asn Cys Tyr Glu Lys Phe Gln Ser
305                 310                 315                 320

Ser Gly Ile Glu Pro Asn Ile Arg Thr Phe Asn Ile Leu Leu Asp Ser
                325                 330                 335

Tyr Gly Lys Ser Gly Asn Tyr Lys Lys Met Ser Ala Val Met Glu Tyr
            340                 345                 350

Met Gln Lys Tyr His Tyr Ser Trp Thr Ile Val Thr Tyr Asn Val Val
        355                 360                 365

Ile Asp Ala Phe Gly Arg Ala Gly Asp Leu Lys Gln Met Glu Tyr Leu
    370                 375                 380

Phe Arg Leu Met Gln Ser Glu Arg Ile Phe Pro Ser Cys Val Thr Leu
385                 390                 395                 400

Cys Ser Leu Val Arg Ala Tyr Gly Arg Ala Ser Lys Ala Asp Lys Ile
                405                 410                 415

Gly Gly Val Leu Arg Phe Ile Glu Asn Ser Asp Ile Arg Leu Asp Leu
            420                 425                 430

Val Phe Phe Asn Cys Leu Val Asp Ala Tyr Gly Arg Met Glu Lys Phe
        435                 440                 445

Ala Glu Met Lys Gly Val Leu Glu Leu Met Glu Lys Lys Gly Phe Lys
    450                 455                 460

Pro Asp Lys Ile Thr Tyr Arg Thr Met Val Lys Ala Tyr Arg Ile Ser
465                 470                 475                 480

Gly Met Thr Thr His Val Lys Glu Leu His Gly Val Val Glu Ser Val
                485                 490                 495

Gly Glu Ala Gln Val Val Val Lys Lys Pro Asp Phe
            500                 505

<210>  308
<211>  723
<212>  PRT
<213>  Arabidopsis thaliana

<400>  308

Met Ser Met Ala Ser Ser Ser Leu Ala Thr Gln Ser Phe Phe Ser Ser
1               5                   10                  15

Phe Pro Leu Ser His Arg Leu His Phe Pro Val Pro Tyr Leu Leu Leu
            20                  25                  30

Arg Ser Ser Phe Phe Arg Lys Pro Leu Ser Leu Ser Ala Thr Ser Pro
        35                  40                  45

Ser Ser Ser Ser Ser Ser Pro Ser Ile Phe Leu Ser Cys Phe Asp Asp
    50                  55                  60

Ala Leu Pro Asp Lys Ile Gln Gln Pro Glu Asn Ser Thr Ile Asn Ser
65                  70                  75                  80

Glu Glu Ser Glu Cys Glu Glu Glu Asp Asp Glu Glu Gly Asp Asp Phe
                85                  90                  95

Thr Asp Pro Ile Leu Lys Phe Phe Lys Ser Arg Thr Leu Thr Ser Glu
            100                 105                 110

Ser Thr Ala Asp Pro Ala Arg Glu Ser Lys Phe Ser Leu Gln Lys Asn
        115                 120                 125

Arg Arg Thr Ser Trp His Leu Ala Pro Asp Phe Ala Asp Pro Glu Thr
    130                 135                 140

Glu Ile Glu Ser Lys Pro Glu Glu Ser Val Phe Val Thr Asn Gln Gln
145                 150                 155                 160

Thr Leu Gly Val His Ile Pro Phe Glu Ser Gly Val Ala Arg Glu Ile
                165                 170                 175

Leu Glu Leu Ala Lys Asn Leu Lys Glu Asn Gln Thr Leu Gly Glu Met
            180                 185                 190

Leu Ser Gly Phe Glu Arg Arg Val Ser Asp Thr Glu Cys Val Glu Ala
        195                 200                 205

Leu Val Met Met Gly Glu Ser Gly Phe Val Lys Ser Cys Leu Tyr Phe
    210                 215                 220

Tyr Glu Trp Met Ser Leu Gln Glu Pro Ser Leu Ala Ser Pro Arg Ala
225                 230                 235                 240

Cys Ser Val Leu Phe Thr Leu Leu Gly Arg Glu Arg Met Ala Asp Tyr
                245                 250                 255

Ile Leu Leu Leu Leu Ser Asn Leu Pro Asp Lys Glu Glu Phe Arg Asp
            260                 265                 270

Val Arg Leu Tyr Asn Ala Ala Ile Ser Gly Leu Ser Ala Ser Gln Arg
        275                 280                 285

Tyr Asp Asp Ala Trp Glu Val Tyr Glu Ala Met Asp Lys Ile Asn Val
    290                 295                 300

Tyr Pro Asp Asn Val Thr Cys Ala Ile Leu Ile Thr Thr Leu Arg Lys
305                 310                 315                 320

Ala Gly Arg Ser Ala Lys Glu Val Trp Glu Ile Phe Glu Lys Met Ser
                325                 330                 335

Glu Lys Gly Val Lys Trp Ser Gln Asp Val Phe Gly Gly Leu Val Lys
            340                 345                 350

Ser Phe Cys Asp Glu Gly Leu Lys Glu Glu Ala Leu Val Ile Gln Thr
        355                 360                 365

Glu Met Glu Lys Lys Gly Ile Arg Ser Asn Thr Ile Val Tyr Asn Thr
    370                 375                 380

Leu Met Asp Ala Tyr Asn Lys Ser Asn His Ile Glu Glu Val Glu Gly
385                 390                 395                 400

Leu Phe Thr Glu Met Arg Asp Lys Gly Leu Lys Pro Ser Ala Ala Thr
                405                 410                 415

Tyr Asn Ile Leu Met Asp Ala Tyr Ala Arg Arg Met Gln Pro Asp Ile
            420                 425                 430

Val Glu Thr Leu Leu Arg Glu Met Glu Asp Leu Gly Leu Glu Pro Asn
        435                 440                 445

Val Lys Ser Tyr Thr Cys Leu Ile Ser Ala Tyr Gly Arg Thr Lys Lys
    450                 455                 460

Met Ser Asp Met Ala Ala Asp Ala Phe Leu Arg Met Lys Lys Val Gly
465                 470                 475                 480

Leu Lys Pro Ser Ser His Ser Tyr Thr Ala Leu Ile His Ala Tyr Ser
                485                 490                 495

Val Ser Gly Trp His Glu Lys Ala Tyr Ala Ser Phe Glu Glu Met Cys
            500                 505                 510

Lys Glu Gly Ile Lys Pro Ser Val Glu Thr Tyr Thr Ser Val Leu Asp
        515                 520                 525

Ala Phe Arg Arg Ser Gly Asp Thr Gly Lys Leu Met Glu Ile Trp Lys
    530                 535                 540

Leu Met Leu Arg Glu Lys Ile Lys Gly Thr Arg Ile Thr Tyr Asn Thr
545                 550                 555                 560

Leu Leu Asp Gly Phe Ala Lys Gln Gly Leu Tyr Ile Glu Ala Arg Asp
                565                 570                 575

Val Val Ser Glu Phe Ser Lys Met Gly Leu Gln Pro Ser Val Met Thr
            580                 585                 590

Tyr Asn Met Leu Met Asn Ala Tyr Ala Arg Gly Gly Gln Asp Ala Lys
        595                 600                 605

Leu Pro Gln Leu Leu Lys Glu Met Ala Ala Leu Asn Leu Lys Pro Asp
    610                 615                 620

Ser Ile Thr Tyr Ser Thr Met Ile Tyr Ala Phe Val Arg Val Arg Asp
625                 630                 635                 640

Phe Lys Arg Ala Phe Phe Tyr His Lys Met Met Val Lys Ser Gly Gln
                645                 650                 655

Val Pro Asp Pro Arg Ser Tyr Glu Lys Leu Arg Ala Ile Leu Glu Asp
            660                 665                 670

Lys Ala Lys Thr Lys Asn Arg Lys Asp Lys Thr Ala Ile Leu Gly Ile
        675                 680                 685

Ile Asn Ser Lys Phe Gly Arg Val Lys Ala Lys Thr Lys Gly Lys Lys
    690                 695                 700

Asp Glu Phe Trp Lys Tyr Lys Thr Asn Arg Thr Thr Ser Pro Gly Arg

705                 710                 715                 720

His Arg Ser

<210>  309
<211>  4
<212>  PRT
<213>  Artificial Sequence

<220>
<223>  crPPR N terminal side

<400>  309

Met Gly Asn Ser
1

<210>  310
<211>  14
<212>  PRT
<213>  Artificial Sequence

<220>
<223>  crPPR C terminal side

<400>  310

Val Thr Tyr Thr Thr Leu Ile Ser Gly Leu Gly Lys Ala Gly
1               5                   10

<210>  311
<211>  35
<212>  PRT

<213>  Artificial Sequence

<220>
<223>  crPPR(7L/31F)

<400>  311

Val Thr Tyr Thr Thr Leu Leu Ser Gly Leu Gly Lys Ala Gly Arg Leu
1               5                   10                  15

Glu Glu Ala Leu Glu Leu Phe Glu Glu Met Lys Glu Lys Gly Phe Val
            20                  25                  30

Pro Asn Val
        35

<210>  312
<211>  14
<212>  PRT

<213>  Artificial Sequence

<220>
<223>  crPPR(7L/31F)

<400>  312

Val Thr Tyr Thr Thr Leu Leu Ser Gly Leu Gly Lys Ala Gly
1               5                   10


<210>  313
<211>  35
<212>  PRT
<213>  Artificial Sequence

<220>
<223>  MODIFIED TYPE crPPR(7L/31F)

<400>  313

Val Thr Tyr Thr Thr Leu Ile Ser Gly Leu Gly Lys Ala Gly Arg Leu
1               5                   10                  15


Glu Glu Ala Leu Glu Leu Phe Glu Glu Met Lys Glu Lys Gly Phe Val
            20                  25                  30


Pro Asn Val
        35


<210>  314
<211>  35
<212>  PRT
<213>  Artificial Sequence

<220>
<223>  MODIFIED TYPE crPPR(7L/31F)

<400>  314

Val Thr Tyr Thr Thr Leu Leu Ser Ala Leu Gly Lys Ala Gly Arg Leu
1               5                   10                  15


Glu Glu Ala Leu Glu Leu Phe Glu Glu Met Lys Glu Lys Gly Phe Val
            20                  25                  30


Pro Asn Val
        35


<210>  315
<211>  14
<212>  PRT
<213>  Artificial Sequence

<220>
<223>  MODIFIED TYPE crPPR(7L/31F)

<400>  315

Val Thr Tyr Thr Thr Leu Leu Ser Ala Leu Gly Lys Ala Gly
1               5                   10

<210>  316
<211>  35
<212>  PRT
<213>  Artificial Sequence

<220>
<223>  MODIFIED TYPE crPPR(7L/31F)

<400>  316

Val Thr Tyr Thr Thr Leu Leu Ser Gly Tyr Gly Lys Ala Gly Arg Leu
1               5                   10                  15

Glu Glu Ala Leu Glu Leu Phe Glu Glu Met Lys Glu Lys Gly Phe Val
            20                  25                  30

Pro Asn Val
        35

<210>  317
<211>  14
<212>  PRT
<213>  Artificial Sequence

<220>
<223>  MODIFIED TYPE crPPR(7L/31F)

<400>  317

Val Thr Tyr Thr Thr Leu Leu Ser Gly Tyr Gly Lys Ala Gly
1               5                   10

<210>  318
<211>  35
<212>  PRT
<213>  Artificial Sequence

<220>
<223>  MODIFIED TYPE crPPR(7L/31F)

<400>  318

Val Thr Tyr Thr Thr Leu Leu Ser Gly Leu Gly Lys Ala Gly Arg Leu
1               5                   10                  15

Glu Lys Ala Leu Glu Leu Phe Glu Glu Met Lys Glu Lys Gly Phe Val
            20                  25                  30

Pro Asn Val
        35

<210>  319
<211>  35
<212>  PRT
<213>  Artificial Sequence

<220>
<223>  MODIFIED TYPE crPPR(7L/31F)

<400>  319

Val Thr Tyr Thr Thr Leu Leu Ser Gly Leu Gly Lys Ala Gly Arg Leu
1               5                   10                  15

Glu Glu Ala Glu Glu Leu Phe Glu Glu Met Lys Glu Lys Gly Phe Val
            20                  25                  30

Pro Asn Val
        35

<210>  320
<211>  35
<212>  PRT
<213>  Artificial Sequence

<220>
<223>  MODIFIED TYPE crPPR(7L/31F)

<400>  320

Val Thr Tyr Thr Thr Leu Leu Ser Gly Leu Gly Lys Ala Gly Arg Leu
1               5                   10                  15

Glu Glu Ala Leu Glu Leu Phe Glu Glu Met Lys Glu Glu Gly Phe Val
            20                  25                  30

Pro Asn Val
        35

<210>  321
<211>  35
<212>  PRT
<213>  Artificial Sequence

<220>
<223>  MODIFIED TYPE crPPR(7L/31F)

<400>  321

Val Thr Tyr Thr Thr Leu Leu Ser Gly Leu Gly Lys Ala Gly Arg Leu
1               5                   10                  15

Glu Glu Ala Leu Glu Leu Phe Glu Glu Met Lys Glu Lys Gly Ile Val
            20                  25                  30

Pro Asn Val

        35

<210>  322
<211>  35
<212>  PRT
<213>  Artificial Sequence

<220>
<223>  MODIFIED TYPE crPPR(7L/31F)

<400>  322

Val Thr Tyr Thr Thr Leu Leu Ser Gly Leu Gly Lys Ala Gly Arg Leu
1               5                   10                  15

Glu Glu Ala Leu Glu Leu Phe Glu Glu Met Lys Glu Lys Gly Phe Lys
            20                  25                  30

Pro Asn Val
        35

<210>  323
<211>  35
<212>  PRT
<213>  Artificial Sequence

<220>
<223>  MODIFIED TYPE crPPR(7L/31F)

<400>  323

Val Thr Tyr Thr Thr Leu Leu Ser Ala Tyr Gly Lys Ala Gly Arg Leu
1               5                   10                  15

Glu Glu Ala Leu Glu Leu Phe Glu Glu Met Lys Glu Lys Gly Phe Val
            20                  25                  30

Pro Asn Val
        35

<210>  324
<211>  14
<212>  PRT
<213>  Artificial Sequence

<220>
<223>  MODIFIED TYPE crPPR(7L/31F)

<400>  324

Val Thr Tyr Thr Thr Leu Leu Ser Ala Tyr Gly Lys Ala Gly
1               5                   10

<210>  325
<211>  35

<212>  PRT
<<213>  Artificial Sequence

<220>
<223>  MODIFIED TYPE crPPR(7L/31F)

<400>  325

Val Thr Tyr Thr Thr Leu Leu Ser Gly Leu Gly Lys Ala Gly Arg Leu
1               5                   10                  15

Glu His Ala Leu Glu Leu Phe Glu Glu Met Lys Glu Lys Gly Phe Val
            20                  25                  30

Pro Asn Val
        35

<210>  326
<211>  35
<212>  PRT
<213>  Artificial Sequence

<220>
<223>  MODIFIED TYPE crPPR(7L/31F)

<400>  326

Val Thr Tyr Thr Thr Leu Leu Ser Gly Leu Gly Lys Ala Gly Arg Leu
1               5                   10                  15

Glu Arg Ala Leu Glu Leu Phe Glu Glu Met Lys Glu Lys Gly Phe Val
            20                  25                  30

Pro Asn Val
        35

<210>  327
<211>  35
<212>  PRT
<213>  Artificial Sequence

<220>
<223>  MODIFIED TYPE crPPR(7L/31F)

<400>  327

Val Thr Tyr Thr Thr Leu Leu Ser Gly Leu Gly Lys Ala Gly Arg Leu
1               5                   10                  15

Glu Lys Ala Leu Glu Leu Phe Glu Glu Met Lys Glu Lys Gly Val Val
            20                  25                  30

Pro Asn Val
        35

<210>  328
<211>  35
<212>  PRT
<213>  Artificial Sequence

<220>
<223>  MODIFIED TYPE crPPR(7L/31F)

<400>  328

Val Thr Tyr Thr Thr Leu Leu Ser Gly Leu Gly Lys Ala Gly Arg Leu
1               5                   10                  15

Glu Lys Ala Leu Glu Leu Phe Glu Glu Met Lys Glu Lys Gly Leu Val
            20                  25                  30

Pro Asn Val
        35

<210>  329
<211>  35
<212>  PRT
<213>  Artificial Sequence

<220>
<223>  MODIFIED TYPE crPPR(7L/31F)

<400>  329

Val Thr Tyr Thr Thr Leu Leu Ser Gly Leu Gly Lys Ala Gly Arg Leu
1               5                   10                  15

Glu Glu Ala Leu Glu Leu Phe Glu Glu Met Lys Glu Lys Gly Phe His
            20                  25                  30

Pro Asn Val
        35

<210>  330
<211>  35
<212>  PRT
<213>  Artificial Sequence

<220>
<223>  MODIFIED TYPE crPPR(7L/31F)

<400>  330

Val Thr Tyr Thr Thr Leu Leu Ser Gly Leu Gly Lys Ala Gly Arg Leu
1               5                   10                  15

Glu Glu Ala Leu Glu Leu Phe Glu Glu Met Lys Glu Lys Gly Phe Arg
            20                  25                  30

Pro Asn Val
        35

<210>  331
<211>  35
<212>  PRT
<213>  Artificial Sequence

<220>
<223>  MODIFIED TYPE crPPR(7L/31F)

<400>  331

Val Thr Tyr Thr Thr Leu Leu Ser Ala Phe Gly Lys Ala Gly Arg Leu
1               5                   10                  15

Glu Glu Ala Leu Glu Leu Phe Glu Glu Met Lys Glu Lys Gly Phe Val
            20                  25                  30

Pro Asn Val
        35

<210>  332
<211>  14
<212>  PRT
<213>  Artificial Sequence

<220>
<223>  MODIFIED TYPE crPPR(7L/31F)

<400>  332

Val Thr Tyr Thr Thr Leu Leu Ser Ala Phe Gly Lys Ala Gly
1               5                   10

<210>  333
<211>  35
<212>  PRT
<213>  Artificial Sequence

<220>
<223>  MODIFIED TYPE crPPR(7L/31F)

<400>  333

Val Thr Tyr Thr Thr Leu Leu Ser Ala Trp Gly Lys Ala Gly Arg Leu
1               5                   10                  15

Glu Glu Ala Leu Glu Leu Phe Glu Glu Met Lys Glu Lys Gly Phe Val
            20                  25                  30

Pro Asn Val
        35

<210>  334

<211>  14
<212>  PRT
<213>  Artificial Sequence

<220>
<223>  MODIFIED TYPE crPPR(7L/31F)

<400>  334

Val Thr Tyr Thr Thr Leu Leu Ser Ala Trp Gly Lys Ala Gly
1               5                   10


<210>  335
<211>  298
<212>  PRT
<213>  Artificial Sequence

<220>
<223>  crPPR

<400>  335

Met Gly Asn Ser Val Thr Tyr Thr Thr Leu Ile Ser Gly Leu Gly Lys
1               5                   10                  15


Ala Gly Arg Leu Glu Glu Ala Leu Glu Leu Phe Glu Glu Met Lys Glu
            20                  25                  30


Lys Gly Ile Val Pro Asn Val Val Thr Tyr Thr Thr Leu Ile Ser Gly
        35                  40                  45


Leu Gly Lys Ala Gly Arg Leu Glu Glu Ala Leu Glu Leu Phe Glu Glu
    50                  55                  60


Met Lys Glu Lys Gly Ile Val Pro Asn Val Val Thr Tyr Thr Thr Leu
65                  70                  75                  80


Ile Ser Gly Leu Gly Lys Ala Gly Arg Leu Glu Glu Ala Leu Glu Leu
                85                  90                  95


Phe Glu Glu Met Lys Glu Lys Gly Ile Val Pro Asn Val Val Thr Tyr
            100                 105                 110


Thr Thr Leu Ile Ser Gly Leu Gly Lys Ala Gly Arg Leu Glu Glu Ala
        115                 120                 125


Leu Glu Leu Phe Glu Glu Met Lys Glu Lys Gly Ile Val Pro Asn Val
    130                 135                 140


Val Thr Tyr Thr Thr Leu Ile Ser Gly Leu Gly Lys Ala Gly Arg Leu
145                 150                 155                 160

Glu Glu Ala Leu Glu Leu Phe Glu Glu Met Lys Glu Lys Gly Ile Val
                165                 170                 175

Pro Asn Val Val Thr Tyr Thr Thr Leu Ile Ser Gly Leu Gly Lys Ala
            180                 185                 190

Gly Arg Leu Glu Glu Ala Leu Glu Leu Phe Glu Glu Met Lys Glu Lys
        195                 200                 205

Gly Ile Val Pro Asn Val Val Thr Tyr Thr Thr Leu Ile Ser Gly Leu
    210                 215                 220

Gly Lys Ala Gly Arg Leu Glu Glu Ala Leu Glu Leu Phe Glu Glu Met
225                 230                 235                 240

Lys Glu Lys Gly Ile Val Pro Asn Val Val Thr Tyr Thr Thr Leu Ile
                245                 250                 255

Ser Gly Leu Gly Lys Ala Gly Arg Leu Glu Glu Ala Leu Glu Leu Phe
            260                 265                 270

Glu Glu Met Lys Glu Lys Gly Ile Val Pro Asn Val Val Thr Tyr Thr
        275                 280                 285

Thr Leu Ile Ser Gly Leu Gly Lys Ala Gly
    290                 295

<210>  336
<211>  298
<212>  PRT
<213>  Artificial Sequence

<220>
<223>  MODIFIED TYPE crPPR(7L/31F)

<400>  336

Met Gly Asn Ser Val Thr Tyr Thr Thr Leu Leu Ser Gly Leu Gly Lys
1               5                   10                  15

Ala Gly Arg Leu Glu Glu Ala Leu Glu Leu Phe Glu Glu Met Lys Glu
            20                  25                  30

Lys Gly Phe Val Pro Asn Val Val Thr Tyr Thr Thr Leu Leu Ser Gly
        35                  40                  45

Leu Gly Lys Ala Gly Arg Leu Glu Glu Ala Leu Glu Leu Phe Glu Glu
    50                  55                  60

Met Lys Glu Lys Gly Phe Val Pro Asn Val Val Thr Tyr Thr Thr Leu

65                  70                  75                  80

Leu Ser Gly Leu Gly Lys Ala Gly Arg Leu Glu Glu Ala Leu Glu Leu
                85                  90                  95

Phe Glu Glu Met Lys Glu Lys Gly Phe Val Pro Asn Val Val Thr Tyr
            100                 105                 110

Thr Thr Leu Leu Ser Gly Leu Gly Lys Ala Gly Arg Leu Glu Glu Ala
        115                 120                 125

Leu Glu Leu Phe Glu Glu Met Lys Glu Lys Gly Phe Val Pro Asn Val
    130                 135                 140

Val Thr Tyr Thr Thr Leu Leu Ser Gly Leu Gly Lys Ala Gly Arg Leu
145                 150                 155                 160

Glu Glu Ala Leu Glu Leu Phe Glu Glu Met Lys Glu Lys Gly Phe Val
                165                 170                 175

Pro Asn Val Val Thr Tyr Thr Thr Leu Leu Ser Gly Leu Gly Lys Ala
            180                 185                 190

Gly Arg Leu Glu Glu Ala Leu Glu Leu Phe Glu Glu Met Lys Glu Lys
        195                 200                 205

Gly Phe Val Pro Asn Val Val Thr Tyr Thr Thr Leu Leu Ser Gly Leu
    210                 215                 220

Gly Lys Ala Gly Arg Leu Glu Glu Ala Leu Glu Leu Phe Glu Glu Met
225                 230                 235                 240

Lys Glu Lys Gly Phe Val Pro Asn Val Val Thr Tyr Thr Thr Leu Leu
                245                 250                 255

Ser Gly Leu Gly Lys Ala Gly Arg Leu Glu Glu Ala Leu Glu Leu Phe
            260                 265                 270

Glu Glu Met Lys Glu Lys Gly Phe Val Pro Asn Val Val Thr Tyr Thr
        275                 280                 285

Thr Leu Leu Ser Gly Leu Gly Lys Ala Gly
    290                 295

<210>  337
<211>  298
<212>  PRT
<213>  Artificial Sequence

<220>
<223>  MODIFIED TYPE crPPR(7L/31F)

<400>  337

Met Gly Asn Ser Val Thr Tyr Thr Thr Leu Ile Ser Gly Leu Gly Lys
1               5                   10                  15

Ala Gly Arg Leu Glu Glu Ala Leu Glu Leu Phe Glu Glu Met Lys Glu
            20                  25                  30

Lys Gly Phe Val Pro Asn Val Val Thr Tyr Thr Thr Leu Ile Ser Gly
        35                  40                  45

Leu Gly Lys Ala Gly Arg Leu Glu Glu Ala Leu Glu Leu Phe Glu Glu
    50                  55                  60

Met Lys Glu Lys Gly Phe Val Pro Asn Val Val Thr Tyr Thr Thr Leu
65                  70                  75                  80

Ile Ser Gly Leu Gly Lys Ala Gly Arg Leu Glu Glu Ala Leu Glu Leu
                85                  90                  95

Phe Glu Glu Met Lys Glu Lys Gly Phe Val Pro Asn Val Val Thr Tyr
            100                 105                 110

Thr Thr Leu Ile Ser Gly Leu Gly Lys Ala Gly Arg Leu Glu Glu Ala
        115                 120                 125

Leu Glu Leu Phe Glu Glu Met Lys Glu Lys Gly Phe Val Pro Asn Val
    130                 135                 140

Val Thr Tyr Thr Thr Leu Ile Ser Gly Leu Gly Lys Ala Gly Arg Leu
145                 150                 155                 160

Glu Glu Ala Leu Glu Leu Phe Glu Glu Met Lys Glu Lys Gly Phe Val
                165                 170                 175

Pro Asn Val Val Thr Tyr Thr Thr Leu Ile Ser Gly Leu Gly Lys Ala
            180                 185                 190

Gly Arg Leu Glu Glu Ala Leu Glu Leu Phe Glu Glu Met Lys Glu Lys
        195                 200                 205

Gly Phe Val Pro Asn Val Val Thr Tyr Thr Thr Leu Ile Ser Gly Leu
    210                 215                 220

Gly Lys Ala Gly Arg Leu Glu Glu Ala Leu Glu Leu Phe Glu Glu Met

225                 230                 235                 240

Lys Glu Lys Gly Phe Val Pro Asn Val Val Thr Tyr Thr Thr Leu Ile
                245                 250                 255

Ser Gly Leu Gly Lys Ala Gly Arg Leu Glu Glu Ala Leu Glu Leu Phe
            260                 265                 270

Glu Glu Met Lys Glu Lys Gly Phe Val Pro Asn Val Val Thr Tyr Thr
        275                 280                 285

Thr Leu Ile Ser Gly Leu Gly Lys Ala Gly
    290                 295

<210>  338
<211>  298
<212>  PRT
<213>  Artificial Sequence

<220>
<223>  MODIFIED TYPE crPPR(7L/31F)

<400>  338

Met Gly Asn Ser Val Thr Tyr Thr Thr Leu Leu Ser Ala Leu Gly Lys
1               5                   10                  15

Ala Gly Arg Leu Glu Glu Ala Leu Glu Leu Phe Glu Glu Met Lys Glu
            20                  25                  30

Lys Gly Phe Val Pro Asn Val Val Thr Tyr Thr Thr Leu Leu Ser Ala
        35                  40                  45

Leu Gly Lys Ala Gly Arg Leu Glu Glu Ala Leu Glu Leu Phe Glu Glu
    50                  55                  60

Met Lys Glu Lys Gly Phe Val Pro Asn Val Val Thr Tyr Thr Thr Leu
65                  70                  75                  80

Leu Ser Ala Leu Gly Lys Ala Gly Arg Leu Glu Glu Ala Leu Glu Leu
                85                  90                  95

Phe Glu Glu Met Lys Glu Lys Gly Phe Val Pro Asn Val Val Thr Tyr
            100                 105                 110

Thr Thr Leu Leu Ser Ala Leu Gly Lys Ala Gly Arg Leu Glu Glu Ala
        115                 120                 125

Leu Glu Leu Phe Glu Glu Met Lys Glu Lys Gly Phe Val Pro Asn Val
    130                 135                 140

Val Thr Tyr Thr Thr Leu Leu Ser Ala Leu Gly Lys Ala Gly Arg Leu
145                 150                 155                 160

Glu Glu Ala Leu Glu Leu Phe Glu Glu Met Lys Glu Lys Gly Phe Val
                165                 170                 175

Pro Asn Val Val Thr Tyr Thr Thr Leu Leu Ser Ala Leu Gly Lys Ala
            180                 185                 190

Gly Arg Leu Glu Glu Ala Leu Glu Leu Phe Glu Glu Met Lys Glu Lys
        195                 200                 205

Gly Phe Val Pro Asn Val Val Thr Tyr Thr Thr Leu Leu Ser Ala Leu
    210                 215                 220

Gly Lys Ala Gly Arg Leu Glu Glu Ala Leu Glu Leu Phe Glu Glu Met
225                 230                 235                 240

Lys Glu Lys Gly Phe Val Pro Asn Val Val Thr Tyr Thr Thr Leu Leu
                245                 250                 255

Ser Ala Leu Gly Lys Ala Gly Arg Leu Glu Glu Ala Leu Glu Leu Phe
            260                 265                 270

Glu Glu Met Lys Glu Lys Gly Phe Val Pro Asn Val Val Thr Tyr Thr
        275                 280                 285

Thr Leu Leu Ser Ala Leu Gly Lys Ala Gly
    290                 295

<210>  339
<211>  298
<212>  PRT
<213>  Artificial Sequence

<220>
<223>  MODIFIED TYPE crPPR(7L/31F)

<400>  339

Met Gly Asn Ser Val Thr Tyr Thr Thr Leu Leu Ser Gly Tyr Gly Lys
1               5                   10                  15

Ala Gly Arg Leu Glu Glu Ala Leu Glu Leu Phe Glu Glu Met Lys Glu
            20                  25                  30

Lys Gly Phe Val Pro Asn Val Val Thr Tyr Thr Thr Leu Leu Ser Gly
        35                  40                  45

Tyr Gly Lys Ala Gly Arg Leu Glu Glu Ala Leu Glu Leu Phe Glu Glu
    50                  55                  60

Met Lys Glu Lys Gly Phe Val Pro Asn Val Val Thr Tyr Thr Thr Leu
65                  70                  75                  80

Leu Ser Gly Tyr Gly Lys Ala Gly Arg Leu Glu Glu Ala Leu Glu Leu
                85                  90                  95

Phe Glu Glu Met Lys Glu Lys Gly Phe Val Pro Asn Val Val Thr Tyr
            100                 105                 110

Thr Thr Leu Leu Ser Gly Tyr Gly Lys Ala Gly Arg Leu Glu Glu Ala
        115                 120                 125

Leu Glu Leu Phe Glu Glu Met Lys Glu Lys Gly Phe Val Pro Asn Val
    130                 135                 140

Val Thr Tyr Thr Thr Leu Leu Ser Gly Tyr Gly Lys Ala Gly Arg Leu
145                 150                 155                 160

Glu Glu Ala Leu Glu Leu Phe Glu Glu Met Lys Glu Lys Gly Phe Val
                165                 170                 175

Pro Asn Val Val Thr Tyr Thr Thr Leu Leu Ser Gly Tyr Gly Lys Ala
            180                 185                 190

Gly Arg Leu Glu Glu Ala Leu Glu Leu Phe Glu Glu Met Lys Glu Lys
        195                 200                 205

Gly Phe Val Pro Asn Val Val Thr Tyr Thr Thr Leu Leu Ser Gly Tyr
    210                 215                 220

Gly Lys Ala Gly Arg Leu Glu Glu Ala Leu Glu Leu Phe Glu Glu Met
225                 230                 235                 240

Lys Glu Lys Gly Phe Val Pro Asn Val Val Thr Tyr Thr Thr Leu Leu
                245                 250                 255

Ser Gly Tyr Gly Lys Ala Gly Arg Leu Glu Glu Ala Leu Glu Leu Phe
            260                 265                 270

Glu Glu Met Lys Glu Lys Gly Phe Val Pro Asn Val Val Thr Tyr Thr
        275                 280                 285

Thr Leu Leu Ser Gly Tyr Gly Lys Ala Gly
    290                 295

<210>  340
<211>  298
<212>  PRT
<213>  Artificial Sequence

<220>
<223>  MODIFIED TYPE crPPR(7L/31F)

<400>  340

Met Gly Asn Ser Val Thr Tyr Thr Thr Leu Leu Ser Gly Leu Gly Lys
1               5                   10                  15

Ala Gly Arg Leu Glu Lys Ala Leu Glu Leu Phe Glu Glu Met Lys Glu
            20                  25                  30

Lys Gly Phe Val Pro Asn Val Val Thr Tyr Thr Thr Leu Leu Ser Gly
        35                  40                  45

Leu Gly Lys Ala Gly Arg Leu Glu Lys Ala Leu Glu Leu Phe Glu Glu
    50                  55                  60

Met Lys Glu Lys Gly Phe Val Pro Asn Val Val Thr Tyr Thr Thr Leu
65                  70                  75                  80

Leu Ser Gly Leu Gly Lys Ala Gly Arg Leu Glu Lys Ala Leu Glu Leu
                85                  90                  95

Phe Glu Glu Met Lys Glu Lys Gly Phe Val Pro Asn Val Val Thr Tyr
            100                 105                 110

Thr Thr Leu Leu Ser Gly Leu Gly Lys Ala Gly Arg Leu Glu Lys Ala
        115                 120                 125

Leu Glu Leu Phe Glu Glu Met Lys Glu Lys Gly Phe Val Pro Asn Val
    130                 135                 140

Val Thr Tyr Thr Thr Leu Leu Ser Gly Leu Gly Lys Ala Gly Arg Leu
145                 150                 155                 160

Glu Lys Ala Leu Glu Leu Phe Glu Glu Met Lys Glu Lys Gly Phe Val
                165                 170                 175

Pro Asn Val Val Thr Tyr Thr Thr Leu Leu Ser Gly Leu Gly Lys Ala
            180                 185                 190

Gly Arg Leu Glu Lys Ala Leu Glu Leu Phe Glu Glu Met Lys Glu Lys
        195                 200                 205

Gly Phe Val Pro Asn Val Val Thr Tyr Thr Thr Leu Leu Ser Gly Leu
    210                 215                 220

Gly Lys Ala Gly Arg Leu Glu Lys Ala Leu Glu Leu Phe Glu Glu Met
225                 230                 235                 240

Lys Glu Lys Gly Phe Val Pro Asn Val Val Thr Tyr Thr Thr Leu Leu
                245                 250                 255

Ser Gly Leu Gly Lys Ala Gly Arg Leu Glu Lys Ala Leu Glu Leu Phe
            260                 265                 270

Glu Glu Met Lys Glu Lys Gly Phe Val Pro Asn Val Val Thr Tyr Thr
        275                 280                 285

Thr Leu Leu Ser Gly Leu Gly Lys Ala Gly
    290                 295

<210>  341
<211>  298
<212>  PRT
<213>  Artificial Sequence

<220>
<223>  MODIFIED TYPE crPPR(7L/31F)

<400>  341

Met Gly Asn Ser Val Thr Tyr Thr Thr Leu Leu Ser Gly Leu Gly Lys
1               5                   10                  15

Ala Gly Arg Leu Glu Glu Ala Glu Glu Leu Phe Glu Glu Met Lys Glu
            20                  25                  30

Lys Gly Phe Val Pro Asn Val Val Thr Tyr Thr Thr Leu Leu Ser Gly
        35                  40                  45

Leu Gly Lys Ala Gly Arg Leu Glu Glu Ala Glu Glu Leu Phe Glu Glu
    50                  55                  60

Met Lys Glu Lys Gly Phe Val Pro Asn Val Val Thr Tyr Thr Thr Leu
65                  70                  75                  80

Leu Ser Gly Leu Gly Lys Ala Gly Arg Leu Glu Glu Ala Glu Glu Leu
                85                  90                  95

Phe Glu Glu Met Lys Glu Lys Gly Phe Val Pro Asn Val Val Thr Tyr
            100                 105                 110

Thr Thr Leu Leu Ser Gly Leu Gly Lys Ala Gly Arg Leu Glu Glu Ala
        115                 120                 125

Glu Glu Leu Phe Glu Glu Met Lys Glu Lys Gly Phe Val Pro Asn Val
    130                 135                 140

Val Thr Tyr Thr Thr Leu Leu Ser Gly Leu Gly Lys Ala Gly Arg Leu
145                 150                 155                 160

Glu Glu Ala Glu Glu Leu Phe Glu Glu Met Lys Glu Lys Gly Phe Val
                165                 170                 175

Pro Asn Val Val Thr Tyr Thr Thr Leu Leu Ser Gly Leu Gly Lys Ala
            180                 185                 190

Gly Arg Leu Glu Glu Ala Glu Glu Leu Phe Glu Glu Met Lys Glu Lys
        195                 200                 205

Gly Phe Val Pro Asn Val Val Thr Tyr Thr Thr Leu Leu Ser Gly Leu
    210                 215                 220

Gly Lys Ala Gly Arg Leu Glu Glu Ala Glu Glu Leu Phe Glu Glu Met
225                 230                 235                 240

Lys Glu Lys Gly Phe Val Pro Asn Val Val Thr Tyr Thr Thr Leu Leu
                245                 250                 255

Ser Gly Leu Gly Lys Ala Gly Arg Leu Glu Glu Ala Glu Glu Leu Phe
            260                 265                 270

Glu Glu Met Lys Glu Lys Gly Phe Val Pro Asn Val Val Thr Tyr Thr
        275                 280                 285

Thr Leu Leu Ser Gly Leu Gly Lys Ala Gly
    290                 295

<210>  342
<211>  298
<212>  PRT
<213>  Artificial Sequence

<220>
<223>  MODIFIED TYPE crPPR(7L/31F)

<400>  342

Met Gly Asn Ser Val Thr Tyr Thr Thr Leu Leu Ser Gly Leu Gly Lys
1               5                   10                  15

Ala Gly Arg Leu Glu Glu Ala Leu Glu Leu Phe Glu Glu Met Lys Glu

20                  25                  30

Glu Gly Phe Val Pro Asn Val Val Thr Tyr Thr Thr Leu Leu Ser Gly
        35                  40                  45

Leu Gly Lys Ala Gly Arg Leu Glu Glu Ala Leu Glu Leu Phe Glu Glu
    50                  55                  60

Met Lys Glu Glu Gly Phe Val Pro Asn Val Val Thr Tyr Thr Thr Leu
65                  70                  75                  80

Leu Ser Gly Leu Gly Lys Ala Gly Arg Leu Glu Glu Ala Leu Glu Leu
                85                  90                  95

Phe Glu Glu Met Lys Glu Glu Gly Phe Val Pro Asn Val Val Thr Tyr
            100                 105                 110

Thr Thr Leu Leu Ser Gly Leu Gly Lys Ala Gly Arg Leu Glu Glu Ala
        115                 120                 125

Leu Glu Leu Phe Glu Glu Met Lys Glu Glu Gly Phe Val Pro Asn Val
    130                 135                 140

Val Thr Tyr Thr Thr Leu Leu Ser Gly Leu Gly Lys Ala Gly Arg Leu
145                 150                 155                 160

Glu Glu Ala Leu Glu Leu Phe Glu Glu Met Lys Glu Glu Gly Phe Val
                165                 170                 175

Pro Asn Val Val Thr Tyr Thr Thr Leu Leu Ser Gly Leu Gly Lys Ala
            180                 185                 190

Gly Arg Leu Glu Glu Ala Leu Glu Leu Phe Glu Glu Met Lys Glu Glu
        195                 200                 205

Gly Phe Val Pro Asn Val Val Thr Tyr Thr Thr Leu Leu Ser Gly Leu
    210                 215                 220

Gly Lys Ala Gly Arg Leu Glu Glu Ala Leu Glu Leu Phe Glu Glu Met
225                 230                 235                 240

Lys Glu Glu Gly Phe Val Pro Asn Val Val Thr Tyr Thr Thr Leu Leu
                245                 250                 255

Ser Gly Leu Gly Lys Ala Gly Arg Leu Glu Glu Ala Leu Glu Leu Phe
            260                 265                 270

Glu Glu Met Lys Glu Glu Gly Phe Val Pro Asn Val Val Thr Tyr Thr
        275                 280                 285

Thr Leu Leu Ser Gly Leu Gly Lys Ala Gly
    290                 295

<210>  343
<211>  298
<212>  PRT
<213>  Artificial Sequence

<220>
<223>  MODIFIED TYPE crPPR(7L/31F)

<400>  343

Met Gly Asn Ser Val Thr Tyr Thr Thr Leu Leu Ser Gly Leu Gly Lys
1               5                   10                  15

Ala Gly Arg Leu Glu Glu Ala Leu Glu Leu Phe Glu Glu Met Lys Glu
            20                  25                  30

Lys Gly Ile Val Pro Asn Val Val Thr Tyr Thr Thr Leu Leu Ser Gly
        35                  40                  45

Leu Gly Lys Ala Gly Arg Leu Glu Glu Ala Leu Glu Leu Phe Glu Glu
    50                  55                  60

Met Lys Glu Lys Gly Ile Val Pro Asn Val Val Thr Tyr Thr Thr Leu
65                  70                  75                  80

Leu Ser Gly Leu Gly Lys Ala Gly Arg Leu Glu Glu Ala Leu Glu Leu
                85                  90                  95

Phe Glu Glu Met Lys Glu Lys Gly Ile Val Pro Asn Val Val Thr Tyr
            100                 105                 110

Thr Thr Leu Leu Ser Gly Leu Gly Lys Ala Gly Arg Leu Glu Glu Ala
        115                 120                 125

Leu Glu Leu Phe Glu Glu Met Lys Glu Lys Gly Ile Val Pro Asn Val
    130                 135                 140

Val Thr Tyr Thr Thr Leu Leu Ser Gly Leu Gly Lys Ala Gly Arg Leu
145                 150                 155                 160

Glu Glu Ala Leu Glu Leu Phe Glu Glu Met Lys Glu Lys Gly Ile Val
                165                 170                 175

Pro Asn Val Val Thr Tyr Thr Thr Leu Leu Ser Gly Leu Gly Lys Ala

180                 185                 190

Gly Arg Leu Glu Glu Ala Leu Glu Leu Phe Glu Glu Met Lys Glu Lys
        195                 200                 205

Gly Ile Val Pro Asn Val Val Thr Tyr Thr Thr Leu Leu Ser Gly Leu
    210                 215                 220

Gly Lys Ala Gly Arg Leu Glu Glu Ala Leu Glu Leu Phe Glu Glu Met
225                 230                 235                 240

Lys Glu Lys Gly Ile Val Pro Asn Val Val Thr Tyr Thr Thr Leu Leu
                245                 250                 255

Ser Gly Leu Gly Lys Ala Gly Arg Leu Glu Glu Ala Leu Glu Leu Phe
            260                 265                 270

Glu Glu Met Lys Glu Lys Gly Ile Val Pro Asn Val Val Thr Tyr Thr
        275                 280                 285

Thr Leu Leu Ser Gly Leu Gly Lys Ala Gly
    290                 295

<210>  344
<211>  298
<212>  PRT
<213>  Artificial Sequence

<220>
<223>  MODIFIED TYPE crPPR(7L/31F)

<400>  344

Met Gly Asn Ser Val Thr Tyr Thr Thr Leu Leu Ser Gly Leu Gly Lys
1               5                   10                  15

Ala Gly Arg Leu Glu Glu Ala Leu Glu Leu Phe Glu Glu Met Lys Glu
            20                  25                  30

Lys Gly Phe Lys Pro Asn Val Val Thr Tyr Thr Thr Leu Leu Ser Gly
        35                  40                  45

Leu Gly Lys Ala Gly Arg Leu Glu Glu Ala Leu Glu Leu Phe Glu Glu
    50                  55                  60

Met Lys Glu Lys Gly Phe Lys Pro Asn Val Val Thr Tyr Thr Thr Leu
65                  70                  75                  80

Leu Ser Gly Leu Gly Lys Ala Gly Arg Leu Glu Glu Ala Leu Glu Leu
                85                  90                  95

Phe Glu Glu Met Lys Glu Lys Gly Phe Lys Pro Asn Val Val Thr Tyr
                100                 105                 110

Thr Thr Leu Leu Ser Gly Leu Gly Lys Ala Gly Arg Leu Glu Glu Ala
        115                 120                 125

Leu Glu Leu Phe Glu Glu Met Lys Glu Lys Gly Phe Lys Pro Asn Val
    130                 135                 140

Val Thr Tyr Thr Thr Leu Leu Ser Gly Leu Gly Lys Ala Gly Arg Leu
145                 150                 155                 160

Glu Glu Ala Leu Glu Leu Phe Glu Glu Met Lys Glu Lys Gly Phe Lys
                165                 170                 175

Pro Asn Val Val Thr Tyr Thr Thr Leu Leu Ser Gly Leu Gly Lys Ala
                180                 185                 190

Gly Arg Leu Glu Glu Ala Leu Glu Leu Phe Glu Glu Met Lys Glu Lys
        195                 200                 205

Gly Phe Lys Pro Asn Val Val Thr Tyr Thr Thr Leu Leu Ser Gly Leu
    210                 215                 220

Gly Lys Ala Gly Arg Leu Glu Glu Ala Leu Glu Leu Phe Glu Glu Met
225                 230                 235                 240

Lys Glu Lys Gly Phe Lys Pro Asn Val Val Thr Tyr Thr Thr Leu Leu
                245                 250                 255

Ser Gly Leu Gly Lys Ala Gly Arg Leu Glu Glu Ala Leu Glu Leu Phe
                260                 265                 270

Glu Glu Met Lys Glu Lys Gly Phe Lys Pro Asn Val Val Thr Tyr Thr
        275                 280                 285

Thr Leu Leu Ser Gly Leu Gly Lys Ala Gly
    290                 295

<210>  345
<211>  298
<212>  PRT
<213>  Artificial Sequence

<220>
<223>  MODIFIED TYPE crPPR(7L/31F)

<400>  345

Met Gly Asn Ser Val Thr Tyr Thr Thr Leu Leu Ser Ala Tyr Gly Lys
1               5                   10                  15

Ala Gly Arg Leu Glu Glu Ala Leu Glu Leu Phe Glu Glu Met Lys Glu
            20                  25                  30

Lys Gly Phe Val Pro Asn Val Val Thr Tyr Thr Thr Leu Leu Ser Ala
        35                  40                  45

Tyr Gly Lys Ala Gly Arg Leu Glu Glu Ala Leu Glu Leu Phe Glu Glu
    50                  55                  60

Met Lys Glu Lys Gly Phe Val Pro Asn Val Val Thr Tyr Thr Thr Leu
65                  70                  75                  80

Leu Ser Ala Tyr Gly Lys Ala Gly Arg Leu Glu Glu Ala Leu Glu Leu
                85                  90                  95

Phe Glu Glu Met Lys Glu Lys Gly Phe Val Pro Asn Val Val Thr Tyr
            100                 105                 110

Thr Thr Leu Leu Ser Ala Tyr Gly Lys Ala Gly Arg Leu Glu Glu Ala
        115                 120                 125

Leu Glu Leu Phe Glu Glu Met Lys Glu Lys Gly Phe Val Pro Asn Val
    130                 135                 140

Val Thr Tyr Thr Thr Leu Leu Ser Ala Tyr Gly Lys Ala Gly Arg Leu
145                 150                 155                 160

Glu Glu Ala Leu Glu Leu Phe Glu Glu Met Lys Glu Lys Gly Phe Val
                165                 170                 175

Pro Asn Val Val Thr Tyr Thr Thr Leu Leu Ser Ala Tyr Gly Lys Ala
            180                 185                 190

Gly Arg Leu Glu Glu Ala Leu Glu Leu Phe Glu Glu Met Lys Glu Lys
        195                 200                 205

Gly Phe Val Pro Asn Val Val Thr Tyr Thr Thr Leu Leu Ser Ala Tyr
    210                 215                 220

Gly Lys Ala Gly Arg Leu Glu Glu Ala Leu Glu Leu Phe Glu Glu Met
225                 230                 235                 240

Lys Glu Lys Gly Phe Val Pro Asn Val Val Thr Tyr Thr Thr Leu Leu
                245                 250                 255

Ser Ala Tyr Gly Lys Ala Gly Arg Leu Glu Glu Ala Leu Glu Leu Phe
            260                 265                 270

Glu Glu Met Lys Glu Lys Gly Phe Val Pro Asn Val Val Thr Tyr Thr
        275                 280                 285

Thr Leu Leu Ser Ala Tyr Gly Lys Ala Gly
    290                 295

<210>  346
<211>  298
<212>  PRT
<213>  Artificial Sequence

<220>
<223>  MODIFIED TYPE crPPR(7L/31F)

<400>  346

Met Gly Asn Ser Val Thr Tyr Thr Thr Leu Leu Ser Gly Leu Gly Lys
1               5                   10                  15

Ala Gly Arg Leu Glu His Ala Leu Glu Leu Phe Glu Glu Met Lys Glu
            20                  25                  30

Lys Gly Phe Val Pro Asn Val Val Thr Tyr Thr Thr Leu Leu Ser Gly
        35                  40                  45

Leu Gly Lys Ala Gly Arg Leu Glu His Ala Leu Glu Leu Phe Glu Glu
    50                  55                  60

Met Lys Glu Lys Gly Phe Val Pro Asn Val Val Thr Tyr Thr Thr Leu
65                  70                  75                  80

Leu Ser Gly Leu Gly Lys Ala Gly Arg Leu Glu His Ala Leu Glu Leu
                85                  90                  95

Phe Glu Glu Met Lys Glu Lys Gly Phe Val Pro Asn Val Val Thr Tyr
            100                 105                 110

Thr Thr Leu Leu Ser Gly Leu Gly Lys Ala Gly Arg Leu Glu His Ala
        115                 120                 125

Leu Glu Leu Phe Glu Glu Met Lys Glu Lys Gly Phe Val Pro Asn Val
    130                 135                 140

Val Thr Tyr Thr Thr Leu Leu Ser Gly Leu Gly Lys Ala Gly Arg Leu
145                 150                 155                 160

Glu His Ala Leu Glu Leu Phe Glu Glu Met Lys Glu Lys Gly Phe Val
                165                 170                 175

Pro Asn Val Val Thr Tyr Thr Thr Leu Leu Ser Gly Leu Gly Lys Ala
            180                 185                 190

Gly Arg Leu Glu His Ala Leu Glu Leu Phe Glu Glu Met Lys Glu Lys
        195                 200                 205

Gly Phe Val Pro Asn Val Val Thr Tyr Thr Thr Leu Leu Ser Gly Leu
    210                 215                 220

Gly Lys Ala Gly Arg Leu Glu His Ala Leu Glu Leu Phe Glu Glu Met
225                 230                 235                 240

Lys Glu Lys Gly Phe Val Pro Asn Val Val Thr Tyr Thr Thr Leu Leu
                245                 250                 255

Ser Gly Leu Gly Lys Ala Gly Arg Leu Glu His Ala Leu Glu Leu Phe
            260                 265                 270

Glu Glu Met Lys Glu Lys Gly Phe Val Pro Asn Val Val Thr Tyr Thr
        275                 280                 285

Thr Leu Leu Ser Gly Leu Gly Lys Ala Gly
    290                 295

<210>  347
<211>  298
<212>  PRT
<213>  Artificial Sequence

<220>
<223>  MODIFIED TYPE crPPR(7L/31F)

<400>  347

Met Gly Asn Ser Val Thr Tyr Thr Thr Leu Leu Ser Gly Leu Gly Lys
1               5                   10                  15

Ala Gly Arg Leu Glu Arg Ala Leu Glu Leu Phe Glu Glu Met Lys Glu
            20                  25                  30

Lys Gly Phe Val Pro Asn Val Val Thr Tyr Thr Thr Leu Leu Ser Gly
        35                  40                  45

Leu Gly Lys Ala Gly Arg Leu Glu Arg Ala Leu Glu Leu Phe Glu Glu
    50                  55                  60

Met Lys Glu Lys Gly Phe Val Pro Asn Val Val Thr Tyr Thr Thr Leu
65                  70                  75                  80

Leu Ser Gly Leu Gly Lys Ala Gly Arg Leu Glu Arg Ala Leu Glu Leu
                85                  90                  95

Phe Glu Glu Met Lys Glu Lys Gly Phe Val Pro Asn Val Val Thr Tyr
            100                 105                 110

Thr Thr Leu Leu Ser Gly Leu Gly Lys Ala Gly Arg Leu Glu Arg Ala
        115                 120                 125

Leu Glu Leu Phe Glu Glu Met Lys Glu Lys Gly Phe Val Pro Asn Val
    130                 135                 140

Val Thr Tyr Thr Thr Leu Leu Ser Gly Leu Gly Lys Ala Gly Arg Leu
145                 150                 155                 160

Glu Arg Ala Leu Glu Leu Phe Glu Glu Met Lys Glu Lys Gly Phe Val
                165                 170                 175

Pro Asn Val Val Thr Tyr Thr Thr Leu Leu Ser Gly Leu Gly Lys Ala
            180                 185                 190

Gly Arg Leu Glu Arg Ala Leu Glu Leu Phe Glu Glu Met Lys Glu Lys
        195                 200                 205

Gly Phe Val Pro Asn Val Val Thr Tyr Thr Thr Leu Leu Ser Gly Leu
    210                 215                 220

Gly Lys Ala Gly Arg Leu Glu Arg Ala Leu Glu Leu Phe Glu Glu Met
225                 230                 235                 240

Lys Glu Lys Gly Phe Val Pro Asn Val Val Thr Tyr Thr Thr Leu Leu
                245                 250                 255

Ser Gly Leu Gly Lys Ala Gly Arg Leu Glu Arg Ala Leu Glu Leu Phe
            260                 265                 270

Glu Glu Met Lys Glu Lys Gly Phe Val Pro Asn Val Val Thr Tyr Thr
        275                 280                 285

Thr Leu Leu Ser Gly Leu Gly Lys Ala Gly
    290                 295

<210>  348
<211>  298
<212>  PRT

<213>  Artificial Sequence

<220>
<223>  MODIFIED TYPE crPPR(7L/31F)

<400>  348

Met Gly Asn Ser Val Thr Tyr Thr Thr Leu Leu Ser Gly Leu Gly Lys
1               5                   10                  15

Ala Gly Arg Leu Glu Lys Ala Leu Glu Leu Phe Glu Glu Met Lys Glu
            20                  25                  30

Lys Gly Val Val Pro Asn Val Val Thr Tyr Thr Thr Leu Leu Ser Gly
        35                  40                  45

Leu Gly Lys Ala Gly Arg Leu Glu Lys Ala Leu Glu Leu Phe Glu Glu
    50                  55                  60

Met Lys Glu Lys Gly Val Val Pro Asn Val Val Thr Tyr Thr Thr Leu
65                  70                  75                  80

Leu Ser Gly Leu Gly Lys Ala Gly Arg Leu Glu Lys Ala Leu Glu Leu
                85                  90                  95

Phe Glu Glu Met Lys Glu Lys Gly Val Val Pro Asn Val Val Thr Tyr
            100                 105                 110

Thr Thr Leu Leu Ser Gly Leu Gly Lys Ala Gly Arg Leu Glu Lys Ala
        115                 120                 125

Leu Glu Leu Phe Glu Glu Met Lys Glu Lys Gly Val Val Pro Asn Val
    130                 135                 140

Val Thr Tyr Thr Thr Leu Leu Ser Gly Leu Gly Lys Ala Gly Arg Leu
145                 150                 155                 160

Glu Lys Ala Leu Glu Leu Phe Glu Glu Met Lys Glu Lys Gly Val Val
                165                 170                 175

Pro Asn Val Val Thr Tyr Thr Thr Leu Leu Ser Gly Leu Gly Lys Ala
            180                 185                 190

Gly Arg Leu Glu Lys Ala Leu Glu Leu Phe Glu Glu Met Lys Glu Lys
        195                 200                 205

Gly Val Val Pro Asn Val Val Thr Tyr Thr Thr Leu Leu Ser Gly Leu
    210                 215                 220

Gly Lys Ala Gly Arg Leu Glu Lys Ala Leu Glu Leu Phe Glu Glu Met
225                 230                 235                 240

Lys Glu Lys Gly Val Val Pro Asn Val Val Thr Tyr Thr Thr Leu Leu
                245                 250                 255

Ser Gly Leu Gly Lys Ala Gly Arg Leu Glu Lys Ala Leu Glu Leu Phe
            260                 265                 270

Glu Glu Met Lys Glu Lys Gly Val Val Pro Asn Val Val Thr Tyr Thr
        275                 280                 285

Thr Leu Leu Ser Gly Leu Gly Lys Ala Gly
    290                 295

<210>  349
<211>  298
<212>  PRT
<213>  Artificial Sequence

<220>
<223>  MODIFIED TYPE crPPR(7L/31F)

<400>  349

Met Gly Asn Ser Val Thr Tyr Thr Thr Leu Leu Ser Gly Leu Gly Lys
1               5                   10                  15

Ala Gly Arg Leu Glu Lys Ala Leu Glu Leu Phe Glu Glu Met Lys Glu
            20                  25                  30

Lys Gly Leu Val Pro Asn Val Val Thr Tyr Thr Thr Leu Leu Ser Gly
        35                  40                  45

Leu Gly Lys Ala Gly Arg Leu Glu Lys Ala Leu Glu Leu Phe Glu Glu
    50                  55                  60

Met Lys Glu Lys Gly Leu Val Pro Asn Val Val Thr Tyr Thr Thr Leu
65                  70                  75                  80

Leu Ser Gly Leu Gly Lys Ala Gly Arg Leu Glu Lys Ala Leu Glu Leu
                85                  90                  95

Phe Glu Glu Met Lys Glu Lys Gly Leu Val Pro Asn Val Val Thr Tyr
            100                 105                 110

Thr Thr Leu Leu Ser Gly Leu Gly Lys Ala Gly Arg Leu Glu Lys Ala
        115                 120                 125

Leu Glu Leu Phe Glu Glu Met Lys Glu Lys Gly Leu Val Pro Asn Val

130                 135                 140

Val Thr Tyr Thr Thr Leu Leu Ser Gly Leu Gly Lys Ala Gly Arg Leu
145                 150                 155                 160

Glu Lys Ala Leu Glu Leu Phe Glu Glu Met Lys Glu Lys Gly Leu Val
                165                 170                 175

Pro Asn Val Val Thr Tyr Thr Thr Leu Leu Ser Gly Leu Gly Lys Ala
            180                 185                 190

Gly Arg Leu Glu Lys Ala Leu Glu Leu Phe Glu Glu Met Lys Glu Lys
        195                 200                 205

Gly Leu Val Pro Asn Val Val Thr Tyr Thr Thr Leu Leu Ser Gly Leu
    210                 215                 220

Gly Lys Ala Gly Arg Leu Glu Lys Ala Leu Glu Leu Phe Glu Glu Met
225                 230                 235                 240

Lys Glu Lys Gly Leu Val Pro Asn Val Val Thr Tyr Thr Thr Leu Leu
                245                 250                 255

Ser Gly Leu Gly Lys Ala Gly Arg Leu Glu Lys Ala Leu Glu Leu Phe
            260                 265                 270

Glu Glu Met Lys Glu Lys Gly Leu Val Pro Asn Val Val Thr Tyr Thr
        275                 280                 285

Thr Leu Leu Ser Gly Leu Gly Lys Ala Gly
    290                 295

<210>  350
<211>  298
<212>  PRT
<213>  Artificial Sequence

<220>
<223>  MODIFIED TYPE crPPR(7L/31F)

<400>  350

Met Gly Asn Ser Val Thr Tyr Thr Thr Leu Leu Ser Gly Leu Gly Lys
1               5                   10                  15

Ala Gly Arg Leu Glu Glu Ala Leu Glu Leu Phe Glu Glu Met Lys Glu
            20                  25                  30

Lys Gly Phe His Pro Asn Val Val Thr Tyr Thr Thr Leu Leu Ser Gly
        35                  40                  45

Leu Gly Lys Ala Gly Arg Leu Glu Glu Ala Leu Glu Leu Phe Glu Glu
    50                  55                  60

Met Lys Glu Lys Gly Phe His Pro Asn Val Val Thr Tyr Thr Thr Leu
65                  70                  75                  80

Leu Ser Gly Leu Gly Lys Ala Gly Arg Leu Glu Glu Ala Leu Glu Leu
                85                  90                  95

Phe Glu Glu Met Lys Glu Lys Gly Phe His Pro Asn Val Val Thr Tyr
            100                 105                 110

Thr Thr Leu Leu Ser Gly Leu Gly Lys Ala Gly Arg Leu Glu Glu Ala
        115                 120                 125

Leu Glu Leu Phe Glu Glu Met Lys Glu Lys Gly Phe His Pro Asn Val
    130                 135                 140

Val Thr Tyr Thr Thr Leu Leu Ser Gly Leu Gly Lys Ala Gly Arg Leu
145                 150                 155                 160

Glu Glu Ala Leu Glu Leu Phe Glu Glu Met Lys Glu Lys Gly Phe His
                165                 170                 175

Pro Asn Val Val Thr Tyr Thr Thr Leu Leu Ser Gly Leu Gly Lys Ala
            180                 185                 190

Gly Arg Leu Glu Glu Ala Leu Glu Leu Phe Glu Glu Met Lys Glu Lys
        195                 200                 205

Gly Phe His Pro Asn Val Val Thr Tyr Thr Thr Leu Leu Ser Gly Leu
    210                 215                 220

Gly Lys Ala Gly Arg Leu Glu Glu Ala Leu Glu Leu Phe Glu Glu Met
225                 230                 235                 240

Lys Glu Lys Gly Phe His Pro Asn Val Val Thr Tyr Thr Thr Leu Leu
                245                 250                 255

Ser Gly Leu Gly Lys Ala Gly Arg Leu Glu Glu Ala Leu Glu Leu Phe
            260                 265                 270

Glu Glu Met Lys Glu Lys Gly Phe His Pro Asn Val Val Thr Tyr Thr
        275                 280                 285

Thr Leu Leu Ser Gly Leu Gly Lys Ala Gly

290                 295


<210>  351
<211>  298
<212>  PRT
<213>  Artificial Sequence

<220>
<223>  MODIFIED TYPE crPPR(7L/31F)

<400>  351

Met Gly Asn Ser Val Thr Tyr Thr Thr Leu Leu Ser Gly Leu Gly Lys
1               5                   10                  15

Ala Gly Arg Leu Glu Glu Ala Leu Glu Leu Phe Glu Glu Met Lys Glu
            20                  25                  30

Lys Gly Phe Arg Pro Asn Val Val Thr Tyr Thr Thr Leu Leu Ser Gly
        35                  40                  45

Leu Gly Lys Ala Gly Arg Leu Glu Glu Ala Leu Glu Leu Phe Glu Glu
    50                  55                  60

Met Lys Glu Lys Gly Phe Arg Pro Asn Val Val Thr Tyr Thr Thr Leu
65                  70                  75                  80

Leu Ser Gly Leu Gly Lys Ala Gly Arg Leu Glu Glu Ala Leu Glu Leu
                85                  90                  95

Phe Glu Glu Met Lys Glu Lys Gly Phe Arg Pro Asn Val Val Thr Tyr
            100                 105                 110

Thr Thr Leu Leu Ser Gly Leu Gly Lys Ala Gly Arg Leu Glu Glu Ala
        115                 120                 125

Leu Glu Leu Phe Glu Glu Met Lys Glu Lys Gly Phe Arg Pro Asn Val
    130                 135                 140

Val Thr Tyr Thr Thr Leu Leu Ser Gly Leu Gly Lys Ala Gly Arg Leu
145                 150                 155                 160

Glu Glu Ala Leu Glu Leu Phe Glu Glu Met Lys Glu Lys Gly Phe Arg
                165                 170                 175

Pro Asn Val Val Thr Tyr Thr Thr Leu Leu Ser Gly Leu Gly Lys Ala
            180                 185                 190

Gly Arg Leu Glu Glu Ala Leu Glu Leu Phe Glu Glu Met Lys Glu Lys
        195                 200                 205

Gly Phe Arg Pro Asn Val Val Thr Tyr Thr Thr Leu Leu Ser Gly Leu
    210                 215                 220

Gly Lys Ala Gly Arg Leu Glu Glu Ala Leu Glu Leu Phe Glu Glu Met
225                 230                 235                 240

Lys Glu Lys Gly Phe Arg Pro Asn Val Val Thr Tyr Thr Thr Leu Leu
                245                 250                 255

Ser Gly Leu Gly Lys Ala Gly Arg Leu Glu Glu Ala Leu Glu Leu Phe
            260                 265                 270

Glu Glu Met Lys Glu Lys Gly Phe Arg Pro Asn Val Val Thr Tyr Thr
        275                 280                 285

Thr Leu Leu Ser Gly Leu Gly Lys Ala Gly
    290                 295

<210>  352
<211>  298
<212>  PRT
<213>  Artificial Sequence

<220>
<223>  MODIFIED TYPE crPPR(7L/31F)

<400>  352

Met Gly Asn Ser Val Thr Tyr Thr Thr Leu Leu Ser Ala Phe Gly Lys
1               5                   10                  15

Ala Gly Arg Leu Glu Glu Ala Leu Glu Leu Phe Glu Glu Met Lys Glu
            20                  25                  30

Lys Gly Phe Val Pro Asn Val Val Thr Tyr Thr Thr Leu Leu Ser Ala
        35                  40                  45

Phe Gly Lys Ala Gly Arg Leu Glu Glu Ala Leu Glu Leu Phe Glu Glu
    50                  55                  60

Met Lys Glu Lys Gly Phe Val Pro Asn Val Val Thr Tyr Thr Thr Leu
65                  70                  75                  80

Leu Ser Ala Phe Gly Lys Ala Gly Arg Leu Glu Glu Ala Leu Glu Leu
                85                  90                  95

Phe Glu Glu Met Lys Glu Lys Gly Phe Val Pro Asn Val Val Thr Tyr
            100                 105                 110

Thr Thr Leu Leu Ser Ala Phe Gly Lys Ala Gly Arg Leu Glu Glu Ala
        115                 120                 125

Leu Glu Leu Phe Glu Glu Met Lys Glu Lys Gly Phe Val Pro Asn Val
    130                 135                 140

Val Thr Tyr Thr Thr Leu Leu Ser Ala Phe Gly Lys Ala Gly Arg Leu
145                 150                 155                 160

Glu Glu Ala Leu Glu Leu Phe Glu Glu Met Lys Glu Lys Gly Phe Val
                165                 170                 175

Pro Asn Val Val Thr Tyr Thr Thr Leu Leu Ser Ala Phe Gly Lys Ala
            180                 185                 190

Gly Arg Leu Glu Glu Ala Leu Glu Leu Phe Glu Glu Met Lys Glu Lys
        195                 200                 205

Gly Phe Val Pro Asn Val Val Thr Tyr Thr Thr Leu Leu Ser Ala Phe
    210                 215                 220

Gly Lys Ala Gly Arg Leu Glu Glu Ala Leu Glu Leu Phe Glu Glu Met
225                 230                 235                 240

Lys Glu Lys Gly Phe Val Pro Asn Val Val Thr Tyr Thr Thr Leu Leu
                245                 250                 255

Ser Ala Phe Gly Lys Ala Gly Arg Leu Glu Glu Ala Leu Glu Leu Phe
            260                 265                 270

Glu Glu Met Lys Glu Lys Gly Phe Val Pro Asn Val Val Thr Tyr Thr
        275                 280                 285

Thr Leu Leu Ser Ala Phe Gly Lys Ala Gly
    290                 295

<210>  353
<211>  298
<212>  PRT
<213>  Artificial Sequence

<220>
<223>  MODIFIED TYPE crPPR(7L/31F)

<400>  353

Met Gly Asn Ser Val Thr Tyr Thr Thr Leu Leu Ser Ala Trp Gly Lys
1               5                   10                  15

Ala Gly Arg Leu Glu Glu Ala Leu Glu Leu Phe Glu Glu Met Lys Glu
            20                  25                  30

Lys Gly Phe Val Pro Asn Val Val Thr Tyr Thr Thr Leu Leu Ser Ala
        35                  40                  45

Trp Gly Lys Ala Gly Arg Leu Glu Glu Ala Leu Glu Leu Phe Glu Glu
    50                  55                  60

Met Lys Glu Lys Gly Phe Val Pro Asn Val Val Thr Tyr Thr Thr Leu
65                  70                  75                  80

Leu Ser Ala Trp Gly Lys Ala Gly Arg Leu Glu Glu Ala Leu Glu Leu
                85                  90                  95

Phe Glu Glu Met Lys Glu Lys Gly Phe Val Pro Asn Val Val Thr Tyr
            100                 105                 110

Thr Thr Leu Leu Ser Ala Trp Gly Lys Ala Gly Arg Leu Glu Glu Ala
        115                 120                 125

Leu Glu Leu Phe Glu Glu Met Lys Glu Lys Gly Phe Val Pro Asn Val
    130                 135                 140

Val Thr Tyr Thr Thr Leu Leu Ser Ala Trp Gly Lys Ala Gly Arg Leu
145                 150                 155                 160

Glu Glu Ala Leu Glu Leu Phe Glu Glu Met Lys Glu Lys Gly Phe Val
                165                 170                 175

Pro Asn Val Val Thr Tyr Thr Thr Leu Leu Ser Ala Trp Gly Lys Ala
            180                 185                 190

Gly Arg Leu Glu Glu Ala Leu Glu Leu Phe Glu Glu Met Lys Glu Lys
        195                 200                 205

Gly Phe Val Pro Asn Val Val Thr Tyr Thr Thr Leu Leu Ser Ala Trp
    210                 215                 220

Gly Lys Ala Gly Arg Leu Glu Glu Ala Leu Glu Leu Phe Glu Glu Met
225                 230                 235                 240

Lys Glu Lys Gly Phe Val Pro Asn Val Val Thr Tyr Thr Thr Leu Leu
                245                 250                 255

Ser Ala Trp Gly Lys Ala Gly Arg Leu Glu Glu Ala Leu Glu Leu Phe
            260                 265                 270

Glu Glu Met Lys Glu Lys Gly Phe Val Pro Asn Val Val Thr Tyr Thr
        275                 280                 285

Thr Leu Leu Ser Ala Trp Gly Lys Ala Gly
    290                 295

<210>  354
<211>  298
<212>  PRT
<213>  Artificial Sequence

<220>
<223>  MODIFIED TYPE crPPR(7L/31F)

<400>  354

Met Gly Asn Ser Val Thr Tyr Thr Thr Leu Leu Ser Gly Leu Gly Lys
1               5                   10                  15

Ala Gly Arg Leu Glu Lys Ala Leu Glu Leu Phe Glu Glu Met Lys Glu
            20                  25                  30

Lys Gly Phe Val Pro Asn Val Val Thr Tyr Thr Thr Leu Leu Ser Gly
        35                  40                  45

Leu Gly Lys Ala Gly Arg Leu Glu Lys Ala Leu Glu Leu Phe Glu Glu
    50                  55                  60

Met Lys Glu Lys Gly Phe Val Pro Asn Val Val Thr Tyr Thr Thr Leu
65                  70                  75                  80

Leu Ser Gly Leu Gly Lys Ala Gly Arg Leu Glu Lys Ala Leu Glu Leu
                85                  90                  95

Phe Glu Glu Met Lys Glu Lys Gly Phe Val Pro Asn Val Val Thr Tyr
            100                 105                 110

Thr Thr Leu Leu Ser Gly Leu Gly Lys Ala Gly Arg Leu Glu Lys Ala
        115                 120                 125

Leu Glu Leu Phe Glu Glu Met Lys Glu Lys Gly Phe Val Pro Asn Val
    130                 135                 140

Val Thr Tyr Thr Thr Leu Leu Ser Gly Leu Gly Lys Ala Gly Arg Leu
145                 150                 155                 160

Glu Glu Ala Leu Glu Leu Phe Glu Glu Met Lys Glu Lys Gly Phe Val
                165                 170                 175

Pro Asn Val Val Thr Tyr Thr Thr Leu Leu Ser Gly Leu Gly Lys Ala
            180                 185                 190

Gly Arg Leu Glu Glu Ala Leu Glu Leu Phe Glu Glu Met Lys Glu Lys
        195                 200                 205

Gly Phe Val Pro Asn Val Val Thr Tyr Thr Thr Leu Leu Ser Gly Leu
    210                 215                 220

Gly Lys Ala Gly Arg Leu Glu Glu Ala Leu Glu Leu Phe Glu Glu Met
225                 230                 235                 240

Lys Glu Lys Gly Phe Val Pro Asn Val Val Thr Tyr Thr Thr Leu Leu
                245                 250                 255

Ser Gly Leu Gly Lys Ala Gly Arg Leu Glu Glu Ala Leu Glu Leu Phe
            260                 265                 270

Glu Glu Met Lys Glu Lys Gly Phe Val Pro Asn Val Val Thr Tyr Thr
        275                 280                 285

Thr Leu Leu Ser Gly Leu Gly Lys Ala Gly
    290                 295

<210>  355
<211>  298
<212>  PRT
<213>  Artificial Sequence

<220>
<223>  MODIFIED TYPE crPPR(7L/31F)

<400>  355

Met Gly Asn Ser Val Thr Tyr Thr Thr Leu Leu Ser Gly Leu Gly Lys
1               5                   10                  15

Ala Gly Arg Leu Glu Lys Ala Leu Glu Leu Phe Glu Glu Met Lys Glu
            20                  25                  30

Lys Gly Phe Val Pro Asn Val Val Thr Tyr Thr Thr Leu Leu Ser Gly
        35                  40                  45

Leu Gly Lys Ala Gly Arg Leu Glu Lys Ala Leu Glu Leu Phe Glu Glu
    50                  55                  60

Met Lys Glu Lys Gly Phe Val Pro Asn Val Val Thr Tyr Thr Thr Leu
65                  70                  75                  80

Leu Ser Gly Leu Gly Lys Ala Gly Arg Leu Glu Glu Ala Leu Glu Leu

85                  90                  95

Phe Glu Glu Met Lys Glu Lys Gly Phe Val Pro Asn Val Val Thr Tyr
            100                 105                 110

Thr Thr Leu Leu Ser Gly Leu Gly Lys Ala Gly Arg Leu Glu Glu Ala
        115                 120                 125

Leu Glu Leu Phe Glu Glu Met Lys Glu Lys Gly Phe Val Pro Asn Val
    130                 135                 140

Val Thr Tyr Thr Thr Leu Leu Ser Gly Leu Gly Lys Ala Gly Arg Leu
145                 150                 155                 160

Glu Glu Ala Leu Glu Leu Phe Glu Glu Met Lys Glu Lys Gly Phe Val
                165                 170                 175

Pro Asn Val Val Thr Tyr Thr Thr Leu Leu Ser Gly Leu Gly Lys Ala
            180                 185                 190

Gly Arg Leu Glu Glu Ala Leu Glu Leu Phe Glu Glu Met Lys Glu Lys
        195                 200                 205

Gly Phe Val Pro Asn Val Val Thr Tyr Thr Thr Leu Leu Ser Gly Leu
    210                 215                 220

Gly Lys Ala Gly Arg Leu Glu Glu Ala Leu Glu Leu Phe Glu Glu Met
225                 230                 235                 240

Lys Glu Lys Gly Phe Val Pro Asn Val Val Thr Tyr Thr Thr Leu Leu
                245                 250                 255

Ser Gly Leu Gly Lys Ala Gly Arg Leu Glu Glu Ala Leu Glu Leu Phe
            260                 265                 270

Glu Glu Met Lys Glu Lys Gly Phe Val Pro Asn Val Val Thr Tyr Thr
        275                 280                 285

Thr Leu Leu Ser Gly Leu Gly Lys Ala Gly
    290                 295

<210>  356
<211>  298
<212>  PRT
<213>  Artificial Sequence

<220>
<223>  MODIFIED TYPE crPPR(7L/31F)

<400> 356

Met Gly Asn Ser Val Thr Tyr Thr Thr Leu Leu Ser Gly Leu Gly Lys
1               5                   10                  15

Ala Gly Arg Leu Glu Glu Ala Leu Glu Leu Phe Glu Glu Met Lys Glu
            20                  25                  30

Lys Gly Ile Val Pro Asn Val Val Thr Tyr Thr Thr Leu Leu Ser Gly
        35                  40                  45

Leu Gly Lys Ala Gly Arg Leu Glu Glu Ala Leu Glu Leu Phe Glu Glu
    50                  55                  60

Met Lys Glu Lys Gly Ile Val Pro Asn Val Val Thr Tyr Thr Thr Leu
65                  70                  75                  80

Leu Ser Gly Leu Gly Lys Ala Gly Arg Leu Glu Glu Ala Leu Glu Leu
                85                  90                  95

Phe Glu Glu Met Lys Glu Lys Gly Ile Val Pro Asn Val Val Thr Tyr
            100                 105                 110

Thr Thr Leu Leu Ser Gly Leu Gly Lys Ala Gly Arg Leu Glu Glu Ala
        115                 120                 125

Leu Glu Leu Phe Glu Glu Met Lys Glu Lys Gly Ile Val Pro Asn Val
    130                 135                 140

Val Thr Tyr Thr Thr Leu Leu Ser Gly Leu Gly Lys Ala Gly Arg Leu
145                 150                 155                 160

Glu Glu Ala Leu Glu Leu Phe Glu Glu Met Lys Glu Lys Gly Phe Val
                165                 170                 175

Pro Asn Val Val Thr Tyr Thr Thr Leu Leu Ser Gly Leu Gly Lys Ala
            180                 185                 190

Gly Arg Leu Glu Glu Ala Leu Glu Leu Phe Glu Glu Met Lys Glu Lys
        195                 200                 205

Gly Phe Val Pro Asn Val Val Thr Tyr Thr Thr Leu Leu Ser Gly Leu
    210                 215                 220

Gly Lys Ala Gly Arg Leu Glu Glu Ala Leu Glu Leu Phe Glu Glu Met
225                 230                 235                 240

Lys Glu Lys Gly Phe Val Pro Asn Val Val Thr Tyr Thr Thr Leu Leu

245                 250                 255

Ser Gly Leu Gly Lys Ala Gly Arg Leu Glu Glu Ala Leu Glu Leu Phe
            260                 265                 270

Glu Glu Met Lys Glu Lys Gly Phe Val Pro Asn Val Val Thr Tyr Thr
        275                 280                 285

Thr Leu Leu Ser Gly Leu Gly Lys Ala Gly
    290                 295

<210>  357
<211>  298
<212>  PRT
<213>  Artificial Sequence

<220>
<223>  MODIFIED TYPE crPPR(7L/31F)

<400>  357

Met Gly Asn Ser Val Thr Tyr Thr Thr Leu Leu Ser Gly Leu Gly Lys
1               5                   10                  15

Ala Gly Arg Leu Glu Glu Ala Leu Glu Leu Phe Glu Glu Met Lys Glu
            20                  25                  30

Lys Gly Ile Val Pro Asn Val Val Thr Tyr Thr Thr Leu Leu Ser Gly
        35                  40                  45

Leu Gly Lys Ala Gly Arg Leu Glu Glu Ala Leu Glu Leu Phe Glu Glu
    50                  55                  60

Met Lys Glu Lys Gly Ile Val Pro Asn Val Val Thr Tyr Thr Thr Leu
65                  70                  75                  80

Leu Ser Gly Leu Gly Lys Ala Gly Arg Leu Glu Glu Ala Leu Glu Leu
                85                  90                  95

Phe Glu Glu Met Lys Glu Lys Gly Phe Val Pro Asn Val Val Thr Tyr
            100                 105                 110

Thr Thr Leu Leu Ser Gly Leu Gly Lys Ala Gly Arg Leu Glu Glu Ala
        115                 120                 125

Leu Glu Leu Phe Glu Glu Met Lys Glu Lys Gly Phe Val Pro Asn Val
    130                 135                 140

Val Thr Tyr Thr Thr Leu Leu Ser Gly Leu Gly Lys Ala Gly Arg Leu
145                 150                 155                 160

Glu Glu Ala Leu Glu Leu Phe Glu Glu Met Lys Glu Lys Gly Phe Val
                165                 170                 175

Pro Asn Val Val Thr Tyr Thr Thr Leu Leu Ser Gly Leu Gly Lys Ala
            180                 185                 190

Gly Arg Leu Glu Glu Ala Leu Glu Leu Phe Glu Glu Met Lys Glu Lys
        195                 200                 205

Gly Phe Val Pro Asn Val Val Thr Tyr Thr Thr Leu Leu Ser Gly Leu
    210                 215                 220

Gly Lys Ala Gly Arg Leu Glu Glu Ala Leu Glu Leu Phe Glu Glu Met
225                 230                 235                 240

Lys Glu Lys Gly Phe Val Pro Asn Val Val Thr Tyr Thr Thr Leu Leu
                245                 250                 255

Ser Gly Leu Gly Lys Ala Gly Arg Leu Glu Glu Ala Leu Glu Leu Phe
            260                 265                 270

Glu Glu Met Lys Glu Lys Gly Phe Val Pro Asn Val Val Thr Tyr Thr
        275                 280                 285

Thr Leu Leu Ser Gly Leu Gly Lys Ala Gly
    290                 295

<210>  358
<211>  298
<212>  PRT
<213>  Artificial Sequence

<220>
<223>  MODIFIED TYPE crPPR(7L/31F)

<400>  358

Met Gly Asn Ser Val Thr Tyr Thr Thr Leu Leu Ser Gly Leu Gly Lys
1               5                   10                  15

Ala Gly Arg Leu Glu Glu Ala Leu Glu Leu Phe Glu Glu Met Lys Glu
            20                  25                  30

Lys Gly Phe Lys Pro Asn Val Val Thr Tyr Thr Thr Leu Leu Ser Gly
        35                  40                  45

Leu Gly Lys Ala Gly Arg Leu Glu Glu Ala Leu Glu Leu Phe Glu Glu
    50                  55                  60

Met Lys Glu Lys Gly Phe Lys Pro Asn Val Val Thr Tyr Thr Thr Leu
65                  70                  75                  80

Leu Ser Gly Leu Gly Lys Ala Gly Arg Leu Glu Glu Ala Leu Glu Leu
                85                  90                  95

Phe Glu Glu Met Lys Glu Lys Gly Phe Lys Pro Asn Val Val Thr Tyr
            100                 105                 110

Thr Thr Leu Leu Ser Gly Leu Gly Lys Ala Gly Arg Leu Glu Glu Ala
        115                 120                 125

Leu Glu Leu Phe Glu Glu Met Lys Glu Lys Gly Phe Lys Pro Asn Val
    130                 135                 140

Val Thr Tyr Thr Thr Leu Leu Ser Gly Leu Gly Lys Ala Gly Arg Leu
145                 150                 155                 160

Glu Glu Ala Leu Glu Leu Phe Glu Glu Met Lys Glu Lys Gly Phe Val
                165                 170                 175

Pro Asn Val Val Thr Tyr Thr Thr Leu Leu Ser Gly Leu Gly Lys Ala
            180                 185                 190

Gly Arg Leu Glu Glu Ala Leu Glu Leu Phe Glu Glu Met Lys Glu Lys
        195                 200                 205

Gly Phe Val Pro Asn Val Val Thr Tyr Thr Thr Leu Leu Ser Gly Leu
    210                 215                 220

Gly Lys Ala Gly Arg Leu Glu Glu Ala Leu Glu Leu Phe Glu Glu Met
225                 230                 235                 240

Lys Glu Lys Gly Phe Val Pro Asn Val Val Thr Tyr Thr Thr Leu Leu
                245                 250                 255

Ser Gly Leu Gly Lys Ala Gly Arg Leu Glu Glu Ala Leu Glu Leu Phe
            260                 265                 270

Glu Glu Met Lys Glu Lys Gly Phe Val Pro Asn Val Val Thr Tyr Thr
        275                 280                 285

Thr Leu Leu Ser Gly Leu Gly Lys Ala Gly
    290                 295

<210>  359
<211>  298

<212>  PRT
<213>  Artificial Sequence

<220>
<223>  MODIFIED TYPE crPPR(7L/31F)

<400>  359

Met Gly Asn Ser Val Thr Tyr Thr Thr Leu Leu Ser Gly Leu Gly Lys
1               5                   10                  15

Ala Gly Arg Leu Glu Glu Ala Leu Glu Leu Phe Glu Glu Met Lys Glu
            20                  25                  30

Lys Gly Phe Lys Pro Asn Val Val Thr Tyr Thr Thr Leu Leu Ser Gly
        35                  40                  45

Leu Gly Lys Ala Gly Arg Leu Glu Glu Ala Leu Glu Leu Phe Glu Glu
    50                  55                  60

Met Lys Glu Lys Gly Phe Lys Pro Asn Val Val Thr Tyr Thr Thr Leu
65                  70                  75                  80

Leu Ser Gly Leu Gly Lys Ala Gly Arg Leu Glu Glu Ala Leu Glu Leu
                85                  90                  95

Phe Glu Glu Met Lys Glu Lys Gly Phe Val Pro Asn Val Val Thr Tyr
            100                 105                 110

Thr Thr Leu Leu Ser Gly Leu Gly Lys Ala Gly Arg Leu Glu Glu Ala
        115                 120                 125

Leu Glu Leu Phe Glu Glu Met Lys Glu Lys Gly Phe Val Pro Asn Val
    130                 135                 140

Val Thr Tyr Thr Thr Leu Leu Ser Gly Leu Gly Lys Ala Gly Arg Leu
145                 150                 155                 160

Glu Glu Ala Leu Glu Leu Phe Glu Glu Met Lys Glu Lys Gly Phe Val
                165                 170                 175

Pro Asn Val Val Thr Tyr Thr Thr Leu Leu Ser Gly Leu Gly Lys Ala
            180                 185                 190

Gly Arg Leu Glu Glu Ala Leu Glu Leu Phe Glu Glu Met Lys Glu Lys
        195                 200                 205

Gly Phe Val Pro Asn Val Val Thr Tyr Thr Thr Leu Leu Ser Gly Leu
    210                 215                 220

Gly Lys Ala Gly Arg Leu Glu Glu Ala Leu Glu Leu Phe Glu Glu Met
225                 230                 235                 240

Lys Glu Lys Gly Phe Val Pro Asn Val Val Thr Tyr Thr Thr Leu Leu
                245                 250                 255

Ser Gly Leu Gly Lys Ala Gly Arg Leu Glu Glu Ala Leu Glu Leu Phe
            260                 265                 270

Glu Glu Met Lys Glu Lys Gly Phe Val Pro Asn Val Val Thr Tyr Thr
        275                 280                 285

Thr Leu Leu Ser Gly Leu Gly Lys Ala Gly
    290                 295

<210>  360
<211>  298
<212>  PRT
<213>  Artificial Sequence

<220>
<223>  MODIFIED TYPE crPPR(7L/31F)

<400>  360

Met Gly Asn Ser Val Thr Tyr Thr Thr Leu Leu Ser Ala Tyr Gly Lys
1               5                   10                  15

Ala Gly Arg Leu Glu Glu Ala Leu Glu Leu Phe Glu Glu Met Lys Glu
            20                  25                  30

Lys Gly Phe Val Pro Asn Val Val Thr Tyr Thr Thr Leu Leu Ser Ala
        35                  40                  45

Tyr Gly Lys Ala Gly Arg Leu Glu Glu Ala Leu Glu Leu Phe Glu Glu
    50                  55                  60

Met Lys Glu Lys Gly Phe Val Pro Asn Val Val Thr Tyr Thr Thr Leu
65                  70                  75                  80

Leu Ser Ala Tyr Gly Lys Ala Gly Arg Leu Glu Glu Ala Leu Glu Leu
                85                  90                  95

Phe Glu Glu Met Lys Glu Lys Gly Phe Val Pro Asn Val Val Thr Tyr
            100                 105                 110

Thr Thr Leu Leu Ser Ala Tyr Gly Lys Ala Gly Arg Leu Glu Glu Ala
        115                 120                 125

Leu Glu Leu Phe Glu Glu Met Lys Glu Lys Gly Phe Val Pro Asn Val
    130                 135                 140

Val Thr Tyr Thr Thr Leu Leu Ser Gly Leu Gly Lys Ala Gly Arg Leu
145                 150                 155                 160

Glu Glu Ala Leu Glu Leu Phe Glu Glu Met Lys Glu Lys Gly Phe Val
                165                 170                 175

Pro Asn Val Val Thr Tyr Thr Thr Leu Leu Ser Gly Leu Gly Lys Ala
            180                 185                 190

Gly Arg Leu Glu Glu Ala Leu Glu Leu Phe Glu Glu Met Lys Glu Lys
        195                 200                 205

Gly Phe Val Pro Asn Val Val Thr Tyr Thr Thr Leu Leu Ser Gly Leu
    210                 215                 220

Gly Lys Ala Gly Arg Leu Glu Glu Ala Leu Glu Leu Phe Glu Glu Met
225                 230                 235                 240

Lys Glu Lys Gly Phe Val Pro Asn Val Val Thr Tyr Thr Thr Leu Leu
                245                 250                 255

Ser Gly Leu Gly Lys Ala Gly Arg Leu Glu Glu Ala Leu Glu Leu Phe
            260                 265                 270

Glu Glu Met Lys Glu Lys Gly Phe Val Pro Asn Val Val Thr Tyr Thr
        275                 280                 285

Thr Leu Leu Ser Gly Leu Gly Lys Ala Gly
    290                 295

<210>  361
<211>  298
<212>  PRT
<213>  Artificial Sequence

<220>
<223>  MODIFIED TYPE crPPR(7L/31F)

<400>  361

Met Gly Asn Ser Val Thr Tyr Thr Thr Leu Leu Ser Ala Tyr Gly Lys
1               5                   10                  15

Ala Gly Arg Leu Glu Glu Ala Leu Glu Leu Phe Glu Glu Met Lys Glu
            20                  25                  30

Lys Gly Phe Val Pro Asn Val Val Thr Tyr Thr Thr Leu Leu Ser Ala

35                  40                  45

Tyr Gly Lys Ala Gly Arg Leu Glu Glu Ala Leu Glu Leu Phe Glu Glu
    50                  55                  60

Met Lys Glu Lys Gly Phe Val Pro Asn Val Val Thr Tyr Thr Thr Leu
65                  70                  75                  80

Leu Ser Gly Leu Gly Lys Ala Gly Arg Leu Glu Glu Ala Leu Glu Leu
                85                  90                  95

Phe Glu Glu Met Lys Glu Lys Gly Phe Val Pro Asn Val Val Thr Tyr
            100                 105                 110

Thr Thr Leu Leu Ser Gly Leu Gly Lys Ala Gly Arg Leu Glu Glu Ala
        115                 120                 125

Leu Glu Leu Phe Glu Glu Met Lys Glu Lys Gly Phe Val Pro Asn Val
    130                 135                 140

Val Thr Tyr Thr Thr Leu Leu Ser Gly Leu Gly Lys Ala Gly Arg Leu
145                 150                 155                 160

Glu Glu Ala Leu Glu Leu Phe Glu Glu Met Lys Glu Lys Gly Phe Val
                165                 170                 175

Pro Asn Val Val Thr Tyr Thr Thr Leu Leu Ser Gly Leu Gly Lys Ala
            180                 185                 190

Gly Arg Leu Glu Glu Ala Leu Glu Leu Phe Glu Glu Met Lys Glu Lys
        195                 200                 205

Gly Phe Val Pro Asn Val Val Thr Tyr Thr Thr Leu Leu Ser Gly Leu
    210                 215                 220

Gly Lys Ala Gly Arg Leu Glu Glu Ala Leu Glu Leu Phe Glu Glu Met
225                 230                 235                 240

Lys Glu Lys Gly Phe Val Pro Asn Val Val Thr Tyr Thr Thr Leu Leu
                245                 250                 255

Ser Gly Leu Gly Lys Ala Gly Arg Leu Glu Glu Ala Leu Glu Leu Phe
            260                 265                 270

Glu Glu Met Lys Glu Lys Gly Phe Val Pro Asn Val Val Thr Tyr Thr
        275                 280                 285

Thr Leu Leu Ser Gly Leu Gly Lys Ala Gly
    290                 295

<210>  362
<211>  58
<212>  PRT
<213>  Arabidopsis thaliana

<400>  362

Ala Gly Thr Lys Ser Asp Gln Glu Glu Asp Asp Leu Glu Asp Gly Phe
1               5                   10                  15

Ser Glu Leu Glu Gly Ser Lys Ser Gly Gln Gly Ser Thr Ser Ser Asp
            20                  25                  30

Glu Asp Glu Gly Lys Leu Ser Ala Asp Glu Glu Glu Glu Glu Glu Leu
        35                  40                  45

Asp Leu Ile Glu Thr Asp Val Ser Arg Lys
    50                  55

<210>  363
<211>  35
<212>  PRT
<213>  Arabidopsis thaliana

<400>  363

Val Leu Tyr Arg Thr Leu Leu Ala Asn Cys Val Ala Ala Gly Asn Val
1               5                   10                  15

Lys Lys Ser Glu Leu Val Phe Asn Lys Met Lys Asp Leu Gly Phe Pro
            20                  25                  30

Leu Ser Gly
        35

<210>  364
<211>  34
<212>  PRT
<213>  Arabidopsis thaliana

<400>  364

Phe Thr Cys Asp Gln Met Leu Leu Leu His Lys Arg Ile Asp Arg Lys
1               5                   10                  15

Lys Ile Ala Asp Val Leu Leu Leu Met Glu Lys Glu Asn Ile Lys Pro
            20                  25                  30

Ser Leu

<210>  365
<211>  35
<212>  PRT
<213>  Arabidopsis thaliana

<400>  365

Leu Thr Tyr Lys Ile Leu Ile Asp Val Lys Gly Ala Thr Asn Asp Ile
1               5                   10                  15

Ser Gly Met Glu Gln Ile Leu Glu Thr Met Lys Asp Glu Gly Val Glu
            20                  25                  30

Leu Asp Phe
        35

<210>  366
<211>  35
<212>  PRT
<213>  Arabidopsis thaliana

<400>  366

Gln Thr Gln Ala Leu Thr Ala Arg His Tyr Ser Gly Ala Gly Leu Lys
1               5                   10                  15

Asp Lys Ala Glu Lys Val Leu Lys Glu Met Glu Gly Glu Ser Leu Glu
            20                  25                  30

Ala Asn Arg
        35

<210>  367
<211>  32
<212>  PRT
<213>  Arabidopsis thaliana

<400>  367

Arg Ala Phe Lys Asp Leu Leu Ser Ile Tyr Ala Ser Leu Gly Arg Glu
1               5                   10                  15

Asp Glu Val Lys Arg Ile Trp Lys Ile Cys Glu Ser Lys Pro Tyr Phe
            20                  25                  30

<210>  368
<211>  35
<212>  PRT
<213>  Arabidopsis thaliana

<400>  368

Glu Glu Ser Leu Ala Ala Ile Gln Ala Phe Gly Lys Leu Asn Lys Val

1               5                   10                  15

Gln Glu Ala Glu Ala Ile Phe Glu Lys Ile Val Lys Met Asp Arg Arg
            20                  25                  30

Ala Ser Ser
        35

<210>  369
<211>  35
<212>  PRT
<213>  Arabidopsis thaliana

<400>  369

Ser Thr Tyr Ser Val Leu Leu Arg Val Tyr Val Asp His Lys Met Leu
1               5                   10                  15

Ser Lys Gly Lys Asp Leu Val Lys Arg Met Ala Glu Ser Gly Cys Arg
            20                  25                  30

Ile Glu Ala
        35

<210>  370
<211>  36
<212>  PRT
<213>  Arabidopsis thaliana

<400>  370

Thr Thr Trp Asp Ala Leu Ile Lys Leu Tyr Val Glu Ala Gly Glu Val
1               5                   10                  15

Glu Lys Ala Asp Ser Leu Leu Asp Lys Ala Ser Lys Gln Ser His Thr
            20                  25                  30

Lys Leu Met Met
        35

<210>  371
<211>  35
<212>  PRT
<213>  Arabidopsis thaliana

<400>  371

Asn Ser Phe Met Tyr Ile Met Asp Glu Tyr Ser Lys Arg Gly Asp Val
1               5                   10                  15

His Asn Thr Glu Lys Ile Phe Leu Lys Met Arg Glu Ala Gly Tyr Thr
            20                  25                  30

Ser Arg Leu
        35

<210>  372
<211>  55
<212>  PRT
<213>  Arabidopsis thaliana

<400>  372

Arg Gln Phe Gln Ala Leu Met Gln Ala Tyr Ile Asn Ala Lys Ser Pro
1               5                   10                  15

Ala Tyr Gly Met Arg Asp Arg Leu Lys Ala Asp Asn Ile Phe Pro Asn
            20                  25                  30

Lys Ser Met Ala Ala Gln Leu Ala Gln Gly Asp Pro Phe Lys Lys Thr
        35                  40                  45

Ala Ile Ser Asp Ile Leu Asp
    50                  55

<210>  373
<211>  35
<212>  PRT
<213>  Artificial Sequence

<220>
<223>  MODIFIED TYPE P63

<400>  373

Val Leu Tyr Arg Thr Leu Leu Ala Asn Cys Val Ala Ala Gly Asn Val
1               5                   10                  15

Lys Lys Ser Glu Leu Val Phe Asn Lys Met Lys Asp Leu Gly Ile Lys
            20                  25                  30

Leu Ser Gly
        35

<210>  374
<211>  35
<212>  PRT
<213>  Artificial Sequence

<220>
<223>  MODIFIED TYPE P63

<400>  374

Leu Thr Tyr Lys Ile Leu Ile Asp Val Lys Gly Ala Thr Asn Asp Ile
1               5                   10                  15

Ser Gly Met Glu Gln Ile Leu Glu Thr Met Lys Asp Glu Gly Ile Lys
            20                  25                  30

Leu Asp Phe
        35

<210>  375
<211>  35
<212>  PRT
<213>  Artificial Sequence

<220>
<223>  MODIFIED TYPE P63

<400>  375

Gln Thr Gln Ala Leu Thr Ala Arg His Tyr Ser Gly Ala Gly Leu Lys
1               5                   10                  15

Asp Lys Ala Glu Lys Val Leu Lys Glu Met Glu Gly Glu Ser Ile Lys
            20                  25                  30

Ala Asn Arg
        35

<210>  376
<211>  32
<212>  PRT
<213>  Artificial Sequence

<220>
<223>  MODIFIED TYPE P63

<400>  376

Arg Ala Phe Lys Asp Leu Leu Ser Ile Tyr Ala Ser Leu Gly Arg Glu
1               5                   10                  15

Asp Glu Val Lys Arg Ile Trp Lys Ile Cys Glu Ile Lys Pro Tyr Phe
            20                  25                  30

<210>  377
<211>  35
<212>  PRT
<213>  Artificial Sequence

<220>
<223>  MODIFIED TYPE P63

<400>  377

Glu Glu Ser Leu Ala Ala Ile Gln Ala Phe Gly Lys Leu Asn Lys Val
1               5                   10                  15

Gln Glu Ala Glu Ala Ile Phe Glu Lys Ile Val Lys Met Asp Ile Lys
            20                  25                  30

Ala Ser Ser
        35

<210>  378
<211>  35
<212>  PRT
<213>  Artificial Sequence

<220>
<223>  MODIFIED TYPE P63

<400>  378

Ser Thr Tyr Ser Val Leu Leu Arg Val Tyr Val Asp His Lys Met Leu
1               5                   10                  15

Ser Lys Gly Lys Asp Leu Val Lys Arg Met Ala Glu Ser Gly Ile Lys
            20                  25                  30

Ile Glu Ala
        35

<210>  379
<211>  36
<212>  PRT
<213>  Artificial Sequence

<220>
<223>  MODIFIED TYPE P63

<400>  379

Thr Thr Trp Asp Ala Leu Ile Lys Leu Tyr Val Glu Ala Gly Glu Val
1               5                   10                  15

Glu Lys Ala Asp Ser Leu Leu Asp Lys Ala Ser Lys Gln Ser His Ile
            20                  25                  30

Lys Leu Met Met
        35

<210>  380
<211>  35
<212>  PRT
<213>  Artificial Sequence

<220>
<223>  MODIFIED TYPE P63

<400>  380

Asn Ser Phe Met Tyr Ile Met Asp Glu Tyr Ser Lys Arg Gly Asp Val

1               5                   10                  15

His Asn Thr Glu Lys Ile Phe Leu Lys Met Arg Glu Ala Gly Ile Lys
            20                  25                  30

Ser Arg Leu
        35

<210>  381
<211>  35
<212>  PRT
<213>  Artificial Sequence

<220>
<223>  MODIFIED TYPE P63

<400>  381

Val Leu Tyr Arg Thr Leu Leu Ala Ala Tyr Val Ala Ala Gly Asn Val
1               5                   10                  15

Lys Lys Ser Glu Leu Val Phe Asn Lys Met Lys Asp Leu Gly Ile Pro
            20                  25                  30

Leu Ser Gly
        35

<210>  382
<211>  34
<212>  PRT
<213>  Artificial Sequence

<220>
<223>  MODIFIED TYPE P63

<400>  382

Phe Thr Cys Asp Gln Met Leu Leu Ala Tyr Lys Arg Ile Asp Arg Lys
1               5                   10                  15

Lys Lys Ala Asp Val Leu Leu Leu Met Glu Lys Glu Asn Ile Lys Pro
            20                  25                  30

Ser Leu

<210>  383
<211>  35
<212>  PRT
<213>  Artificial Sequence

<220>
<223>  MODIFIED TYPE P63

<400>  383

Leu Thr Tyr Lys Ile Leu Ile Asp Ala Tyr Gly Ala Thr Asn Asp Ile
1               5                   10                  15

Ser Lys Met Glu Gln Ile Leu Glu Thr Met Lys Asp Glu Gly Ile Glu
            20                  25                  30

Leu Asp Phe
        35

<210>  384
<211>  35
<212>  PRT
<213>  Artificial Sequence

<220>
<223>  MODIFIED TYPE P63

<400>  384

Gln Thr Gln Ala Leu Thr Ala Arg Ala Tyr Ser Gly Ala Gly Leu Lys
1               5                   10                  15

Asp Lys Ala Glu Lys Val Leu Lys Glu Met Glu Gly Glu Ser Ile Glu
            20                  25                  30

Ala Asn Arg
        35

<210>  385
<211>  32
<212>  PRT
<<213>  Artificial Sequence

<220>
<223>  MODIFIED TYPE P63

<400>  385

Arg Ala Phe Lys Asp Leu Leu Ser Ala Tyr Ala Ser Leu Gly Arg Glu
1               5                   10                  15

Asp Lys Val Lys Arg Ile Trp Lys Ile Cys Glu Ile Lys Pro Tyr Phe
            20                  25                  30

<210>  386
<211>  35
<212>  PRT
<213>  Artificial Sequence

<220>
<223>  MODIFIED TYPE P63

<400>  386

Glu Glu Ser Leu Ala Ala Ile Gln Ala Tyr Gly Lys Leu Asn Lys Val
1               5                   10                  15

Gln Lys Ala Glu Ala Ile Phe Glu Lys Ile Val Lys Met Asp Ile Arg
            20                  25                  30

Ala Ser Ser
        35

<210>  387
<211>  35
<212>  PRT
<213>  Artificial Sequence

<220>
<223>  MODIFIED TYPE P63

<400>  387

Ser Thr Tyr Ser Val Leu Leu Arg Ala Tyr Val Asp His Lys Met Leu
1               5                   10                  15

Ser Lys Gly Lys Asp Leu Val Lys Arg Met Ala Glu Ser Gly Ile Arg
            20                  25                  30

Ile Glu Ala
        35

<210>  388
<211>  36
<212>  PRT
<213>  Artificial Sequence

<220>
<223>  MODIFIED TYPE P63

<400>  388

Thr Thr Trp Asp Ala Leu Ile Lys Ala Tyr Val Glu Ala Gly Glu Val
1               5                   10                  15

Glu Lys Ala Asp Ser Leu Leu Asp Lys Ala Ser Lys Gln Ser His Ile
            20                  25                  30

Lys Leu Met Met
        35

<210>  389
<211>  35
<212>  PRT
<213>  Artificial Sequence

<220>

<223>  MODIFIED TYPE P63

<400>  389

Asn Ser Phe Met Tyr Ile Met Asp Ala Tyr Ser Lys Arg Gly Asp Val
1               5                   10                  15

His Lys Thr Glu Lys Ile Phe Leu Lys Met Arg Glu Ala Gly Ile Thr
            20                  25                  30

Ser Arg Leu
        35

<210>  390
<211>  58
<212>  PRT
<213>  Arabidopsis thaliana

<400>  390

Ser Ala His Leu Ser Gln Thr Thr Pro Asn Phe Ser Pro Leu Gln Thr
1               5                   10                  15

Pro Lys Ser Asp Phe Ser Gly Arg Gln Ser Thr Arg Phe Val Ser Pro
            20                  25                  30

Ala Thr Asn Asn His Arg Gln Thr Arg Gln Asn Pro Asn Tyr Asn His
        35                  40                  45

Arg Pro Tyr Gly Ala Ser Ser Ser Pro Arg
    50                  55

<210>  391
<211>  35
<212>  PRT
<213>  Arabidopsis thaliana

<400>  391

Lys Leu Ala Ser Ala Met Ile Ser Thr Leu Gly Arg Tyr Gly Lys Val
1               5                   10                  15

Thr Ile Ala Lys Arg Ile Phe Glu Thr Ala Phe Ala Gly Gly Tyr Gly
            20                  25                  30

Asn Thr Val
        35

<210>  392
<211>  35
<212>  PRT
<213>  Arabidopsis thaliana

<400>  392

Tyr Ala Phe Ser Ala Leu Ile Ser Ala Tyr Gly Arg Ser Gly Leu His
1               5                   10                  15

Glu Glu Ala Ile Ser Val Phe Asn Ser Met Lys Glu Tyr Gly Leu Arg
            20                  25                  30

Pro Asn Leu
        35

<210>  393
<211>  36
<212>  PRT
<213>  Arabidopsis thaliana

<400>  393

Val Thr Tyr Asn Ala Val Ile Asp Ala Cys Gly Lys Gly Gly Met Glu
1               5                   10                  15

Phe Lys Gln Val Ala Lys Phe Phe Asp Glu Met Gln Arg Asn Gly Val
            20                  25                  30

Gln Pro Asp Arg
        35

<210>  394
<211>  35
<212>  PRT
<213>  Arabidopsis thaliana

<400>  394

Ile Thr Phe Asn Ser Leu Leu Ala Val Cys Ser Arg Gly Gly Leu Trp
1               5                   10                  15

Glu Ala Ala Arg Asn Leu Phe Asp Glu Met Thr Asn Arg Arg Ile Glu
            20                  25                  30

Gln Asp Val
        35

<210>  395
<211>  35
<212>  PRT
<213>  Arabidopsis thaliana

<400>  395

Phe Ser Tyr Asn Thr Leu Leu Asp Ala Ile Cys Lys Gly Gly Gln Met
1               5                   10                  15

Asp Leu Ala Phe Glu Ile Leu Ala Gln Met Pro Val Lys Arg Ile Met
            20                  25                  30

Pro Asn Val
        35

<210>  396
<211>  35
<212>  PRT
<213>  Arabidopsis thaliana

<400>  396

Val Ser Tyr Ser Thr Val Ile Asp Gly Phe Ala Lys Ala Gly Arg Phe
1               5                   10                  15

Asp Glu Ala Leu Asn Leu Phe Gly Glu Met Arg Tyr Leu Gly Ile Ala
            20                  25                  30

Leu Asp Arg
        35

<210>  397
<211>  35
<212>  PRT
<213>  Arabidopsis thaliana

<400>  397

Val Ser Tyr Asn Thr Leu Leu Ser Ile Tyr Thr Lys Val Gly Arg Ser
1               5                   10                  15

Glu Glu Ala Leu Asp Ile Leu Arg Glu Met Ala Ser Val Gly Ile Lys
            20                  25                  30

Lys Asp Val
        35

<210>  398
<211>  35
<212>  PRT
<213>  Arabidopsis thaliana

<400>  398

Val Thr Tyr Asn Ala Leu Leu Gly Gly Tyr Gly Lys Gln Gly Lys Tyr
1               5                   10                  15

Asp Glu Val Lys Lys Val Phe Thr Glu Met Lys Arg Glu His Val Leu
            20                  25                  30

Pro Asn Leu
        35

<210>  399
<211>  35
<212>  PRT
<213>  Arabidopsis thaliana

<400>  399

Leu Thr Tyr Ser Thr Leu Ile Asp Gly Tyr Ser Lys Gly Gly Leu Tyr
1               5                   10                  15

Lys Glu Ala Met Glu Ile Phe Arg Glu Phe Lys Ser Ala Gly Leu Arg
            20                  25                  30

Ala Asp Val
        35

<210>  400
<211>  35
<212>  PRT
<213>  Arabidopsis thaliana

<400>  400

Val Leu Tyr Ser Ala Leu Ile Asp Ala Leu Cys Lys Asn Gly Leu Val
1               5                   10                  15

Gly Ser Ala Val Ser Leu Ile Asp Glu Met Thr Lys Glu Gly Ile Ser
            20                  25                  30

Pro Asn Val
        35

<210>  401
<211>  37
<212>  PRT
<213>  Arabidopsis thaliana

<400>  401

Val Thr Tyr Asn Ser Ile Ile Asp Ala Phe Gly Arg Ser Ala Thr Met
1               5                   10                  15

Asp Arg Ser Ala Asp Tyr Ser Asn Gly Gly Ser Leu Pro Phe Ser Ser
            20                  25                  30

Ser Ala Leu Ser Ala
        35

<210>  402
<211>  58
<212>  PRT
<213>  Arabidopsis thaliana

<400>  402

Leu Thr Glu Thr Glu Gly Asn Arg Val Ile Gln Leu Phe Gly Gln Leu
1               5                   10                  15

Thr Thr Glu Ser Asn Asn Arg Thr Thr Lys Asp Cys Glu Glu Gly Met
            20                  25                  30

Gln Glu Leu Ser Cys Ile Leu Glu Val Phe Arg Lys Met His Gln Leu
        35                  40                  45

Glu Ile Lys Pro Asn Val Val Thr Phe Ser
    50                  55

<210>  403
<211>  35
<212>  PRT
<213>  Artificial Sequence

<220>
<223>  MODIFIED TYPE GUN1

<400>  403

Lys Leu Ala Ser Ala Met Ile Ser Thr Leu Gly Arg Tyr Gly Lys Val
1               5                   10                  15

Thr Ile Ala Lys Arg Ile Phe Glu Thr Ala Phe Ala Gly Gly Ile Lys
            20                  25                  30

Asn Thr Val
        35

<210>  404
<211>  35
<212>  PRT
<213>  Artificial Sequence

<220>
<223>  MODIFIED TYPE GUN1

<400>  404

Tyr Ala Phe Ser Ala Leu Ile Ser Ala Tyr Gly Arg Ser Gly Leu His
1               5                   10                  15

Glu Glu Ala Ile Ser Val Phe Asn Ser Met Lys Glu Tyr Gly Ile Lys
            20                  25                  30

Pro Asn Leu
        35

<210>  405
<211>  36
<212>  PRT
<213>  Artificial Sequence

<220>
<223>  MODIFIED TYPE GUN1

<400>  405

Val Thr Tyr Asn Ala Val Ile Asp Ala Cys Gly Lys Gly Gly Met Glu
1               5                   10                  15

Phe Lys Gln Val Ala Lys Phe Phe Asp Glu Met Gln Arg Asn Gly Ile
            20                  25                  30

Lys Pro Asp Arg
        35

<210>  406
<211>  35
<212>  PRT
<213>  Artificial Sequence

<220>
<223>  MODIFIED TYPE GUN1

<400>  406

Ile Thr Phe Asn Ser Leu Leu Ala Val Cys Ser Arg Gly Gly Leu Trp
1               5                   10                  15

Glu Ala Ala Arg Asn Leu Phe Asp Glu Met Thr Asn Arg Arg Ile Lys
            20                  25                  30

Gln Asp Val
        35

<210>  407
<211>  35
<212>  PRT
<213>  Artificial Sequence

<220>
<223>  MODIFIED TYPE GUN1

<400>  407

Phe Ser Tyr Asn Thr Leu Leu Asp Ala Ile Cys Lys Gly Gly Gln Met
1               5                   10                  15

Asp Leu Ala Phe Glu Ile Leu Ala Gln Met Pro Val Lys Arg Ile Lys
            20                  25                  30

Pro Asn Val

        35

<210>  408
<211>  35
<212>  PRT
<213>  Artificial Sequence

<220>
<223>  MODIFIED TYPE GUN1

<400>  408

Val Ser Tyr Ser Thr Val Ile Asp Gly Phe Ala Lys Ala Gly Arg Phe
1               5                   10                  15

Asp Glu Ala Leu Asn Leu Phe Gly Glu Met Arg Tyr Leu Gly Ile Lys
            20                  25                  30

Leu Asp Arg
        35

<210>  409
<211>  35
<212>  PRT
<213>  Artificial Sequence

<220>
<223>  MODIFIED TYPE GUN1

<400>  409

Val Thr Tyr Asn Ala Leu Leu Gly Gly Tyr Gly Lys Gln Gly Lys Tyr
1               5                   10                  15

Asp Glu Val Lys Lys Val Phe Thr Glu Met Lys Arg Glu His Ile Lys
            20                  25                  30

Pro Asn Leu
        35

<210>  410
<211>  35
<212>  PRT
<213>  Artificial Sequence

<220>
<223>  MODIFIED TYPE GUN1

<400>  410

Leu Thr Tyr Ser Thr Leu Ile Asp Gly Tyr Ser Lys Gly Gly Leu Tyr
1               5                   10                  15

Lys Glu Ala Met Glu Ile Phe Arg Glu Phe Lys Ser Ala Gly Ile Lys
            20                  25                  30

Ala Asp Val
        35

<210>  411
<211>  35
<212>  PRT
<213>  Artificial Sequence

<220>
<223>  MODIFIED TYPE GUN1

<400>  411

Val Leu Tyr Ser Ala Leu Ile Asp Ala Leu Cys Lys Asn Gly Leu Val
1               5                   10                  15

Gly Ser Ala Val Ser Leu Ile Asp Glu Met Thr Lys Glu Gly Ile Lys
            20                  25                  30

Pro Asn Val
        35

<210>  412
<211>  37
<212>  PRT
<213>  Artificial Sequence

<220>
<223>  MODIFIED TYPE GUN1

<400>  412

Val Thr Tyr Asn Ser Ile Ile Asp Ala Phe Gly Arg Ser Ala Thr Met
1               5                   10                  15

Asp Arg Ser Ala Asp Tyr Ser Asn Gly Gly Ser Leu Pro Phe Ser Ser
            20                  25                  30

Ile Lys Leu Ser Ala
        35

<210>  413
<211>  35
<212>  PRT
<213>  Artificial Sequence

<220>
<223>  MODIFIED TYPE GUN1

<400>  413

Lys Leu Ala Ser Ala Met Ile Ser Ala Tyr Gly Arg Tyr Gly Lys Val
1               5                   10                  15

Thr Lys Ala Lys Arg Ile Phe Glu Thr Ala Phe Ala Gly Gly Ile Gly
            20                  25                  30

Asn Thr Val
        35

<210>  414
<211>  35
<212>  PRT
<213>  Artificial Sequence

<220>
<223>  MODIFIED TYPE GUN1

<400>  414

Tyr Ala Phe Ser Ala Leu Ile Ser Ala Tyr Gly Arg Ser Gly Leu His
1               5                   10                  15

Glu Lys Ala Ile Ser Val Phe Asn Ser Met Lys Glu Tyr Gly Ile Arg
            20                  25                  30

Pro Asn Leu
        35

<210>  415
<211>  36
<212>  PRT
<213>  Artificial Sequence

<220>
<223>  MODIFIED TYPE GUN1

<400>  415

Val Thr Tyr Asn Ala Val Ile Asp Ala Tyr Gly Lys Gly Gly Met Glu
1               5                   10                  15

Phe Lys Gln Val Ala Lys Phe Phe Asp Glu Met Gln Arg Asn Gly Ile
            20                  25                  30

Gln Pro Asp Arg
        35

<210>  416
<211>  35
<212>  PRT
<213>  Artificial Sequence

<220>
<223>  MODIFIED TYPE GUN1

<400>  416

Ile Thr Phe Asn Ser Leu Leu Ala Ala Tyr Ser Arg Gly Gly Leu Trp
1               5                   10                  15

Glu Lys Ala Arg Asn Leu Phe Asp Glu Met Thr Asn Arg Arg Ile Glu
            20                  25                  30

Gln Asp Val
        35

<210>  417
<211>  35
<212>  PRT
<213>  Artificial Sequence

<220>
<223>  MODIFIED TYPE GUN1

<400>  417

Phe Ser Tyr Asn Thr Leu Leu Asp Ala Tyr Cys Lys Gly Gly Gln Met
1               5                   10                  15

Asp Lys Ala Phe Glu Ile Leu Ala Gln Met Pro Val Lys Arg Ile Met
            20                  25                  30

Pro Asn Val
        35

<210>  418
<211>  35
<212>  PRT
<213>  Artificial Sequence

<220>
<223>  MODIFIED TYPE GUN1

<400>  418

Val Ser Tyr Ser Thr Val Ile Asp Ala Tyr Ala Lys Ala Gly Arg Phe
1               5                   10                  15

Asp Lys Ala Leu Asn Leu Phe Gly Glu Met Arg Tyr Leu Gly Ile Ala
            20                  25                  30

Leu Asp Arg
        35

<210>  419
<211>  35
<212>  PRT
<213>  Artificial Sequence

<220>
<223>  MODIFIED TYPE GUN1

<400>  419

Val Ser Tyr Asn Thr Leu Leu Ser Ala Tyr Thr Lys Val Gly Arg Ser
1               5                   10                  15

Glu Lys Ala Leu Asp Ile Leu Arg Glu Met Ala Ser Val Gly Ile Lys
            20                  25                  30

Lys Asp Val
        35

<210>  420
<211>  35
<212>  PRT
<213>  Artificial Sequence

<220>
<223>  MODIFIED TYPE GUN1

<400>  420

Val Thr Tyr Asn Ala Leu Leu Gly Ala Tyr Gly Lys Gln Gly Lys Tyr
1               5                   10                  15

Asp Lys Val Lys Lys Val Phe Thr Glu Met Lys Arg Glu His Ile Leu
            20                  25                  30

Pro Asn Leu
        35

<210>  421
<211>  35
<212>  PRT
<213>  Artificial Sequence

<220>
<223>  MODIFIED TYPE GUN1

<400>  421

Leu Thr Tyr Ser Thr Leu Ile Asp Ala Tyr Ser Lys Gly Gly Leu Tyr
1               5                   10                  15

Lys Lys Ala Met Glu Ile Phe Arg Glu Phe Lys Ser Ala Gly Ile Arg
            20                  25                  30

Ala Asp Val
        35

<210>  422
<211>  35
<212>  PRT
<213>  Artificial Sequence

<220>
<223>  MODIFIED TYPE GUN1

<400>  422

Val Leu Tyr Ser Ala Leu Ile Asp Ala Tyr Cys Lys Asn Gly Leu Val
1               5                   10                  15

Gly Lys Ala Val Ser Leu Ile Asp Glu Met Thr Lys Glu Gly Ile Ser
            20                  25                  30

Pro Asn Val
        35

<210>  423
<211>  37
<212>  PRT
<213>  Artificial Sequence

<220>
<223>  MODIFIED TYPE GUN1

<400>  423

Val Thr Tyr Asn Ser Ile Ile Asp Ala Tyr Gly Arg Ser Ala Thr Met
1               5                   10                  15

Asp Lys Ser Ala Asp Tyr Ser Asn Gly Gly Ser Leu Pro Phe Ser Ser
            20                  25                  30

Ile Ala Leu Ser Ala
        35

<210>  424
<211>  531
<212>  PRT
<213>  Artificial Sequence

<220>
<223>  MODIFIED TYPE P63

<400>  424

Ala Gly Thr Lys Ser Asp Gln Glu Glu Asp Asp Leu Glu Asp Gly Phe
1               5                   10                  15

Ser Glu Leu Glu Gly Ser Lys Ser Gly Gln Gly Ser Thr Ser Ser Asp
            20                  25                  30

Glu Asp Glu Gly Lys Leu Ser Ala Asp Glu Glu Glu Glu Glu Glu Leu
        35                  40                  45

Asp Leu Ile Glu Thr Asp Val Ser Arg Lys Thr Val Glu Lys Lys Gln

50                  55                  60

Ser Glu Leu Phe Lys Thr Ile Val Ser Ala Pro Gly Leu Ser Ile Gly
65                  70                  75                  80

Ser Ala Leu Asp Lys Trp Val Glu Glu Gly Asn Glu Ile Thr Arg Val
                85                  90                  95

Glu Ile Ala Lys Ala Met Leu Gln Leu Arg Arg Arg Arg Met Tyr Gly
            100                 105                 110

Arg Ala Leu Gln Met Ser Glu Trp Leu Glu Ala Asn Lys Lys Ile Glu
        115                 120                 125

Met Thr Glu Arg Asp Tyr Ala Ser Arg Leu Asp Leu Thr Val Lys Ile
    130                 135                 140

Arg Gly Leu Glu Lys Gly Glu Ala Cys Met Gln Lys Ile Pro Lys Ser
145                 150                 155                 160

Phe Lys Gly Glu Val Leu Tyr Arg Thr Leu Leu Ala Asn Cys Val Ala
                165                 170                 175

Ala Gly Asn Val Lys Lys Ser Glu Leu Val Phe Asn Lys Met Lys Asp
            180                 185                 190

Leu Gly Ile Lys Leu Ser Gly Phe Thr Cys Asp Gln Met Leu Leu Leu
        195                 200                 205

His Lys Arg Ile Asp Arg Lys Lys Ile Ala Asp Val Leu Leu Leu Met
    210                 215                 220

Glu Lys Glu Asn Ile Lys Pro Ser Leu Leu Thr Tyr Lys Ile Leu Ile
225                 230                 235                 240

Asp Val Lys Gly Ala Thr Asn Asp Ile Ser Gly Met Glu Gln Ile Leu
                245                 250                 255

Glu Thr Met Lys Asp Glu Gly Ile Lys Leu Asp Phe Gln Thr Gln Ala
            260                 265                 270

Leu Thr Ala Arg His Tyr Ser Gly Ala Gly Leu Lys Asp Lys Ala Glu
        275                 280                 285

Lys Val Leu Lys Glu Met Glu Gly Glu Ser Ile Lys Ala Asn Arg Arg
    290                 295                 300

Ala Phe Lys Asp Leu Leu Ser Ile Tyr Ala Ser Leu Gly Arg Glu Asp
305                 310                 315                 320

Glu Val Lys Arg Ile Trp Lys Ile Cys Glu Ile Lys Pro Tyr Phe Glu
                325                 330                 335

Glu Ser Leu Ala Ala Ile Gln Ala Phe Gly Lys Leu Asn Lys Val Gln
            340                 345                 350

Glu Ala Glu Ala Ile Phe Glu Lys Ile Val Lys Met Asp Ile Lys Ala
        355                 360                 365

Ser Ser Ser Thr Tyr Ser Val Leu Leu Arg Val Tyr Val Asp His Lys
    370                 375                 380

Met Leu Ser Lys Gly Lys Asp Leu Val Lys Arg Met Ala Glu Ser Gly
385                 390                 395                 400

Ile Lys Ile Glu Ala Thr Thr Trp Asp Ala Leu Ile Lys Leu Tyr Val
                405                 410                 415

Glu Ala Gly Glu Val Glu Lys Ala Asp Ser Leu Leu Asp Lys Ala Ser
            420                 425                 430

Lys Gln Ser His Ile Lys Leu Met Met Asn Ser Phe Met Tyr Ile Met
        435                 440                 445

Asp Glu Tyr Ser Lys Arg Gly Asp Val His Asn Thr Glu Lys Ile Phe
    450                 455                 460

Leu Lys Met Arg Glu Ala Gly Ile Lys Ser Arg Leu Arg Gln Phe Gln
465                 470                 475                 480

Ala Leu Met Gln Ala Tyr Ile Asn Ala Lys Ser Pro Ala Tyr Gly Met
                485                 490                 495

Arg Asp Arg Leu Lys Ala Asp Asn Ile Phe Pro Asn Lys Ser Met Ala
            500                 505                 510

Ala Gln Leu Ala Gln Gly Asp Pro Phe Lys Lys Thr Ala Ile Ser Asp
        515                 520                 525

Ile Leu Asp
    530

<210>  425
<211>  531
<212>  PRT

<213>  Artificial Sequence

<220>
<223>  MODIFIED TYPE P63

<400>  425

Ala Gly Thr Lys Ser Asp Gln Glu Glu Asp Asp Leu Glu Asp Gly Phe
1               5                   10                  15

Ser Glu Leu Glu Gly Ser Lys Ser Gly Gln Gly Ser Thr Ser Ser Asp
            20                  25                  30

Glu Asp Glu Gly Lys Leu Ser Ala Asp Glu Glu Glu Glu Glu Glu Leu
        35                  40                  45

Asp Leu Ile Glu Thr Asp Val Ser Arg Lys Thr Val Glu Lys Lys Gln
    50                  55                  60

Ser Glu Leu Phe Lys Thr Ile Val Ser Ala Pro Gly Leu Ser Ile Gly
65                  70                  75                  80

Ser Ala Leu Asp Lys Trp Val Glu Glu Gly Asn Glu Ile Thr Arg Val
                85                  90                  95

Glu Ile Ala Lys Ala Met Leu Gln Leu Arg Arg Arg Arg Met Tyr Gly
            100                 105                 110

Arg Ala Leu Gln Met Ser Glu Trp Leu Glu Ala Asn Lys Lys Ile Glu
        115                 120                 125

Met Thr Glu Arg Asp Tyr Ala Ser Arg Leu Asp Leu Thr Val Lys Ile
    130                 135                 140

Arg Gly Leu Glu Lys Gly Glu Ala Cys Met Gln Lys Ile Pro Lys Ser
145                 150                 155                 160

Phe Lys Gly Glu Val Leu Tyr Arg Thr Leu Leu Ala Ala Tyr Val Ala
                165                 170                 175

Ala Gly Asn Val Lys Lys Ser Glu Leu Val Phe Asn Lys Met Lys Asp
            180                 185                 190

Leu Gly Ile Pro Leu Ser Gly Phe Thr Cys Asp Gln Met Leu Leu Ala
        195                 200                 205

Tyr Lys Arg Ile Asp Arg Lys Lys Lys Ala Asp Val Leu Leu Leu Met
    210                 215                 220

Glu Lys Glu Asn Ile Lys Pro Ser Leu Leu Thr Tyr Lys Ile Leu Ile
225                 230                 235                 240

Asp Ala Tyr Gly Ala Thr Asn Asp Ile Ser Lys Met Glu Gln Ile Leu
                245                 250                 255

Glu Thr Met Lys Asp Glu Gly Ile Glu Leu Asp Phe Gln Thr Gln Ala
            260                 265                 270

Leu Thr Ala Arg Ala Tyr Ser Gly Ala Gly Leu Lys Asp Lys Ala Glu
        275                 280                 285

Lys Val Leu Lys Glu Met Glu Gly Glu Ser Ile Glu Ala Asn Arg Arg
    290                 295                 300

Ala Phe Lys Asp Leu Leu Ser Ala Tyr Ala Ser Leu Gly Arg Glu Asp
305                 310                 315                 320

Lys Val Lys Arg Ile Trp Lys Ile Cys Glu Ile Lys Pro Tyr Phe Glu
                325                 330                 335

Glu Ser Leu Ala Ala Ile Gln Ala Tyr Gly Lys Leu Asn Lys Val Gln
            340                 345                 350

Lys Ala Glu Ala Ile Phe Glu Lys Ile Val Lys Met Asp Ile Arg Ala
        355                 360                 365

Ser Ser Ser Thr Tyr Ser Val Leu Leu Arg Ala Tyr Val Asp His Lys
    370                 375                 380

Met Leu Ser Lys Gly Lys Asp Leu Val Lys Arg Met Ala Glu Ser Gly
385                 390                 395                 400

Ile Arg Ile Glu Ala Thr Thr Trp Asp Ala Leu Ile Lys Ala Tyr Val
                405                 410                 415

Glu Ala Gly Glu Val Glu Lys Ala Asp Ser Leu Leu Asp Lys Ala Ser
            420                 425                 430

Lys Gln Ser His Ile Lys Leu Met Met Asn Ser Phe Met Tyr Ile Met
        435                 440                 445

Asp Ala Tyr Ser Lys Arg Gly Asp Val His Lys Thr Glu Lys Ile Phe
    450                 455                 460

Leu Lys Met Arg Glu Ala Gly Ile Thr Ser Arg Leu Arg Gln Phe Gln
465                 470                 475                 480

Ala Leu Met Gln Ala Tyr Ile Asn Ala Lys Ser Pro Ala Tyr Gly Met
                485                 490                 495

Arg Asp Arg Leu Lys Ala Asp Asn Ile Phe Pro Asn Lys Ser Met Ala
            500                 505                 510

Ala Gln Leu Ala Gln Gly Asp Pro Phe Lys Lys Thr Ala Ile Ser Asp
        515                 520                 525

Ile Leu Asp
    530

<210>  426
<211>  669
<212>  PRT
<213>  Artificial Sequence

<220>
<223>  MODIFIED TYPE GUN1

<400>  426

Ser Ala His Leu Ser Gln Thr Thr Pro Asn Phe Ser Pro Leu Gln Thr
1               5                   10                  15

Pro Lys Ser Asp Phe Ser Gly Arg Gln Ser Thr Arg Phe Val Ser Pro
            20                  25                  30

Ala Thr Asn Asn His Arg Gln Thr Arg Gln Asn Pro Asn Tyr Asn His
        35                  40                  45

Arg Pro Tyr Gly Ala Ser Ser Ser Pro Arg Gly Ser Ala Pro Pro Pro
    50                  55                  60

Ser Ser Val Ala Thr Val Ala Pro Ala Gln Leu Ser Gln Pro Pro Asn
65                  70                  75                  80

Phe Ser Pro Leu Gln Thr Pro Lys Ser Asp Leu Ser Ser Asp Phe Ser
                85                  90                  95

Gly Arg Arg Ser Thr Arg Phe Val Ser Lys Met His Phe Gly Arg Gln
            100                 105                 110

Lys Thr Thr Met Ala Thr Arg His Ser Ser Ala Ala Glu Asp Ala Leu
        115                 120                 125

Gln Asn Ala Ile Asp Phe Ser Gly Asp Asp Glu Met Phe His Ser Leu
    130                 135                 140

Met Leu Ser Phe Glu Ser Lys Leu Cys Gly Ser Asp Asp Cys Thr Tyr
145                 150                 155                 160

Ile Ile Arg Glu Leu Gly Asn Arg Asn Glu Cys Asp Lys Ala Val Gly
                165                 170                 175

Phe Tyr Glu Phe Ala Val Lys Arg Glu Arg Arg Lys Asn Glu Gln Gly
            180                 185                 190

Lys Leu Ala Ser Ala Met Ile Ser Thr Leu Gly Arg Tyr Gly Lys Val
        195                 200                 205

Thr Ile Ala Lys Arg Ile Phe Glu Thr Ala Phe Ala Gly Gly Ile Lys
    210                 215                 220

Asn Thr Val Tyr Ala Phe Ser Ala Leu Ile Ser Ala Tyr Gly Arg Ser
225                 230                 235                 240

Gly Leu His Glu Glu Ala Ile Ser Val Phe Asn Ser Met Lys Glu Tyr
                245                 250                 255

Gly Ile Lys Pro Asn Leu Val Thr Tyr Asn Ala Val Ile Asp Ala Cys
            260                 265                 270

Gly Lys Gly Gly Met Glu Phe Lys Gln Val Ala Lys Phe Phe Asp Glu
        275                 280                 285

Met Gln Arg Asn Gly Ile Lys Pro Asp Arg Ile Thr Phe Asn Ser Leu
    290                 295                 300

Leu Ala Val Cys Ser Arg Gly Gly Leu Trp Glu Ala Ala Arg Asn Leu
305                 310                 315                 320

Phe Asp Glu Met Thr Asn Arg Arg Ile Lys Gln Asp Val Phe Ser Tyr
                325                 330                 335

Asn Thr Leu Leu Asp Ala Ile Cys Lys Gly Gly Gln Met Asp Leu Ala
            340                 345                 350

Phe Glu Ile Leu Ala Gln Met Pro Val Lys Arg Ile Lys Pro Asn Val
        355                 360                 365

Val Ser Tyr Ser Thr Val Ile Asp Gly Phe Ala Lys Ala Gly Arg Phe
    370                 375                 380

Asp Glu Ala Leu Asn Leu Phe Gly Glu Met Arg Tyr Leu Gly Ile Lys
385                 390                 395                 400

Leu Asp Arg Val Ser Tyr Asn Thr Leu Leu Ser Ile Tyr Thr Lys Val
                405                 410                 415

Gly Arg Ser Glu Glu Ala Leu Asp Ile Leu Arg Glu Met Ala Ser Val
            420                 425                 430

Gly Ile Lys Lys Asp Val Val Thr Tyr Asn Ala Leu Leu Gly Gly Tyr
        435                 440                 445

Gly Lys Gln Gly Lys Tyr Asp Glu Val Lys Lys Val Phe Thr Glu Met
    450                 455                 460

Lys Arg Glu His Ile Lys Pro Asn Leu Leu Thr Tyr Ser Thr Leu Ile
465                 470                 475                 480

Asp Gly Tyr Ser Lys Gly Gly Leu Tyr Lys Glu Ala Met Glu Ile Phe
                485                 490                 495

Arg Glu Phe Lys Ser Ala Gly Ile Lys Ala Asp Val Val Leu Tyr Ser
            500                 505                 510

Ala Leu Ile Asp Ala Leu Cys Lys Asn Gly Leu Val Gly Ser Ala Val
        515                 520                 525

Ser Leu Ile Asp Glu Met Thr Lys Glu Gly Ile Lys Pro Asn Val Val
    530                 535                 540

Thr Tyr Asn Ser Ile Ile Asp Ala Phe Gly Arg Ser Ala Thr Met Asp
545                 550                 555                 560

Arg Ser Ala Asp Tyr Ser Asn Gly Gly Ser Leu Pro Phe Ser Ser Ile
                565                 570                 575

Lys Leu Ser Ala Leu Thr Glu Thr Glu Gly Asn Arg Val Ile Gln Leu
            580                 585                 590

Phe Gly Gln Leu Thr Thr Glu Ser Asn Asn Arg Thr Thr Lys Asp Cys
        595                 600                 605

Glu Glu Gly Met Gln Glu Leu Ser Cys Ile Leu Glu Val Phe Arg Lys
    610                 615                 620

Met His Gln Leu Glu Ile Lys Pro Asn Val Val Thr Phe Ser Ala Ile
625                 630                 635                 640

Leu Asn Ala Cys Ser Arg Cys Asn Ser Phe Glu Asp Ala Ser Met Leu
                645                 650                 655

Leu Glu Glu Leu Arg Leu Phe Asp Asn Lys Val Tyr Gly
            660                 665

<210> 427
<211> 669
<212> PRT
<213> Artificial Sequence

<220>
<223> MODIFIED TYPE GUN1

<400> 427

Ser Ala His Leu Ser Gln Thr Thr Pro Asn Phe Ser Pro Leu Gln Thr
1               5                   10                  15

Pro Lys Ser Asp Phe Ser Gly Arg Gln Ser Thr Arg Phe Val Ser Pro
            20                  25                  30

Ala Thr Asn Asn His Arg Gln Thr Arg Gln Asn Pro Asn Tyr Asn His
        35                  40                  45

Arg Pro Tyr Gly Ala Ser Ser Ser Pro Arg Gly Ser Ala Pro Pro Pro
    50                  55                  60

Ser Ser Val Ala Thr Val Ala Pro Ala Gln Leu Ser Gln Pro Pro Asn
65                  70                  75                  80

Phe Ser Pro Leu Gln Thr Pro Lys Ser Asp Leu Ser Ser Asp Phe Ser
                85                  90                  95

Gly Arg Arg Ser Thr Arg Phe Val Ser Lys Met His Phe Gly Arg Gln
            100                 105                 110

Lys Thr Thr Met Ala Thr Arg His Ser Ser Ala Ala Glu Asp Ala Leu
        115                 120                 125

Gln Asn Ala Ile Asp Phe Ser Gly Asp Asp Glu Met Phe His Ser Leu
    130                 135                 140

Met Leu Ser Phe Glu Ser Lys Leu Cys Gly Ser Asp Asp Cys Thr Tyr
145                 150                 155                 160

Ile Ile Arg Glu Leu Gly Asn Arg Asn Glu Cys Asp Lys Ala Val Gly
                165                 170                 175

Phe Tyr Glu Phe Ala Val Lys Arg Glu Arg Arg Lys Asn Glu Gln Gly
            180                 185                 190

Lys Leu Ala Ser Ala Met Ile Ser Ala Tyr Gly Arg Tyr Gly Lys Val
        195                 200                 205

Thr Lys Ala Lys Arg Ile Phe Glu Thr Ala Phe Ala Gly Gly Ile Gly
    210                 215                 220

Asn Thr Val Tyr Ala Phe Ser Ala Leu Ile Ser Ala Tyr Gly Arg Ser
225                 230                 235                 240

Gly Leu His Glu Lys Ala Ile Ser Val Phe Asn Ser Met Lys Glu Tyr
                245                 250                 255

Gly Ile Arg Pro Asn Leu Val Thr Tyr Asn Ala Val Ile Asp Ala Tyr
            260                 265                 270

Gly Lys Gly Gly Met Glu Phe Lys Gln Val Ala Lys Phe Phe Asp Glu
        275                 280                 285

Met Gln Arg Asn Gly Ile Gln Pro Asp Arg Ile Thr Phe Asn Ser Leu
    290                 295                 300

Leu Ala Ala Tyr Ser Arg Gly Gly Leu Trp Glu Lys Ala Arg Asn Leu
305                 310                 315                 320

Phe Asp Glu Met Thr Asn Arg Arg Ile Glu Gln Asp Val Phe Ser Tyr
                325                 330                 335

Asn Thr Leu Leu Asp Ala Tyr Cys Lys Gly Gly Gln Met Asp Lys Ala
            340                 345                 350

Phe Glu Ile Leu Ala Gln Met Pro Val Lys Arg Ile Met Pro Asn Val
        355                 360                 365

Val Ser Tyr Ser Thr Val Ile Asp Ala Tyr Ala Lys Ala Gly Arg Phe
    370                 375                 380

Asp Lys Ala Leu Asn Leu Phe Gly Glu Met Arg Tyr Leu Gly Ile Ala
385                 390                 395                 400

Leu Asp Arg Val Ser Tyr Asn Thr Leu Leu Ser Ala Tyr Thr Lys Val
                405                 410                 415

Gly Arg Ser Glu Lys Ala Leu Asp Ile Leu Arg Glu Met Ala Ser Val
            420                 425                 430

Gly Ile Lys Lys Asp Val Val Thr Tyr Asn Ala Leu Leu Gly Ala Tyr
        435                 440                 445

Gly Lys Gln Gly Lys Tyr Asp Lys Val Lys Lys Val Phe Thr Glu Met
    450                 455                 460

Lys Arg Glu His Ile Leu Pro Asn Leu Leu Thr Tyr Ser Thr Leu Ile
465                 470                 475                 480

Asp Ala Tyr Ser Lys Gly Gly Leu Tyr Lys Lys Ala Met Glu Ile Phe
                485                 490                 495

Arg Glu Phe Lys Ser Ala Gly Ile Arg Ala Asp Val Val Leu Tyr Ser
            500                 505                 510

Ala Leu Ile Asp Ala Tyr Cys Lys Asn Gly Leu Val Gly Lys Ala Val
        515                 520                 525

Ser Leu Ile Asp Glu Met Thr Lys Glu Gly Ile Ser Pro Asn Val Val
    530                 535                 540

Thr Tyr Asn Ser Ile Ile Asp Ala Tyr Gly Arg Ser Ala Thr Met Asp
545                 550                 555                 560

Lys Ser Ala Asp Tyr Ser Asn Gly Gly Ser Leu Pro Phe Ser Ser Ile
                565                 570                 575

Ala Leu Ser Ala Leu Thr Glu Thr Glu Gly Asn Arg Val Ile Gln Leu
            580                 585                 590

Phe Gly Gln Leu Thr Thr Glu Ser Asn Asn Arg Thr Thr Lys Asp Cys
        595                 600                 605

Glu Glu Gly Met Gln Glu Leu Ser Cys Ile Leu Glu Val Phe Arg Lys
    610                 615                 620

Met His Gln Leu Glu Ile Lys Pro Asn Val Val Thr Phe Ser Ala Ile
625                 630                 635                 640

Leu Asn Ala Cys Ser Arg Cys Asn Ser Phe Glu Asp Ala Ser Met Leu
                645                 650                 655

Leu Glu Glu Leu Arg Leu Phe Asp Asn Lys Val Tyr Gly
            660                 665